# EUROPEAN PATENT SPECIFICATION

(11) **EP 0 479 903 B1**
(45) Date of publication and mention of the grant of the patent: **21.02.1996**
(21) Application number: 90911154.4
(22) Date of filing: 28.06.1990
(51) Int. Cl.: C07D 233/64, C07D 233/90, C07D 403/10, C07D 405/14

(54) **SUBSTITUTED IMIDAZOLES USEFUL AS ANGIOTENSIN II BLOCKERS**
SUBSTITUIERTE IMIDAZOLE UND IHRE VERWENDUNG ALS HEMMMSTOFF FUR ANGIOTENSIN II
IMIDAZOLES SUBSTITUES UTILES COMME INHIBITEURS DE L'ANGIOTENSIN II

(30) Priority: 30.06.1989 US 373755; 27.06.1990 US 544557; 27.06.1990 US 545240
(43) Date of publication of application: 15.04.1992
(73) Proprietor: E.I. DU PONT DE NEMOURS AND COMPANY, Wilmington Delaware 19898 (US)
(72) Inventor: ARDECKY, Robert, John, Landenburg, PA 19350 (US); CARINI, David, John, Wilmington, DE 19803 (US); DUNCIA, John, Jonas, Vytautas, Newark, DE 19711 (US); WONG, Pancras, Chor-Bun, Wilmington, DE 19808 (US)
(74) Representative: von Kreisler, Alek, Dipl.-Chem.
(86) International application number: US9003683
(87) International publication number: WO9100277

(56) References cited:
- EP-A- 0 253 310
- EP-A- 0 324 377

## Description

### BACKGROUND OF THE INVENTION

### Field of the Invention

This invention relates to novel substituted imidazoles, and processes for their preparation. The invention also relates to pharmaceutical compositions containing the novel imidazoles, alone and in conjunction with other drugs, especially diuretics and non-steroidal anti-inflammatory drugs (NSAID's).

The compounds of this invention inhibit the action of the hormone angiotensin II (AII) and are useful therefore in alleviating angiotensin induced hypertension. The enzyme renin acts on a blood plasma α₂-globulin, angiotensinogen, to produce angiotensin I, which is then converted by angiotensin converting-enzyme to All. The latter substance is a powerful vasopressor agent which has been implicated as a causitive agent for producing high blood pressure in various mammalian species, such as the rat, dog, and man. The compounds of this invention inhibit the action of AII at its receptors on target cells and thus prevent the increase in blood pressure produced by this hormone-receptor interaction. By administering a compound of this invention to a species of mammal with hypertension due to AII, the blood pressure is reduced. The compounds of this invention are also useful for the treatment of congestive heart failure. Administration of a compound of this invention with a diuretic such as furosemide or hydrochlorothiazide, either as a stepwise combined therapy (diuretic first) or as a physical mixture, enhances the antihypertensive effect of the compound. Administration of a compound of this invention with a non-steroidal anti-inflammatory drug (NSAID) can prevent renal failure which sometimes results from administration of a NSAID.

European Published Application 0 253 310, published January 20, 1988, discloses that certain substituted Imidazoles block the AII receptor and are useful therefore In alleviating angiotensin induced hypertension as well as in treating congestive heart failure. The Imidazoles disclosed have the formula (I) 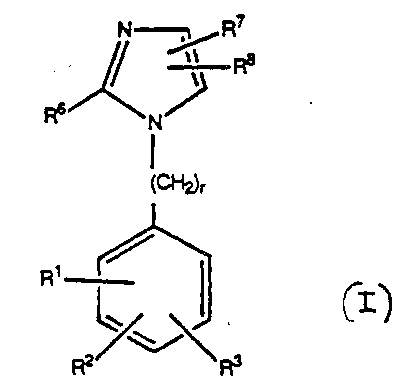 The imidazoles of the present invention differ from those of EPA 0 253 310 in the substituents R⁷ at positions 4 or 5 of the imidazole ring. In EPA 0 253 310, R⁷ and R⁸ are defined as follows:
- R⁷: is H, F, Cl, Br, I, NO₂, CF₃ or CN;
- R⁸: is H, CN, alkyl of 1 to 10 carbon atoms, alkenyl of 3 to 10 carbon atoms, or the same groups substituted with F: phenylalkenyl wherein the aliphatic portion is 2 to 6 carbon atoms; -(CH₂)ₘ-imidazol-1-yl; -(CH₂)ₘ-1,2,3-triazolyl optionally substituted with one or two groups selected from CO₂CH₃ or alkyl of 1 to 4 carbon atoms; -(CH₂)ₘ-tetrazolyl; -(CH₂)ₙOR¹¹ ; 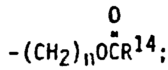 -(CH₂)ₙSR¹⁵; 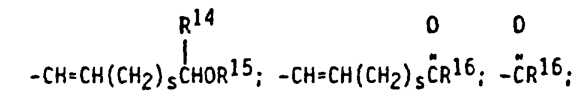 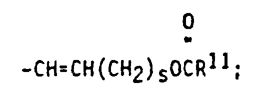 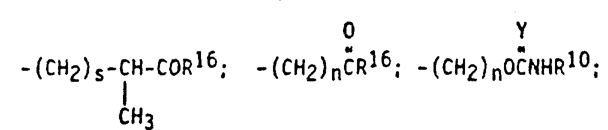 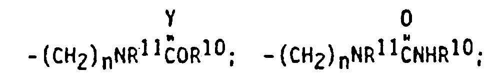 -(CH₂)ₙNR¹¹SO₂R¹⁰; 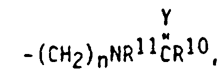 etc., where R¹⁰, R¹¹, R¹⁴, R¹⁵, R¹⁶, and
- Y: are as defined below for the present invention.

Imidazoles of the above formula (I) are further known from EP-A-0 324 377 (cited under Art. 54(3) EPC), which imidazoles are differing from the compounds of the present invention in that R⁷ is defined as follows:
- R⁷: is H, F, Cl, Br, I, NO₂, CᵥF₂ᵥ₊₁, where v = 1-6, C₆F₅; CN; 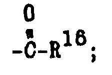 straight or branched alkyl of 1 to 6 carbon atoms; phenyl or phenylalkyl, where alkyl is 1 to 3 carbon atoms; or substituted phenyl or substituted phenylalkyl, where alkyl is 1 to 3 carbon atoms, substituted with one or two substituents selected from alkyl of 1 to 4 carbon atoms, F, Cl, Br, OH, OCH₃, CF₃, and COOR, where R is H, alkyl of 1 to 4 carbon atoms, or phenyl.

Pals et al., Circulation Research, 29, 673 (1971) describe the introduction of a sarcosine residue in position 1 and alanine in position 8 of the endogenous vasoconstrictor hormone All to yield an (octa)peptide that blocks the effects of All on the blood pressure of pithed rats. This analog, [Sar¹, Ala⁸] AII, initially called "P-113" and subsequently "Saralasin", was found to be one of the most potent competitive antagonists of the actions of All, although, like most of the so-called peptide-AII-antagonists, it also possesses agonistic actions of its own. Saralasin has been demonstrated to lower arterial pressure in mammals and man when the (elevated) pressure is dependent on circulating AII (Pals et al., Circulation Research, 29*,* 673 (1971); Streeten and Anderson, Handbook of Hypertension, Vol. 5, Clinical Pharmacology of Antihypertensive Drugs, A. E. Doyle (Editor), Elsevier Science Publishers B.V., p. 246 (1984)). However, due to its agonistic character, saralasin generally elicits pressor effects when the pressure is not sustained by All. Being a peptide, the pharmacological effects to saralasin are relatively short-lasting and are only manifest after parenteral administration, oral doses being ineffective. Although the therapeutic uses of peptide AII-blockers, like saralasin, are severely limited due to their oral ineffectiveness and short duration of action, their major utility is as a pharmaceutical standard.

Some known non-peptide antihypertensive agents act by inhibiting an enzyme, called angiotensin converting enzyme (ACE), which is responsible for conversion of angiotensin I to AII. Such agents are thus referred to as ACE inhibitors, or converting enzyme inhibitors (CEI's). Captopril and enalapril are commercially available CEI's. Based on experimental and clinical evidence, about 40% of hypertensive patients are non-responsive to treatment with CEI's. But when a diuretic such as furosemide or hydrochlorothiazide is given together with a CEI, the blood pressure of the majority of hypertensive patients is effectively normalized. Diuretic treatment converts the non-renin dependent state in regulating blood pressure to a renin-dependent state. Although the imidazoles of this invention act by a different mechanism, i.e., by blocking the All receptor rather than by inhibiting the angiotensin converting enzyme, both mechanisms involve interference with the reninangiotensin cascade. A combination of the CEI enalapril maleate and the diuretic hydrochlorothiazide is commercially available under the trademark Vaseretic^{®} from Merck & Co. Publications which relate to the use of diuretics with CEI's to treat hypertension, in either a diuretic-first, stepwise approach or in physical combination, include Keeton, T. K. and Campbell, W. B., Pharmacol. Rev., 31:81 (1981) and Weinberger, M. H., Medical Clinics N. America, 71:979 (1987). Diuretics have also been administered in combination with saralasin to enhance the antihypertensive effect.

Non-steroidal anti-inflammatory drugs (NSAID's) have been reported to induce renal failure in patients with renal underperfusion and high plasma level of AII. (Dunn, M.J., Hospital Practice, 19:99, 1984). Administration of an AII blocking compound of this invention in combination with an NSAID (either stepwise or in physical combination) can prevent such renal failure. Saralasin has been shown to Inhibit the renal vasoconstrictor effect of indomethacin and meclofenamate in dogs (Satoh et al., Circ. Res. 36/37 (Suppl. I):I-89, 1975; Blasingham et al., Am. J. Physiol. 239:F360, 1980). The CEI captopril has been demonstrated to reverse the renal vasoconstrictor effect of indomethacin in dogs with non-hypotensive hemorrhage. (Wong et al., J. Pharmacol, Exp, Ther, 219:104, 1980).

### Summary Of The Invention

According to the present invention there are provided novel compounds of formula (I) which have angiotensin II-antagonizing properties and are useful as antihypertensives. 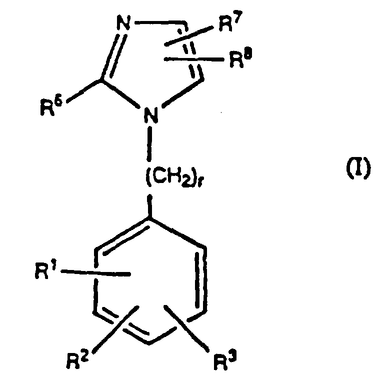 wherein
- R¹: is 4-CO₂H; 4-CO₂R^{9;} 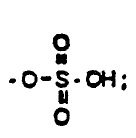 -SO₃H; - C(CF₃)₂OH; 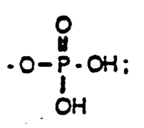 -PO₃H₂; 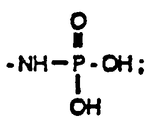 4-NHSO₂CH₃; 4-NHSO₂CF₃ ; -CONHOR^{12;} -SO₂NH₂ ; 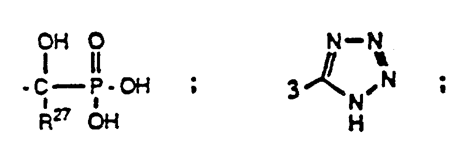 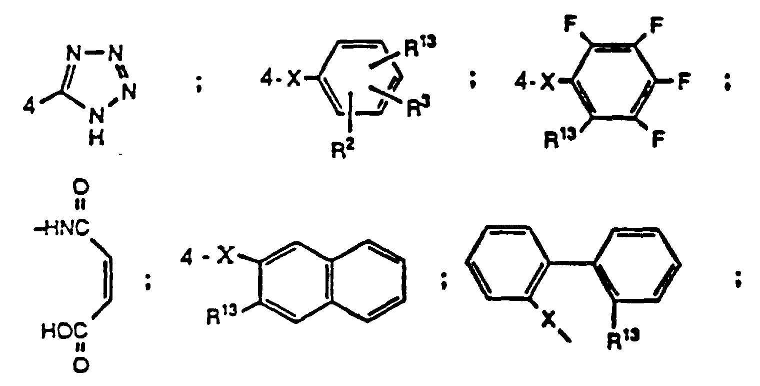 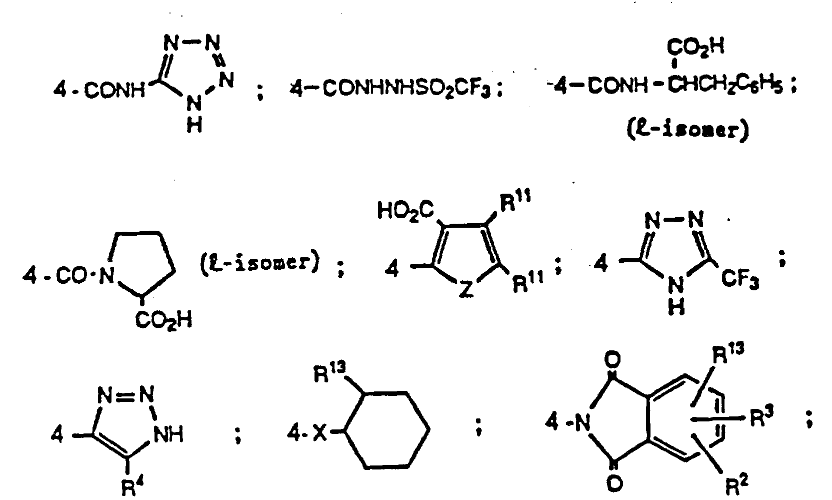 or 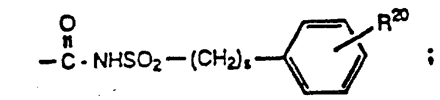
- R: is H; Cl; Br; I; F; NO₂; CN; alkyl of 1 to 4 carbon atoms; acyloxy of 1 to 4 carbon atoms; alkoxy of 1 to 4 carbon atoms; CO₂H; CO₂R⁹; NHSO₂CH₃; NHSO₂CF₃; CONHOR¹; SO₂NH₂; 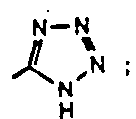 phenyl; or furyl;
- R³: is H; Cl, Br, I or F; alkyl of 1 to 4 carbon atoms or alkoxy of 1 to 4 carbon atoms;
- R⁴: is CN, NO₂ or CO₂R¹¹;
- R⁵: is H, alkyl of 1 to 6 carbon atoms, cycloalkyl of 3 to 6 carbon atoms alkenyl or alkynyl of 2 to 4 carbon atoms;
- R⁶: is alkyl of 2 to 10 carbon atoms, alkenyl or alkynyl of 3 to 10 carbon atoms or the same groups substituted with F or CO₂R¹⁴; cycloalkyl of 3 to 8 carbon atoms, cycloalkylalkyl of 4 to 10 carbon atoms; cycloalkylalkenyl or cycloalkylalkynyl of 5 to 10 carbon atoms; (CH₂)ₛZ(CH₂)ₘR⁵ optionally substituted with F or CO₂R¹⁴; benzyl or benzyl substituted on the phenyl ring with 1 or 2 halogens, alkoxy of 1 to 4 carbon atoms, alkyl of 1 to 4 carbon atoms or nitro;
- R⁷: is vinyl; cycloalkylidenyl; alkynyl of 2-10 carbon atoms; phenylalkynyl where the alkynyl portion is 2-6 carbon atoms; heteroaryl selected from 2- and 3-thienyl, 2- and 3-furyl, 2-, 3-, and 4-pyridyl, 2-pyrazinyl, 2-, 4-, and 5-pyrimidinyl, 3- and 4-pyridazinyl, 2-, 4- and 5-thiazolyl, 2-, 4-, and 5-selenazolyl, and 2-, 4-, and 5-oxazolyl, 2- or 3-pyrrolyl, 3-, 4- or 5-pyrazolyl, 2-, 4- or 5-imidazolyl; o-, m- or p-biphenylyl; o-, m- or p-phenoxyphenyl; 2-oxazolinyl; 2-thiazolinyl; substituted phenylalkynyl, heteroaryl, biphenylyl or phenoxyphenyl as defined above substituted with 1 or 2 substituents selected from halogen, hydroxy, mercapto, alkoxy of 1-5 carbon atoms, alkyl of 1-5 carbon atoms, -NO₂, -CN, -CF₃, -COR¹⁶, -CH₂OR¹⁷, -NHCOR¹⁷, -CONR¹⁸R¹⁹, S(O)ᵣR¹⁷, and SO₂NR¹⁸R¹⁹; pyrrolyl, pyrazolyl or imidazolyl as defined above substituted on ring nitrogen with alkyl of 1-5 carbon atoms, phenyl or benzyl; or substituted alkyl, alkenyl, or alkynyl of 1 to 10 carbon atoms substituted with a substituted or unsubstituted heteroaryl, biphenylyl or phenoxyphenyl group as defined above; -S(O)ᵣ-heteroaryl, -S-(O)ᵣ-biphenylyl, -S(O)ᵣ-phenoxyphenyl, -S-tetrazole, -S(O)ᵣR¹⁷, -NR¹⁸R¹⁹, -NR¹⁸-heteroaryl, -NR¹⁸-phenyl, -NR¹⁸-biphenylyl, -NR¹⁸-phenoxyphenyl, -N-phthalimido, -NH-SO₂-phenoxyphenyl, -NH-SO₂-heteroaryl, -NH-SO₂-biphenylyl, -NH-SO₂-R¹⁷, and -S-(C=O)-R¹⁷, N-imidazolyl, N-1,2,3-triazolyl, N-1,2,4-triazolyl, where heteroaryl is a heterocycle defined in the scope of R⁷ and where the phenyl group in R¹⁷ of -S-(O)ᵣR¹⁷, the N-imidazolyl, N-1,2,3-triazolyl, and N-1,2,4-triazolyls may be substituted with one or two substituents as described above for heteroaryl;
- R⁸: is 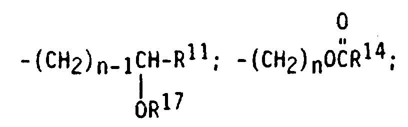 -(CH₂)ₙSR¹⁵; 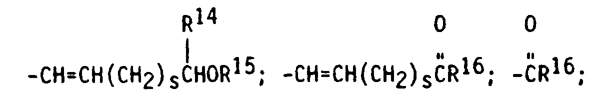 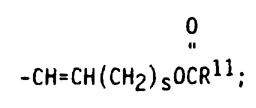 -(CH₂)ₘ-tetrazolyl; 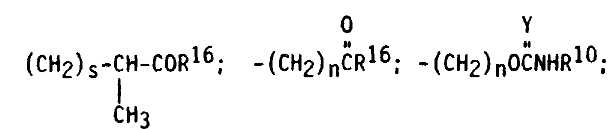 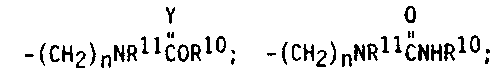 -(CH₂)ₙNR¹¹SO₂R¹⁰; 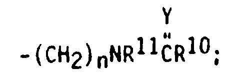
- R⁹: is 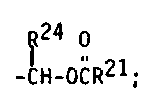
- R¹⁰: is alkyl of 1 to 6 carbon atoms or perfluoroalkyl of 1 to 6 carbon atoms, 1-adamantyl, 1-naphthyl, 1-(1-naphthyl)ethyl, or (CH₂)pC₆H₅;
- R¹¹: is H, alkyl of 1 to 6 carbon atoms, cycloalkyl of 3 to 6 carbon atoms, phenyl or benzyl;
- R¹: is H, methyl or benzyl;
- R¹³: is -CO₂H; -CO₂R⁹; -CH₂CO₂H, -CH₂CO₂R⁹; 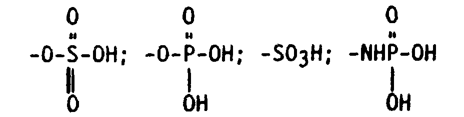 -PO₃H₂; -C(CF₃)₂OH; -NHSO₂CH₃; -NHSO₂CF₃; -NHCOCF₃; -CONHOR¹; -SO₂NH₂; 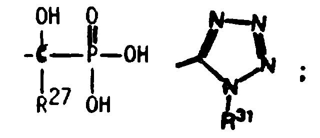 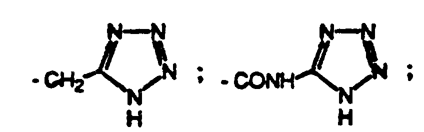 -CONHNHSO₂CF₃; 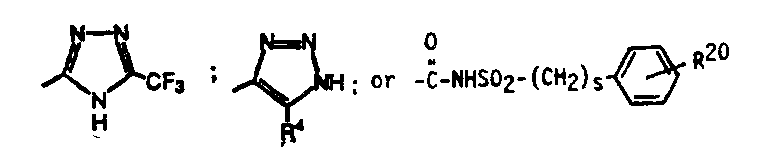
- R¹⁴: is H, alkyl or perfluoroalkyl of 1 to 8 carbon atoms, cycloalkyl of 3 to 6 carbon atoms, phenyl or benzyl;
- R¹⁵: is H, alkyl of 1 to 6 carbon atoms, cycloalkyl of 3 to 6 carbon atoms, phenyl, benzyl, acyl of 1 to 4 carbon atoms, phenacyl;
- R¹⁶: is H, alkyl of 1 to 6 carbon atoms, cycloalkyl of 3 to 6 carbon atoms, (CH₂)ₚC₆H₅, OR¹⁷, or NR¹⁸R¹⁹;
- R¹⁷: is H, alkyl of 1 to 6 carbon atoms, cycloalkyl of 3 to 6 carbon atoms, phenyl or benzyl;
- R¹⁸ and R¹⁹: independently are H, alkyl of 1 to 4 carbon atoms, phenyl, benzyl, α-methylbenzyl, or taken together with the nitrogen form a ring of the formula 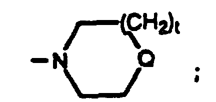 Q is NR⁰, O or CH₂;
- R⁰: is H, alkyl of 1-4 carbon atoms, or phenyl;
- R¹: is alkyl of 1 to 6 carbon atoms, -NRR³, or 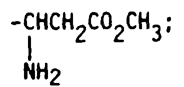
- R and R³: independently are H, alkyl of 1 to 6 carbon atoms, benzyl, or are taken together as (CH₂)ᵤ where u is 3-6;
- R⁴: is H, CH₃ or -C₆H₅;
- R⁵: is NR⁷R⁸, OR⁸, NHCONH₂, NHCSNH₂, 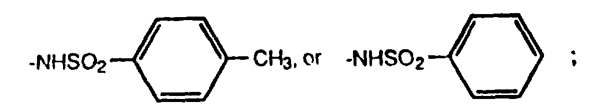
- R⁶: is hydrogen, alkyl with from 1 to 6 carbon atoms, benzyl, or allyl;
- R⁷ and R⁸: are independently hydrogen, alkyl with from 1 to 5 carbon atoms, or phenyl;
- R⁹ and R³⁰: are independently alkyl of 1-4 carbon atoms or taken together are -(CH₂)_{q}-;
- R³¹: is H, alkyl of 1 to 4 carbon atoms, -CH₂CH=CH₂ or -CH₂C₆H₄R³;
- R³: is H, NO₂, NH₂, OH or OCH₃;
X is a carbon-carbon single bond, -CO-, -CH₂-, -O-, -S-, -NH-, 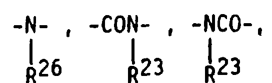 -OCH₂-, -CH₂O-, -SCH₂-, -CH₂S-, -NHC(R⁷)(R⁸), -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 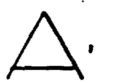 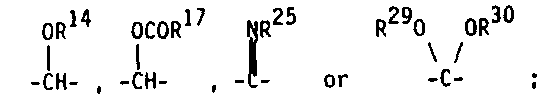
Y is O or S;
Z is O, NR¹¹, or S;
m is 1 to 5;
n is 1 to 10;
p is 0 to 3;
q is 2 to 3;
r is 0 to 2;
s is 0 to 5;
t is 0 or 1;
and pharmaceutically acceptable salts of these compounds;
provided that:
(1) the R¹ group is not in the ortho position;
(2) when R¹ is 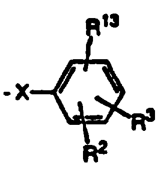 X is a single bond, and R¹³ is CO₂H, or 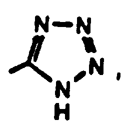 then R¹³ must be in the ortho or meta position; or when R¹ and X are as above and R¹³ is NHSO₂CF₃ or NHSO₂CH₃, R¹³ must be ortho;
(3) when R¹ is 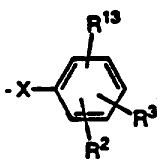 and X is other than a single bond, then R¹³ must be ortho except when X = NR³CO and R¹³ is NHSO₂CF₃ or NHSO₂CH₃, then R¹³ must be ortho or meta;
(4) when R¹ is 4-CO₂H or a salt thereof, R⁶ cannot be S-alkyl;
(5) when R¹ is 4-CO₂H or a salt thereof, the substituent on the 4-position of the imidazole cannot be CH₂OH, CH₂OCOCH₃, or CH₂CO₂H;
(6) when R¹ is 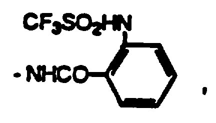 R⁶ is not methoxybenzyl;
(7) the R⁶ group is not 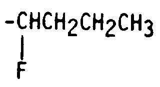 or CH₂OH;

Preferred for their antihypertensive activity are novel compounds having the formula: 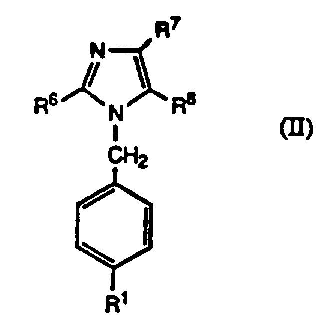 wherein
- R¹: is -CO₂H ; -NHSO₂CF₃; 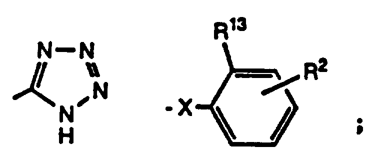 or 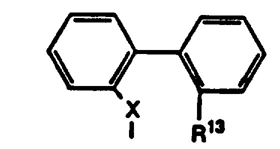
- R⁶: is alkyl of 3 to 10 carbon atoms, alkenyl of 3 to 10 carbon atoms, alkynyl of 3 to 10 carbon atoms, cycloalkyl of 3 to 8 carbon atoms, benzyl substituted on the phenyl ring with up to two groups selected from alkoxy of 1 to 4 carbon atoms, halogen, alkyl of 1 to 4 carbon atoms, and nitro;
- R⁸: is -(CH₂)ₘ-tetrazolyl, -(CH₂)ₙOR¹¹; 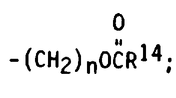 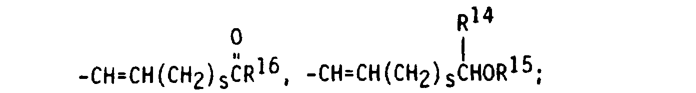 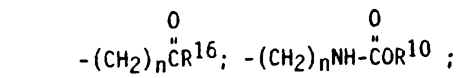 -(CH₂)ₙNHSO₂R¹⁰; 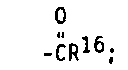
- R¹³: is -CO₂H, CO₂R⁹, NHSO₂CF³; SO₃H; or 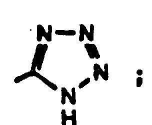
- R¹⁶: is H, alkyl of 1 to 5 carbon atoms, OR¹⁷, or NR¹⁸R¹⁹;
X is carbon-carbon single bond, -CO-, 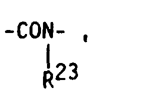 -CH₂CH₂-, 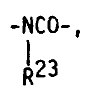 -OCH₂-, -CH₂O-, -SCH₂-, -CH₂S-, -NHCH₂-, -CH₂NH- or -CH=CH-; and pharmaceutically acceptable salts of these compounds.

More preferred are compounds of the preferred scope where:
- R: is H, alkyl of 1 to 4 carbon atoms, halogen, or alkoxy of 1 to 4 carbon atoms;
- R⁶: is alkyl, alkenyl or alkynyl of 3 to 7 carbon atoms;
- R⁷: is heteroaryl selected from 2- and 3-thienyl, 2- and 3-furyl, 2-, 3-, and 4-pyridyl, or p-biphenylyl, 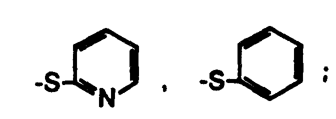
- R⁸: is -(CH₂)ₘOR¹¹; 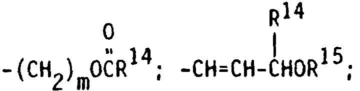 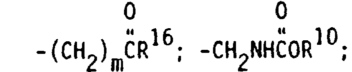 -(CH₂)ₘNHSO₂R¹⁰; or -COR¹⁶;
- R¹⁰: is CF₃, alkyl of 1 to 6 carbon atoms or phenyl;
- R¹¹: is H, or alkyl of 1 to 4 carbon atoms;
- R¹³: is CO₂H; CO₂CH₂OCOC(CH₃)₃; NHSO₂CF₃ or 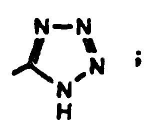
- R¹⁴: is H, or alkyl of 1 to 4 carbon atoms;
- R¹⁵: is H, alkyl of 1 to 4 carbon atoms, or acyl of 1 to 4 carbon atoms;
- R¹⁶: is H, alkyl of 1 to 5 carbon atoms; OR¹⁷; or 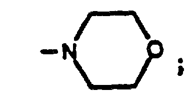
- m: is 1 to 5;
- X =: single bond, -O-; -CO-; -NHCO-; or -OCH₂-; and pharmaceutically acceptable salts.

Note that throughout the text when an alkyl substituent is mentioned, the normal alkyl structure is meant (i,e., butyl is n-butyl) unless otherwise specified.

Pharmaceutically suitable salts include both the metallic (inorganic) salts and organic salts; a list of which is given in Remington's Pharmaceutical Sciences, 17th Edition, pg. 1418 (1985). It is well known to one skilled in the art that an appropriate salt form is chosen based on physical and chemical stability, flowability, hydroscopicity and solubility. Preferred salts of this invention for the reasons cited above include potassium, sodium, calcium and ammonium salts.

Also within the scope of this invention are pharmaceutical compositions comprising a suitable pharmaceutical carrier and a compound of Formula (I), and methods of using the compounds of Formula (I) to treat hypertension and congestive heart failure. The pharmaceutical compositions can optionally contain one or more other therapeutic agents, such as a diuretic or a non-steroidal antiinflammatory drug. Also within the scope of this invention is the use of the above compounds for preparing a medicament for preventing renal failure resulting from administration of a non-steroidal antiinflammatory drug (NSAID).

The compounds of this invention can also be used as diagnostic agents to test the renin angiotensin system.

It should be noted in the foregoing structural formula, when a radical can be a substituent in more than one previously defined radical, that first radical can be selected independently in each previously defined radical. For example, R¹, R and R³ can each be CONHOR¹. R¹ need not be the same substituent in each of R¹, R and R³ but can be selected independently for each of them.

### Detailed Description

### Synthesis

The novel compounds of Formula (I) may be prepared using the reactions and techniques described in this section. The reactions are performed in a solvent appropriate to the reagents and materials employed and suitable for the transformation being effected. It is understood by those skilled in the art of organic synthesis that the functionality present on the imidazole and other portions of the molecule must be consistent with the chemical transformations proposed. This will frequently necessitate judgment as to the order of synthetic steps, protecting groups required, deprotection conditions, and activation of a benzylic position to enable attachment to nitrogen on the imidazole nucleus. Throughout the following section, not all compounds of Formula (I) falling into a given class may necessarily be prepared by all methods described for that class. Substituents on the starting materials may be incompatible with some of the reaction conditions required in some of the methods described. Such restrictions to the substituents which are compatible with the reaction conditions will be readily apparent to one skilled in the art and alternative methods described must then be used.

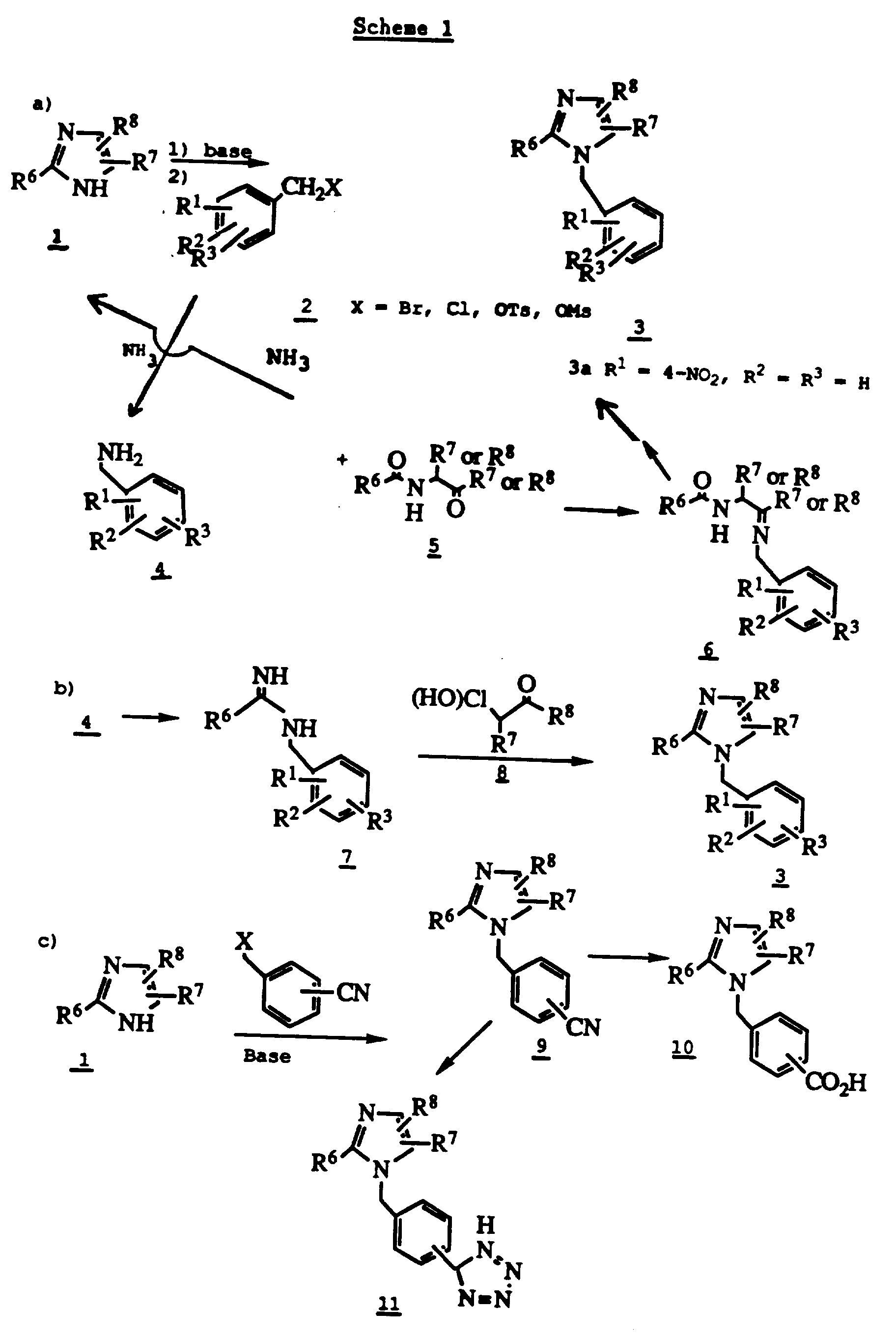

Generally, compounds of Formula (3) can be prepared by direct alkylation onto imidazole (1), with an appropriately protected benzyl halide, tosylate or mesylate (2) in the presence of base, as shown in path a). Preferably, the metallic imidazolide salt is prepared by reacting imidazole (1) with a proton acceptor such as MH where M is lithium, sodium or potassium in a solvent such as dimethylformamide (DMF) or by reacting it with a metal alkoxide of formula MOR where R is methyl, ethyl, t-butyl or the like In an alcohol solvent such as ethanol or t-butanol, or a dipolar aprotic solvent such as dimethylformamide. The imidazole salt is dissolved in an inert aprotic solvent such as DMF, and treated with an appropriate alkylating agent (2). Alternatively, imidazole (1) can be alkylated with a benzyl halide (2, where X=Br, Cl) in the presence of a base such as sodium carbonate, potassium carbonate, triethylamine or pyridine. The reaction is run in an inert solvent such as DMF or DMSO at 20°C to the reflux temperature of the solvent for 1-10 hours.

For example, the 4-nitrobenzyl intermediate (3a, wherein R¹ = 4-NO₂, R = R³ = H) may be obtained by direct alkylation onto imidazole (1) with a 4-nitrobenzyl halide, tosylate or mesylate In the presence of base.

As R⁷ and R⁸ are different, mixtures of two regioisomer alkylation products (3b, and 3c) are obtained in which R⁷ and R⁸ are interchanged. When R⁸ is CHO the alkylation is such that the benzyl group becomes attached to the adjacent nitrogen preferentially. These isomers possess distinct physical and biological properties and can usually be separated and isolated by conventional separation techniques such as chromatography and/or crystallization.

In all series examined, the more rapidly eluted isomer of a given pair has greater biological potency than the less rapidly eluted isomer.

Alternatively, any properly functionalized benzylamine derivative (4) may be converted to imine (6) by treatment with an acylamino ketone (5) in the presence of an inert solvent such as benzene, toluene, or the like, and a catalytic amount of p-toluenesulfonic acid or molecular sieves, N. Engel, and W. Steglich, Liebigs Ann. Chem., 1916, (1978), or in the presence of alumina, F. Texier-Boulet, Synthesis, 679 (1985). The resulting imine (6) can be cyclized to the N-benzyl imidazole (3) with phosphorus pentachloride (PCl₅), phosphorus oxychloride (POCl₃) or triphenylphosphine (PPh₃) in dichloroethane in the presence of a base such as triethylamine, N. Engel and W. Steglich, Liebigs Ann. Chem., 1916, (1978).

Acylamino ketone (5) is readily obtainable from amino acids via the Dakin-West reaction, H.D. Dakin, R. West, J. Biol. Chem., 78, 95 and 745 (1928), and various modifications thereof, W. Steglich, G. Hofle, Angew. Chem. Int. Ed. Engl., 8, 98l (1969); G. Hofle, W. Steglich, H. Vorbruggen, Angew. Chem. Int. Ed. Engl., 17, 569 (1978); W. Steglich, G. Hofle, Ber., 102, 883 (1969), or by selective reduction of acyl cyanides, A. Pfaltz, S. Anwar, Tet. Lett. 2977 (1984), or from α-halo, α-tosyl or α-mesyl ketones via the appropriate substitution reactions that one skilled in the art will readily recognize.

The functionalized benzylamines (4) may be made from the corresponding benzyl halide, tosylate or mesylate (2) via displacement with a nitrogen nucleophile, a procedure familiar to one skilled in the art. This displacement may be achieved using azide ion, ammonia, or phthalimide anion, etc., in a neutral solvent such as dimethylformamide, dimethylsulfoxide etc., or under phase transfer conditions. The benzyl halide (2) may be made by a variety of benzylic halogenation methods familiar to one skilled in the art, for example benzylic bromination of toluene derivatives with N-bromosuccinimide in an inert solvent such as carbon tetrachloride in the presence of a radical initiator such as benzoyl peroxide at temperatures up to reflux conditions.

A wide variety of toluene derivatives may be made from simple electrophilic substitution reactions on an aromatic ring. This includes nitration, sulfonation, phosphorylation, Friedel-Crafts alkylation, Friedel-Crafts acylation, halogenation, and other similar reactions known to one skilled in the art, G. A. Olah, "Friedel-Crafts and Related Reactions," Vol. 1-5, Interscience, New York, (1965).

Another way to synthesize functionalized benzyl halides is via chloromethylation of the corresponding aromatic precursor. Thus, the appropriately substituted benzene ring may be chloromethylated with formaldehyde and hydrochloric acid (HCl) for example with or without an inert solvent such as chloroform, carbon tetrachloride, light petroleum ether or acetic acid. A Lewis acid such as zinc chloride (ZnCl₂) or a mineral acid such as phosphoric acid may also be added as a catalyst or condensing agent, R. C. Fuson, C. H. McKeever, Org. Reactions, 1, 63 (1942).

Alternatively, N-benzylimidazoles (3) can also be prepared as shown in path b) by forming an R⁶ substituted amidine (7) from an appropriately substituted benzylamine (4) which is in turn reacted with an α-haloketone, α-hydroxyketone (8), α-haloaldehyde, or α-hydroxyaldehyde, F. Kunckell, Ber., 34, 637 (1901).

As shown in path a), imidazole (1) may be alkylated by a variety of benzyl derivatives. These include compounds with latent acid functionalities such as o, m, and p-cyanobenzylhalides, mesylates or tosylates as shown in path c). Nitriles of formula (9) may be hydrolyzed to carboxylic acids of formula (10) by treatment with strong acid or alkali. Preferably, treatment with a l:l (v/v) mixture of concentrated aqueous hydrochloric acid/glacial acetic acid at reflux temperatures for 2-96 hours or by treatment with 1N sodium hydroxide in an alcohol solvent such as ethanol or ethylene glycol for 2-96 hours at temperatures from 20°C to reflux can be used. If another nitrile group is present it will also be hydrolyzed. The nitrile functionality can also be hydrolyzed in two steps by first stirring in sulfuric acid to form the amide followed by hydrolysis with sodium hydroxide or a mineral acid to give the carboxylic acid (10).

The nitriles (9) can be converted into the corresponding tetrazole derivative (11) by a variety of methods using hydrazoic acid. For example, the nitrile can be heated with sodium azide and ammonium chloride in DMF at temperatures between 30°C and reflux for l-10 days, J. P. Hurwitz and A. J. Tomson, J. Org. Chem., 26, 3392 (196l). Preferably, the tetrazole is prepared by the 1,3-dipolar cycloaddition of trialkyltin or triaryltin azides to the appropriately substituted nitrile as described in detail by Scheme 15.

The starting imidazole compounds (1) are readily available by any of a number of standard methods. For example, acylaminoketone (5) can be cyclized with ammonia or equivalents thereof, D. Davidson, et al., J. Org. Chem., 2, 319 (1937) to the corresponding imidazole as shown in Scheme l. The corresponding oxazole can also be converted to imidazole (1) by action of ammonia or amines in general, H. Bredereck, et al., Ber., 88, 1351 (1955); J. W. Cornforth and R. H. Cornforth, J. Chem Soc., 96, (1947).

Several alternative routes to imidazoles (1) are illustrated in Scheme 2. As shown in Scheme 2 equation a), reaction of the appropriate R⁶ substituted imidate esters (12) with an appropriately substituted α-hydroxy- or α-haloketone or aldehyde (8) in ammonia leads to imidazoles of formula (1), P. Dziuron, and W. Schunack, Archiv. Pharmaz., 307 and 470 (1974).

The starting imidazole compounds (1) wherein R⁷ and R⁸ are both hydrogen can be prepared as shown in equation b) by reaction of the appropriate R⁶-substituted imidate ester (12) with α-aminoacetaldehyde dimethyl acetal (13), M. R. Grimmett, Adv. Heterocyclic Chem., 12, 103 (1970).

As shown in equation c), imidazole (15; wherein R⁷ = hydrogen and R⁸ = CH₂OH) can be prepared by treatment of the imidate ester (12) with 1,3-dihydroxyacetone (14) in ammonia by the procedure described in Archive der Pharmazie, 307, 470 (1974). Halogenation of imidazole (15) or any imidazole wherein R⁷ or R⁸ is hydrogen is preferably accomplished by reaction with one to two equivalents of N-halosuccinimide in a polar solvent such as dioxane or 2-methoxyethanol at a temperature of 40-100°C for 1-10 hours. Reaction of the halogenated imidazole (16) with a benzylhalide (2) in the manner described in Scheme l affords the corresponding N-benzylimidazole (17); wherein R⁷ is halogen and R⁸ is CH₂OH). This procedure is described in U.S. Patent 4,355,040. Alternatively, imidazole (17) can be prepared by the procedure described in U.S. Patent 4,207,324.

Compounds of formula (17) can also be prepared by treatment of the starting imidazole compound (1) wherein R⁷ and R⁸ are both hydrogen, with the appropriate benzyl halide followed by functionalization of R⁷ and R⁸ by treatment with formaldehyde as described in E. F. Godefroi, et al., Recueil, 91, 1383 (1972) followed by halogenation as was described above.

As shown in equation d) the imidazoles (1) can also be prepared by reaction of R⁶ substituted amidines (18) with an α-hydroxy- or α-haloketone or aldehyde (8) as described by F. Kunckel, Ber., 34, 637, (190l).

Compounds of Formula (1) wherein R⁸= CH₂OH can be prepared as shown in equation e). The imidazoles (1) were prepared as described in L. A. Reiter, J. Org. Chem., 52, 2714 (1987). Hydroxymethylation of (1) as described by U. Kempe, et al. in U.S. Patent 4,278,801 provides the hydroxymethylimidazoles (1a).

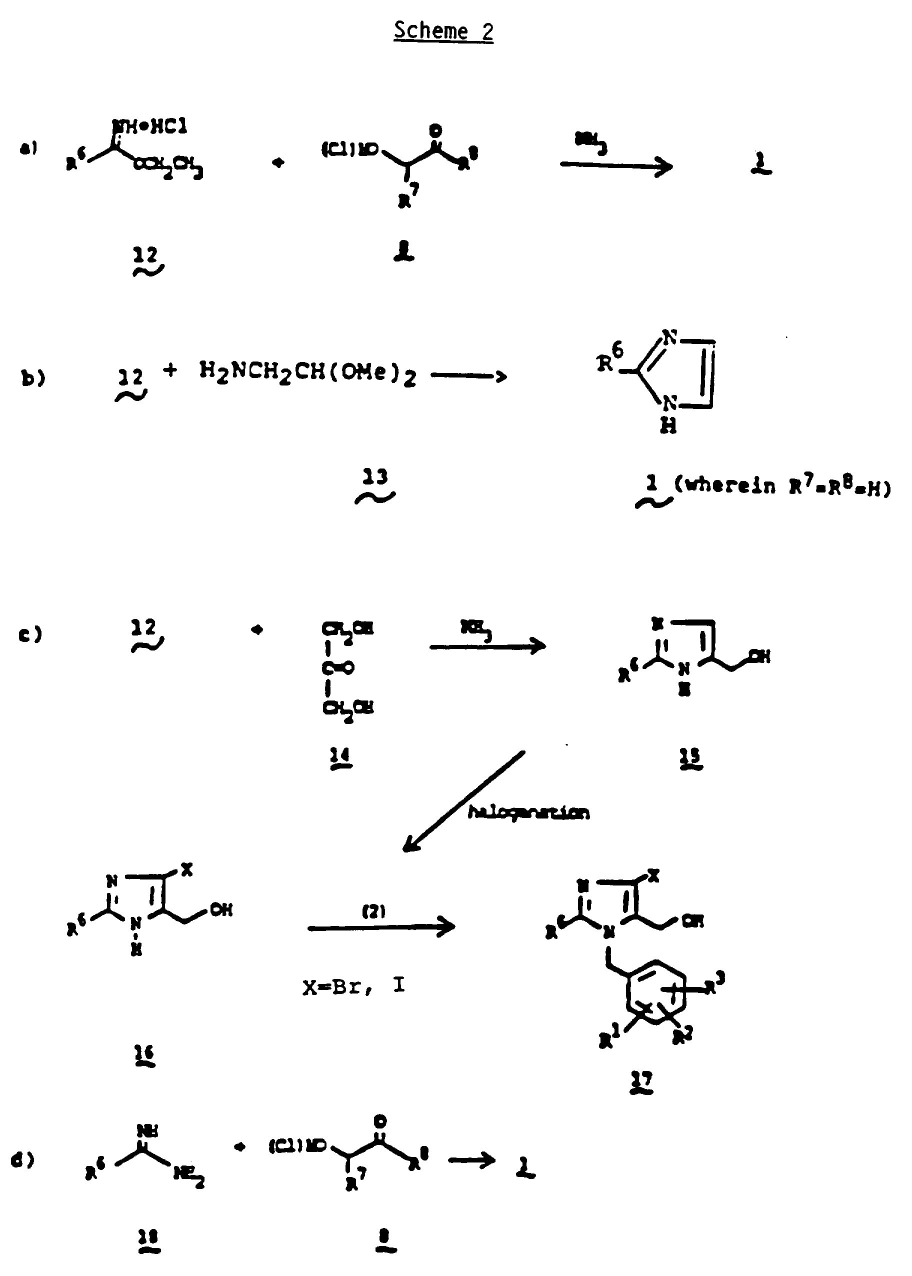

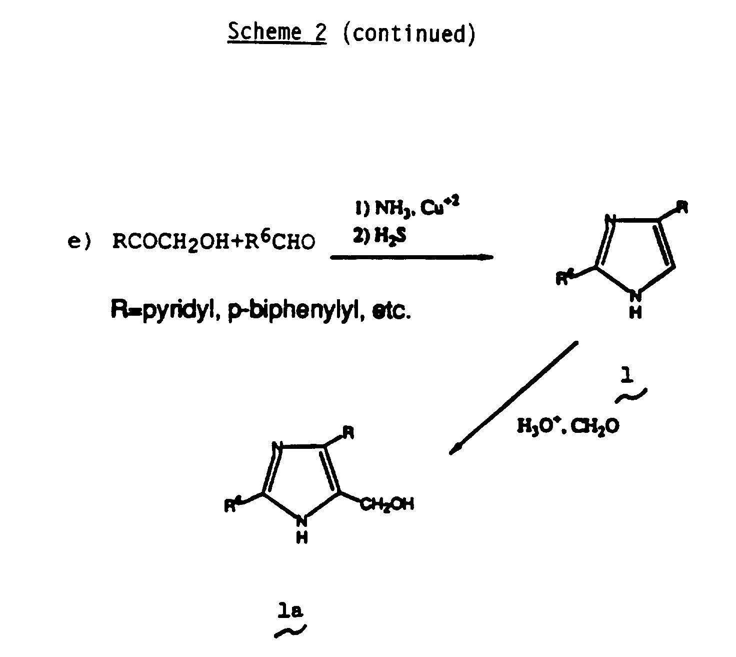

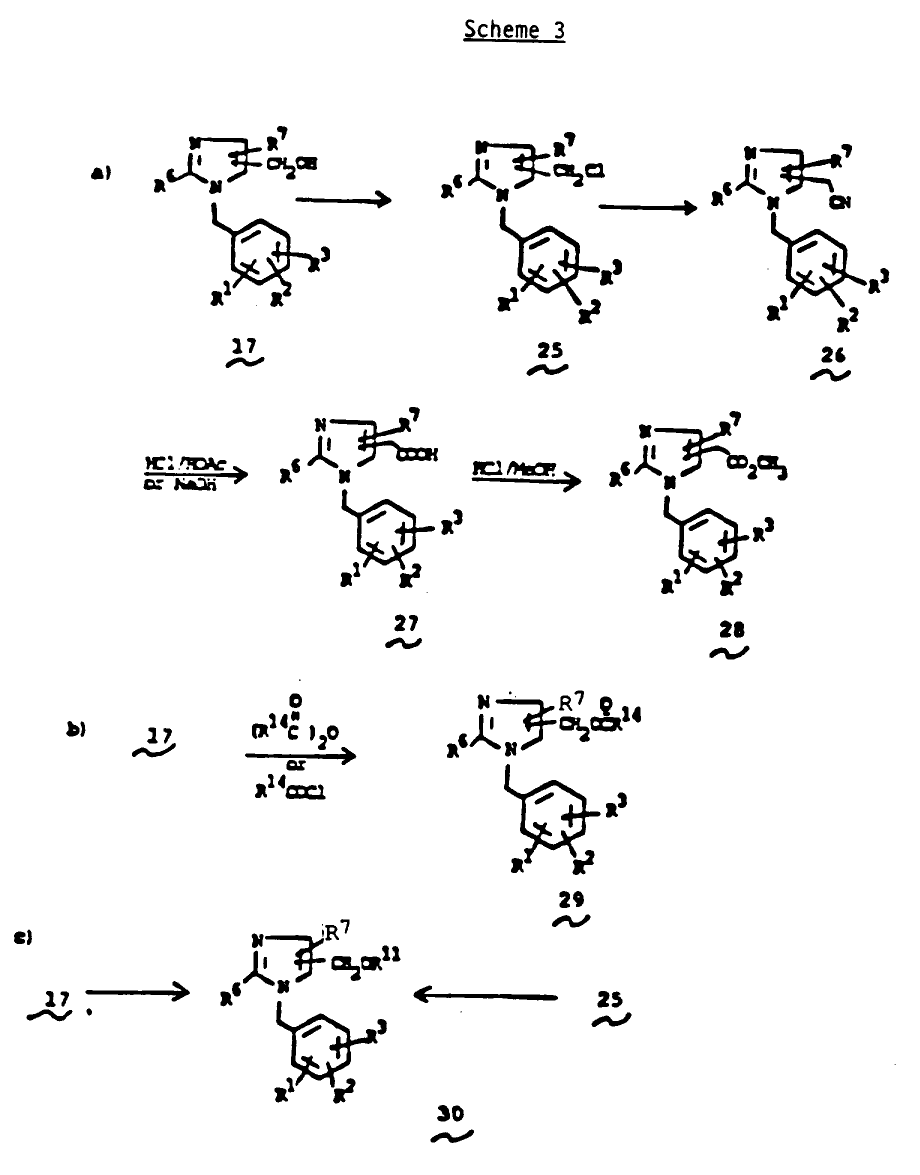

As shown in Scheme 3, path a) for benzylimidazoles (17) where R⁸ = CH₂OH, the hydroxymethyl groups may be easily converted to the corresponding halide, mesylate or tosylate by a variety of methods familiar to one skilled in the art. Preferably, the alcohol (17) is converted to the chloride (25) with thionyl chloride in an inert solvent at temperatures of 20°C to the reflux temperature of the solvent.

Chloride (25) may be displaced by a variety of nucleophiles by nucleophilic displacement reaction procedures familiar to one skilled in the art. For example, excess sodium cyanide in DMSO may be used to form cyanomethyl derivatives (26) at temperatures of 20°C to 100°C.

Nitrile (26) may be hydrolyzed to an acetic acid derivative (27), by a variety of methods. These methods include methods described previously for the hydrolysis of nitriles of formula (9). Examples of desired acids and bases for this hydrolysis include mineral acids such as sulfuric acid, hydrochloric acid, and mixtures of either of the above with 30-50% acetic acid (when solubility is a problem), and alkali metal hydroxides such as sodium hydroxide or potassium hydroxide. The hydrolysis reaction proceeds under heating at temperatures ranging from 50-160°C for 2-48 hours. Carboxylic acid (27) may be esterified by a variety of methods without affecting other parts of the molecule. Preferably, (27) is refluxed in a hydrochloric acid/methanol solution for 2-48 hours to give ester (28).

Ester (28) may be hydrolyzed to carboxylic acid (27), for instance, after R¹, R and R³ have been elaborated. Various methods, acidic or basic, may be used. For example, compound (28) is stirred with 0.5N potassium hydroxide in methanol, or if base soluble, it is stirred in 1.0N sodium hydroxide for l-48 h at 20°C to reflux temperatures.

Hydroxymethyl derivative (17) may be acylated to give (29) by a variety of procedures. As shown in path b) acylation can be achieved with l-3 equivalents of an acyl halide or an anhydride in a solvent such as diethyl ether, tetrahydrofuran, methylene chloride or the like in the presence of a base such as pyridine or triethylamine. Alternatively (17) may be acylated by reaction with a carboxylic acid and dicyclohexylcarbodiimide (DCC) in the presence of a catalytic amount of 4-(N,N-dimethylamino)pyridine (DMAP) via the procedure described by A. Hassner, Tet. Lett., 46, 4475 (1978). Treatment of (17) with a solution of carboxylic acid anhydride in pyridine optionally with a catalytic amount of DMAP at temperatures of 20-100°C for 2-48 hours is the preferred method.

The ether (30) can be prepared from the alcohol (17) as shown in path c) by methods such as treatment of (17) in a solvent such as dimethylformamide or dimethylsulfoxide with potassium t-butoxide, sodium hydride, or the like followed by treatment with R¹¹L at 25°C for 1-20 hours, where L is a halogen, tosylate or mesylate.

Alternatively, treatment of (17) with l-5 equivalents of thionyl chloride in chloroform for 2-6 hours at 25°C followed by treatment of the intermediate (25) with l-3 equivalents of MOR¹¹, where M is sodium or potassium, for 2-10 hours at 25°C either In R¹¹OH as solvent or in a polar solvent such as dimethylformamide or the like will also yield ether (30).

The ether (30) can also be prepared for example by heating (17) for 3-15 hours at 60-160°C in R¹¹OH containing an inorganic acid such as a hydrochloric acid or sulfuric acid.

N-arylimidazoles of formula I (compounds wherein r=o) can be prepared by the following methods, it being understood by one skilled in the art that certain manipulations, protecting and deprotecting steps, and other synthetic procedures disclosed above may be necessary to produce compounds with the desired combinations of R⁶, R⁷, R⁸ and R¹³.

As shown in Scheme 4, equation a) the reaction of aniline derivative (34) with imidate ester (12) to form the substituted amidine (35) provides material which can be cyclized with dihydroxyacetone to form structure (36). Subsequent elaboration into (I) provides the N-arylimidazole compounds of the invention.

Alternatively as shown by equation b) the Marckwald procedure, described by Marckwald et al., Ber., 22, 568, 1353 (1889); Ber., 25, 2354 (1892) can form a 2-mercaptoimidazole (38) from aniline derivative (34) via isothiocyanate (37). Desulfurization of (38) with dilute nitric acid followed by anion formation at the 2-position of the imidazole (39) and reaction with R⁶X where X is Cl, Br, I, allows the formation of (40) which can be subsequently elaborated to I.

A variation of Marckwald's process as shown in equation c) using an α-aminoketone (41) and isothiocyanate (37) can also be employed, see Norris and McKee, J. Amer. Chem. Soc., 77, 1056 (1955) can also be employed. Intermediate (42) can be converted to (I) by known sequences. The general procedure of Carboni et al., J. Amer. Chem. Soc., 89, 2626 (1967) (illustrated by equation d)) can also be used to prepare N-aryl substituted imidazoles from appropriate haloaromatic compounds (43; X=F, Cl, Br) and imidazoles (1):

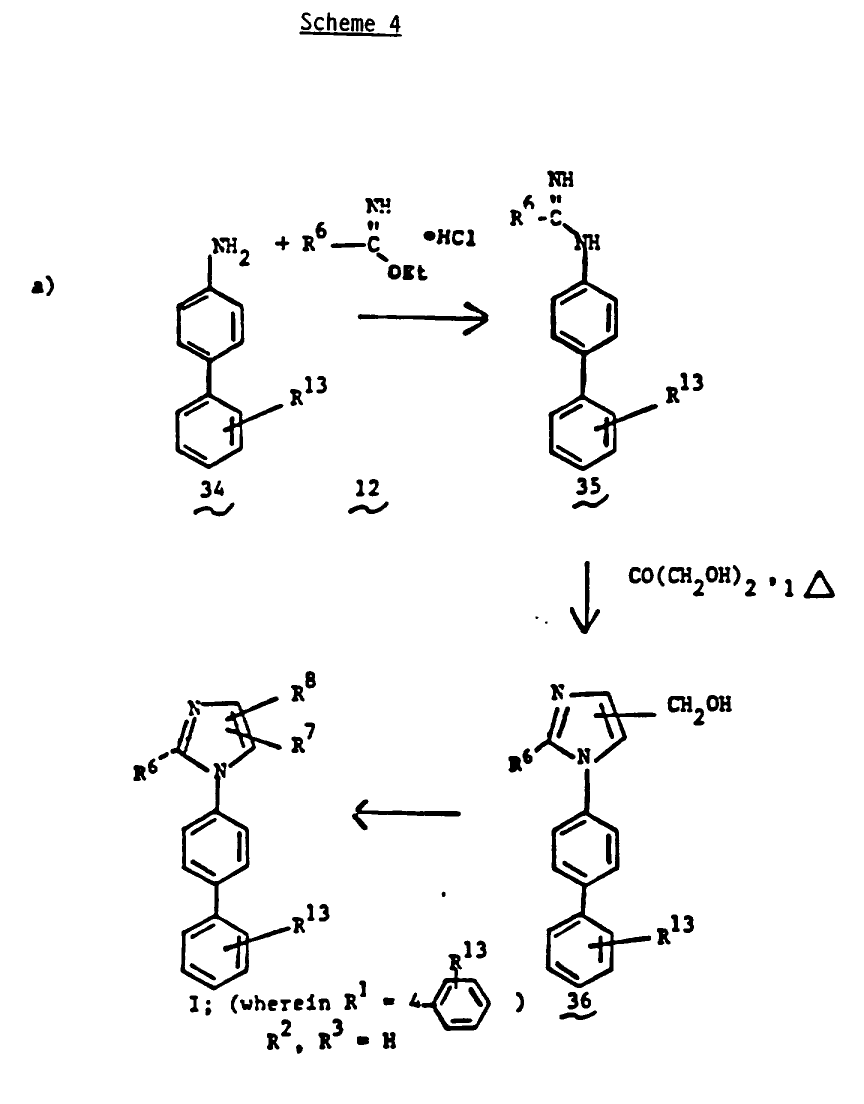

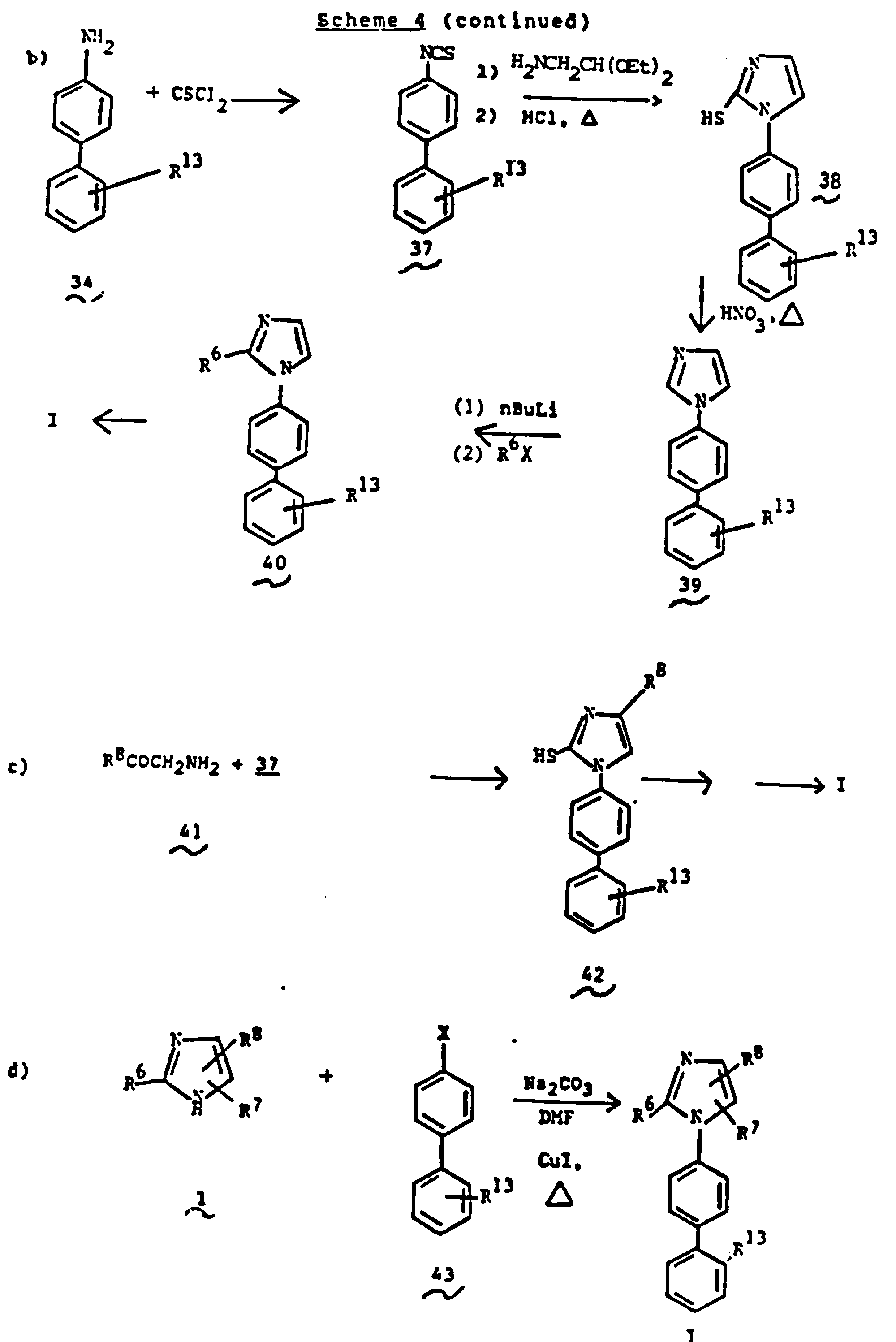

In various synthetic routes R¹, R and R³ do not necessarily remain the same from the starting compound to the final products, but are often manipulated through known reactions in the intermediate steps as shown in Schemes 5-22. All of the transformations shown in Schemes 5-10 and 12 can also be carried out on the terminal aromatic ring (i.e., biphenyl ring).

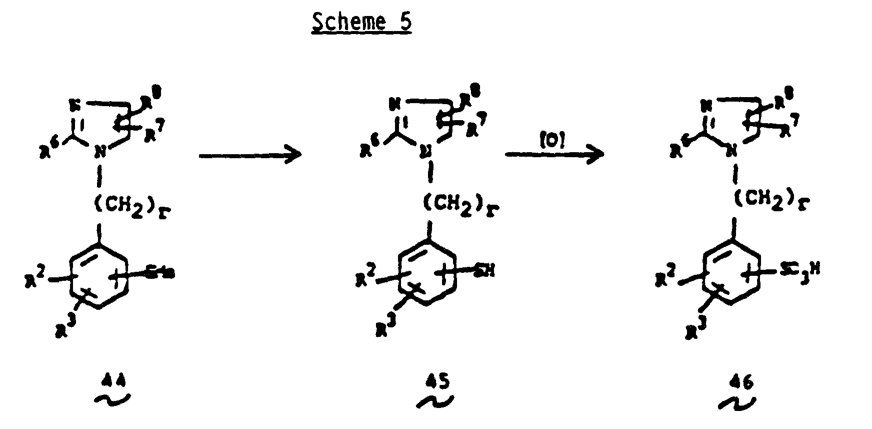

As shown in Scheme 5, compounds where R¹ is a sulfonic acid group may be prepared by oxidation of the corresponding thiol (45). Thus, an N-benzylimidazole derivative bearing a thiol group may be converted into a sulfonic acid (46) by the action of hydrogen peroxide, peroxyacids such as metachloroperoxybenzoic acid, potassium permanganate or by a variety of other oxidizing agents, E. E. Reid, Organic Chemistry of Bivalent Sulfur, l, Chemical Publishing Co., New York, 120-12l (1958).

Aromatic hydroxy or thiol groups are obtained from deprotection of the corresponding alkyl ether or thioethers. Thus, for example, a methyl ether or a methyl thioether derivative (44) of an N-benzylimidazole containing one or more aromatic rings may be converted into the free phenol or thiophenol (45) by the action of boron tribromide methyl sulfide, P. G. Willard and C. F. Fryhle, Tet. Lett., 21, 373l (1980); trimethylsilyl iodide, M. E. Jung and M. A. Lyster, J. Org. Chem., 42, 376l (1977); KSEt and derivatives thereof, G. I. Feutrill, R. N. Mirrington, Tet. Lett., 1327, (1970), and a variety of other reagents.

Alternatively, N-benzylimidazoles may be sulfonated by stirring with H₂SO₄ at a variety of different concentrations or with other sulfonating agents such as chlorosulfonic acid or sulfur trioxide with or without complexing agents such as dioxane or pyridine at temperatures from 0 to 200°C with or without solvent, K. LeRoi Nelson in Friedel-Crafts and Related Reactions, III part 2, G. A. Olah, ed., Interscience Publ., 1355 (1964).

The synthesis of compounds where R¹ is a sulfate, phosphate or phosphonic acid are depicted in Scheme 6:

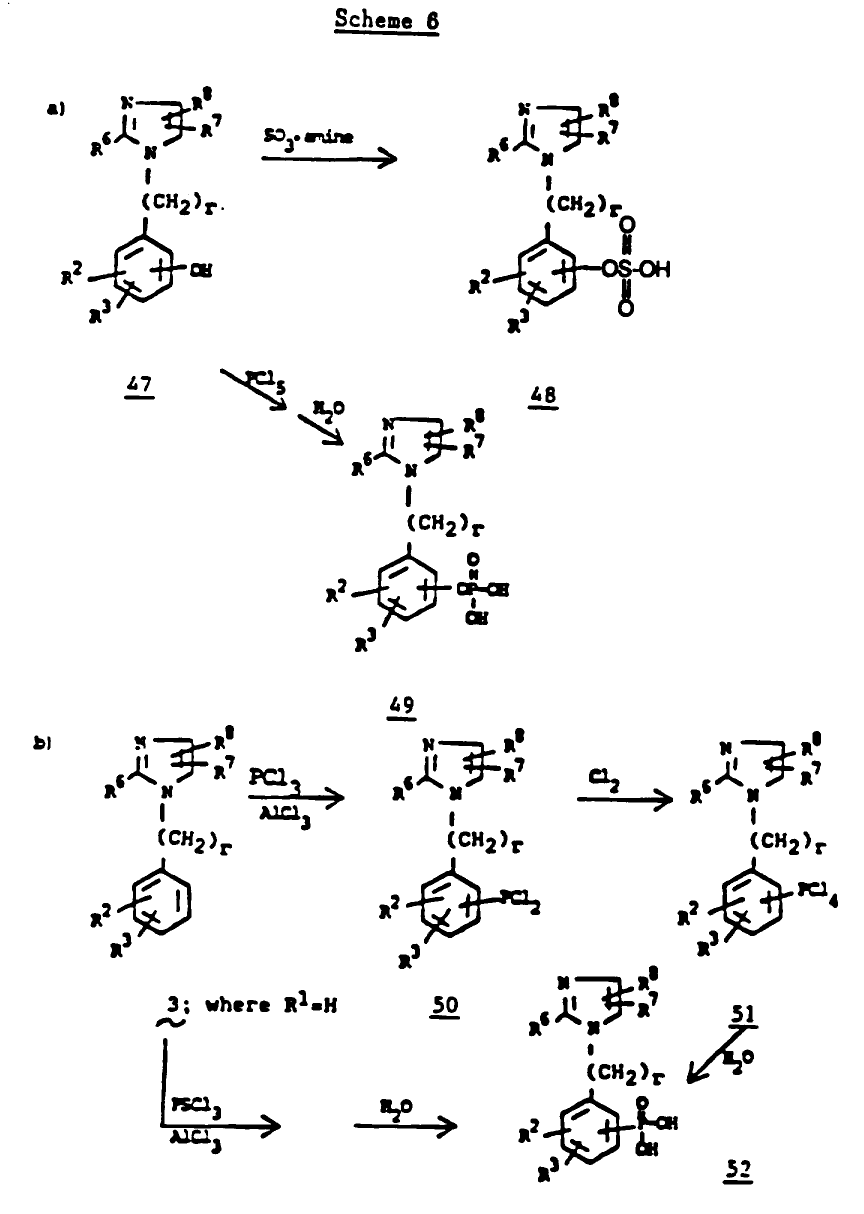

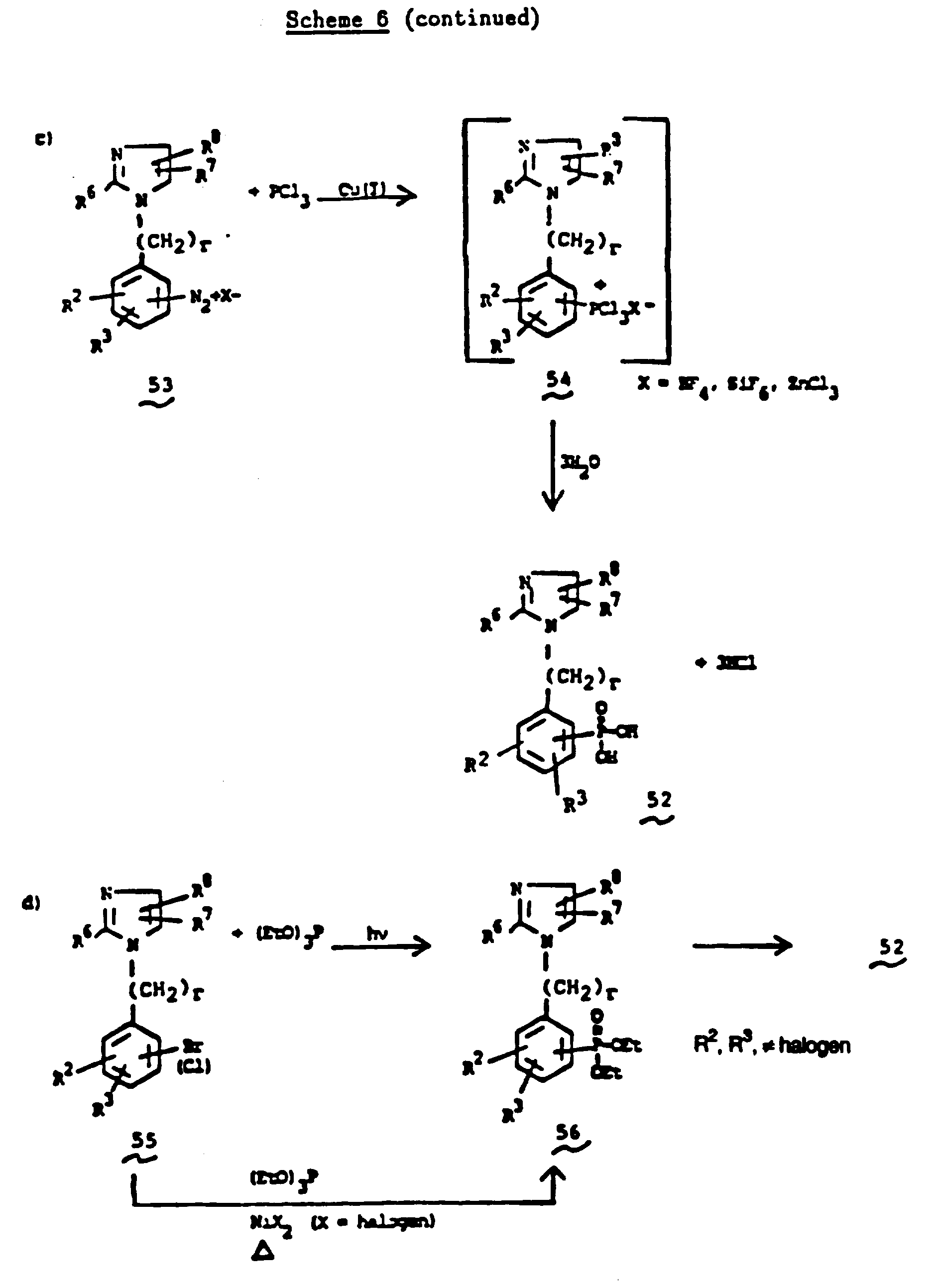

N-Benzylimidazoles containing a phenolic hydroxyl group (47) may be readily converted into the corresponding sulfate (48) or phosphate (49). As shown in equation a), reaction of the phenol with a sulfur trioxide-amine complex will give the corresponding sulfate (48), E. E. Gilbert, Sulfonation and Related Reactions, Interscience, New York, chapter 6 (1965). Reaction of the phenol (47) with phosphorus pentachloride followed by hydrolysis will give the corresponding phosphate (49), G. M. Kosolapoff, Organophosphorus Compounds, John Wiley, New York, 235 (1950).

As shown in equation b) N-benzylimidazoles may be converted into the corresponding phosphonic acids by reaction with phosphorus trichloride (PCl₃) and aluminum chloride (AlCl₃) in an inert solvent for 0.5-96 hours from temperatures of 25°C to the reflux temperatures of the solvent. Appropriate workup followed by reaction with chlorine (Cl₂) and subsequent hydrolysis of the tetrachloride (51) gives the phosphonic acid derivative (52), G. M. Kosolapoff in Org. Reactions, 6, R. Adams, editor, John Wiley and Sons, New York, 297 (1951). Another more direct route involves reaction of the N-benzylimidazole with PSCl₃ and AlCl₃ followed by hydrolysis, R. S. Edmunson in Comprehensive Organic Chemistry, Vol. 2, D. Barton and W. D. Ollis editors, Pergamon Press, New York, 1285 (1979).

Alternatively, equation c) illustrates that aryl phosphonic acids (52) may be formed from reaction of the corresponding diazonium salt (53) with PCl₃ in the presence of Cu(I) followed by hydrolysis with water (ibid, p. 1286).

As shown in equation d), the aryl halides (55) may be photolyzed in the presence of phosphite esters to give phosphonate esters (56), R. Kluger, J. L. W. Chan, J. Am. Chem. Soc., 95, 2362, (1973). These same aryl halides also react with phosphite esters in the presence of nickel or palladium salts to give phosphonate esters, P. Tavs, Chem. Ber., 103, 2428 (1970), which can be subsequently converted to phosphonic acids (52) by procedures known to one skilled in the art.

N-Benzylimidazoles containing an aldehyde or ketone (57) may be reacted with a phosphorus trihalide followed by water hydrolysis to give α-hydroxyphosphonic acid derivatives, G.M. Kosolapoff, op. cit., 304, as shown in Scheme 7.

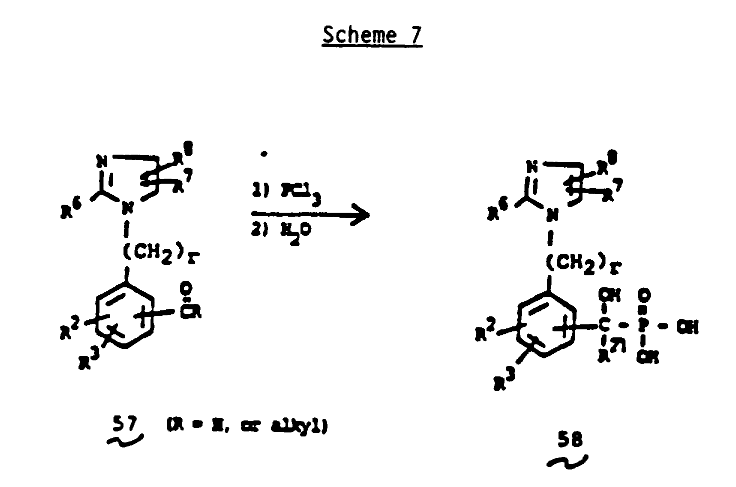

Compounds where R¹ is -CONHOR¹ may be prepared as shown in Scheme 8, by the treatment of a carboxylic acid (10) with 1-4 equivalents of thionyl chloride for 1-10 hours. This reaction can be run without solvent or in a nonreactive solvent such as benzene or chloroform at temperatures of 25-65°C. The intermediate acid chloride is then treated with 2-10 equivalents of the appropriate amine derivative, H₂NOR¹, for 2-18 hours at temperatures of 25-80°C in a polar aprotic solvent such as tetrahydrofuran or dimethylsulfoxide to give the hydroxamic acid (59).

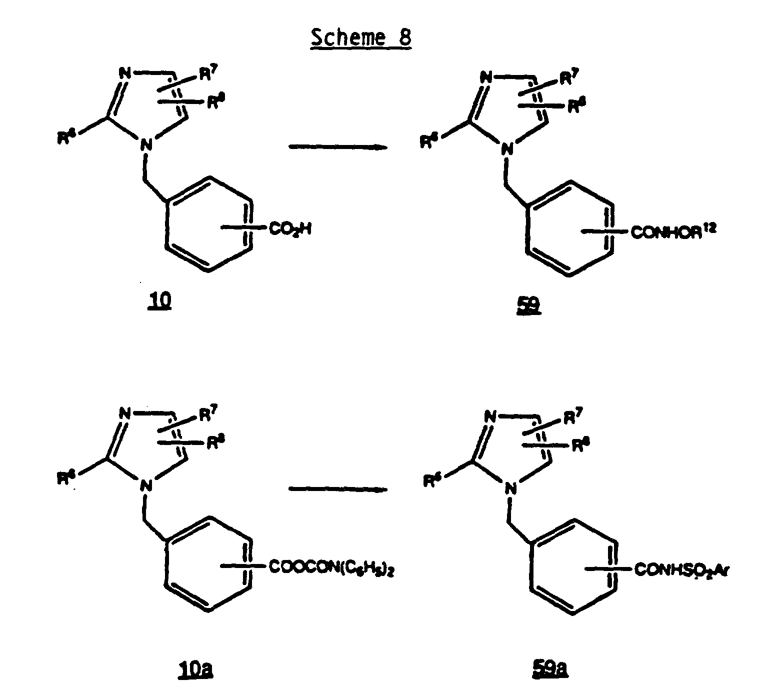

Alternatively, the carboxylic acid (10) can be converted to the hydroxamic acid (59) according to the procedure in J. Med. Chem., 28, 1158 (1985) by employing dicyclohexylcarbodiimide, 1-hydroxybenzotriazole, and H₂NOR¹ or according to the procedure described in Synthesis, 929 (1985) employing the Vilsmeier reagent and H₂NOR¹.

Compounds where R¹ is -CONHSO₂Ar (59a, Ar=phenyl, o-tolyl, etc.) may be produced by treatment of the intermediate acid chlorides from the preparation of the hydroxamic acids (59), with ArSO₂NHNa. Alternatively, these acylsulfonamides (59a) can be prepared from the carboxylic acids (10) through the corresponding N,N-diphenylcarbamoyl anhydrides (10a) as described by F. J. Brown, et al. in Eur. Pat. Appl. EP 199543 (see Scheme 8).

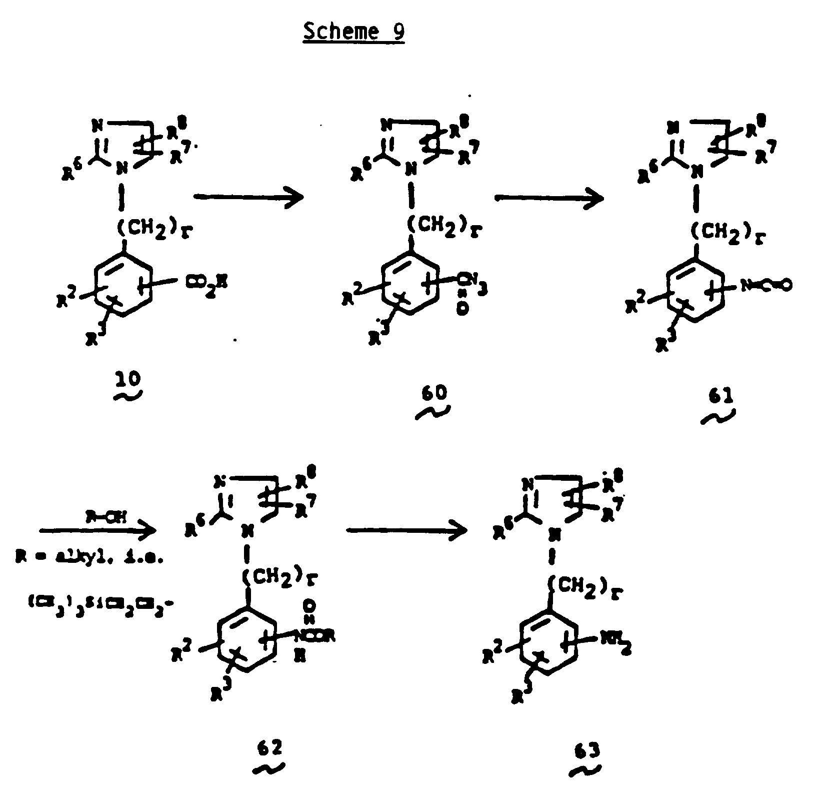

Aniline intermediates (63) are disclosed in U.S. Patent No. 4,355,040 and may be obtained from the corresponding nitro compound precursor by reduction. A variety of reduction procedures may be used such as iron/acetic acid, D. C. Owsley, J. J. Bloomfield, Synthesis, 118, (1977), stannous chloride, F. D. Bellamy, Tet. Lett., 839, (1984) or careful hydrogenation over a metal catalyst such as palladium.

As shown in Scheme 9, aniline intermediates of N-benzylimidazoles may also be prepared from the corresponding carboxylic acid (10) or acid chloride via a Curtius rearrangement of an intermediate acyl azide (60). More modern methods include using diphenylphosphoryl azide as a source of azide, T. Shioiri, K. Ninomiya, S. Yamada, J. Am. Chem. Soc., 94, 6203 (1972), and trapping the intermediate isocyanate (61) produced by the Curtius rearrangement with 2-trimethylsilylethanol and cleaving the resultant carbamate (62) with fluoride to liberate the amine (63), T. L. Capson and C. D. Poulter, Tet. Lett., 25, 3515 (1984). Classical procedures familiar to one skilled in the art may also be employed.

Compounds where R¹ is -SO₂NH₂ may be made as shown in Scheme 10:

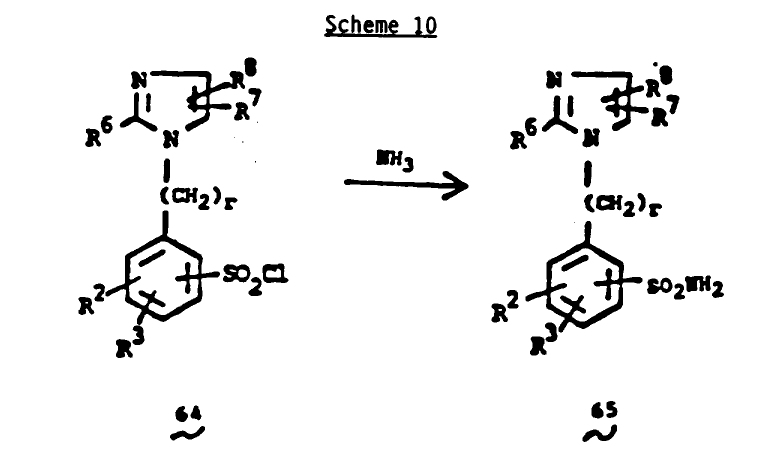

Sulfonamide compounds (65) may be made by reacting an arylsulfonyl chloride (64) with ammonia, or its equivalent. Unsubstituted arylsulfonamides are made by reaction with ammonia in aqueous solution or in an inert organic solvent, F. H. Bergheim and W. Braker, J. Am. Chem. Soc., 66, 1459 (1944), or with dry powdered ammonium carbonate, E. H. Huntress and J. S. Autenrieth, J. Am. Chem. Soc., 63, 3446 (1941); E. H. Huntress and F. H. Carten, J. Am. Chem. Soc., 62, 5ll (1940).

The sulfonyl chloride precursor may be prepared by chlorosulfonation with chlorosulfonic acid on the aromatic ring directly, E. H. Huntress and F. H. Carten, ibid.; E. E. Gilbert, op. cit., 84, or by reacting the corresponding aromatic diazonium chloride salt (53) with sulfur dioxide in the presence of a copper catalyst, H. Meerwein, et al., J. Prakt. Chem., [ii], 152, 25l (1939), or by reacting the aromatic sulfonic acid (46) with PCl₅ or POCl₃, C. M. Suter, The Organic Chemistry of Sulfur, John Wiley, 459 (1948).

Linked ester compounds of formula (I) where R¹ is 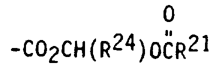 can be made by procedures well known in penicillin and cephalosporin chemistry. The purpose is to provide materials which are more lipophilic and which will be useful orally by rapid transit from the gut into the bloodstream, and which will then cleave at a sufficiently rapid rate to provide therapeutically useful concentrations of the active carboxylic acid form. The following review articles and references cited therein discuss this concept and the chemistry involved in preparing such compounds V. J. Stella, et al., Drugs, 29, 455-473 (1985); H. Ferres, Drugs of Today. 19 (9), 499-538 (1983); A. A. Sirkula, Ann. Repts. Med. Chem., 10, 306-315 (1975).

Experimental procedures which are applicable to the preparation of chemically stable linked esters are illustrated by equations a-e of Scheme 11. 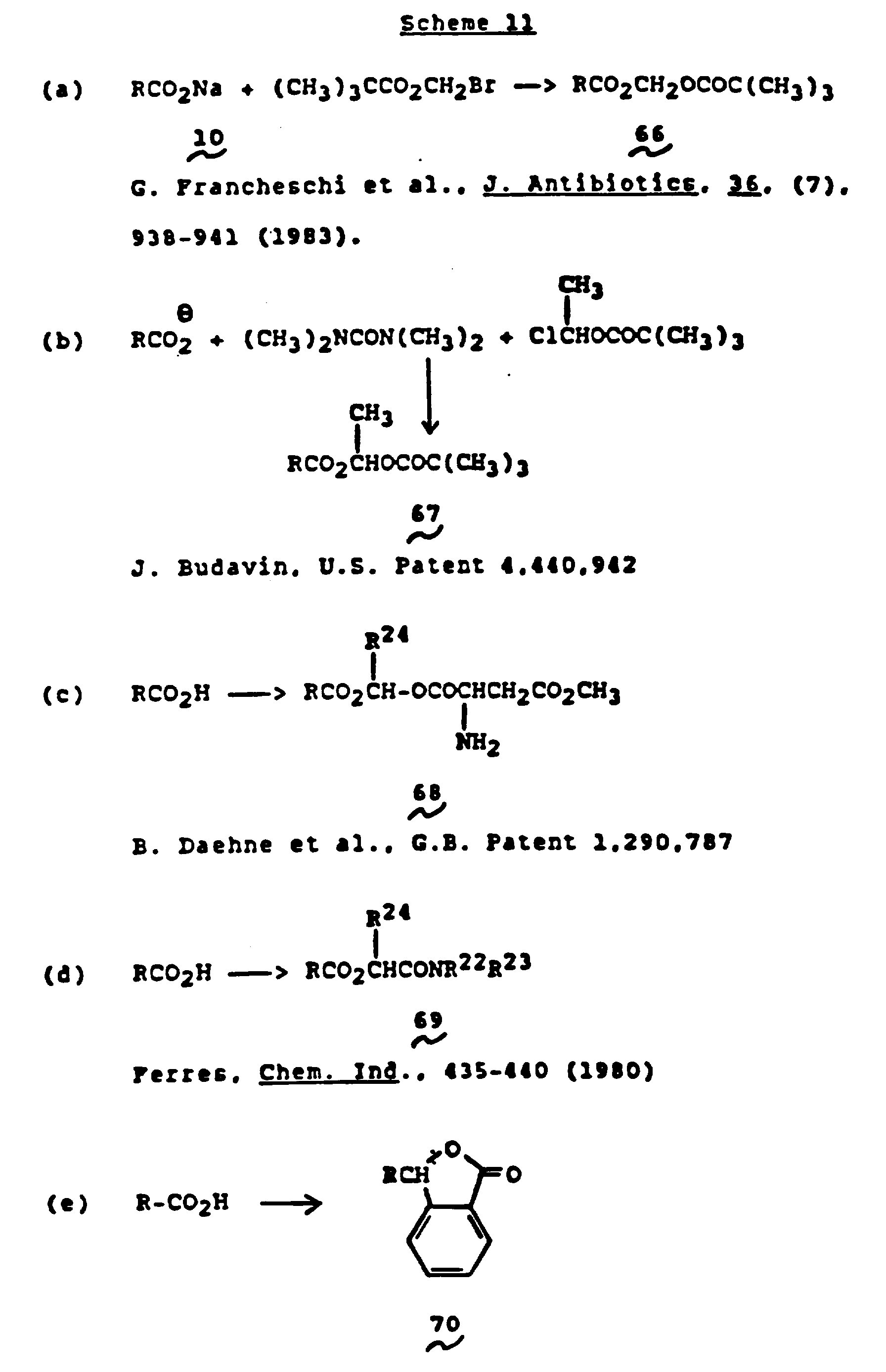 Clayton et al., Antimicrob. Agents Chemotherapy, 5, (6), 670-671 (1974) In equations a-e:

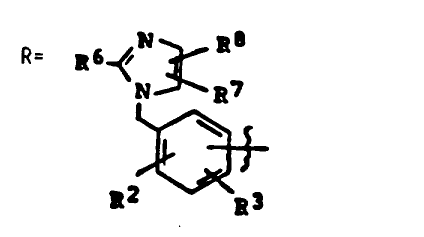

Compounds of Formula I where R¹ is -C(CF₃)₂OH may be prepared as shown in Scheme 12.

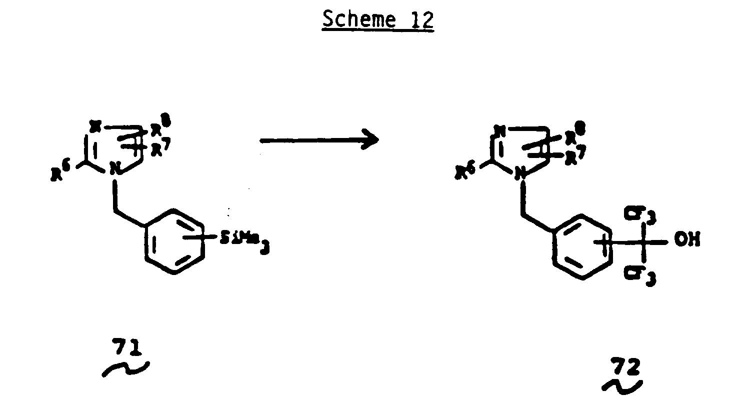

Hexafluoroisopropanol compounds (72) may be prepared by treatment of arylsilane (71) with 1-5 equivalents of hexafluoroacetone in a solvent such as methylene chloride at temperatures ranging from about -50° to 25°C for a period of 2-10 hours. The requisite arylsilane (71) can be prepared using methods known to one skilled in the art such as the procedures described in Chapter 10 of Butterworth's "Silicon in Organic Chemistry".

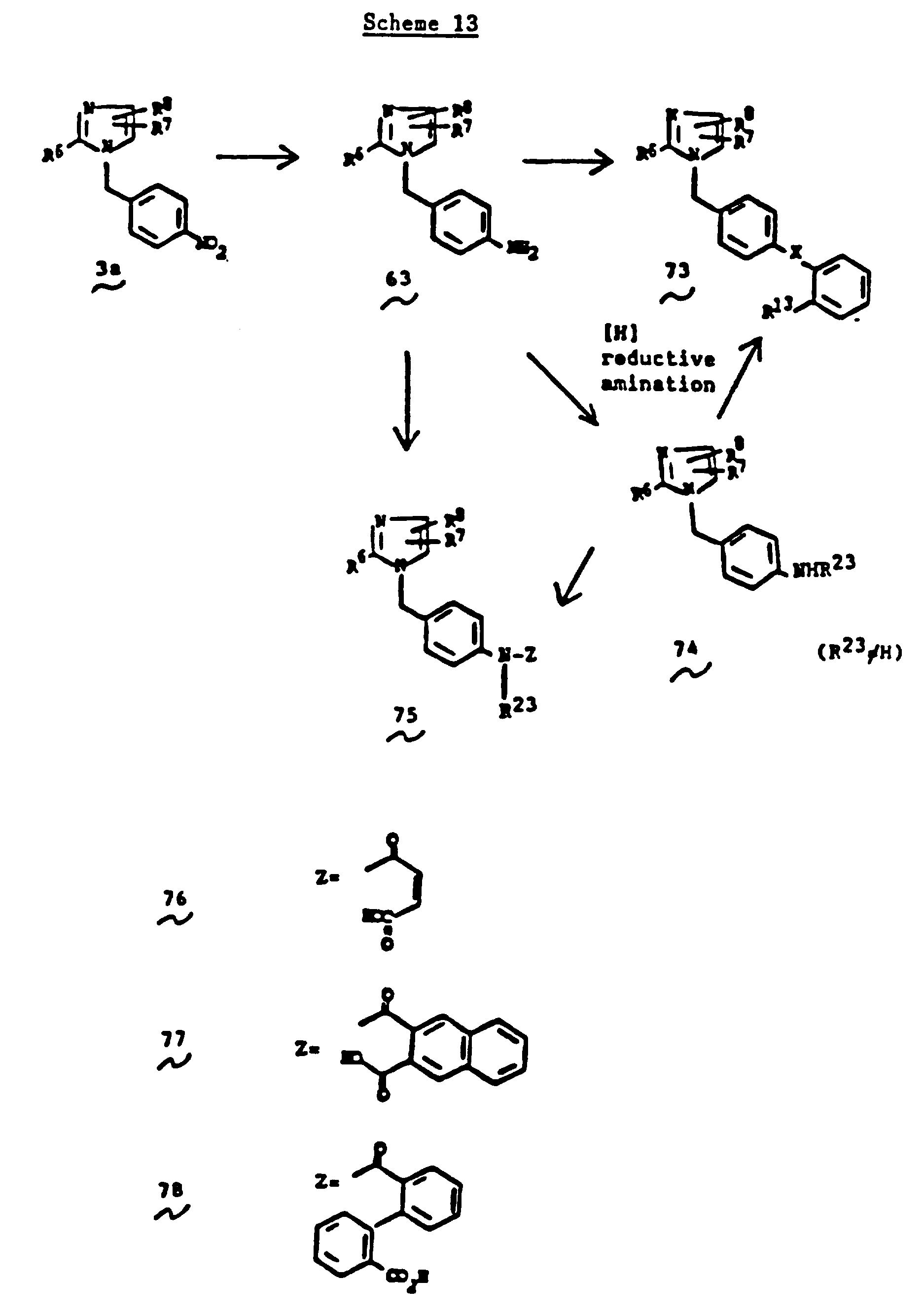

As shown in Scheme 13, compound (73) in which X= -NHCO and R¹³= -COOH may be easily prepared, for example, by reacting aniline precursor (63) with a phthalic anhydride derivative in an appropriate solvent such as benzene, chloroform, ethyl acetate, etc. Often the carboxylic acid product will precipitate from solution with the reactants remaining behind, M.L. Sherrill, F.L. Schaeffer, E.P. Shoyer, J. Am. Chem. Soc., 50, 474 (1928).

When R¹³=NHSO₂CH₃, NHSO₂CF₃ or tetrazolyl (or a variety of other carboxylic acid equivalents), compound (73) may be obtained by reacting aniline (63) with the requisite acid chloride by either a Schotten-Baumann procedure, or simply stirring in a solvent such as methylene chloride in the presence of a base such as sodium bicarbonate, pyridine, or triethylamine.

Likewise, aniline (63) may be coupled with an appropriate carboxylic acid via a variety of amide or peptide bond forming reactions such as DCC coupling, azide coupling, mixed anhydride synthesis, or any other coupling procedure familiar to one skilled in the art.

Aniline derivatives (63) will undergo reductive amination with aldehydes and ketones to form secondary amines (74). Thus the aniline is first stirred with the carbonyl compound in the presence of a dehydration catalyst such as molecular sieves or p-toluenesulfonic acid. Afterwards the resultant imine is reduced to the amine with a borohydride reducing agent such as sodium cyanoborohydride or sodium borohydride. Standard catalytic hydrogenation reagents such as hydrogen and palladium/carbon can also be employed.

Alternatively, aniline (63) may be monoalkylated by reaction with ethyl formate followed by reduction with, for example, lithium aluminum hydride to produce the N-methyl derivative (74). Anilines (74) may in turn be reacted with carboxylic acid anhydrides and acid chlorides or carboxylic acids by any of the coupling procedures described previously to yield (73) where X= -N(CH₃)CO-.

Aniline (63) or (74) or other intermediate anilines where the amino group may be located on another aromatic ring for example, also react with other anhydrides to make amide-carboxylic acid derivatives of formula (75). Thus, for example, maleic anhydride, 2,3-naphthalenedicarboxylic acid anhydride, and diphenic anhydride are reacted in a similar fashion to phthalic anhydride with aniline (63) or (74) to yield carboxylic acids (76), (77), and (78), respectively.

Phthalimide derivatives of aniline (63) may be made by a variety of methods, preferably by stirring aniline (63) with phthalic anhydride in acetic acid at a temperature between 20°C and reflux, G. Wanag, A. Veinbergs, Ber., 75, 1558 (1942), or by stirring (63) with phthaloyl chloride, a base such as triethylamine, and an inert solvent.

Aniline (63) may be converted into its trifluoromethanesulfonamide derivative or its trifluoroacetamido derivative preferably by reacting it with triflic anhydride or trifluoroacetic anhydride and a base such as triethylamine in an inert solvent such as methylene chloride at -78°C followed by warming to room temperature.

Compounds of structure (I) where X is a carbon-carbon linkage which are depicted as (80) can be made as shown in Scheme 14.

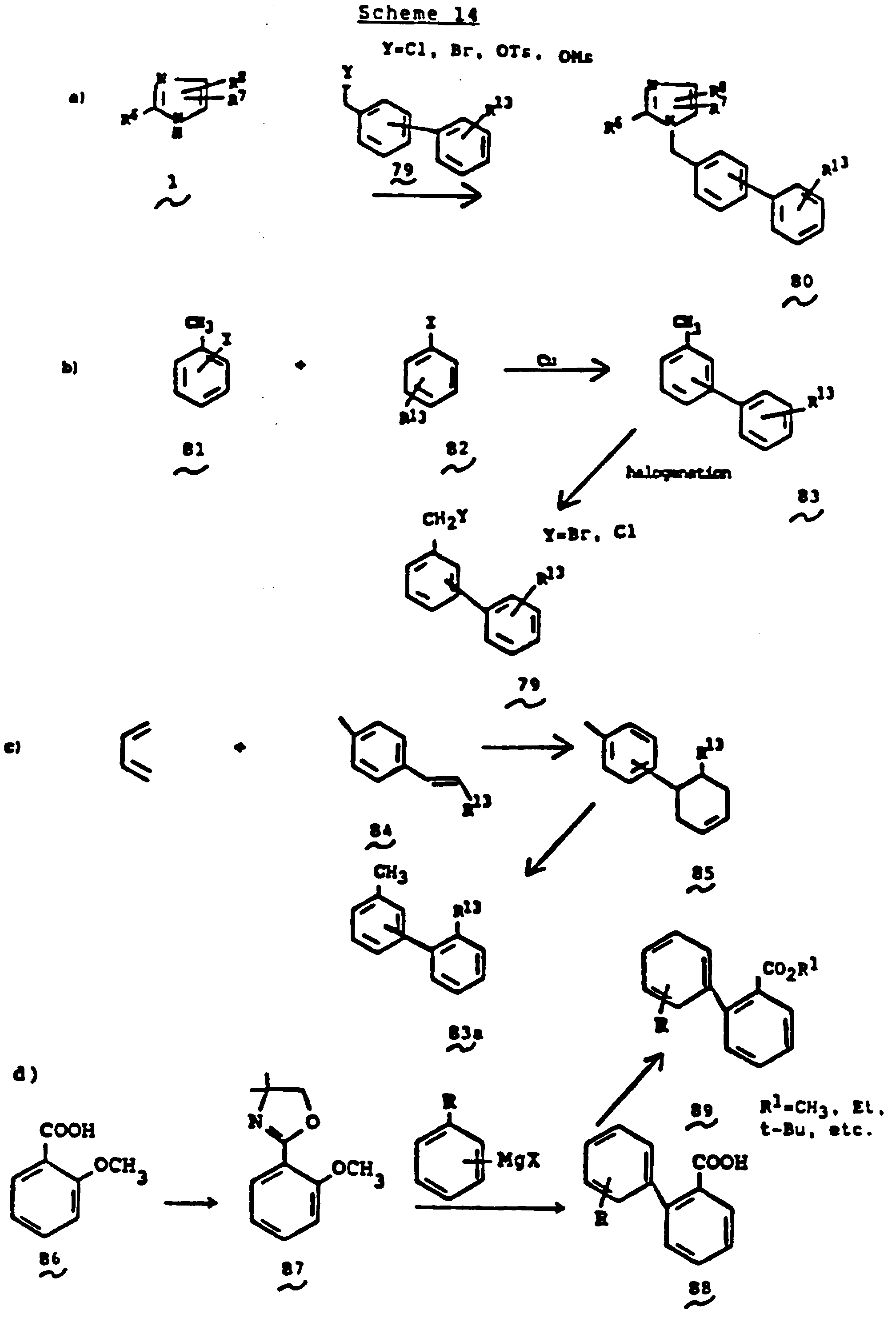

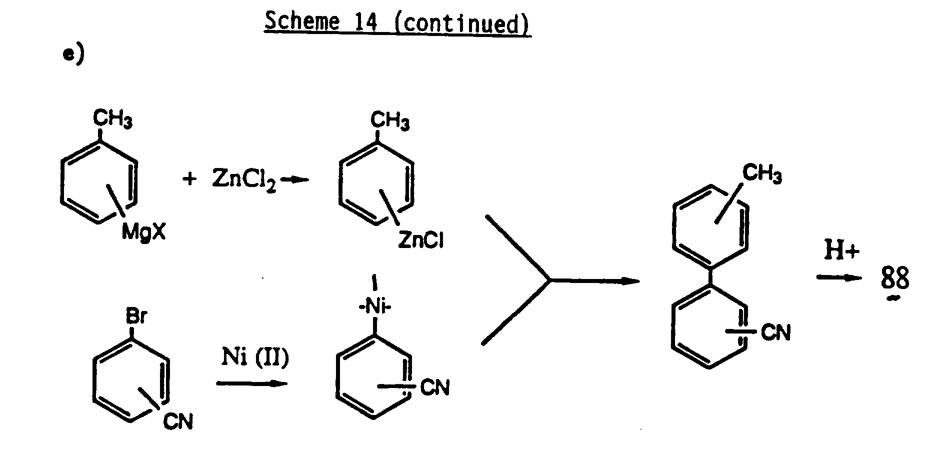

Equation a) illustrates that the biphenyl compounds (80) can be prepared by alkylation of imidazole (1) with the appropriate halomethylbiphenyl compound (79) by the general procedure described in Scheme 1.

The requisite halomethylbiphenyl intermediates (79) are prepared by Ullman Coupling of (81) and (82) as described in "Organic Reactions", 2, 6 (1944) to provide intermediates (83), which are in turn halogenated. Halogenation can be accomplished by refluxing (83) in an inert solvent such as carbon tetrachloride for 1-6 hours in the presence of a N-halosuccinimide and an initiator such as azobisisobutyronitrile (equation b).

As shown in equation c), derivatives of intermediate (83) in which R¹³ is at the 2' position (83a) can also be prepared by the method described in J. Org. Chem., 41, 1320 (1976), that is Diels-Alder addition of a 1,3-butadiene to a styrene (84) followed by aromatization of intermediate (85).

Alternatively, the substituted biphenyl precursors (83; where R¹³ = COOH) and their esters (89) can be prepared as illustrated in equation d), which involves oxazoline compounds as key intermediates, A. I. Meyers and E. D. Mihelich, J. Am. Chem. Soc., 97, 7383 (1975).

Further, as shown in Equation e), nickel-catalyzed cross-coupling of an arylzinc halide with a halobenzonitrile yields a biphenylnitrile which can in turn be hydrolyzed by standard methods to afford acid 88.

The substituted biphenyl tetrazoles (83; where 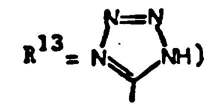 can be prepared from the nitrile precursors (R¹³=CN) by the methods described in Scheme 1, equation c) and Scheme 15, equation c).

However, a preferred method for preparing tetrazoles is described in Scheme 15, equations a) and b). Compounds (90) may be prepared by the 1,3-dipolar cycloaddition of trialkyltin or triphenyltin azides to the appropriately substituted nitrile (83) as in equation a). Alkyl is defined as normal alkyl of 1-6 carbon atoms and cyclohexyl. An example of this technique is described by S. Kozima, et al., J. Organometallic Chemistry, 337 (1971). The required trialkyl or triaryltin azides are made from the requisite commercial trialkyl or triaryl tin chloride and sodium azide. The trialkyl or triaryltin group is removed via acidic or basic hydrolysis and the tetrazole can be protected with the trityl group by reaction with trityl chloride and triethylamine to give (91). Bromination as previously described herein with N-bromosuccinimide and dibenzoylperoxide affords compound (92). Alkylatlon of (1) with the appropriately substituted benzyl halide using conditions previously described followed by deprotection of the trityl group via hydrolysis affords (80; R¹³ = tetrazole). Other protecting groups such as p-nitrobenzyl and 1-ethoxyethyl can be used instead of the trityl group to protect the tetrazole moiety. These groups as ll as the trityl group can be introduced and removed by procedures described in Greene, Protective Groups in Organic Synthesis, Wiley-Interscience, (1980).

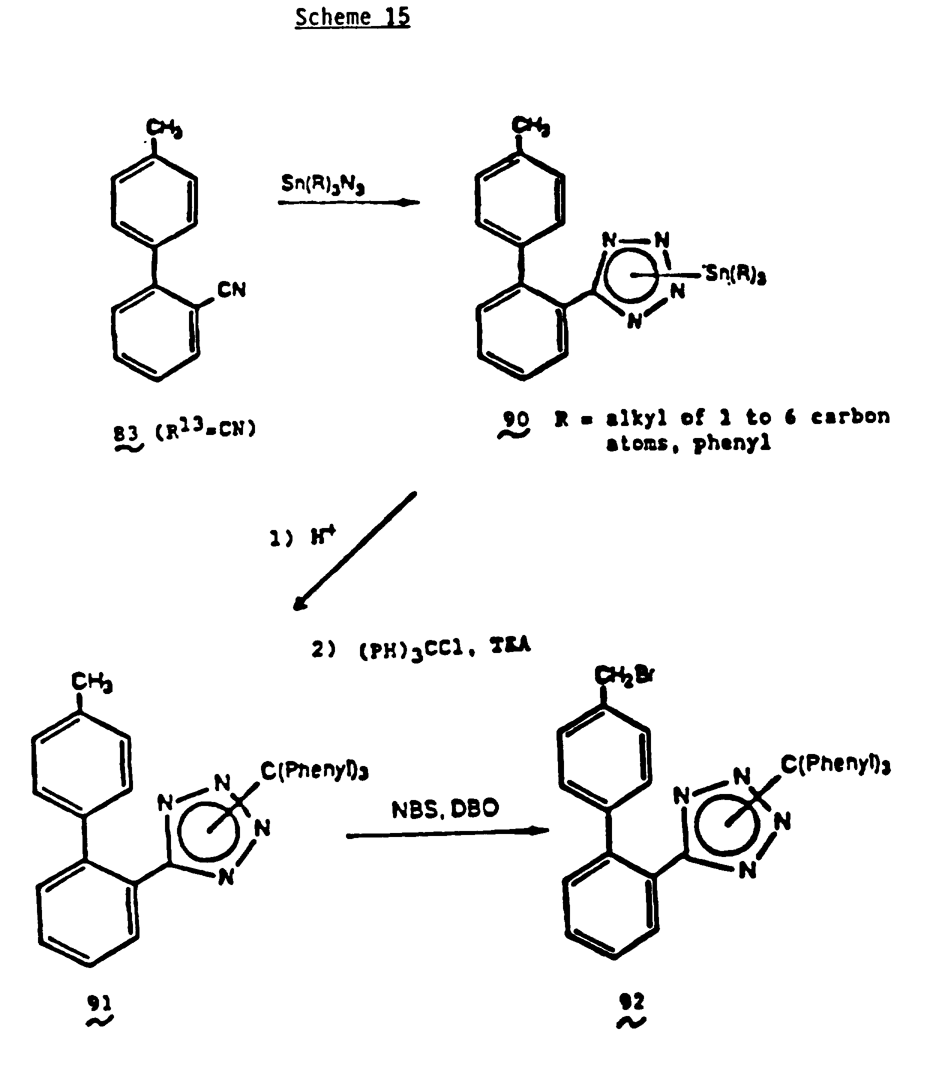

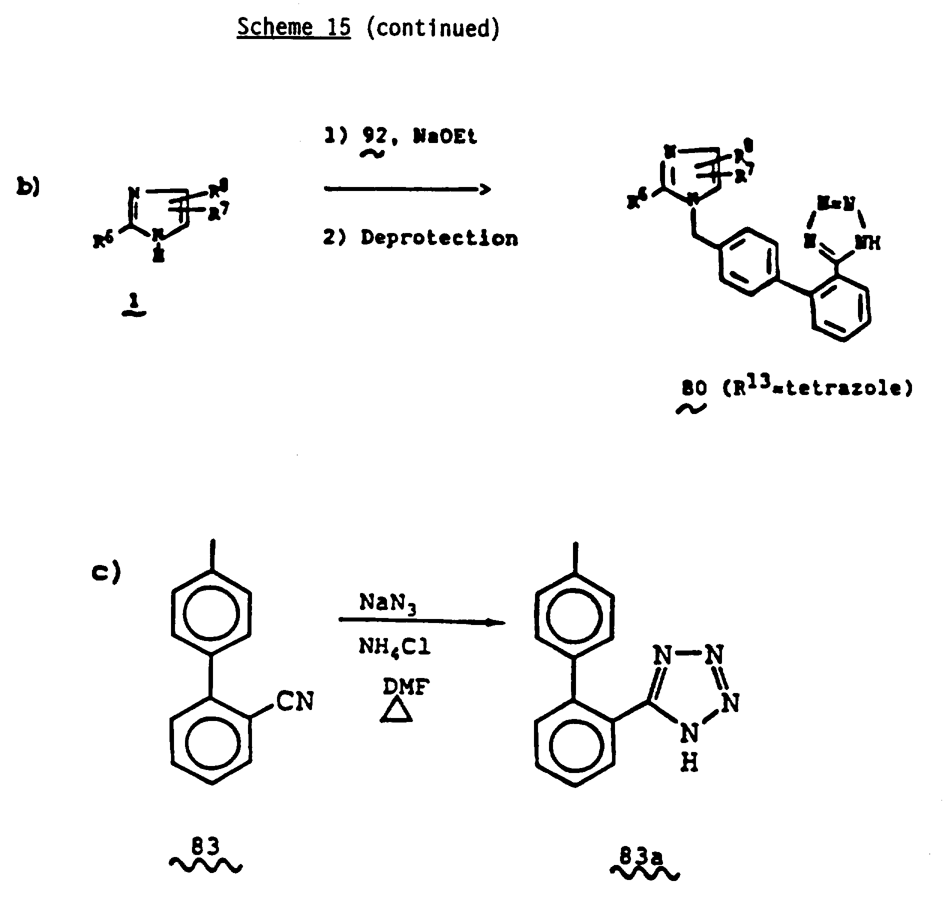

Compounds of structure 93-95 where X is an -O-, -S-, or 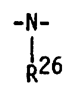 linkage can be prepared as shown in Scheme 16 by alkylation of imidazole (1) with the appropriate benzyl halide (96).

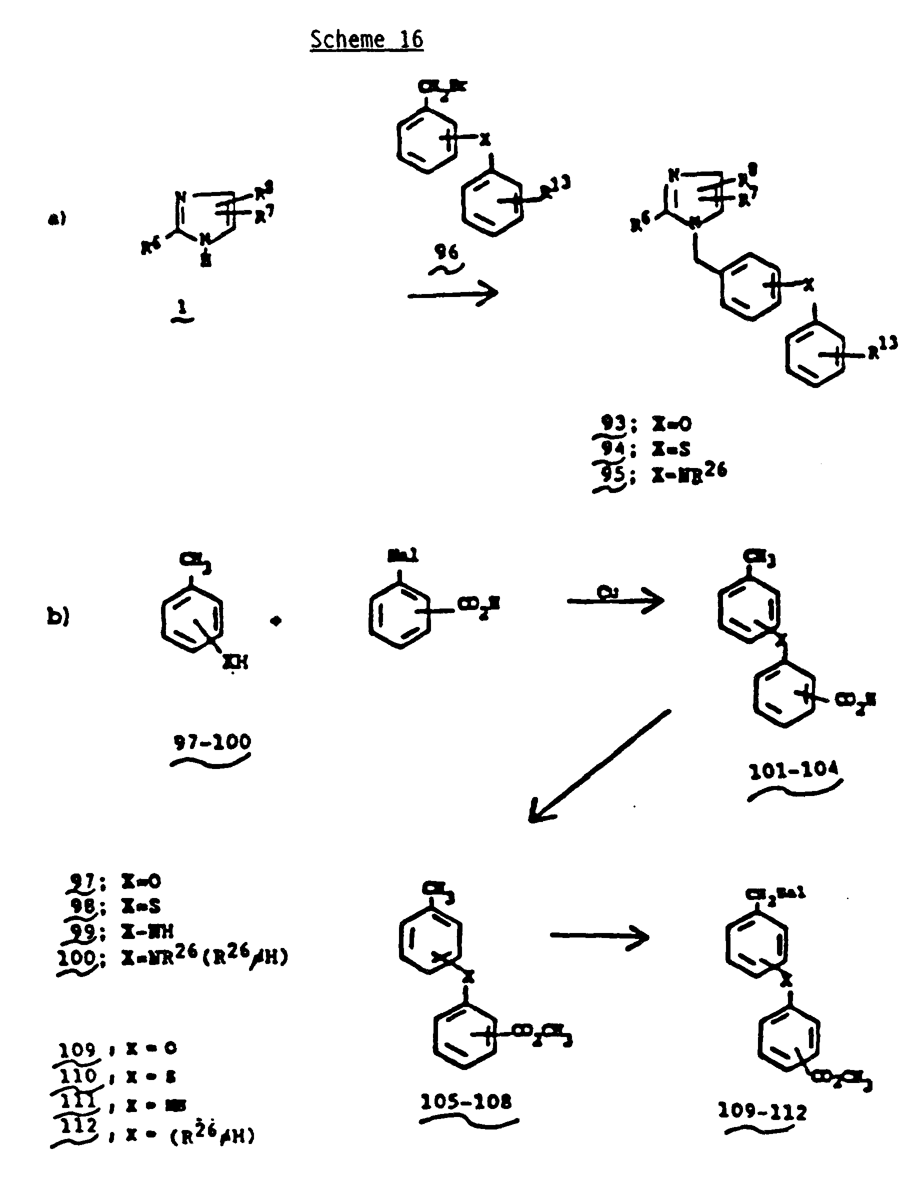

The halomethyldiphenyl ether (109) employed as an alkylating agent in the present invention is prepared as shown in equation b). An Ullman ether condensation of the phenol (97) and a halobenzoic acid as described in Russian Chemical Reviews, 43, 679 (1974) provides the intermediate acid (101). The conversion of (101) into (109) is accomplished by esterification with diazomethane to afford (105) followed by halogenation employing the procedure used in the preparation of (79). The diphenylsulfide (110) and the diphenylamine (111) can be prepared from the appropriate thiophenol (98) or aniline (99) by this procedure.

The tertiary diphenylamine (112) can be prepared from the secondary aniline (100) by the above procedure. Alternatively (107) can be alkylated by one of the following procedures: 1) direct alkylation of (107) with R⁶L where L is a leaving group such as a halogen or tosylate employing phase-transfer conditions and ultrasound as described in Tetrahedron Letters, 24, 5907 (1983), 2) treatment of (107) with 1-1.5 equivalents of an appropriate aldehyde and 0.5-5.0 equivalents of sodium cyanoborohydride in a solvent such as methanol at 25°C at a pH of 3-6 for 1-24 hours, or 3) reductive amination of (107) employing an appropriate carboxylic acid and sodium borohydride as described in J. Am. Chem. Soc., 96, 7812 (1974). The tertiary amine (108) is then halogenated by the procedure previously described to give (112).

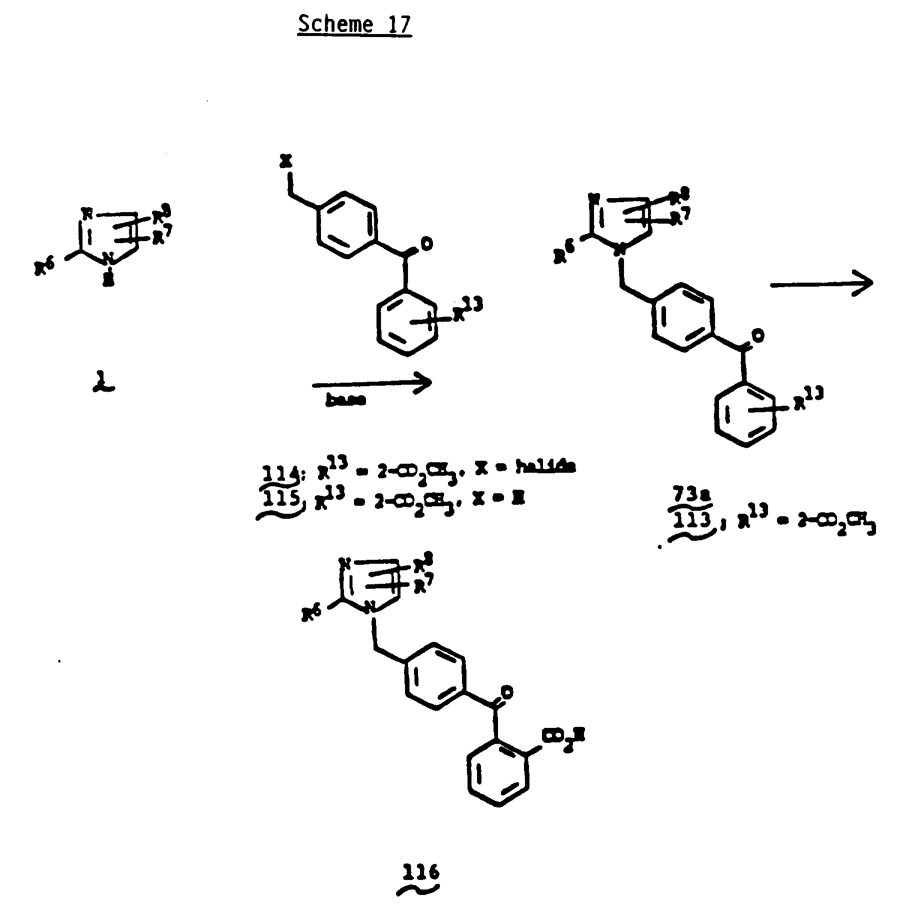

Compounds of structure (73) where X is -CO- are prepared as shown in Scheme 17 by alkylation of imidazole (1) with the requisite benzoylbenzyl halides. For example, esters (113) where R¹³ is 2-CO₂CH₃ are prepared by alkylation of imidazole (1) with carbomethoxybenzoyl benzyl halide (114). Ester (113) may be hydrolyzed to the corresponding carboxylic acid (116) by a variety of methods including hydrolysis with a base such as sodium hydroxide or potassium hydroxide in an alcoholic aqueous solvent such as methanol/H₂O at a temperature from 20°C to the reflux temperature of the solvent.

Carboalkoxybenzoylbenzyl halides (114) are prepared by benzylic halogenation of the corresponding toluoylbenzene precursor by a variety of methods previously described herein. For example, methyl 2-(4-methylbenzoyl)benzoate (115) can be refluxed for 2-48 hours with N-bromosuccinimide, benzoyl peroxide and carbon tetrachloride to effect benzylic bromination.

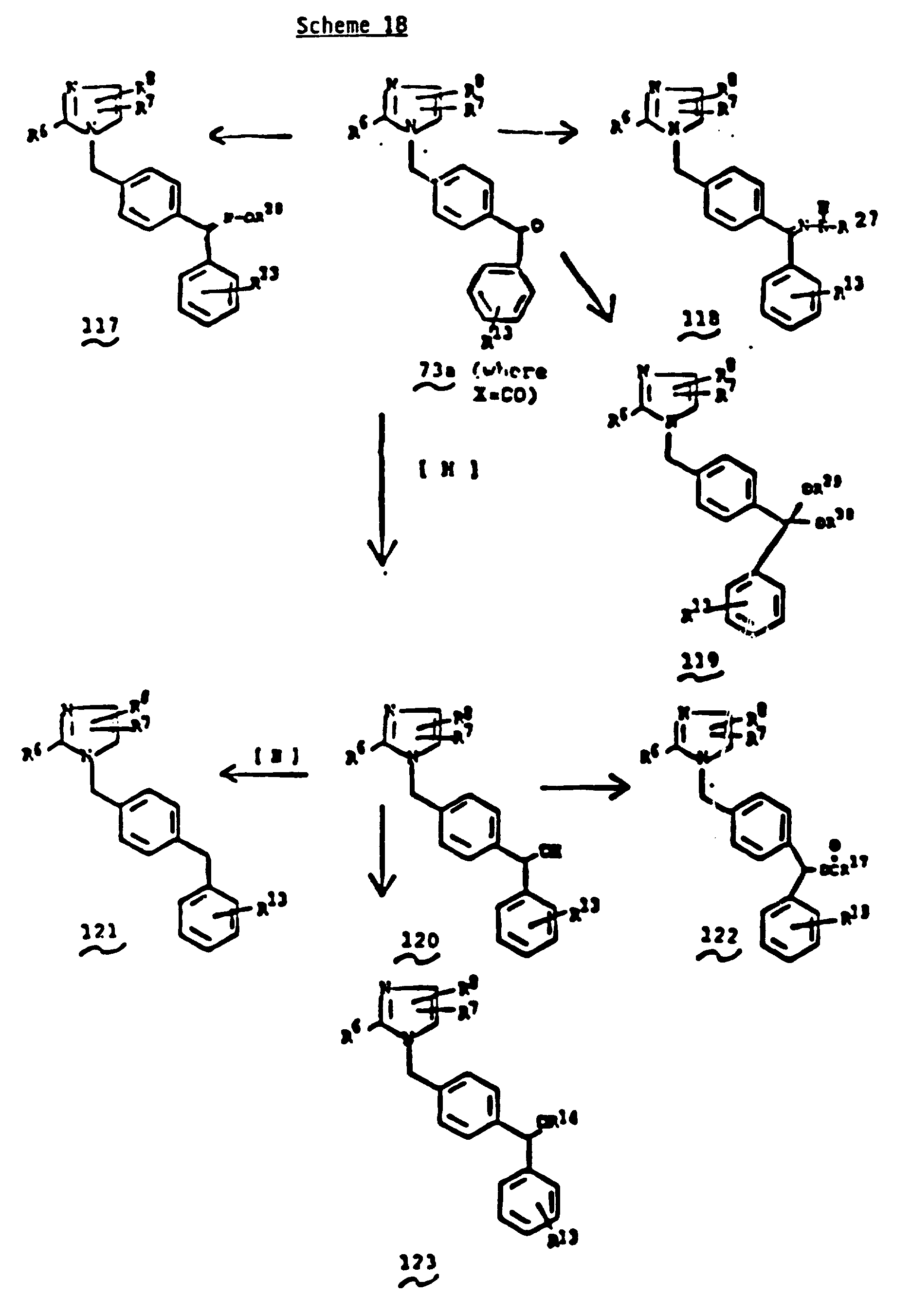

As shown in Scheme 18 the toluoyl ketones (73; where X=CO) may be further transformed into a variety of ketone derivatives including compounds where X is 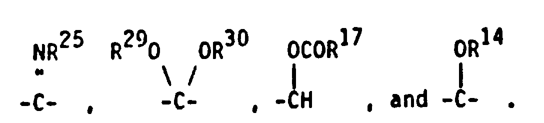 Reaction of ketone (73a) with a hydroxylamine or an appropriately substituted hydrazine will give the requisite oximes (117) and hydrazones (118). Reaction with alcohols in the presence of an acidic catalyst with removal of water will give ketals (119). Reduction, with lithium aluminium hydride, a metal borohydride, zinc/acetic acid or catalytic hydrogenation will give the corresponding alcohol (120) or fully reduced methylene compound (121). These alcohols may be acylated by a variety of anhydrides or acid halides in the presence of a base with or without solvent to give the corresponding esters (122). The alcohols (120) may be converted into their corresponding ethers (123) by reaction of tile metal alkoxide with an alkyl halide, mesylate or tosylate in the appropriate solvent or by treatment with a mineral acid in an alcoholic solvent, or by reaction of the alcohol with diazomethane as described in G. Hilgetag and A. Martini, "Preparative Organic Chemistry", John Wiley, New York, 355-368 (1972).

Compounds of formula (I) where X is -OCH₂-, -SCH₂-, and -NHCH₂- are prepared as shown in Scheme 19.

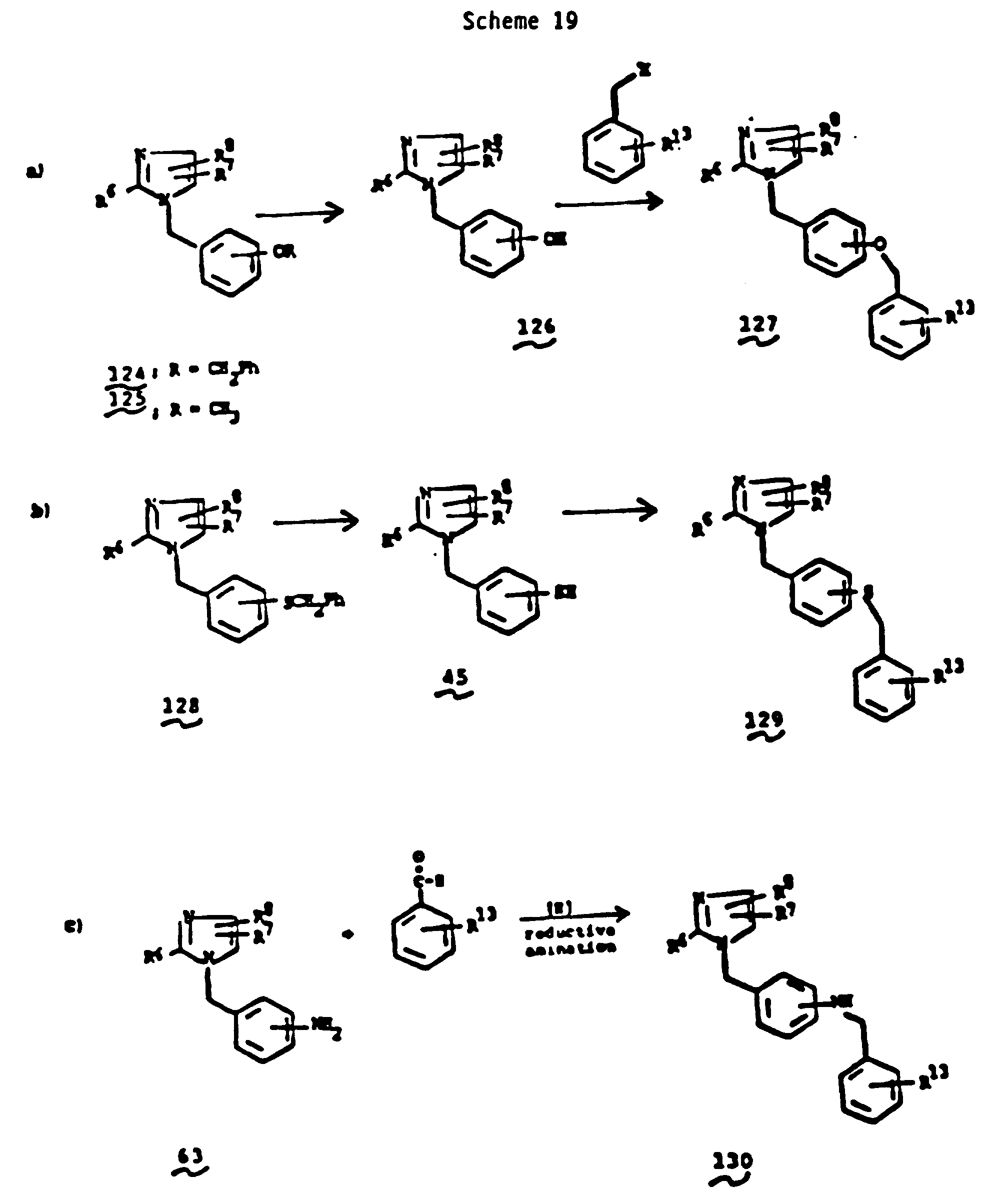

As illustrated in Scheme 19, equation a, hydrolysis of benzyl ether (124) or methyl ether (125) affords hydroxy compound (126) which can be alkylated with the appropriate benzyl halide to give (127). In the case of the methyl ethers (125), the hydrolysis step can be effected by heating the ether at temperatures of 50°-150°C for 1-10 hours in 20-60% hydrobromic acid, or heating at 50°-90°C in acetonitrile with 1-5 equivalents of trimethylsilyl iodide for 10-50 hours followed by treatment with water. Hydrolysis can also be carried out by treatment with 1-2 equivalents of boron tribromide in methylene chloride at 10°-30°C for 1-10 hours followed by treatment with water, or by treatment with an acid such as aluminum chloride and 3-30 equivalents of a sulfur-containing compound such as thiophenol, ethanedithiol, or dimethyl disulfide in methylene chloride at 0-30°C for 1-20 hours followed by treatment with water. For compound (124), hydrolysis can be accomplished by refluxing in trifluoroacetic acid for 0.2-1 hours or by catalytic hydrogenolysis in the presence of a suitable catalyst such as 10% palladium on carbon. Deprotonation of (126) with a base, such as sodium methoxide, sodium hydride or the like in a solvent such as dimethylformamide or dimethylsulfoxide at room temperature followed by alkylation with an appropriate benzyl halide at 25°C for 2-20 hours affords ethers of formula (127), as shown in equation a.

The sulfide (129) can be prepared from the thiophenol (45) by the procedure described above to prepare the ether (127) from the phenol (126). The thiophenol (45) can be prepared for example by treatment of the benzylsulfide (128) with sodium in liquid ammonia.

The amine (130) can be prepared as shown in equation c, from the aniline (63), itself available from reduction of the corresponding p-nitro compound (3a) which has previously been described. The reductive amination can be carried out by the same procedure as described in Scheme 13 for the preparation of compound (74).

Compounds of Formula (I) where the X linkage is -CH=CH-, -CH₂CH₂-, and 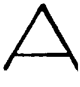 are prepared as shown in Scheme 20.

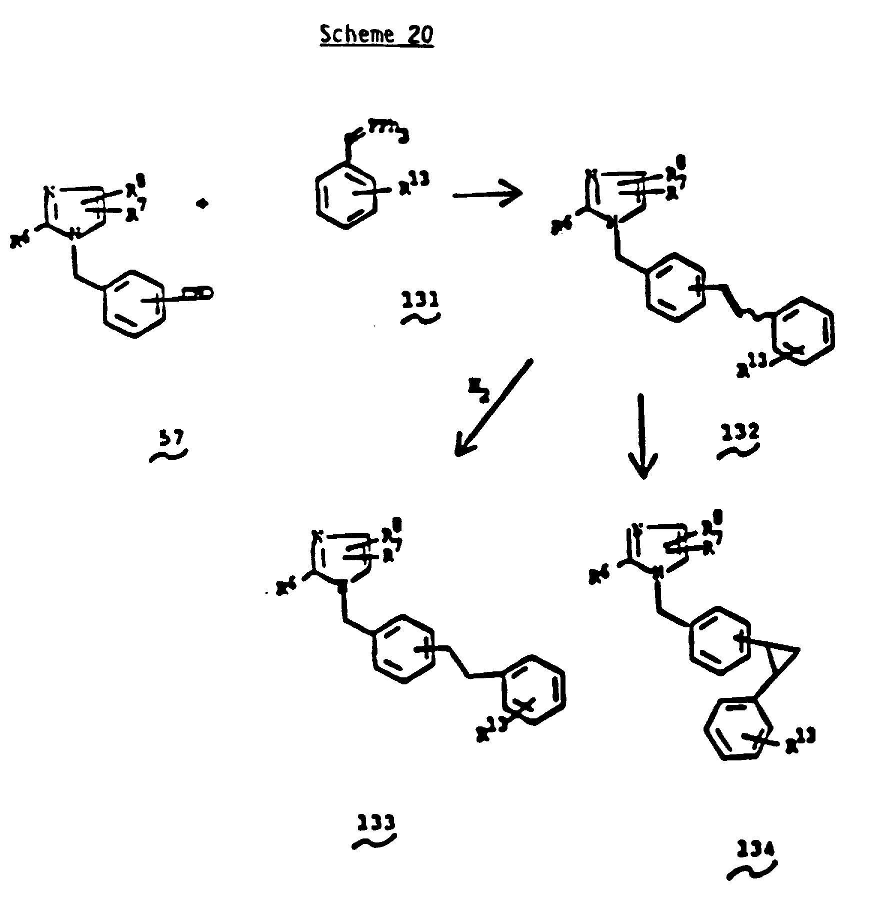

The cis or trans stilbene (132) can be obtained by employing a Wittig reaction between the aldehyde (57) and the phosphorane (131).

The stilbene (132) can readily be converted to the saturated derivative (133) for example by catalytic hydrogenation employing a heterogeneous catalyst such as palladium/carbon or platinum/carbon or alternatively with a homogeneous catalyst such as tristriphenylphosphine rhodium chloride. The reduction is performed in a solvent such as benzene, tetrahydrofuran or ethanol at 25°C under 1-3 atmospheres of hydrogen for 1-24 hours.

The cyclopropane (134) can be prepared by treating the stilbene (132) with the Simmons-Smith reagent as described in J. Am. Chem. Soc., 81, 4256 (1959), or by treating (132) with methylene diiodide and copper powder as described in J. Am. Chem. Soc., 101, 2139 (1979), or by treatment with the iron-containing methylene-transfer reagent described in J. Am. Chem. Soc., 101, 6473 (1979).

The preparation of compounds of formula (I) where X is -CF₂CH₂-, -CF=CH-, -CH=CF-, -CF=CF- and -CF₂CF₂- are depicted in Scheme 21.

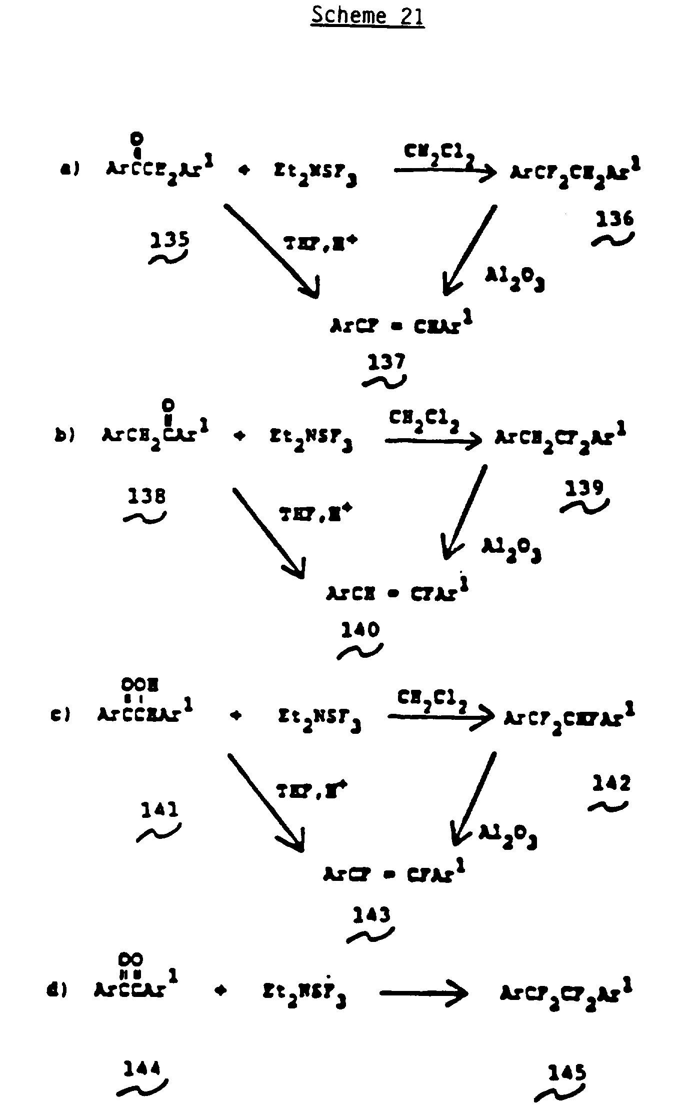

Vinylene fluorides (137) and (140) can be prepared by reaction of SF₄ or Et₂NSF₃ (DAST) with the appropriate ketone (135) or (138) in which Ar bears a methyl group convertible to a benzylic halide suitable for attachment to an imidazole nitrogen, and Ar' bears a cyano, nitro, ester, or other suitable group which can be subsequently converted to CO₂H, NHSO₂CF₃, etc. The initially formed difluoroethylene (136) and (139) can be formed in a non-polar solvent such as methylene chloride and subsequently converted to the vinylene fluoride by means of alumina, or converted directly into the unsaturated fluoride by running the reaction in a polar solvent such as tetrahydrofuran, diglyme or N-methylpyrrolidone in the presence of mineral acid. [Equations a and b]. Experimental details of such procedures are found in D.R. Strobach and G.A. Boswell, J. Org. Chem., 36, 818 (1971); G.A. Boswell, U.S. Patents 3,413,321 (1968) and 4,212,515 (1980).

As shown in equation c) an appropriate benzoin (141) may be similarly converted to the corresponding 1,2-difluorostilbene (143). Likewise as shown in equation d) an appropriate benzil (144) can be converted to a tetrafluorodiarylethylene (145) using DAST or SF₄. Experimental details are described in M.E. Christy, et al., J. Med. Chem., 20, (3), 421-430, (1977).

Compounds of formula 1 where 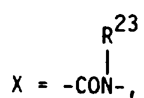 -CH₂O-, -CH₂S-, -CH₂NH-, can be made as shown in Scheme 22.

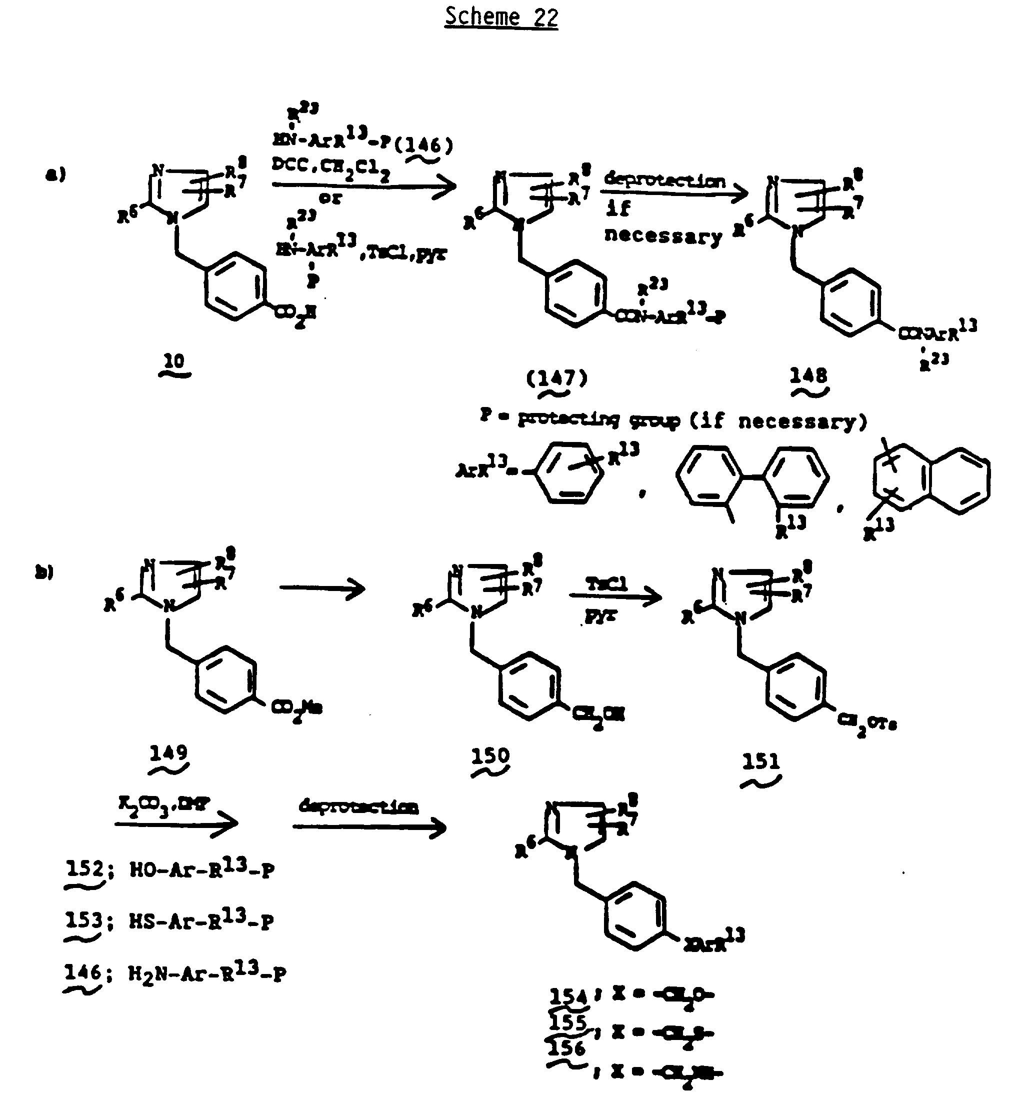

As previously described, acid (10) can be made by alkylating the appropriate imidazole with methyl 4-chloromethylbenzoate in the presence of a base such as potassium carbonate in a polar solvent such as dimethylformamide followed by hydrolysis of the resulting ester. Compound (10) can be converted to (148) by reaction with the requisite amine (146) (R¹³ may need to be protected and subsequently deprotected) and dicyclohexyl carbodiimide (DCC) in methylene chloride [J. R. Beek, et al., J. Am. Chem. Soc, 90, 4706 (1968)] or by reaction with tosyl chloride in pyridine [J. H. Brewster and C. J. Ciotti, Jr., J. Am. Chem. Soc., 77, 6214 (1955)]. Yet another process involves conversion of carboxylic acid (10) to its acid chloride with, for example, thionyl chloride followed by reaction with the amine in aqueous base (Schotten-Baumann conditions) or in an organic solvent in the presence of an acid scavenger such as NaHCO₃, pyridine or triethylamine, or by other procedures known to form an amide bond between an aromatic acid and an amine.

The compounds where X= -CH₂O-, -CH₂S-, and -CH₂NH₂- can be made as shown in pathway b. The ester (149) is reduced with a reducing agent such as lithium aluminum hydride in an inert solvent to form the alcohol (150) which can then be reacted with tosyl chloride in pyridine to form tosylate (151), which is in turn reacted in the presence of base with a corresponding phenol (152) thiophenol (153), or aniline (146; where R³=H) to form compounds (154), (155) or (156). Again this may require that R¹³ be protected with a suitable protecting group, however modifications necessary because of specific functional groups are understood to be incorporated by one skilled in the art of organic synthesis.

Alternatively, the alcohol (150) can be converted to the corresponding halide with SOCl₂, (COCl)₂, etc, and the resulting halide can then be reacted with a phenol, thiophenol or aniline in the presence of base to form the desired compound, where X is -CH₂O-, -CH₂S-, -CH₂NH- respectively.

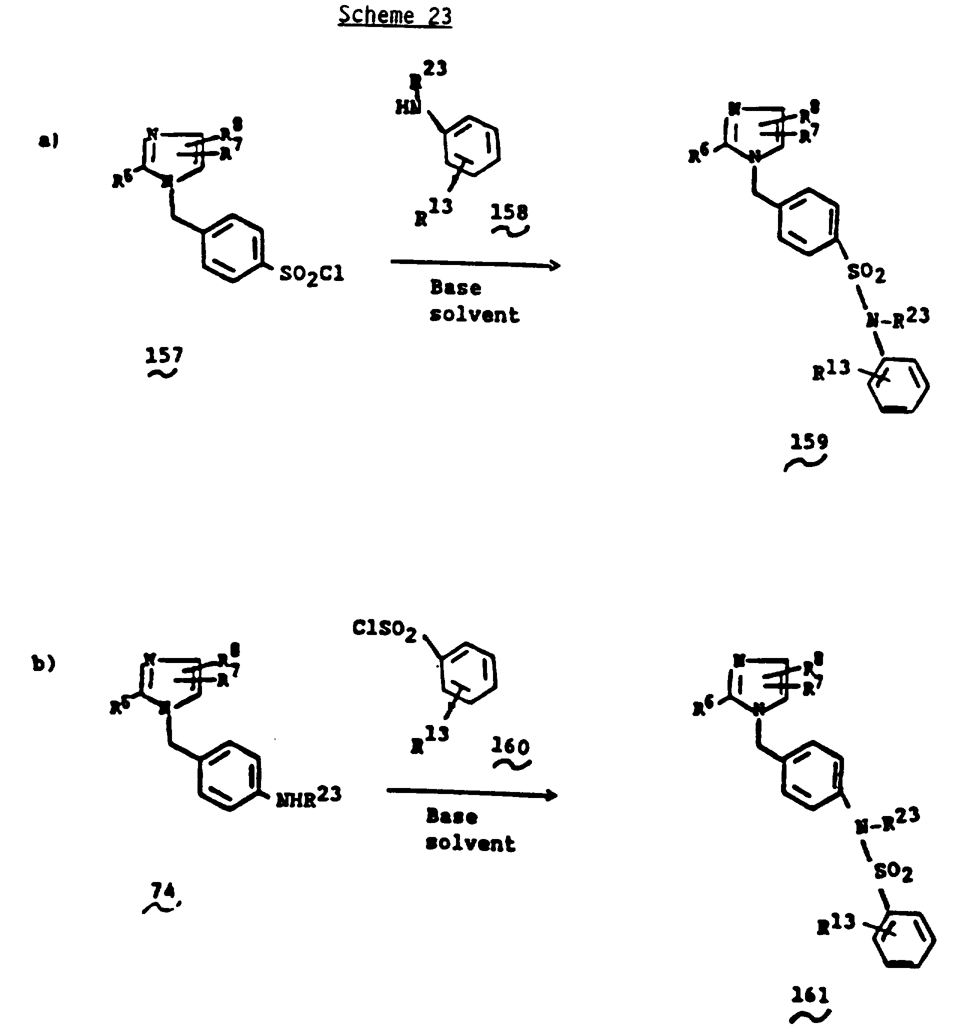

Compounds of Formula (I) where X= -SO₂NR³- and -NR³SO₂- may be prepared as shown in Scheme 23. As shown in equation a, sulfonylchloride derivative (157) can be reacted with aniline derivative (158) in a solvent in the presence of an acid scavenger such as sodium bicarbonate, triethylamine or pyridine or under Schotten-Baumann like conditions to give (159). Sulfonylchloride derivative (157) can be obtained by sulfonation of the corresponding benzyl derivative as described earlier, followed by reaction with PCl₅ or POCl₃. Likewise, aniline (74) may be reacted in the same manner as described above with sulfonylchloride derivative (160) to give (161).

Scheme 24 shows the preparation of furan analogs of the biphenyl compounds (80). Thus, α-ketoester (162), W. Wierenga and H. I. Skulnick, J. Org. Chem., 44, 310 (1979), or the corresponding nitrile (E=CN) can be easily alkylated via standard procedures already mentioned by an alkyl bromide derivative to give (163). The alkene moiety of (163) can be subsequently cleaved by oxidation, for example, with osmium tetroxide, Fieser and Fieser, V.1, p. 812 (Lemleux-Johnson oxidation) to yield dicarbonyl-containing compound (164). Cyclization in mineral acids, acidic ion-exchange resin, POCl₃/pyridine, or trifluoroacetic anhydride with a catalytic amount of trifluoroacetic acid yields furan (165; Z=0). Reaction of (164) with P₄S₁₀, for example, will yield the corresponding thiophene (165; Z=S). Reaction of (164) with an amine In refluxing benzene, with azeotropic removal of water or by using molecular sieves to absorb the water will yield the corresponding pyrrole (165; Z=NR¹¹). Compounds (166) may be prepared from (165) by standard procedures already described.

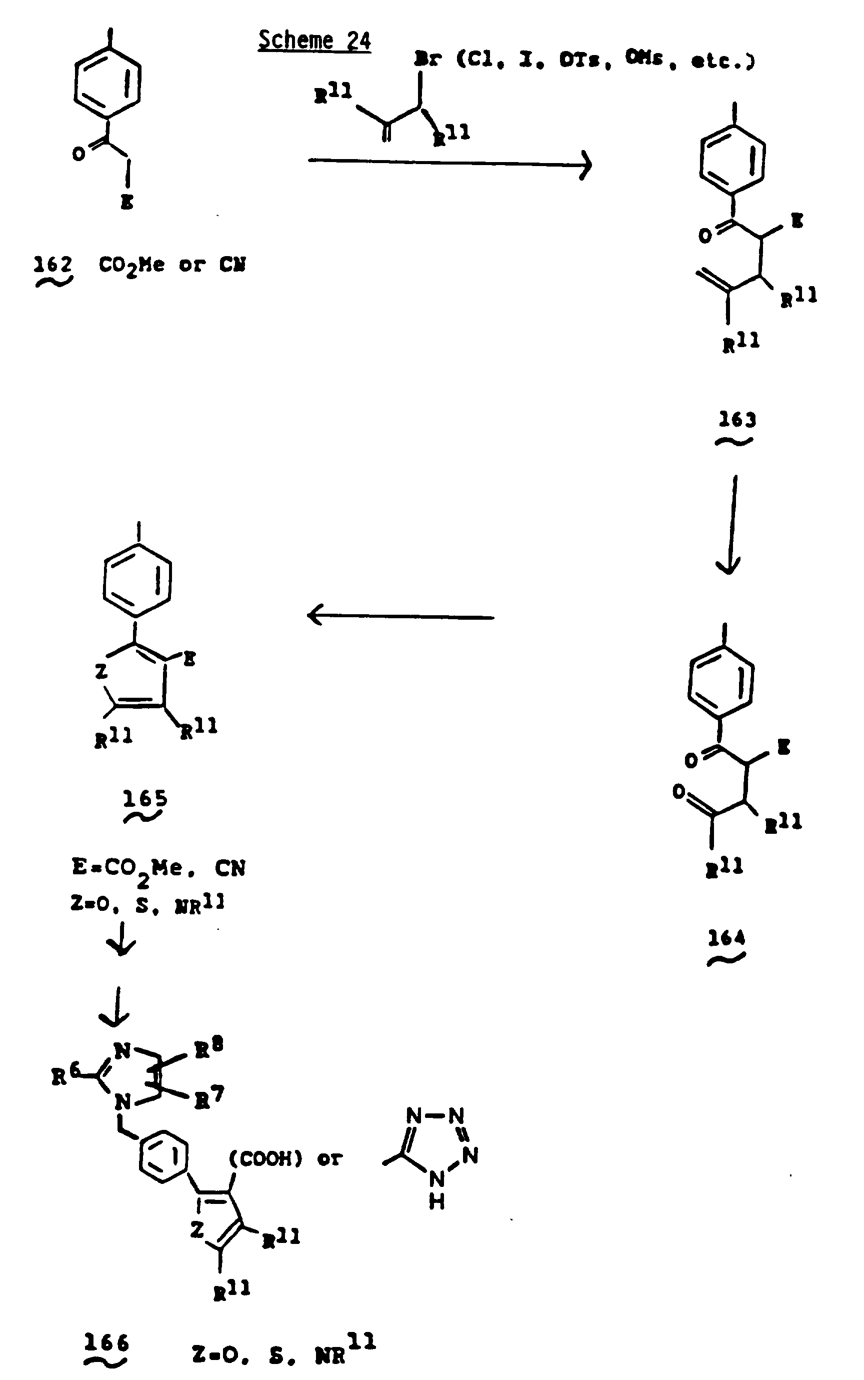

Compounds wherein a methylene group is inserted between the terminal aromatic ring and the acidic functionality may be prepared as shown in Scheme 25, equation a). Thus reduction of ester (167) with, for example, lithium aluminum hydride, gives alcohol (168). Conversion of (168) to the chloride (169) via thionyl chloride followed by reaction with cyanide anion as previously described yields nitrile (170). Compound (170) may be hydrolyzed to carboxylic acid (171) by methods already described or reacted with a hydrazoic acid equivalent to produce tetrazole (172).

Compounds wherein R¹³ is a trifluoromethylsulfonyl hydrazide acidic functional group were prepared by the procedure described in equation b). That is, conversion of ester (167) to the hydrazide (173) by standard hydrazinolysis followed by reaction with triflic anhydride affords hydrazides (174).

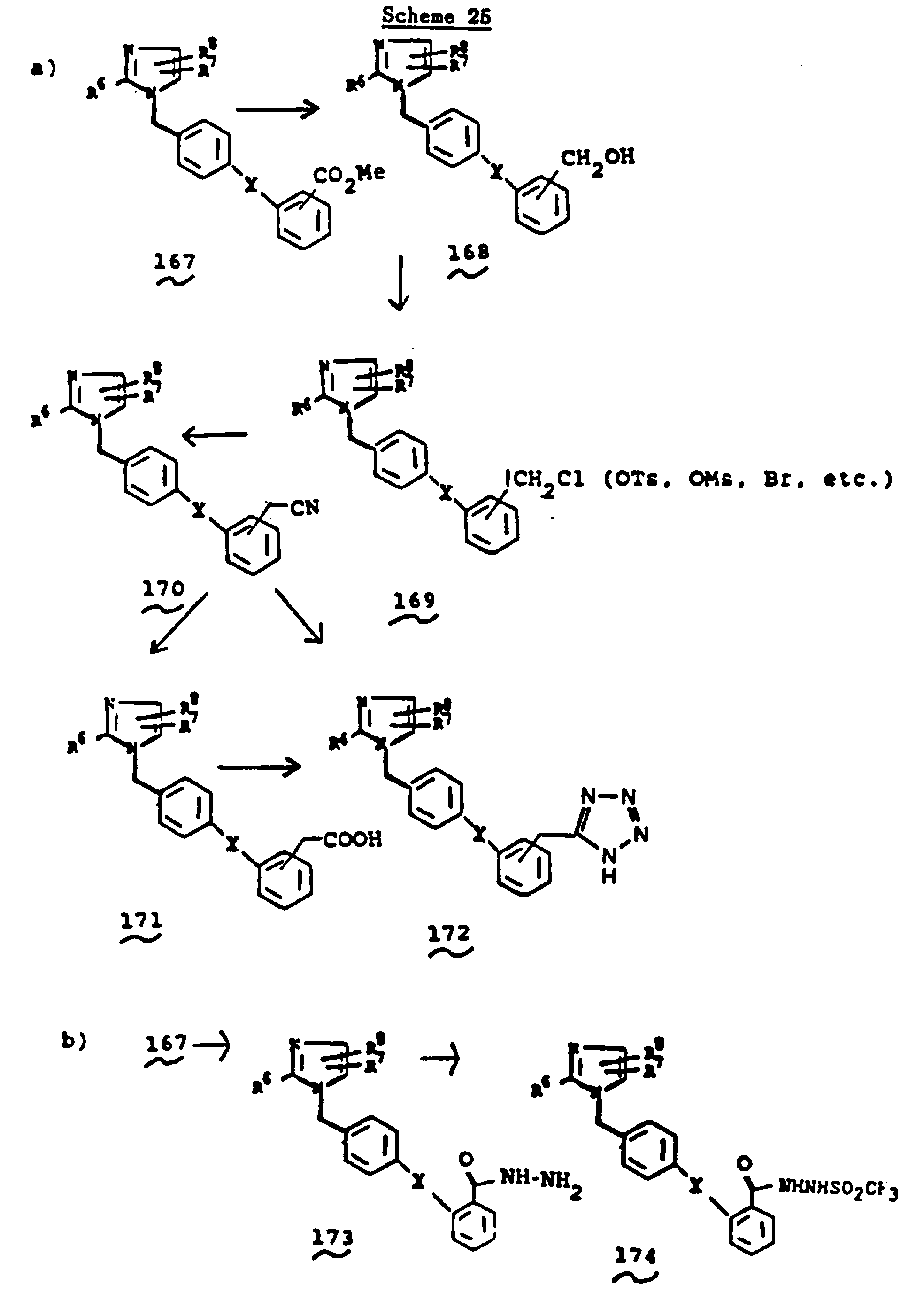

The syntheses of compounds wherein R¹³ is substituted and unsubstituted 1,2,3-triazoles are described in Scheme 26. Thus reduction of ester (175) with a reducing agent such as lithium aluminum hydride or diisobutylaluminum hydride gives alcohol (176). Oxidation with MnO₂ or pyridinium chlorochromate converts (176) into aldehyde (177). Nitroethylene derivative (178) is prepared by condensation of aldehyde (177) with nitromethane in the presence of a catalyst, R. M. Letcher and M. P. Sammes, J. Chem. Ed., 62, 262 (1985). Reaction of (178) with sodium azide produces the 1,2,3-triazole (179), (N. S. Zefirov, et al., J. Chem. Soc. Chem. Comm., 1001 (1971)) which may be transformed via procedures already described into product (180).

Aldehyde (177) can also be converted into substituted 1,2,3-triazoles (183) via the sulfone (181), G. Beck, D. Gunther Chem. Ber. , 106, 2758 (1973), followed by reaction with sodium azide to give the 1,2,3-triazole (182). Subsequent standard manipulations lead to 1,2,3-triazoles (183) where E=CN and CO₂R¹¹. The nitrotriazole (183; E=NO₂) may be synthesized from the unprotected triazole (179; P=H) via nitration, R. Huttel, et al., Chem. Ber. , 88, 1586 (1955), C. L. Habraken and P. Cohen-Fernandes J. Chem. Soc., 37 (1972), or from bromonitroethylene derivative (184), G. Kh. Khisamutdinov, et al., Zh. Org. Khim., 11, 2445 (1975), by reaction with sodium azide.

A variety of protecting groups may be used in the manipulation of the above triazoles, amongst which is the trityl group. This group may be easily attached by reaction of the triazole with triphenylmethyl bromide or chloride in an inert solvent such as methylene chloride in the presence of an acid scavenger such as triethyl amine. The trityl group may be later removed by stirring or refluxing in an acidic medium such as trifluoroacetic acid/water, HCl in methylene chloride, or acetic acid/water. The trityl group may also be hydrogenolyzed using a noble metal catalyst such as palladium and hydrogen.

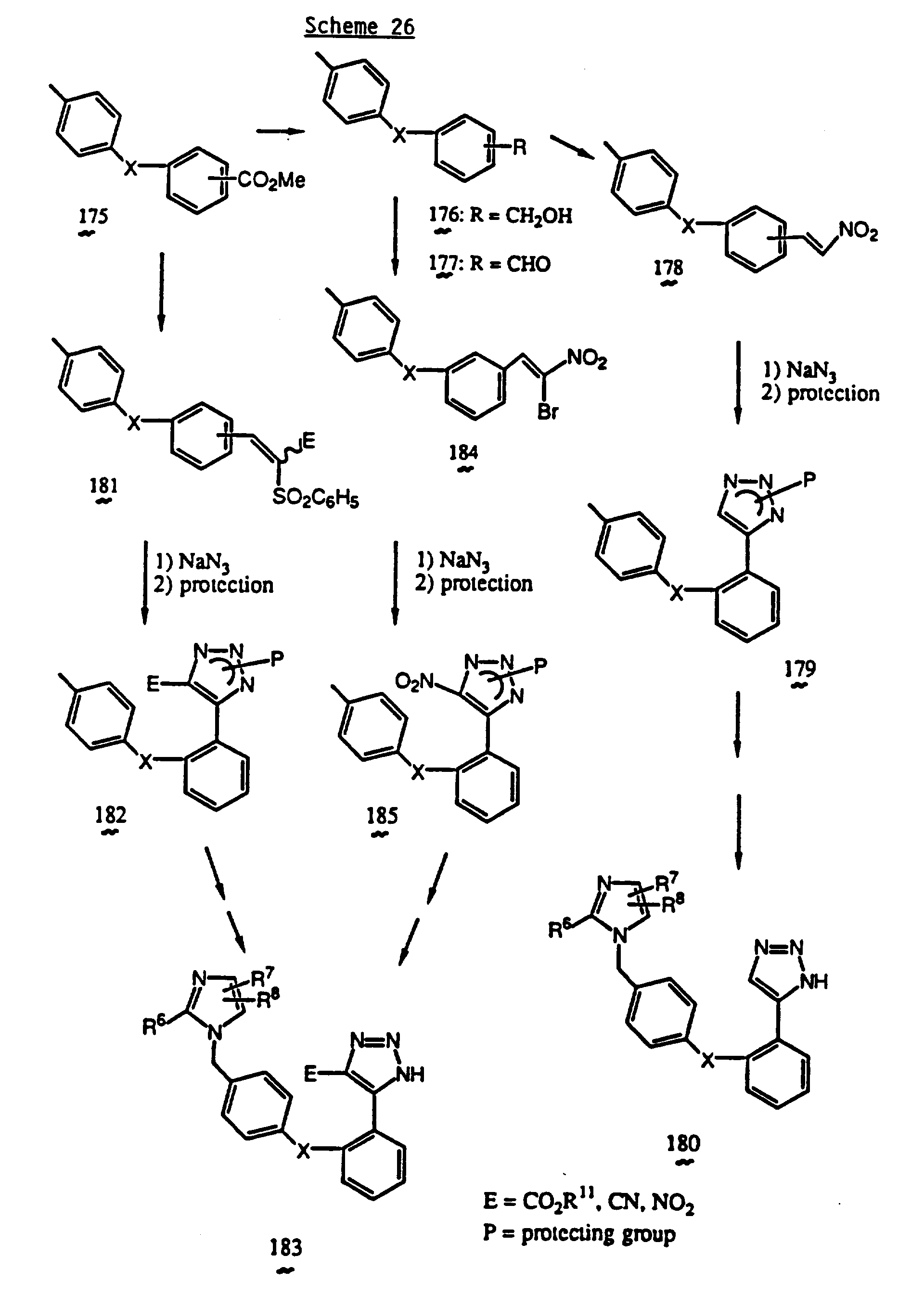

The synthesis of trifluoromethyl-1,2,4-triazoles (190) is depicted in Scheme 27. Acid chloride (186) is converted to amide (187) using standard procedures familiar to one skilled in the art. A preferred protecting group is the 2-propionitrile group (P=CH₂CH₂CN). Thus (187; P=CH₂CH₂CN) can be synthesized from (186) and β-aminopropionitrile under Schotten-Baumann like conditions, using aqueous base in an organic solvent to help solubilize (186) and (187). Amide (187) is converted to amidrazone (188) by reaction with PCl₅ or phosgene to make an iminoyl chloride which then in turn is reacted with excess hydrazine. Amidrazone (188) is cyclized to the trifluoromethyl-1,2,4-triazole (189) with trifluoroacetic anhydride and then converted to 190 via bromination, alkylation and deprotection as previously described.

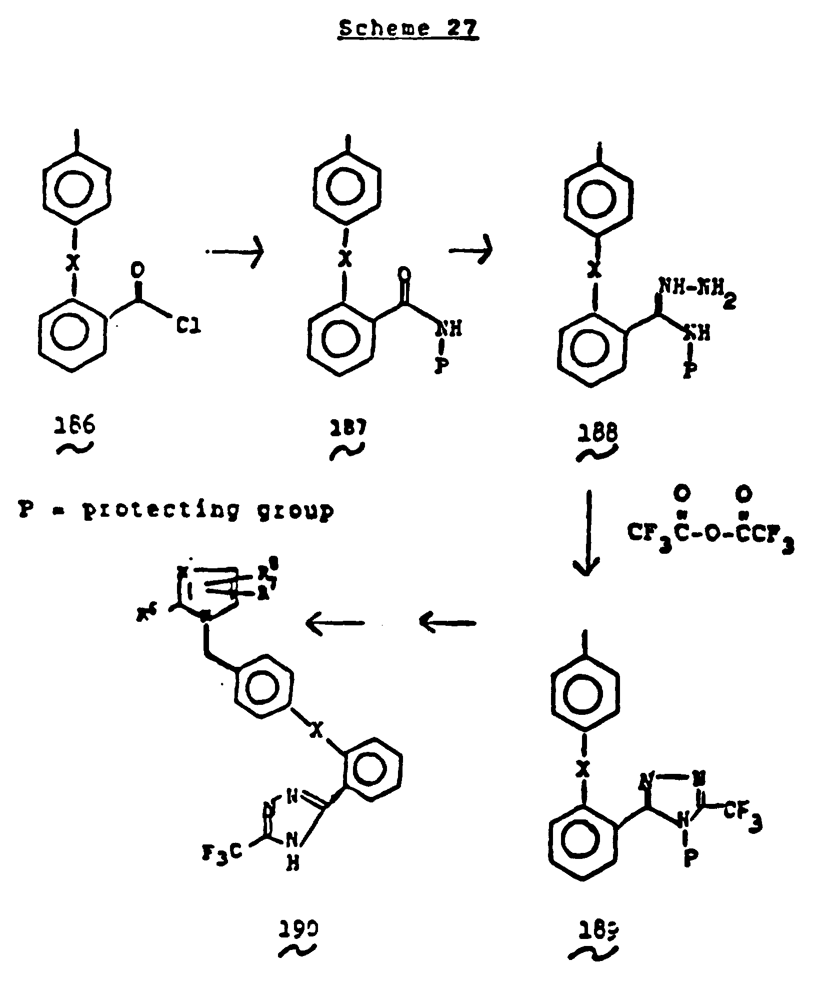

Pertinent R⁶ groups may be variously introduced by many procedures including those described in Scheme 28 which describes imidazole construction.

The R⁶ groups so introduced may stand unchanged or may be further elaborated if appropriately functionalized, according to methods familiar to those skilled in the art such as are illustrated in Scheme 28.

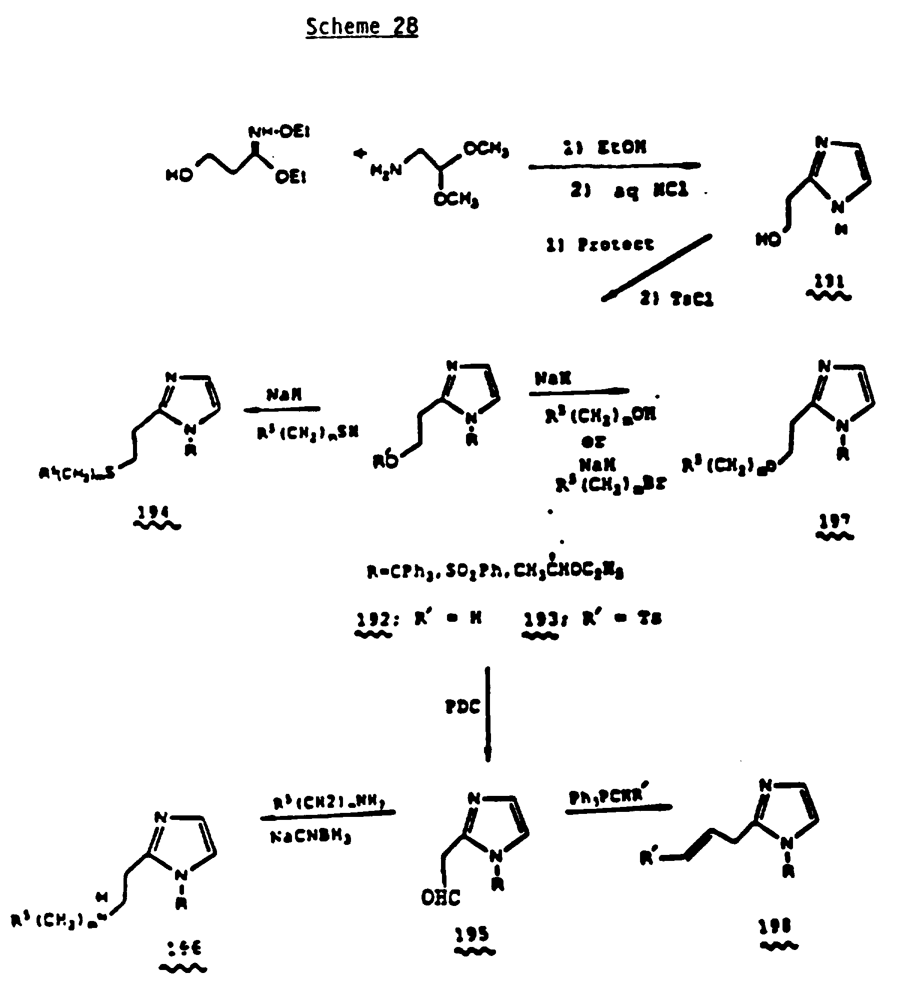

The 2-alkenylimidazoles (201) can be prepared by bromination of the 2-alkylimidazoles (199) followed by elimination of hydrogen bromide. The bromination is preferably accomplished by UV-irradiation for 1-4 hours of imidazole (199) and N-bromosuccinimide, in an inert solvent, such as carbon tetrachloride at 25°C. Treatment of the intermediate bromide (200) with a base, such as DBU, triethylamine, or potassium t-butoxide, affords the trans 2-alkenylimidazoles (201). Cis alkenyl derivatives (203) are prepared from the trans alkenyl compounds by treatment with osmium tetroxide and sodium periodate to afford aldehydes (202) followed by Wittig reaction.

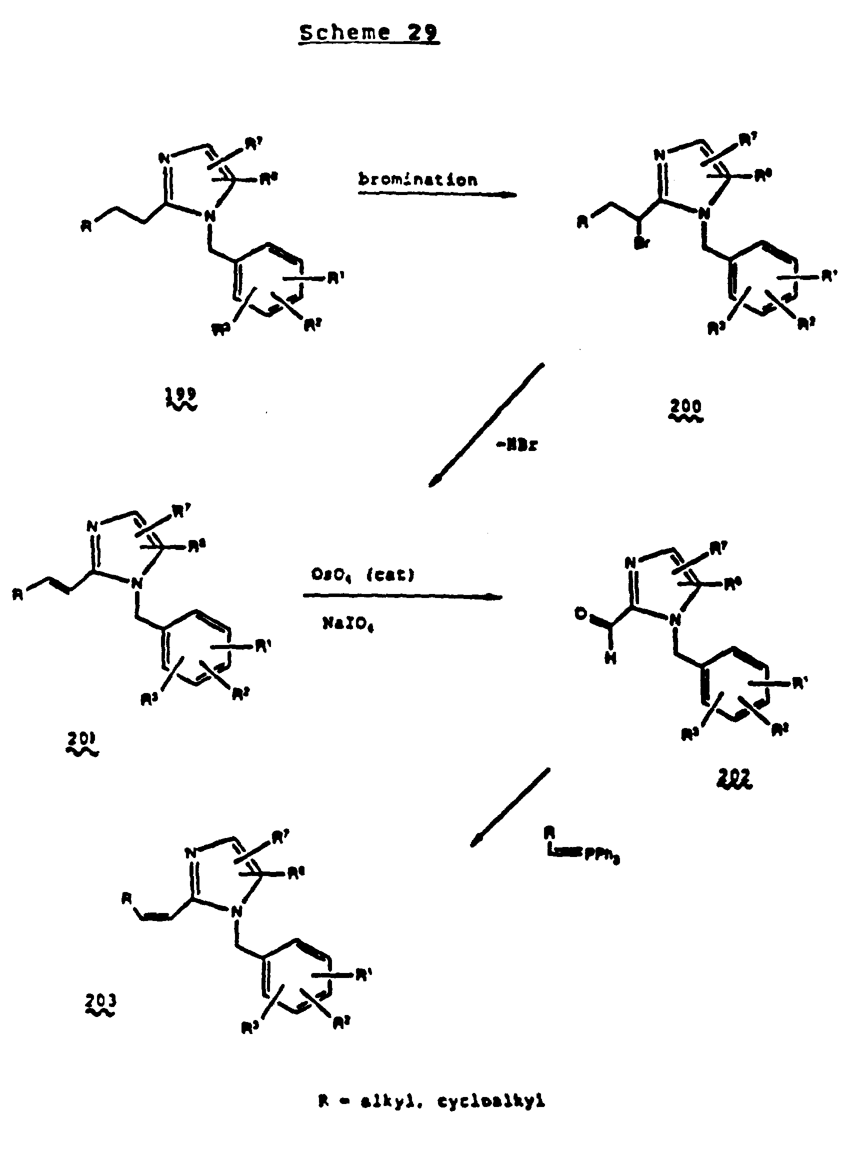

Alternatively, R⁶ groups may be introduced by metallation of a protected imidazole or protected 2-methylimidazole followed by addition of an appropriate electrophile as illustrated in Scheme 30, equations a) and b). The products (alcohols, esters, halides, aldehydes, alkyls) are suitable for further elaboration by methods familiar to those skilled in the art. Metallation of imidazoles is described in K.L. Kirk, J. Org. Chem., 43, 4381 (1978); R.J. Sundberg, J. Het. Chem., 14, 517 (1977); J.V. Hay et al., J. Org. Chem., 38, 4379 (1973); B. Iddon, Heterocycles, 23, 417 (1985).

Condensation of 2-methylimidazole and appropriate electrophiles (equation b) with catalytic acid or base as described in A.R. Katritzky (Ed.), "Comprehensive Heterocyclic Chemistry", Vol. 5, p. 431, Pergamon Press, N.Y., 1984 affords products wherein R₆ is alkenyl which are suitable for further elaboration.

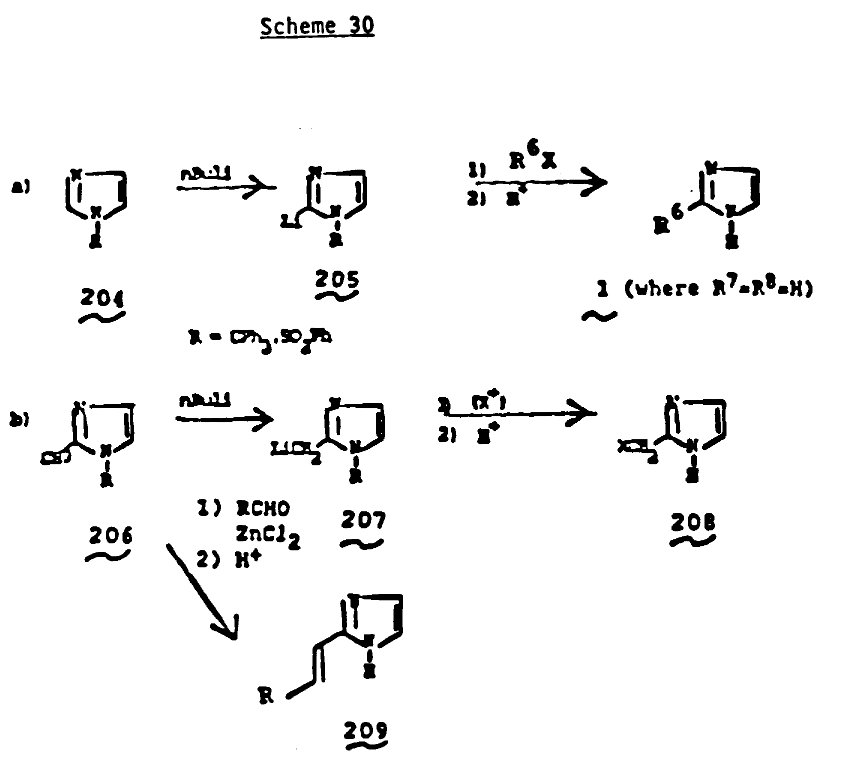

Various 2-substituted imidazoles can be prepared by reaction of a protected 2-trimethylsilylimidazole with a suitable electrophile by the method described by F.H. Pinkerton and S.F. Thames, J.Het. Chem., 9, 67 (1972), which can be further elaborated as desired. Alternatively, R⁶ may also be introduced by nickel catalyzed cross-coupling of Grignard reagents with 2-(methylthio)imidazoles (Scheme 31) as described by E. Wenkert and T.W. Ferreira, J. Chem. Soc., Chem. Commun., 840, (1982); E. Wenkert et al., J. Chem. Soc., Chem. Commun., 637, (1979); and H. Sugimura and H. Takei, Bull. Chem. Soc. Japan, 58, 664 (1985). The 2-(methylthio)imidazoles can be produced by the procedure described in German Patent No. 2,618,370 and the references cited therein.

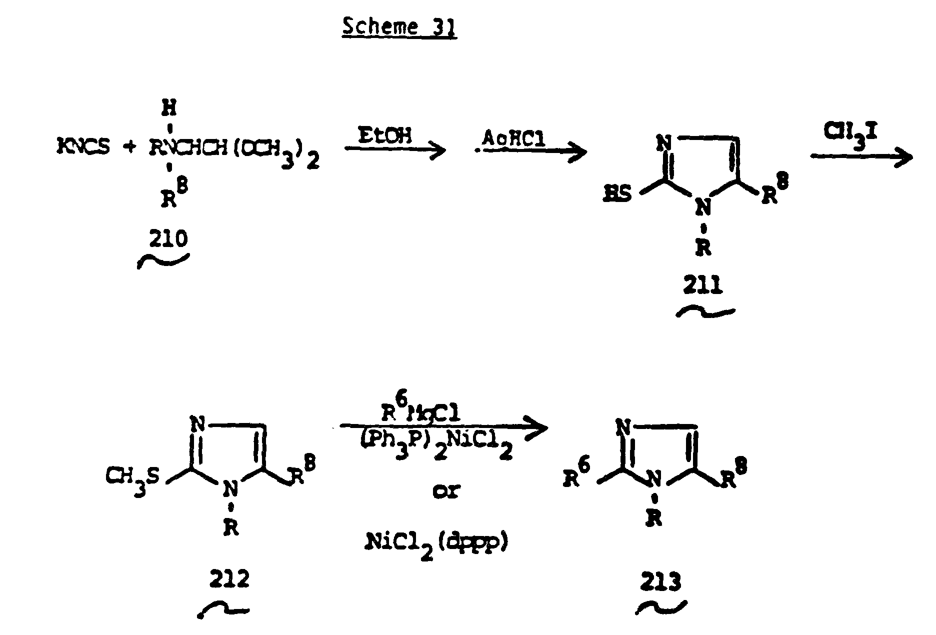

As shown in Schemes 32-36, elaboration of R⁸ can be accomplished by some of the procedures described in Schemes 3 and 28, by chain extension reactions familiar to those skilled in the art, or by degradation reactions such as conversion of an ester to an acid or an alkene to an aldehyde.

Specifically, the hydroxymethyl group can be activated for the displacement reaction by reacting with thionyl chloride, PCl₅ or with carbon tetrachloride/triphenylphosphine to form a corresponding chloro derivative. By a similar reaction bromo and iodo derivatives can be obtained. The hydroxymethyl group can also be activated by forming the corresponding p-toluenesulfonate, methanesulfonate and trifluoromethane sulfonate derivatives.

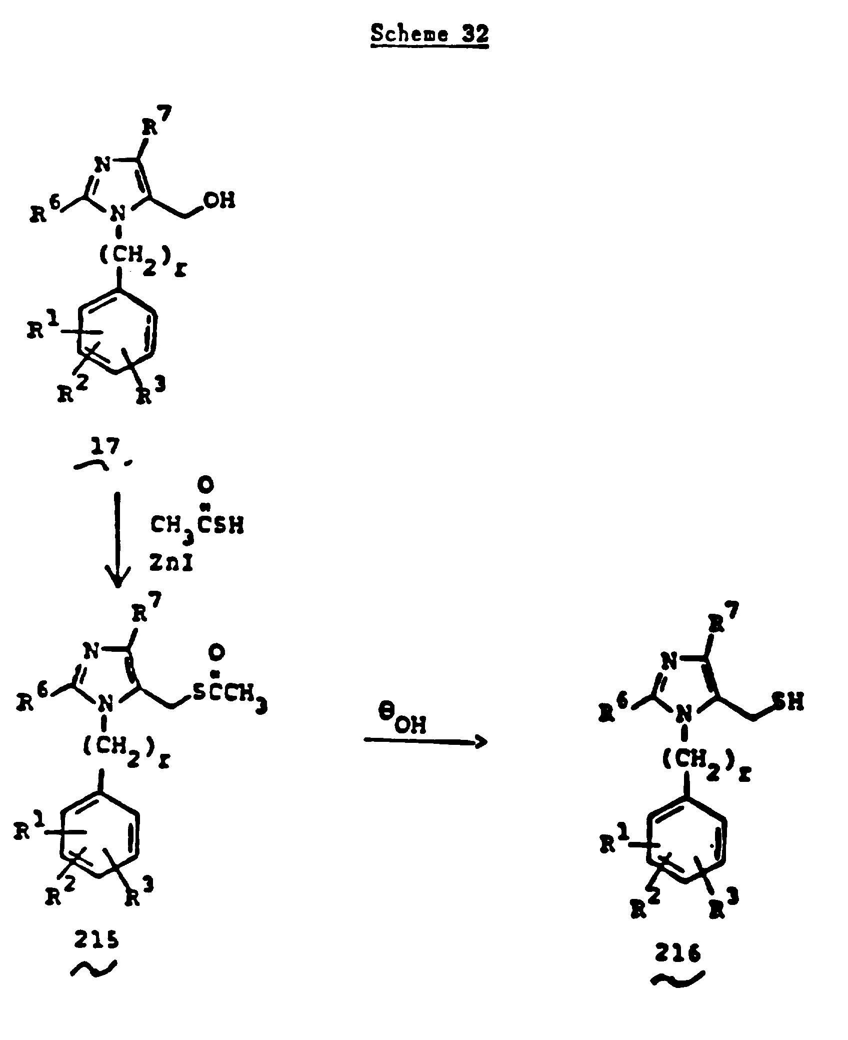

As shown in Scheme 32, the hydroxyl group can be converted to thiolacetic acid derivative (215), J. Y. Gauthier, Tet. Lett., 15 (1986), and to thiol derivative (216) by subsequent hydrolysis.

The hydroxymethyl group on compound (17) can be readily oxidized to an aldehyde group by means of manganese dioxide or ceric ammonium nitrate. The aldehyde group will undergo chain extension reactions such as the Wittig and Wittig-Horner reactions and enter into typical carbon-carbon bond forming reactions with Grignard and lithium reagents as well as with compounds bearing activated methylene groups. Alternatively, the hydroxymethyl group can be oxidized directly to an acid functionality which can in turn be converted to ester and amide derivatives. The esters and amides can be prepared directly from the aldehydes by manganese dioxide oxidation in the presence of sodium cyanide and an alcohol or amine, J. Am. Chem. Sec., 90, 5616 (1968) and J. Chem. Soc. (C), 2355 (1971).

As shown in Scheme 33, the chlorine on compound (25) can be displaced by the anion of dialkyl malonate to give the corresponding malonate derivative (217). The saponification of (217) with NaOH (or KOH) gives the corresponding diacid which can be decarboxylated to give the corresponding propionic acid derivative (218) by heating to 120°C. Alternatively, (218) can be directly obtained by refluxing (217) with a mineral acid such as HCl or sulfuric acid. The free acid (218) can be esterified by heating in a medium of the various alcohols and a catalytic amount of mineral acids such as HCl or sulfuric acid to give the corresponding esters (219). Alternatively the esters can be obtained by reacting the free acid (218) and the corresponding alcohols in the presence of coupling reagents such as DDQ or EEDQ. A similar reaction with various mono-substituted and disubstituted amines produces the corresponding amides (220). A similar reaction with various mercaptans produces the corresponding thioesters.

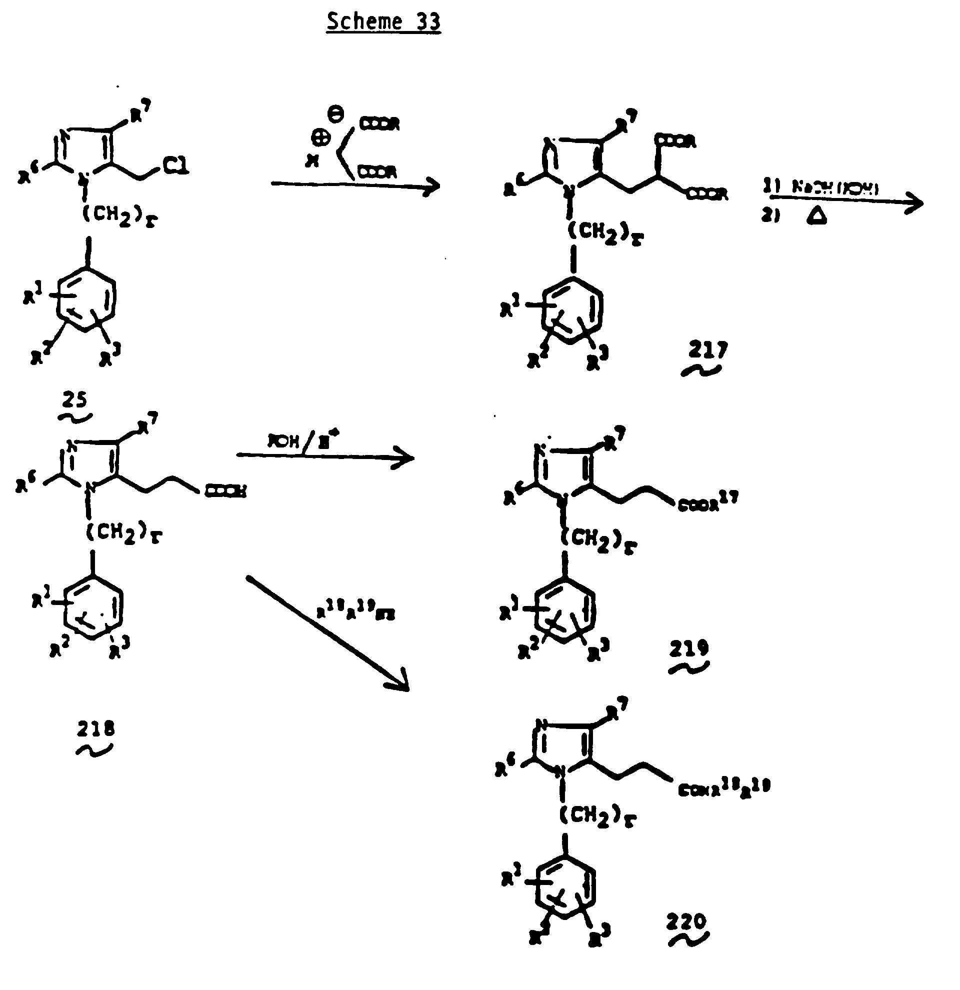

As shown in Scheme 34, the chloro group on (25) can be displaced by the sodium salt or potassium salt of the alkyl, aryl or arylalkyl mercaptans to give the corresponding sulfide derivatives (221). The amine derivative (222) can be obtained by treating (25) with ammonia or with the corresponding mono-substituted amines. Alternatively, the chloro group may be displaced by sodium azide to give an azide intermediate which upon reduction with H₂ over a noble metal catalyst or with a reducing agent such as chromous chloride (W. K. Warburton, J. Chem. Soc., 2651 (1961)) yields (222) where R¹⁰ and R¹¹ are hydrogen. This amine can be subsequently alkylated with alkyl halides, or reductively alkylated with aldehydes and ketones to give alkyl derivatives of (222). The amines (222) are converted to the corresponding carbamates (224), sulfonamides (225), amides (226) or ureas (227) by standard procedures illustrated in Scheme 34 and familiar to one skilled in the art.

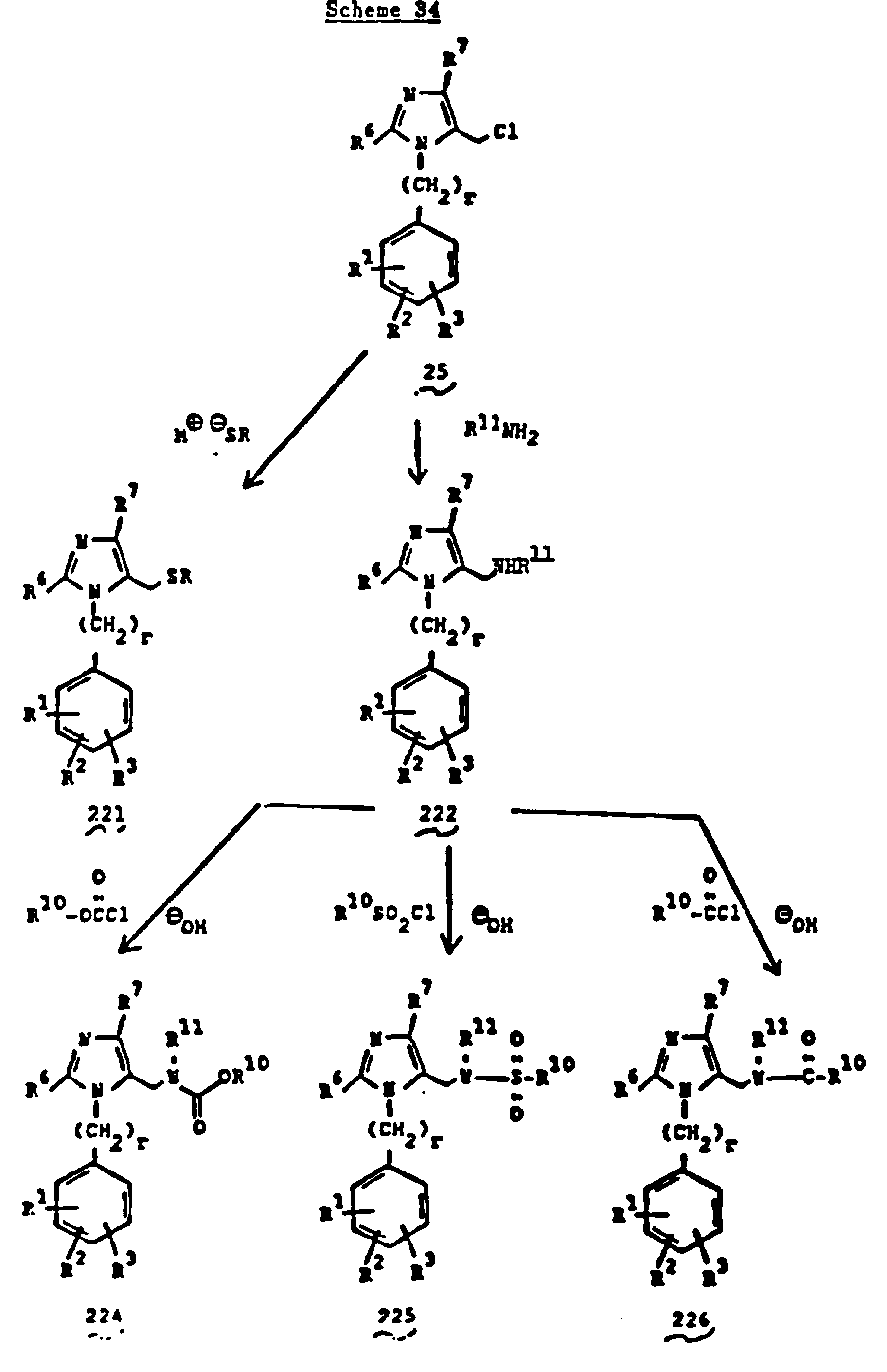

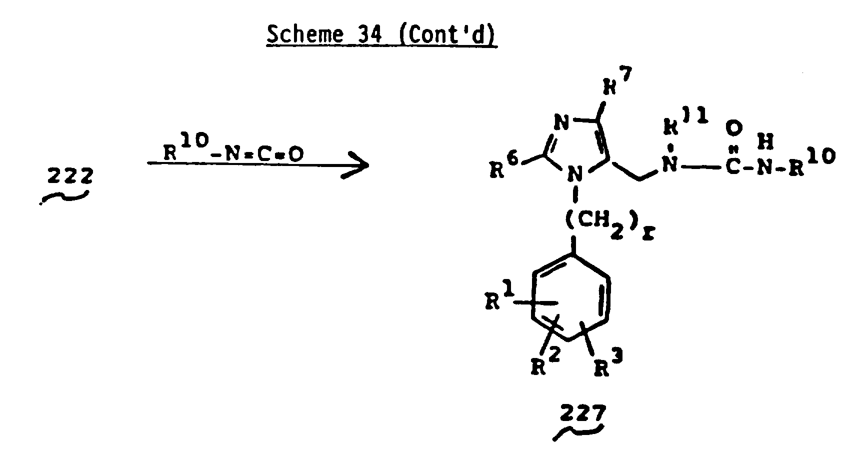

The reaction between the thiopyridyl ester (229) and a suitable Grignard reagent produces the ketones (230).

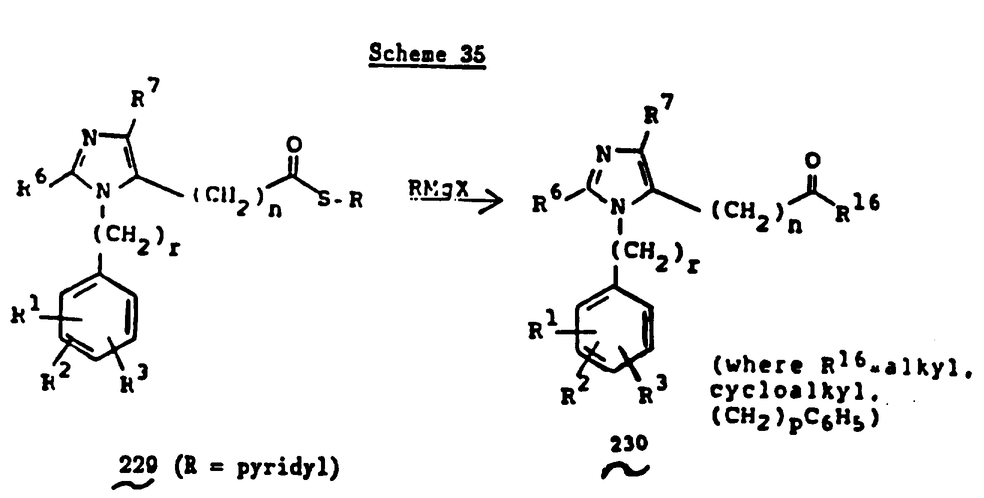

As shown in Scheme 36 when the imidazole 4- and/or 5-position contains an aldehyde (231) then reaction with organometallic reagents such as Grignard or alkyl/aryllithium reagents will yield alcohols (232) which in turn may be transformed into a variety of other functionality familiar to one skilled in the art.

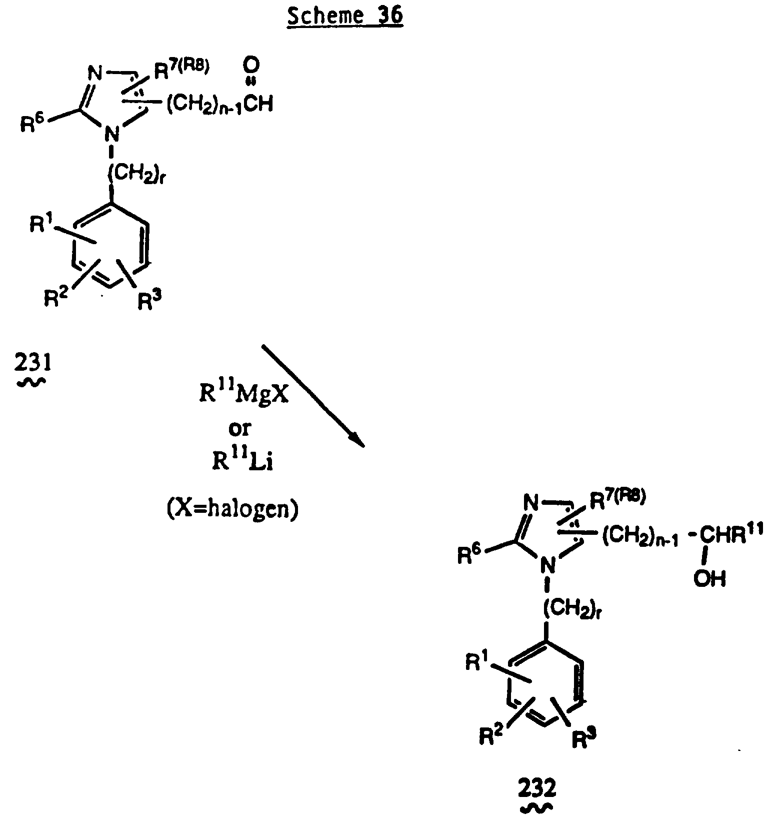

As shown in Scheme 37, ester 234 may be obtained by direct oxidation of aldehyde 233 with NaCN, MnO₂ in methanol (Corey, E. J., et al. J. Am. Chem. Soc. (1968) 90, 5616). Oxidation of 233 with NaCN, MnO₂, and an amine in 2-propanol leads to the corresponding amide 235 (Gilman, N. W. Chem. Comm. (1971) 733).

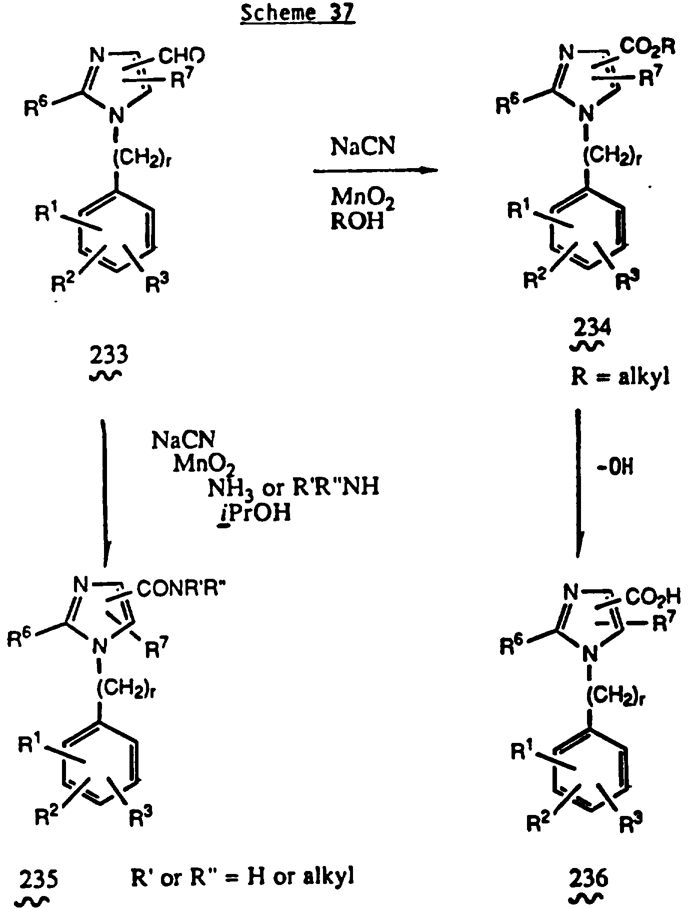

Saponification of ester 234 will lead to carboxylic acid 236.

Aldehyde 233, in turn, may be made from the corresponding alcohol 17 by a variety of methods familiar to one skilled in the art, including pyridium chlorochromate (PCC), Swern and ceric ammonium nitrate (CAN) oxidations.

Likewise, the unalkylated hydroxymethylimidazole derivative 1(R⁸=CH₂OH) may undergo the transformations to the aldehyde, ester, carboxylic acid and carboxamide by the reactions mentioned above for the alkylated case.

Compounds 238 (where Ar = p-biphenylyl, p-phenoxyphenyl, or a heteroaryl group as described in the scope under the definition of R⁷) can be prepared by the coupling of an arylmetal derivative (ArM, where M=ZnBr, Me₃Sn, B(OH)₂, etc.) with a haloimidazole 237 in the presence of a transition metal catalyst such as palladium, nickel, platinum, zirconium, etc. (Scheme 38a). Alternatively an imidazole metal derivative 239 can be coupled to an arylhalide to prepare 238 (Scheme 38b).

The arylmethyl derivatives 240 can be prepared employing the transition metal catalysed coupling of 237 and an arylmethylmetal (ArCH₂M', where M'=ZnBr, etc.), as shown in Scheme 38c.

Compounds 241 may be prepared, as described in Scheme 38d, by the coupling of an alkenyl- or alkylnylmetal derivative (AM) or the corresponding alkene or alkyne (AH) with 237.

Likewise, the unalkylated imidazoles (1, where R⁷=Br or I) may undergo the coupling reactions described in Scheme 38a-d [For references to transition metal catalysed coupling reactions, see: Richard C. Heck, Palladium Reagents in Organic Synthesis, Academic Press, New York, Chapters 6, 7, and 8; and references cited therein.]

The compounds of formula I where R⁷ is an alkynyl group, a substituted alkynyl group, or a substituted alkenyl group and the carbon-carbon double or triple bond is not adjacent to the imidazole ring (e.g., R⁷=(CH₂)₄CH=CH(CH₂)ᵥAr, where v≠0) can be prepared by a variety of chain elongation methods and chain coupling reactions known to one skilled in the art including those described in Schemes 3, 28, 29, 33, 35, 36, and 38.

The compounds of formula I where R⁷ is a substituted alkyl group (R⁷=(CH₂)_{w}Ar, where w=2-10) can be prepared by reduction of the corresponding alkenes (241) by catalytic hydrogenation.

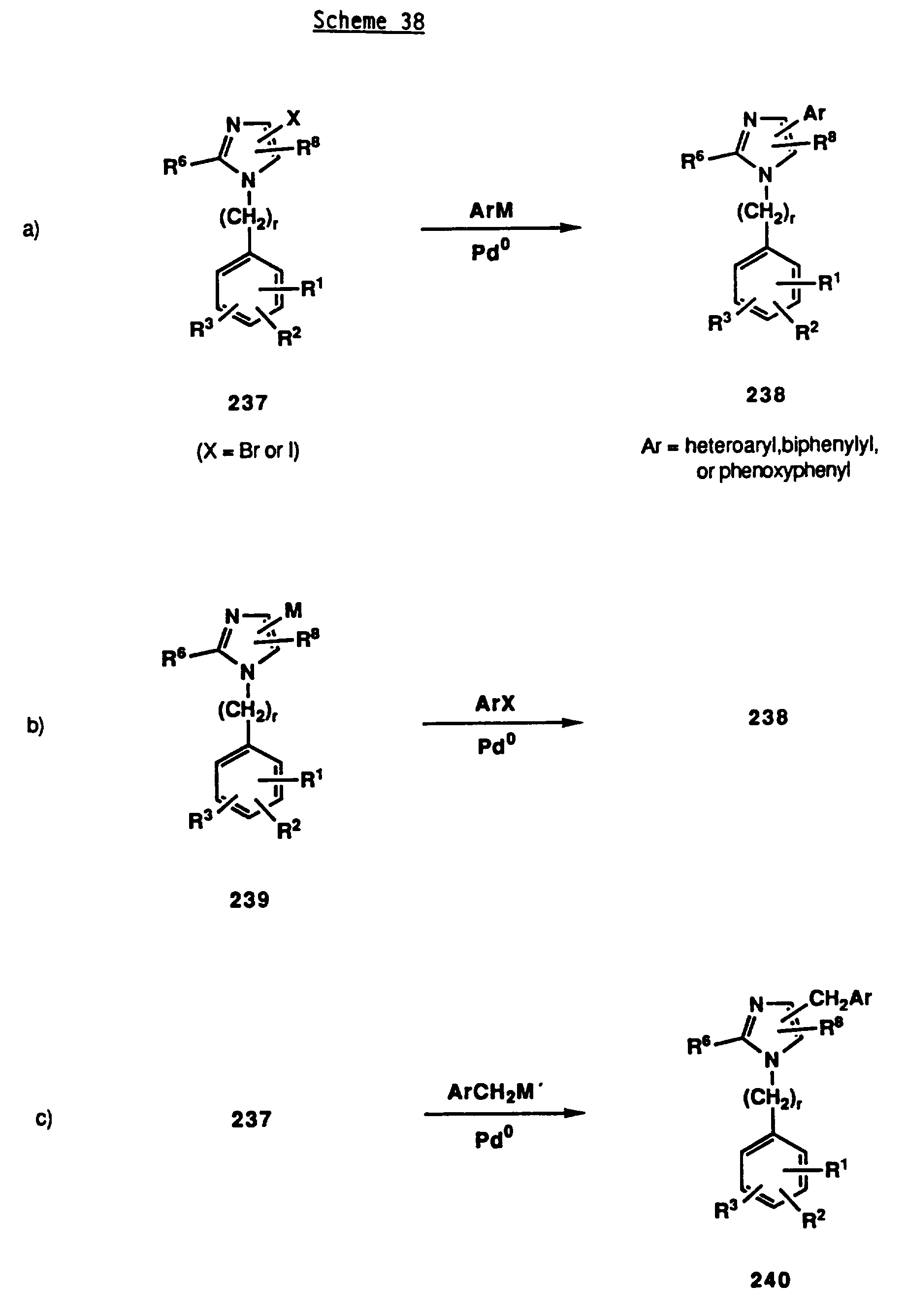

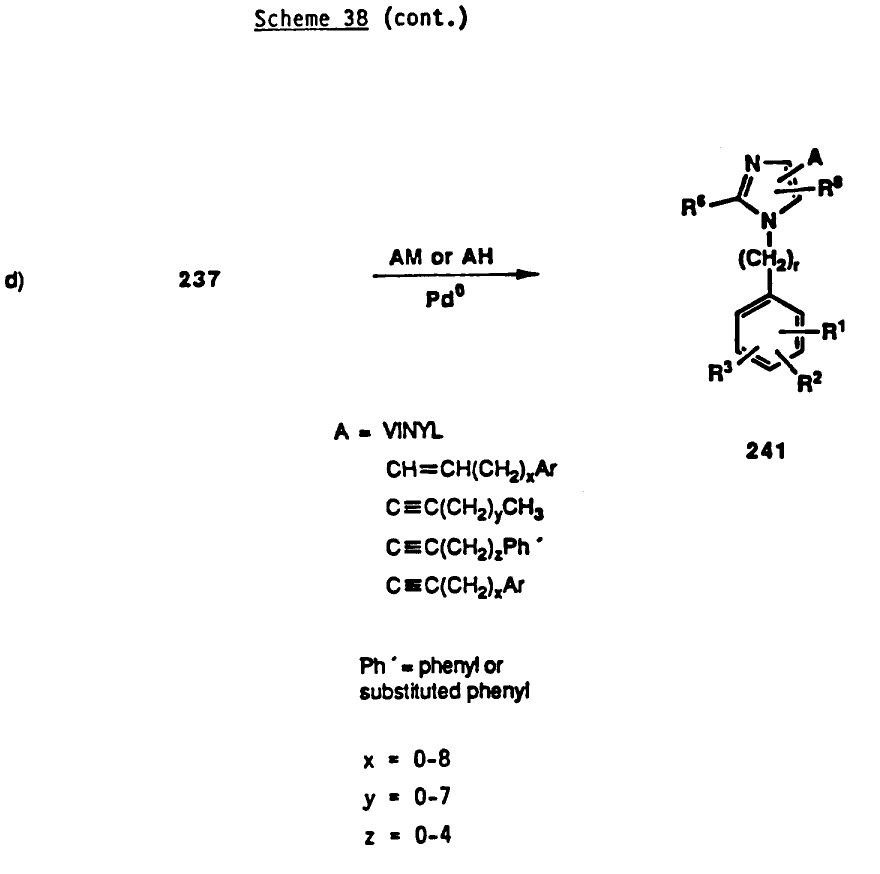

Compounds of formula I where R⁷ = vinyl or arylalkenyl and R⁸ = CH₂OH, aldehyde, or COOH can be prepared as shown in Scheme 39.

2-Alkylimidazole-4,5-dicarboxylic acids (242), prepared by the method of R.G. Fargher and F.L. Pyman (J. Chem. Soc., (1919) 115, 217), can be converted into their corresponding diesters (243) by simply refluxing in an alcohol solvent in the presence of an acid such as HCl, or by many other methods familiar to one skilled in the art.

Diester (243) can then be converted into its metallic salt by reaction with sodium methoxide, sodium ethoxide, sodium hydride, potassium hydride or any other base in an appropriate solvent such as DMF. The resultant salt is then alkylated with the appropriately substituted benzyl derivative (2) to yield benzylimidazole (244). The above alkylation sequence may be also performed by heating or refluxing the benzyl halide (tosylate or niesylate) (2) with imidazole (243) in a solvent such as DMF in the presence of an acid scavenger such as potassium or sodium carbonate.

Diester (244) can be reduced with lithium aluminum hydride in an inert solvent such as THF to the corresponding dialcohol (245). Selective oxidation of dialcohol (245) with manganese dioxide in an inert solvent such as THF yields primarily aldehyde (247) with a minor product dialdehyde (246). Separation of (247) from (246) either by crystallization or chromatographically, followed by Wittig reaction of (247) with methylenetriphenylphosphorane or the appropriately substituted arylalkylidenetriphenylphosphorane in an inert solvent such as THF yields the 4-alkenyl-5-hydroxymethylimidazole (248). Further oxidation of (248) with the Dess-Martin periodinane (J. Org. Chem., (1983) 48, 4155), with manganese dioxide, with pyridinium chlorochromate, with barium manganate or with other oxidants familiar to one skilled in the art, in an inert solvent such as THF or methylene chloride followed by deprotection of either R¹, R, or R³ if necessary yields the 4-alkenylimidazole-5-carboxaldehyde (249).

Oxidation of (249) with, for example, manganese dioxide/cyanide ion (Corey, E.J., et al. J. Am. Chem. Soc., (1968) 90, 5616) or with potassium permanganate (Sam, D.J. et al. J. Am. Chem. Soc., (1972) 94, 4024) yields 4-alkenylimidazole-5-carboxylic acid (250). 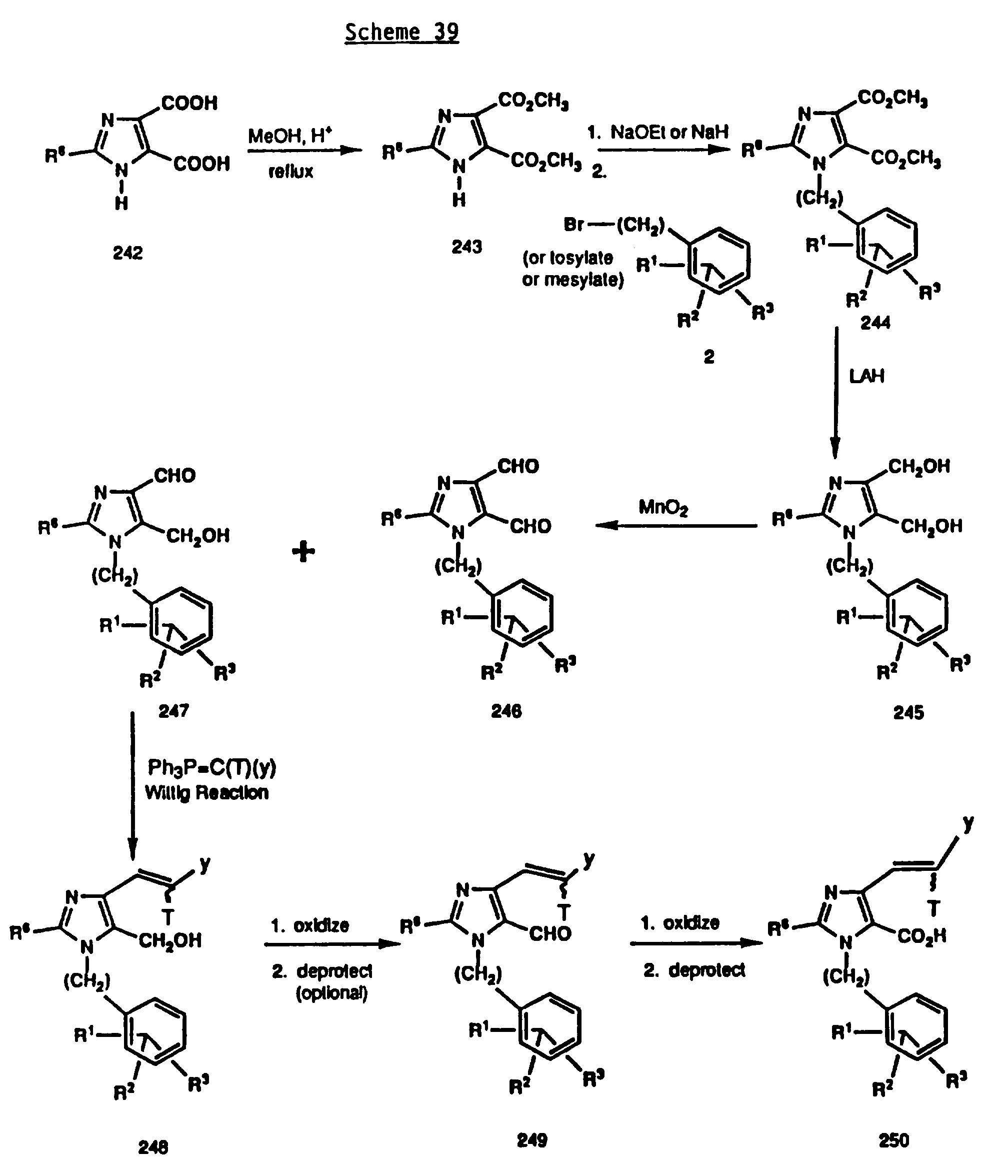 where T=H, y=H, (CH₂)ₓ-Aryl or T and y taken together form a cyclic ring of 3-8 carbons and the regiochemistry about the double bond in 248, 249 and 250 can be Z or E.

Imidazoles represented by structure (251) where X is Cl, Br, or I and E is an electron withdrawing group such as an ester, ketone, nitro, alkylsulfonyl, arylsulfonyl, etc., can undergo the nucleophilic aromatic substitution reaction (H. Schubert, H. Simon, A. Jumar, Z. Chem. (1968) 62-63) where the leaving group X is substituted by a nucleophile such as sulfur, carbon, or nitrogen to yield adducts (252) (Scheme 40). The reaction can be done in hydroxylic solvent such as methanol or non-hydroxylic solvent such as DMSO at room temperature to the reflux temperature of the solvent. The nucleophile sometimes must be converted into its anion to make it more nucleophilic. For example, thiophenol can be refluxed in methanol in the presence of sodium methoxide and the haloimidazole (251). Other nucleophiles include other alkyl and arylthiols, heteroarylthiols, thiolacetic acid, alkyl and arylsulfonamides, heteroarylsulfonamides, diacylamines, alkyl and arylamines, heteroarylamines, etc., familiar to one skilled in the art.

If a sulfur nucleophile is used, the resultant sulfides can be oxidized to the corresponding sulfoxides and sulfones by methods familiar to one skilled in the art.

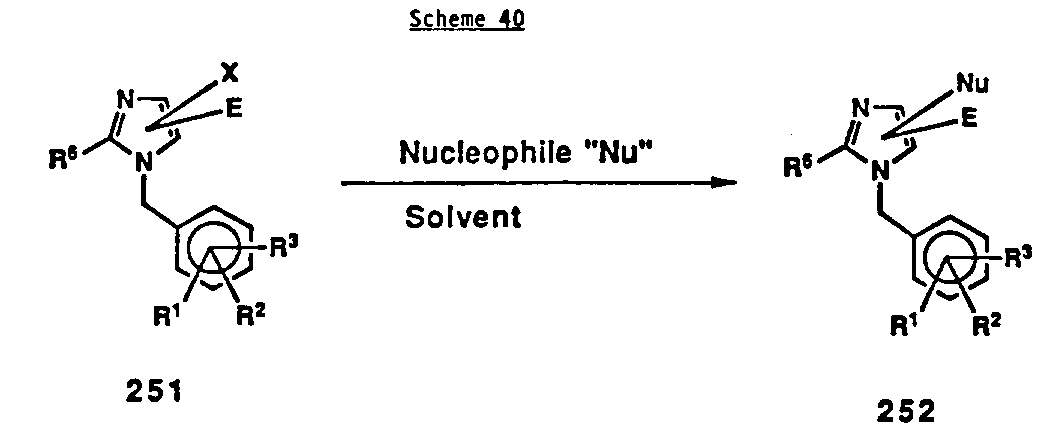

The compounds of this invention and their preparation can be understood further by the following examples, which do not constitute a limitation of the invention. In these examples, unless otherwise indicated, all temperatures are in degrees centigrade and parts and percentages are by weight.

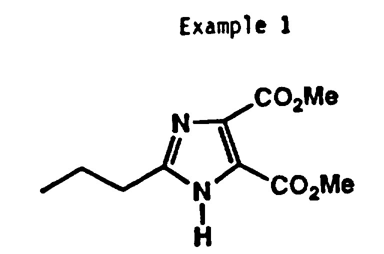

### PART A: Preparation of 2-n-Propyl-4,5-dicarbomethoxyimidazole.

2-n-Propylimidazole-4,5-dicarboxylic acid [prepared by the method of R.G. Fargher and F.L. Pyman (J. Chem. Soc., (1919) 115, 217), mp 257 (dec.) °C] (17.14 g, 86.6 mmol, 1 eq), methanol (400 mL) and acetyl chloride (38.1 mL, 534 mmol, 6 eq) were cautiously mixed (acetyl chloride addition to methanol is very exothermic) and refluxed overnight. The solvent was removed in vacuo and water (100 mL) and 10 N NaOH were added until pH=7. The aqueous mixture was extracted with ethyl acetate (3X), the organic layers combined, dried (MgSO₄) and the solvent removed in vacuo to yield 12.00 g of a white solid. Recrystallization from hexane/ethyl acetate yielded 11.41 g of a white solid (58%); mp: 162.0-164.5°C. NMR (CDCl₃) *δ* 3.95 (s,6H); 2.78 (t,2H); 1.83 (t of t, 2H,J=7,7Hz); 0.97 (t,3H,J=7Hz); IR (neat) 1735 cm⁻¹. Anal. calcd. for C₁₀H₁₄N₂O₄·(H₂O)_{0.25}: C, 52.06; H, 6.28; N, 12.14. Found: C, 52.06; H, 6.17; N, 12.49.

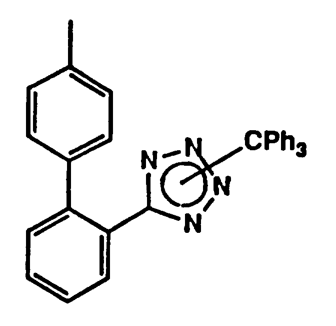

### Part B: Preparation of 4-Methyl-2'-(N-triphenylmethyl-(1H-tetrazol-5-yl))biphenyl

4'-Methylbiphenyl-2-nitrile (preparation described in European patent application 0253310, published on 20.01.88) (10.00 g, 51.7 mmol, 1 eq), trin-butyltin chloride (14.0 mL, 51.7 mmol, 1 eq), sodium azide (3.4 g, 51.7 mmol, 1 eq), and xylene (50 mL) were mixed and refluxed for 64 h after which the reaction mixture was cooled to room temperature. 10.0 N NaOH (6.10 mL, .061 mmol, 1.2 eq) and trityl chloride (14.99 g, 53.8 mmol, 1.04 eq) were then added and the mixture stirred for 24 h after which water (39 mL) and heptane (100 mL) were added. The resultant slurry was stirred at 0°C for 1.5 h. The resultant solids thus obtained were filtered, washed with water (2X 55 mL) washed once with 3:2 heptane/toluene (55 mL) and dried overnight under high vacuum to yield 19.97g of a light yellow powder: mp 148.0-155.0°C (dec.). These solids were slurried in ethyl acetate (75 mL) and filtered to yield 15.0g of a light yellow powder: mp 164.0-165.5°C (dec.). NMR (CDCl₃) δ 7.91 (d,1H,J=9Hz); 7.53-7.18 (m,13H); 7.02-6.84 (m,9H); 2.25 (s,3H).

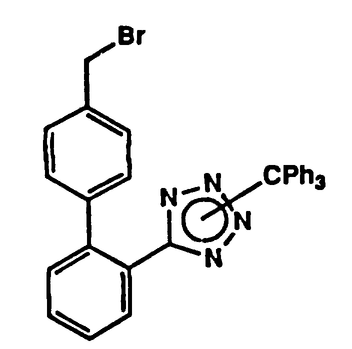

### PART C: Preparation of 4-Bromomethyl-2'-(N-triphenylmethyl-(1H-tetrazol-5-yl))biphenyl, a representative procedure.

4-Metyl-2'-(N-triphenylmethyl-(1H-tetrazol-5-yl))biphenyl (52.07 g, 109 mmol, 1 eq), N-bromosuccinimide (19.4 g, 109 mmol, 1 eq), benzoyl peroxide (1.0 g) and carbon tetrachloride (300 mL) were mixed and refluxed for 2.5 h. The reaction was cooled to room temperature and the succinimide filtered. The filtrate was concentrated and the residue triturated with ether to yield a first crop of 36.0 g: mp 129.5-133.0°C (dec.). NMR (CDCl₃) δ 4.37 (CH₂Br). This material was suitable for further transformation.

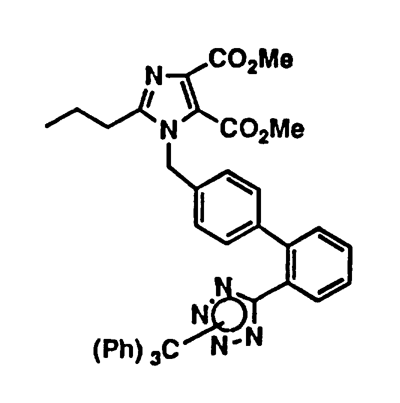

### PART D: Preparation of 4,5-dicarbomethoxy-2-n-propyl-1-[(2'-(N-triphenylmethyl-(1H-tetrazol-5-yl))biphenyl-4-yl)methyl]imidazole.

Sodium hydride (1.06 g, 44.2 mmol, 1 eq) was added to a solution of 4,5-dicarbomethoxy-2-n-propylimidazole (10.00 g, 44.2 mmol, 1 eq) in DMF at room temperature. Foaming and gas evolution occurred. The temperature was increased to 60°C for 15 minutes to dissolve all of the sodium hydride. Gas evolution ceased and the mixture was cooled to room temperature. To this mixture was added a DMF solution of 4-bromomethyl-2'-(N-triphenylmethyl-(1H-tetrazol-5-yl))biphenyl (24.64 g, 44.2 mmol, 1 eq). After 24 h, the solvent was removed in vacuo, and the residue was flash chromatographied in 75:25 hexane/ethyl acetate to 100% ethyl acetate over silica gel to yield 15.78 g (51%) of a white glass which was suitable for further transformation. Recrystallization from ethanol yielded an analytical sample (white crystals); mp: 124.0-125.5°C. NMR (CDCl₃) δ 7.91 (d of d,1H,J=3,9 Hz); 7.59-7.20 (m,12H); 7.09 (d,2H,J=9Hz); 6.94 (m, 6H); 6.76 (d,2H,J=9Hz); 5.30 (s,2H); 3.89 (s,3H); 2.50 (t,2H,J=7Hz); 1.67 (t of t, 2H,J=7,7Hz); 0.85 (t,3H,J=7Hz). IR (neat) 1718 cm⁻¹. Anal. calcd. for C₄₃H₃₈N₆O₄: C, 73.49; H, 5.45; N, 11.96. Found: C, 73.23; H, 5.48; N, 12.22.

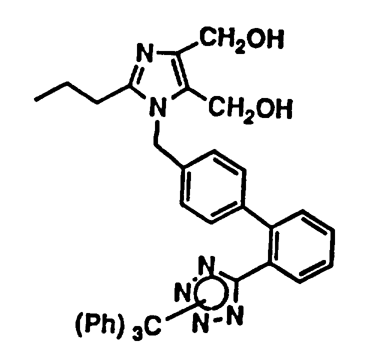

### PART E: Preparation of 4,5-dihydroxymethyl-2-n-propyl-1-[(2'-(N-triphenylmethyl-(1H-tetrazol-5-yl))biphenyl-4-yl)methyl]imidazole.

4,5-Dicarbomethoxy-2-n-propyl-1-[(2'-(N-triphenylmethyl-(1H-tetrazol-5-yl))biphenyl-4-yl)methyl]imidazole (9.88 g, 14.1 mmol, 1 eq) was dissolved in a minimum of THF and to this solution, lithium aluminium hydride (1.0 M in THF) (15.48 mL, 15.48 mmol, 1.1 eq) was slowly added dropwise. The mixture was allowed to stir at room temperature overnight after which it was quenched by the Steinhardt procedure (Fieser & Fieser V.1, p.584) as follows: to the reaction mixture water (0.66 mL) was first carefully added followed by 15% NaOH (0.66 mL) followed by water (1.97 mL). After stirring for 72 h, a very fine suspension of particulate had formed which was slowly filtered through Celite^{TM}. The filtrate was dried (MgSO₄) and the solvent removed in vacuo to yield 8.83 g of a yellow glass which could not be recrystallized. This intermediate was suitable for further transformation. NMR (DMSO-d₆) δ 7.82 (d,1H,J=9Hz); 7.68-7.28 (m,12H); 7.05 (d,2H,J=9Hz); 6.87 (d,6H,J=9Hz); 5.16 (s,2H); 4.94 (t,1H,3=7Hz); 4.66 (t,1H,J=7Hz); 4.37 (d,2H,J=7Hz); 4.32 (d,2H,J=7Hz); 2.34 (t,2H,J=7Hz); 1.52 (t of q,2H,J=7,7Hz); 0.77 (t,3H,J=7Hz). IR (neat) 3300 br; 3061; 1027; 1006; 909; 732; 699 cm⁻¹. Anal. calcd. for C₄₁H₃₈N₆O₂·H₂O: C, 74.07; H, 6.06; N, 12.64. Found: C, 74.06; H, 5.95; N, 11.86.

### PART F: Preparation of 5-hydroxymethyl-2-n-propyl-1-[(2'-(N-triphenylmethyl-(1H-tetrazol-5-yl))biphenyl-4-yl)methyl]imidazole-4-carboxaldehyde and 2-n-propyl-1-[(2'-(N-triphenylmethyl-(1H-tetrazol-5-yl))biphenyl-4-yl)methyl]imidazole-4,5-dicarboxaldehyde.

4,5-Dihydroxymethyl-2-n-propyl-1-[(2'-(N-triphenylmethyl(1H-tetrazol-5-yl))biphenyl-4-yl)methyl]imidazole (8.56 g, 13.2 mmol, 1 eq) was dissolved in a minimum of THF and added to a slurry of manganese dioxide (11.14 g, 128.1 mmol, 9.7 eq) in THF (100 mL) at room temperature. After 24 h, the contents were filtered through Celite^{TM}, the cake washed with THF, and the solvent of the filtrate removed in vacuo. The residue was flash chromatographed in 1:1 hexane/ethyl acetate to 100% ethyl acetate over silica gel to yield the dialdehyde which eluted first; 1.25 g (15%) of a tan glass. NMR (DMSO-d₆) δ 10.27 (s,1H); 10.17 (s,1H); 7.81 (d,1H,J=7Hz); 7.68 (m,2H); 7.50-7.23 (m,10H); 7.09 (d,2H,J=9Hz); 6.96 (d,2H,J=9Hz); 6.86 (m,6H); 5.59 (s,2H); 2.52 (t,2H,J=7Hz); 1.58 (t of q, 2H,J=7,7Hz); 0.77 (t,3H,J=7Hz). IR (neat) 1697; 1672 cm⁻¹. Anal. calcd. for C₄₁H₃₄N₆O₂: C, 76.62; H, 5.33; N, 13.07. Found: C, 76.46; H, 5.54; N, 12.94.

Continued elution yielded the 4-hydroxymethylimidazole-5-carboxaldehyde product as a light yellow solid: mp 164.5-166.0°C. NMR (DMSO-d₆) *δ* 9.86 (s,1H); 7.80 (d,1H,J=9Hz); 7.63 (t,1H,J=9Hz); 7.53 (t,1H,J=7Hz); 7.50-7.25 (m,10H); 7.07 (d,2H,J=9Hz); 6.97-6.80 (m,8H); 5.47 (t,1H,J=7Hz); 5.29 (s,2H); 4.63 (d,2H,J=7Hz); 2.37 (t,2H,J=7Hz); 1.49 (t of q,2H,J=7,7Hz); 0.73 (t,3H,J=7Hz). IR (Nujol) 1688 cm⁻¹. Anal. calcd. for C₄₁H₃₆N₆O₂·(H₂O)_{0.1}: C, 76.16; H, 5.64; N, 12.84. Found: C, 76.02; H, 5.36; N, 12.84.

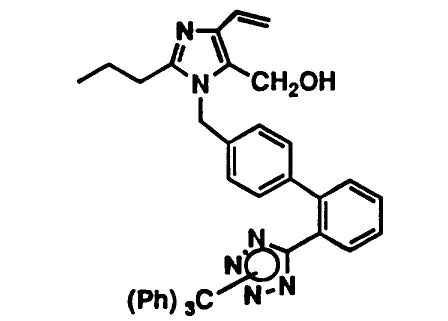

### PART G: Preparation of 5-hydroxymethyl-2-n-propyl-1-[(2'-(N-triphenylmethyl-(1H-tetrazol-5-yl))biphenyl-4-yl)methyl]-4-vinyl-imidazole.

n-BuLi (2.5 M in THF) (1.70 mL, 4.3 mmol, 2.1 eq) was added dropwise to a suspension of methyltriphenylphosphonium bromide (1.53 g, 4.3 mmol, 2.1 eq) in THF (50mL) at 0°C under N₂.

The suspension became a dark yellow solution. Afterwards, a solution of 5-hydroxymethyl-2-n-propyl-1-[(2'-(N-triphenylmethyl-(1H-tetrazol-5-yl))biphenyl-4-yl)methyl]imidazole-4-carboxaldehyde (1.31 g, 2.0 mmol, 1.0 eq) in THF (minimum to dissolve) was added thereto and the resultant light milky yellow solution was stirred overnight at room temperature. The solution was diluted with ethyl acetate and washed with water (3X). The organic layer was dried (MgSO₄), the solvent removed in vacuo, and the residue flash chromatographed over silica gel in 1:1 hexane/ethyl acetate to yield 620 mg (48%) of a white glass: NMR (DMSO-d₆) δ 7.79 (d,1H,J=7Hz); 7.62 (t,1H,J=7Hz); 7.55 (t,1H,J=7Hz); 7.45 (d,1H,J=7Hz); 7.41-7.18 (m,9H); 7.06 (d,2H,J=9Hz); 6.95-6.80 (m,8H); 6.80-6.55 (m,1H); 5.73 (d of d,1H,J=17,3 Hz); 5.17 (s,2H); 5.10 (t,1H,J=7Hz); 5.05 (d of d,1H,J=12,3Hz); 4.28 (d,2H,J=7Hz); 2.37 (t,2H,J=7Hz); 1.50 (t of q,2H,J=7,7Hz); 0.78 (t,3H,J=7Hz). IR (neat) 1029; 1006; 909; 733; 698 cm⁻¹. Anal. calcd. for C₄₂H₃₈N₆O·H₂O: C, 76.34; H, 6.10; N, 12.72. Found: C, 76.49; H, 5.88; N, 12.52.

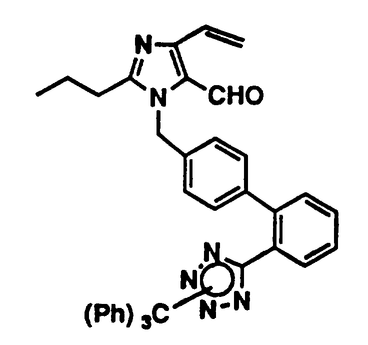

### PART H: Preparation of 2-n-propyl-1-[(2'-(N-triphenylmethyl-(1H-tetrazol-5-yl))biphenyl-4-yl)methyl]-4-vinyl-imidazole-5-carboxaldehyde.

5-hydroxymethyl-2-n-propyl-1-[(2'-(N-triphenylmethyl-(1H-tetrazol-5-yl))biphenyl-4-yl)methyl]-4-vinylimidazole (470 mg, 0.73 mmol, 1 eq), Dess-Martin periodinane (J. 0rg. Chem. (1983) 48, 4155) (341 mg, 0.80 mmol, 1.1 eq) and methylene chloride (10mL) were mixed and stirred under nitrogen overnight. The solvent was removed in vacuo and the residue flash chromatographed in 3:2 hexane ethyl acetate over silica gel to yield 310 mg (66%) of a white glass. NMR (DMSO-d₆) *δ* 9.91 (s,1H); 7.80 (d,1H,J=7Hz); 7.61 (t,1H, J=7Hz); 7.54 (t,1H,J=7Hz); 7.48-7.22(m,10H); 7.20 (d,1H,J=9Hz); 7.06 (d,2H,J=9Hz); 7.00-6.75 (m,8H); 6.15 (d of d,1H,J=17,3 Hz); 5.52 (s,2H); 5.47 (d of d,1H,J=12,3 Hz); 2.49 (t,2H,J=7Hz); 1.57 (t of q,2H,J=7,7Hz); 0.79 (t,3H,J=7Hz). IR (neat) 1658 cm⁻¹. Anal. calcd. for C₄₂H₃₆N₆O·(H₂O)_{0.5}: C, 77.63; H, 5.74; N, 12.93. Found: C, 77.53; H, 5.73; N, 12.64.

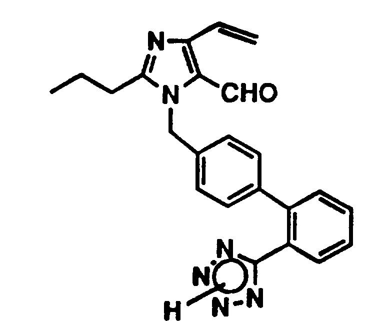

### PART I: Preparatlon of 2-n-propyl-1-[(2'-(1H-tetrazol-5-yl)biphenyl-4-yl)methyl]-4-vinyl-imidazole-5-carboxaldehyde.

2-n-propyl-1-[(2'-(N-triphenylmethyl-(1H-tetrazol-5-yl))biphenyl-4-yl)methyl]-4-vinyl-imidazole-5-carboxaldehyde (330 mg), trifluoroacetic acid (1.65 mL), water (1.65 mL), and THF (1.65 mL) were mixed and stirred at room temperature. After 8 h, the mixture was neutralized to pH=7 with 10N NaOH and the solvents removed in vacuo. The residue was flash chromatographed in 1:1 hexane/ethyl acetate to 100% ethanol to yield 270 mg of a white glass. NMR (DMSO-d₆) *δ* 9.92 (s,1H); 7.65-7.50 (m,1H); 7.50-7.12 (m,3H); 7.09 (d,2H,J=9Hz); 6.89 (d,2H,J=9Hz); 6.11 (d of d,1H,J=7,3Hz); 5.55 (s,2H); 5.45 (d of d, 1H,J=12,3 Hz); 2.63 (t,2H,J=7Hz); 1.64 (t of q,2H,J=7,7Hz); 0.90 (t,3H,J=7Hz). IR (Nujol) 1680 cm⁻¹.

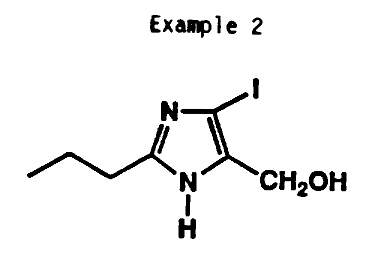

### PART A: Preparation of 5-hydroxymethyl-4-iodo-2-n-propylimidazole.

A solution of 31.5 g of 4(5)-hydroxymethyl-2-n-propylimidazole and 50.6 g of N-iodosuccinimide in 560 mL of 1,4-dioxane and 480 mL of 2-methoxyethanol was stirred at 45°C for 2 h. The solvents then were removed under vacuum. The resulting solids were washed with distilled water and then were dried to afford 54.6 g of the product as a yellow solid; mp 169-170°C. NMR (DMSO-d₆) *δ* 12.06 (br s,1H); 5.08 (t,1H); 4.27 (d,2H); 2.50; (t,2H); 1.59 (sext.,2H); 0.84 (t,3H).

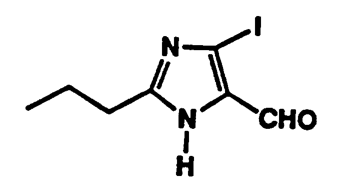

### PART B: Preparatlon of 4-iodo-2-n-propylimidazole-5 carboxaldehyde

To a solution of 35.8 g of 5-hydroxymethyl-4-iodo-2-n-propylimidazole in 325 mL of glacial acetic acid at 20°C was added dropwise over 1 h 290 mL of 1.0 N aqueous ceric ammonium nitrate solution. The resulting mixture was stirred at 20°C for 1 h. The reaction mixture then was diluted with water, adjusted to pH 5-6 employing aqueous sodium hydroxide solution, and extracted with chloroform. The combined organic phases were washed with water and brine, dried over anhydrous sodium sulfate, filtered, and concentrated. The resulting crude solid was recrystallized from 1-chlorobutane to furnish 29.9 g of product as a light yellow solid; mp 141-142°C. NMR (CDCl₃) δ 11.51 (br s,1H); 9.43 (s,1H); 2.81 (t,2H); 1.81 (sext., 2H); 0.97 (t,3H).

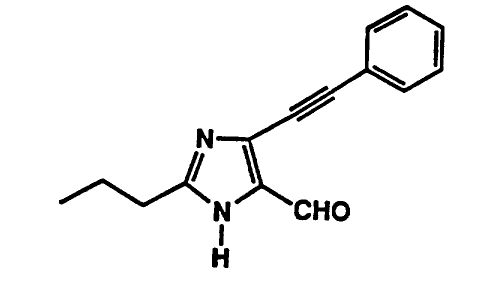

### PART C: Preparation of 3-n-propyl-4-(phenylethynyl)imidazole-5-carboxaldehyde.

A solution of 2.64 g (0.01 mol) of 4-iodo-2-n-propylimidazole-5-carboxaldehyde, 25 mL of dry DMF, 2.5 mL of triethylamine, 1.00 g (0.001426 mol) of bis(triphenylphosphine)palladium chloride and 5.00 g (0.017 mol) of (phenylethynyl)tributyltin was heated to 70°C under nitrogen. The reaction was stirred for 120 hours, then cooled. The precipitate was filtered and washed with methylene chloride, and the resulting filtrate was evaporated under reduced pressure. The residue was dissolved in 200 mL of methylene chloride and extracted three times with 100 mL of 10% HCl. The aqueous layer pH was adjusted to 10 with 50% sodium hydroxide and extracted three times with 100 mL of methylene chloride. The organic phase was dried over sodium sulfate and evaporated under reduced pressure. Yield 0.56 g (0.0023 mol, 23%) of 3-n-propyl-4-(phenylethynyl)imidazole-5-carboxaldehyde. NMR (CDCl₃) *δ* 9.89 (s,1H); 8.22 (s,1H); 7.93 (m,3H); 7.53 (m,2H); 2.87 (t,2H); 1.87 (m,2H); 1.03 (t,3H).

The following intermediates could be prepared by the procedure described in example 2, part C:

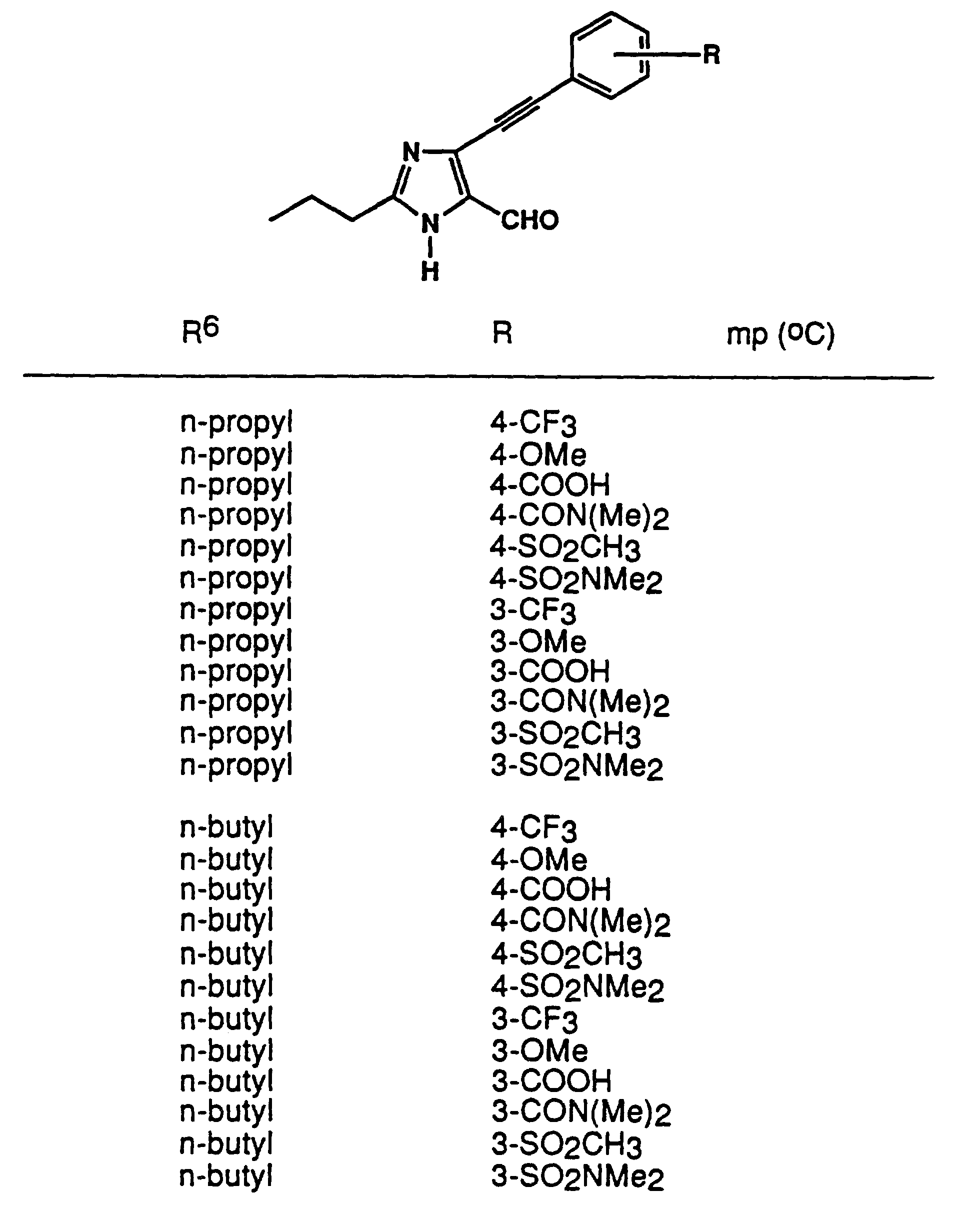

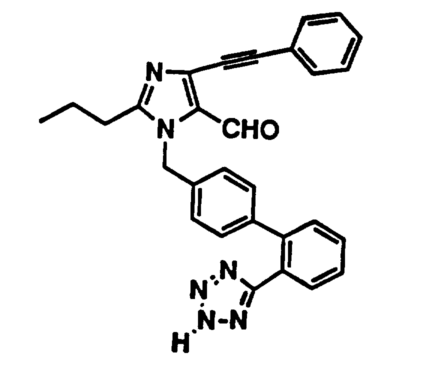

### PART D. Preparation of 4-phenylethynyl-3-n-propyl-1-[(2'-(1H-tetrazol-5-yl)biphenyl-4-yl) methyl]imidazole-5-carboxaldehyde.

3-n-Propyl-4-(phenylethynyl)imidazole-5-carboxaldehyde was alkylated with 4-bromomethyl-2'-(N-triphenylmethyl-(1H-tetrazol-5-yl))biphenyl by the procedure described in example 1, parts D and I, to yield the entitled product. NMR (CDCl₃) δ 9.88 (s,1H); 8.03 (m,1H); 7.57-7.27 (m,8H); 7.17 (m,2H); 7.01 (m,2H); 5.55 (s,2H); 2.61 (t,2H); 1.75 (m,2H); 0.99 (t,3H).

Examples 3-26 (Table 1) could be made by the procedures described in Example 2.

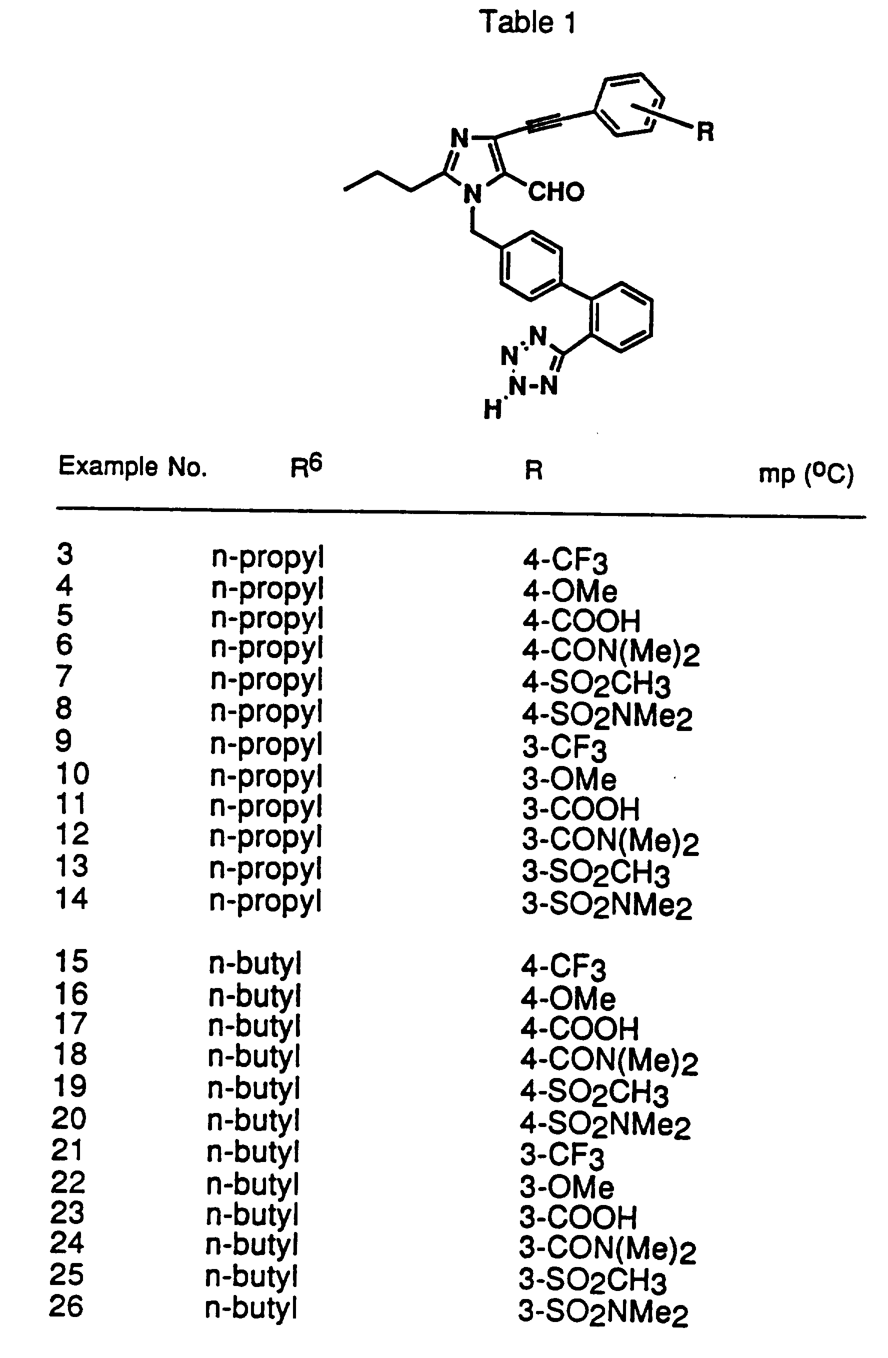

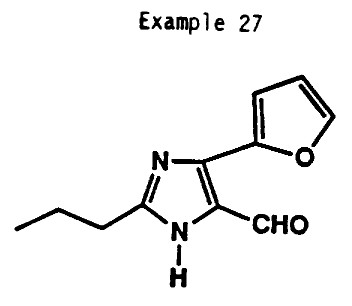

### PART A: Preparatlon of 4-(furan-2-yl)-2-n-propylimidazole-5-carboxaldehyde.

A solution of 2.64 g (0.01 mol) of 4-iodo-3-n-propylimidazole-5-carboxaldehyde, 60 mL of toluene, and 0.33 g (0.00029 mol) of tetrakistriphenylphosphine palladium (0) was stirred at room temperature under nitrogen, while a solution of 2.34 g (0.0174 mol) of furan-2-ylboronic acid in 50 mL of ethanol was slowly added. The reaction was stirred for 5 minutes, after which 12 mL of 2M sodium carbonate was slowly added. After the addition was completed, the reaction was refluxed for 8 h and cooled. The reaction was filtered, the filtrate was evaporated under reduced pressure, and the resulting residue was dissolved in 300 mL of methylene chloride, washed twice with 100 mL of saturated sodium chloride solution, washed twice with 100 mL of distilled water, and washed twice with 300 mL of 10% HCl. The HCl layer was made basic with 50% sodium hydroxide until the pH=10. At this point, the basic water layer was extracted three times with 300 mL of methylene chloride, the methylene chloride layer was dried over sodium sulfate and evaporated under reduced pressure. Yield: 0.34 g (0. 00156 mol, 5-carboxaldehyde. NMR (CDCl₃) δ 10.14 (s,1H); 7.54 (s,1H); 7.00 (d,1H); 6.55 (m,1H); 2.79 (t,2H); 1.80 (m,2H); 1.02 (t,3H).

The following intermediates (Table 2) were or could be prepared by the procedure described in Example 27, part A:

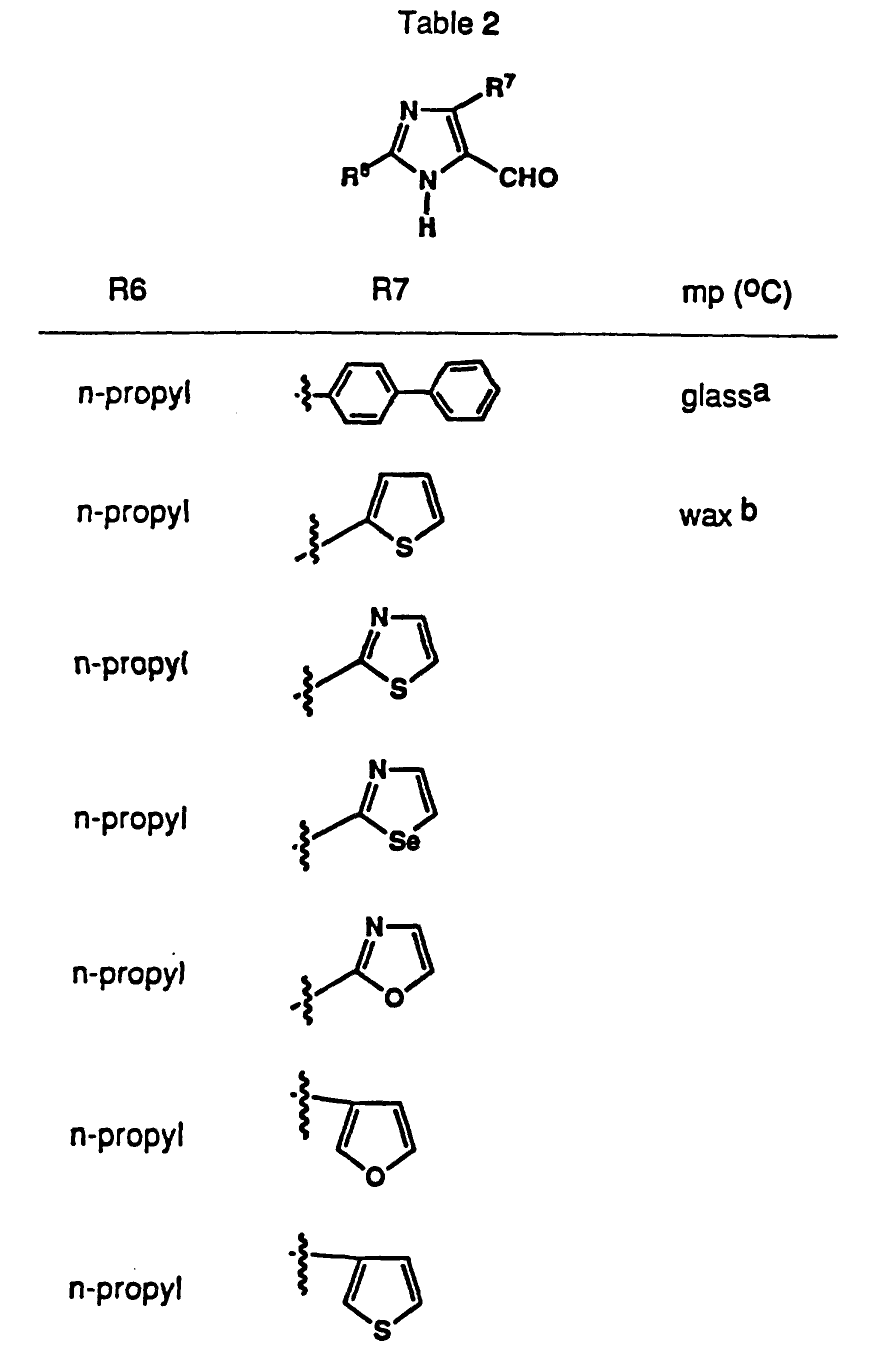

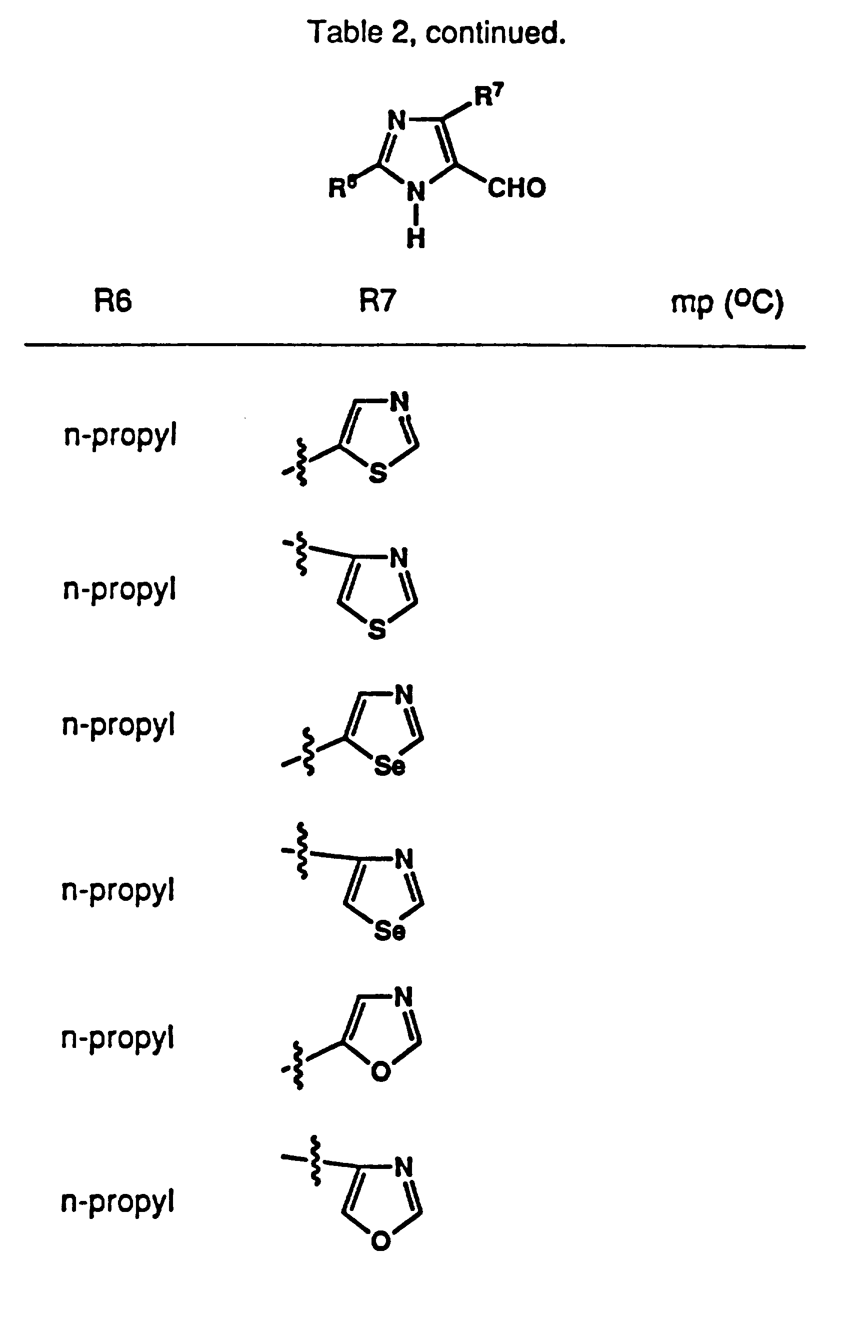

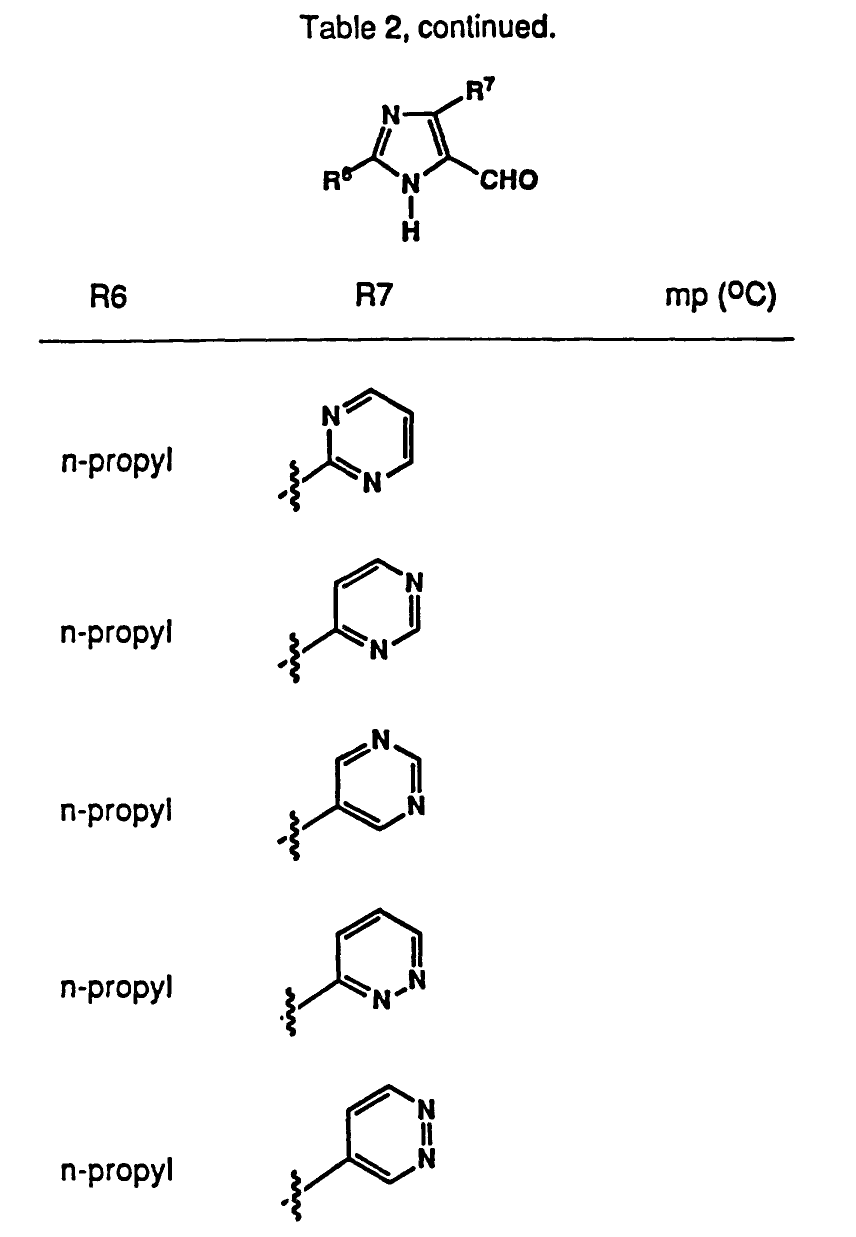

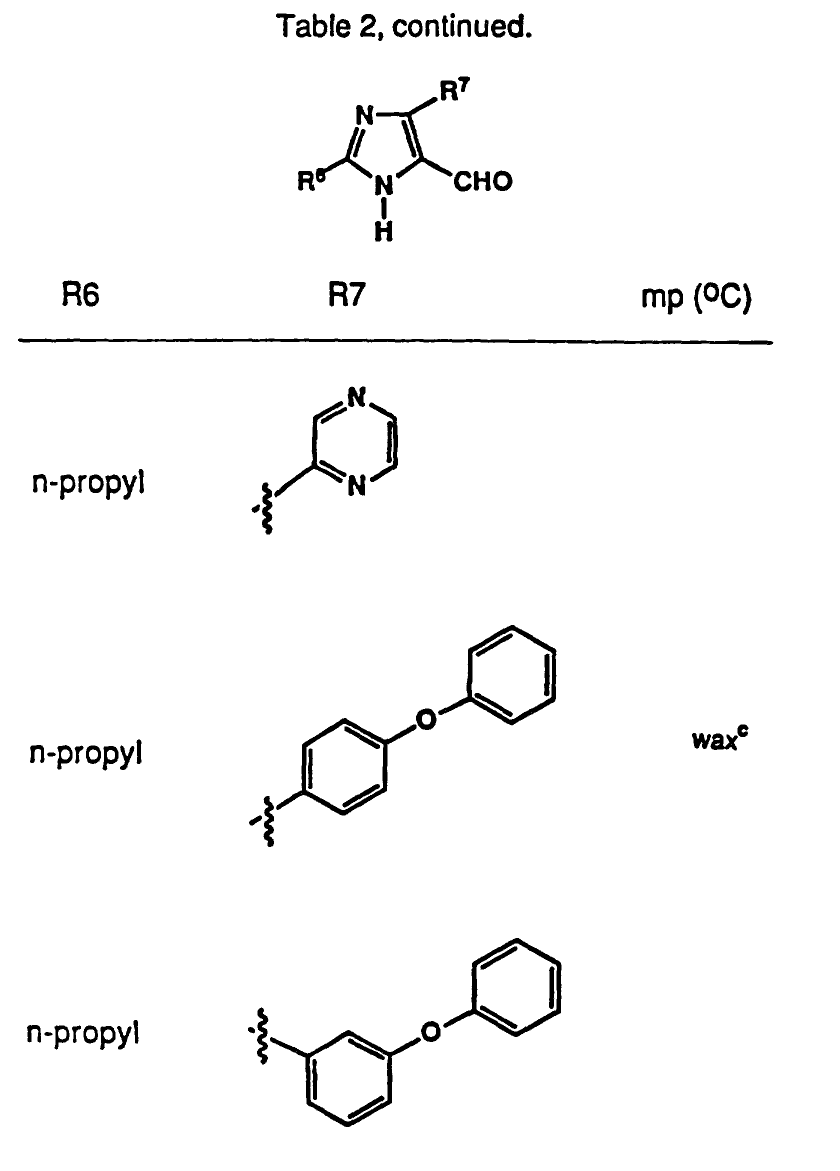

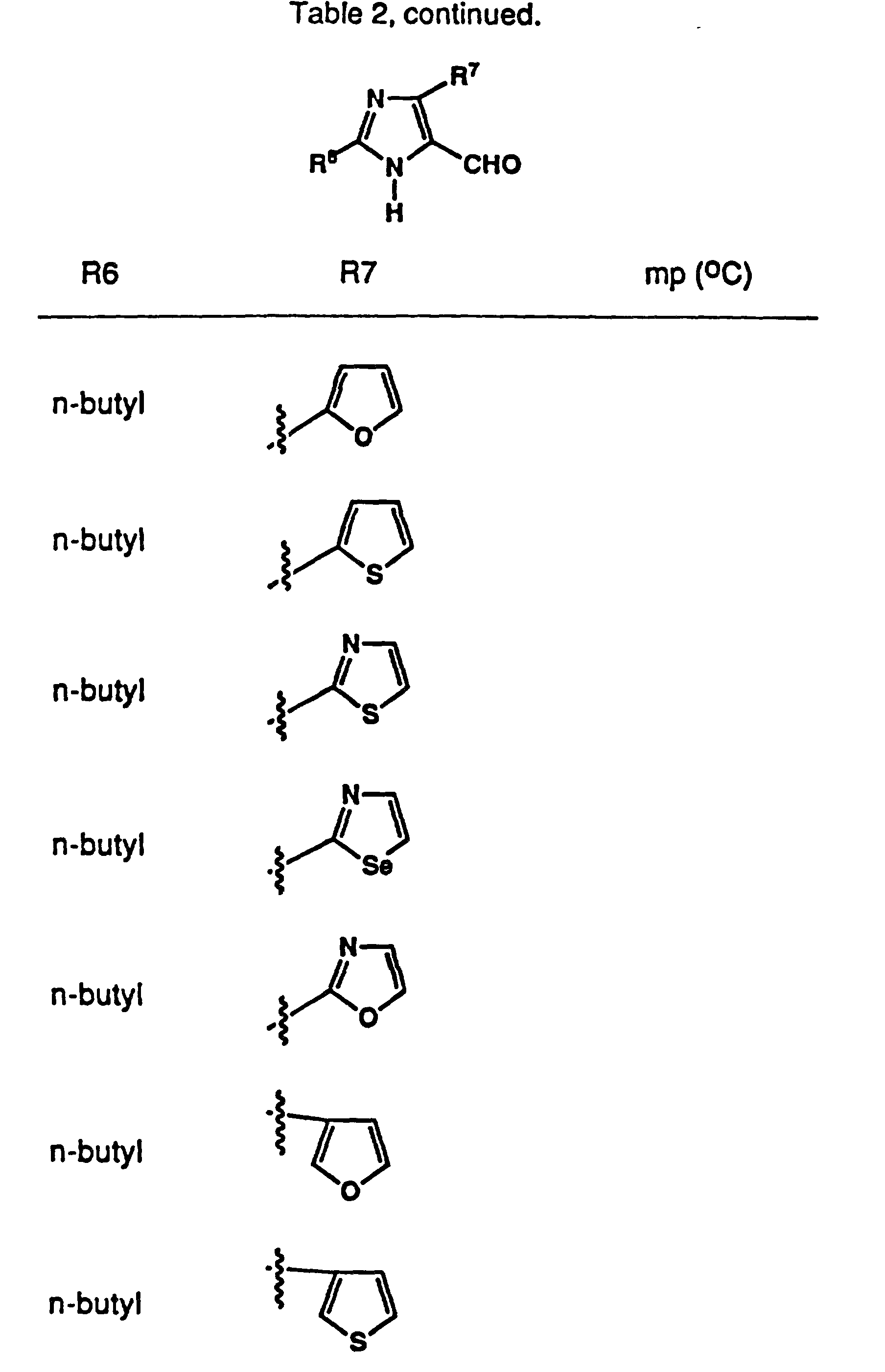

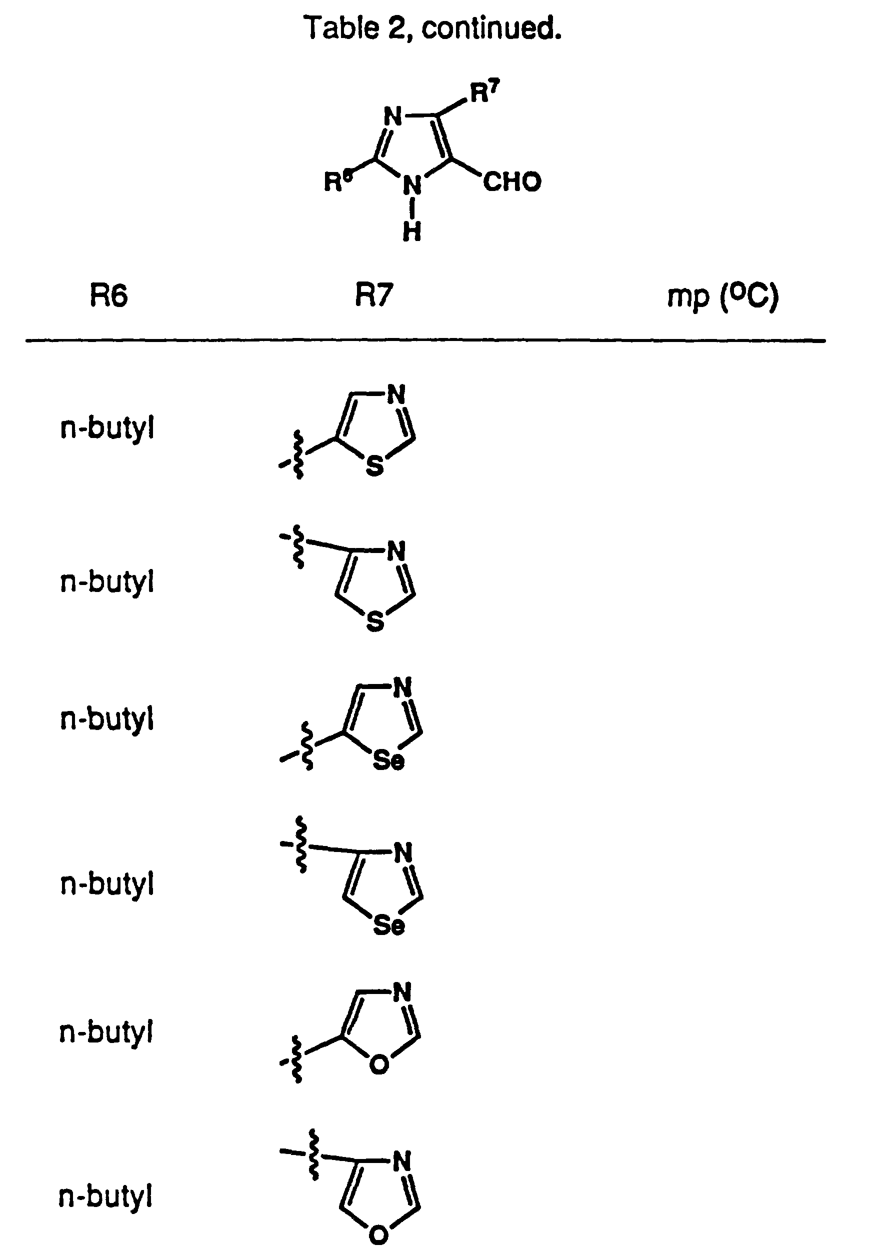

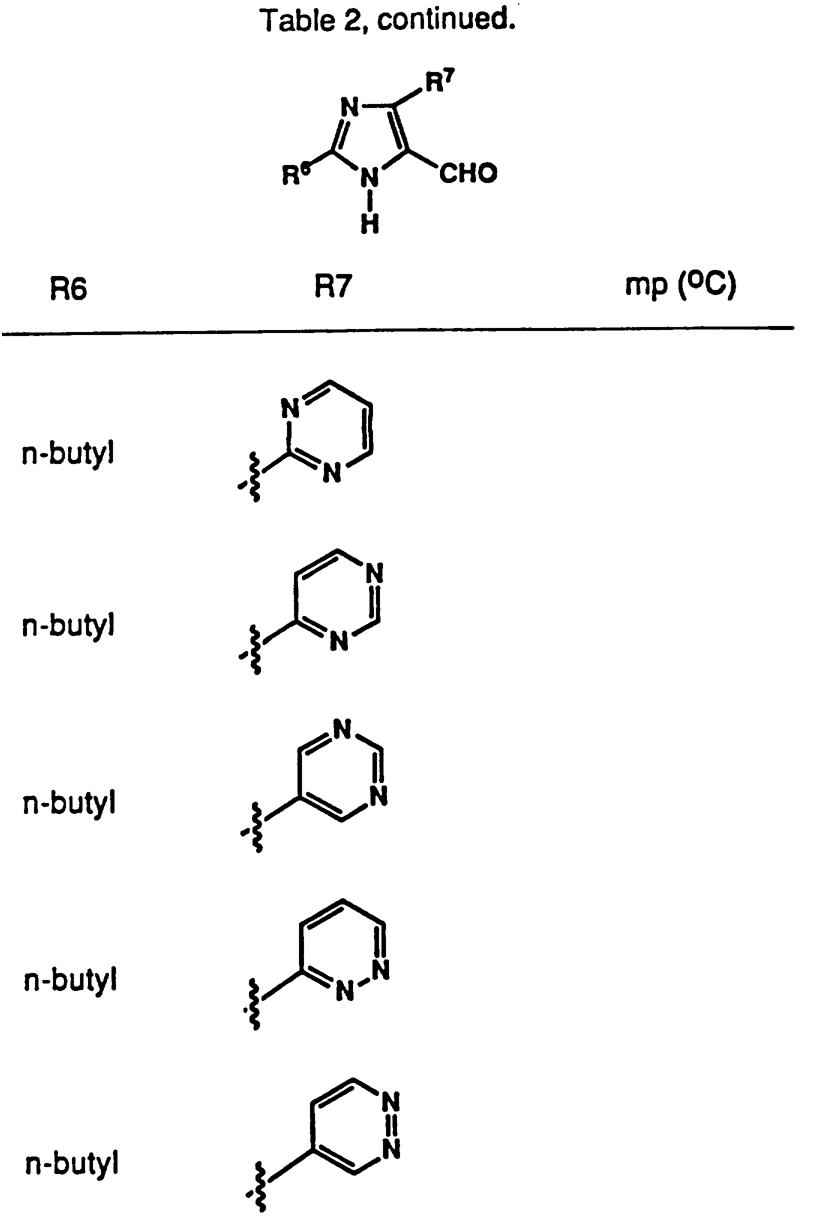

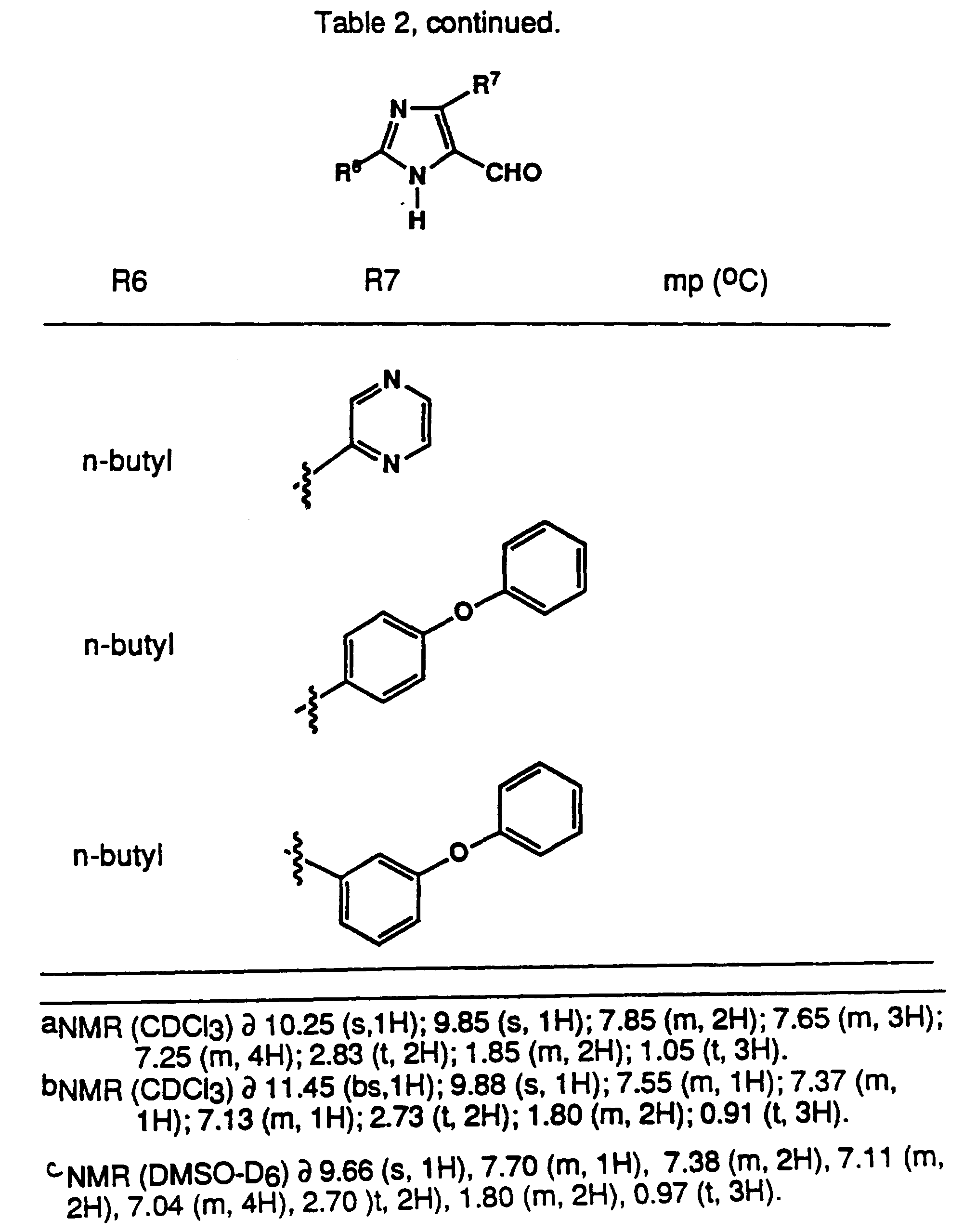

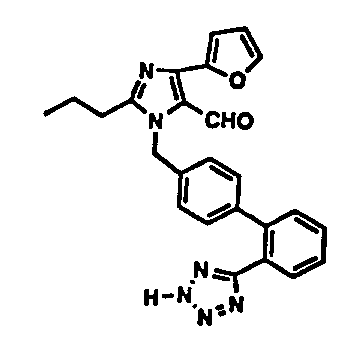

### PART B: Preparation of 4-(furan-2-yl)-2-n-propyl-1-[(2'-(1H-tetrazol-5-yl)biphenyl-4-yl)methyl]imidazole-5-carboxaldehyde.

4-(furan-2-yl)-2-n-propylimidazole-5-carboxaldehyde was transformed into the entitled product by the procedures described in Example 1, parts D and I: mp 129 (dec.). NMR (CDCl₃) δ 10,15 (s,1H); 7.95 (d,1H); 7.55 (m,2H); 7.38 (m,2H); 7.10 (d,2H); 6.98 (d,2H); 6.85 (d,1H); 6.45 (m,1H); 5.55 (s,2H); 2.55 (t,2H); 1.70 (m,2H); 0.91 (t,3H).

The examples in Table 3 could be prepared by the procedures described in example 27 using the appropriate starting materials:

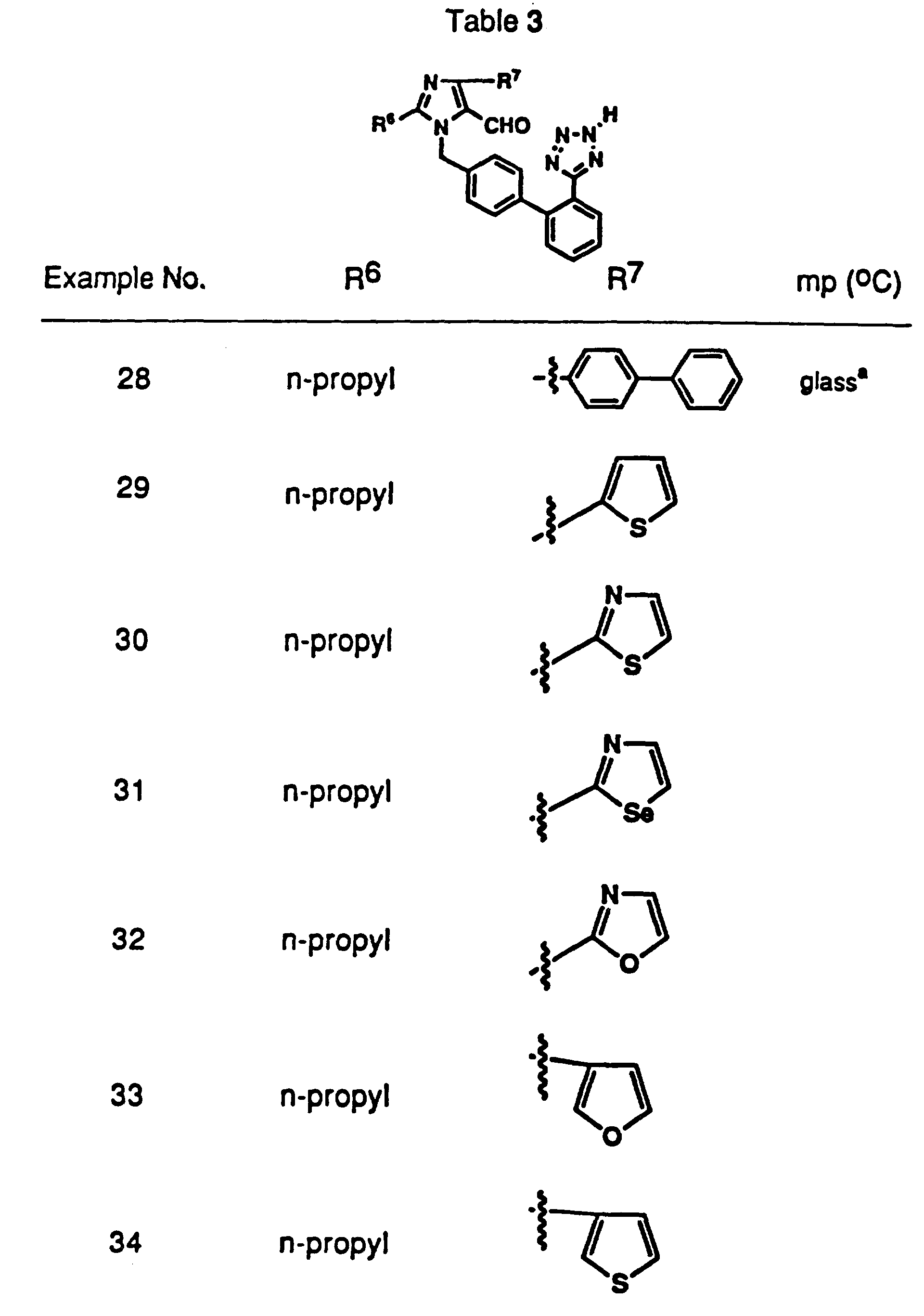

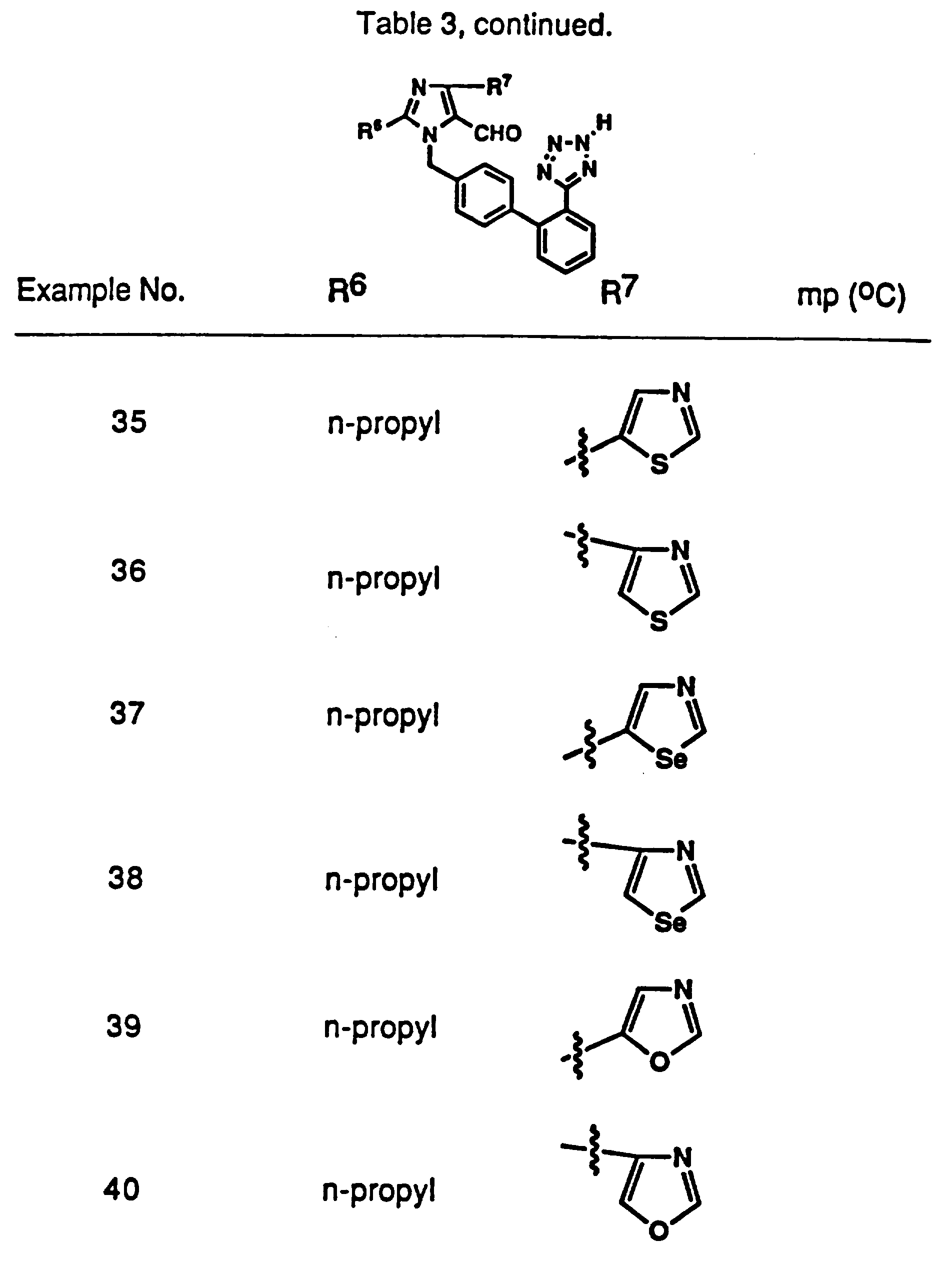

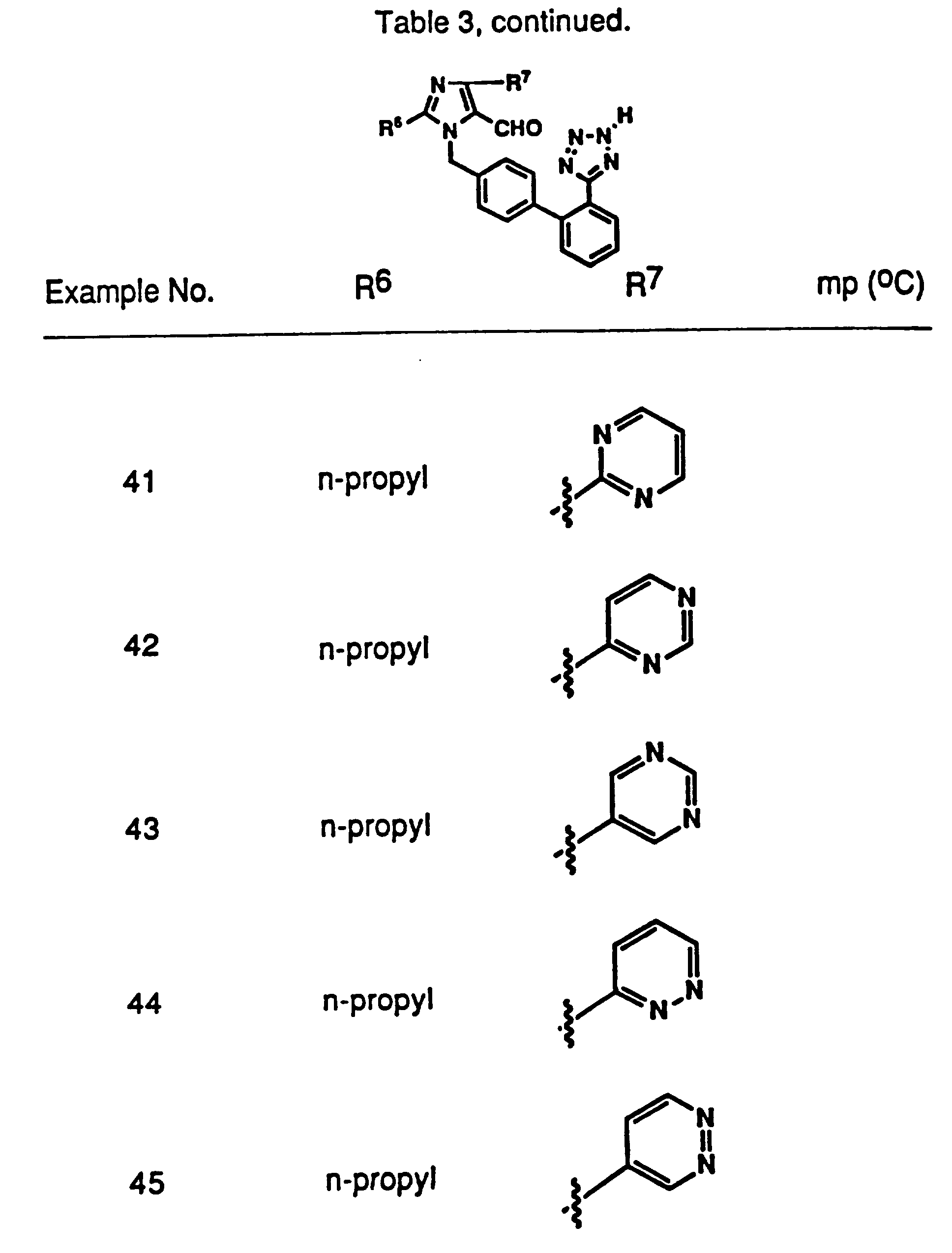

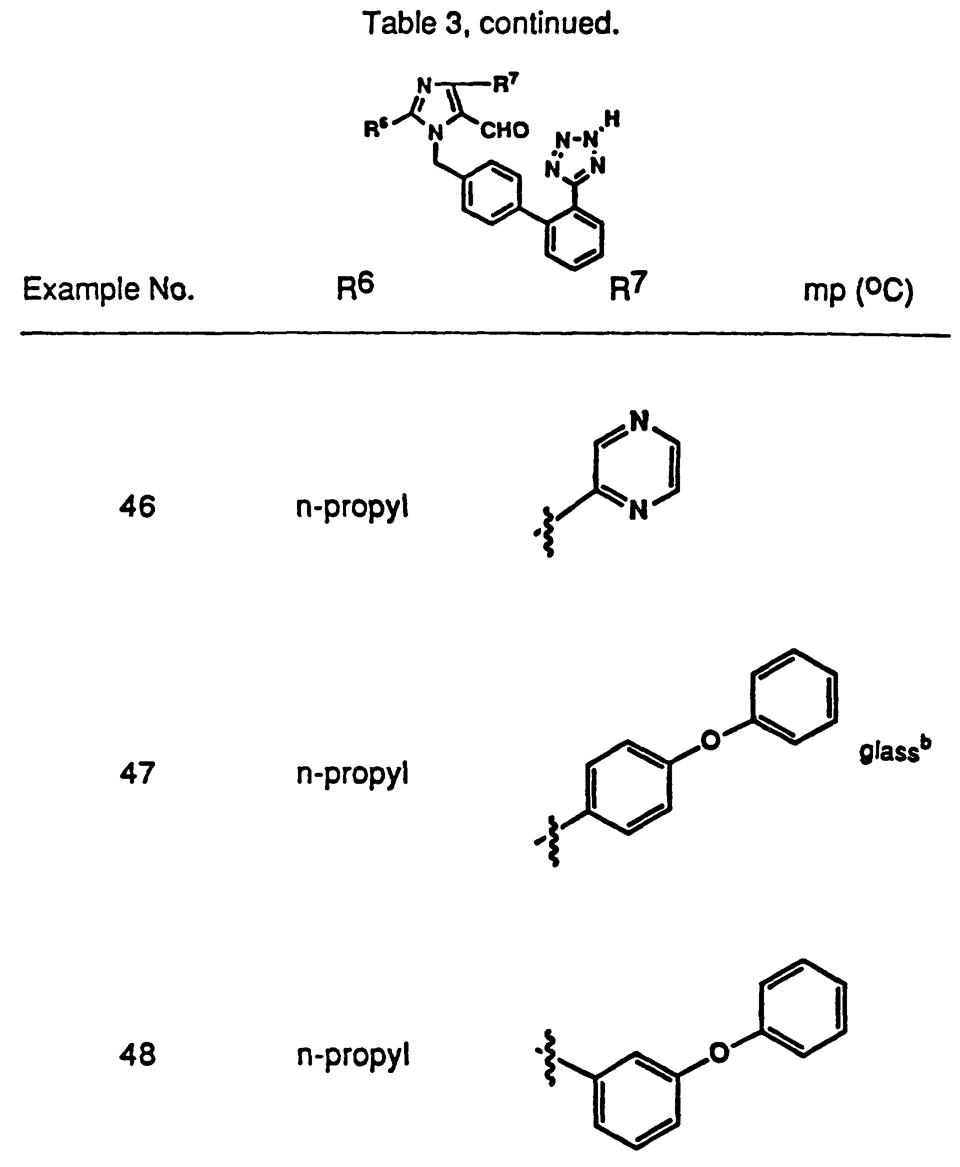

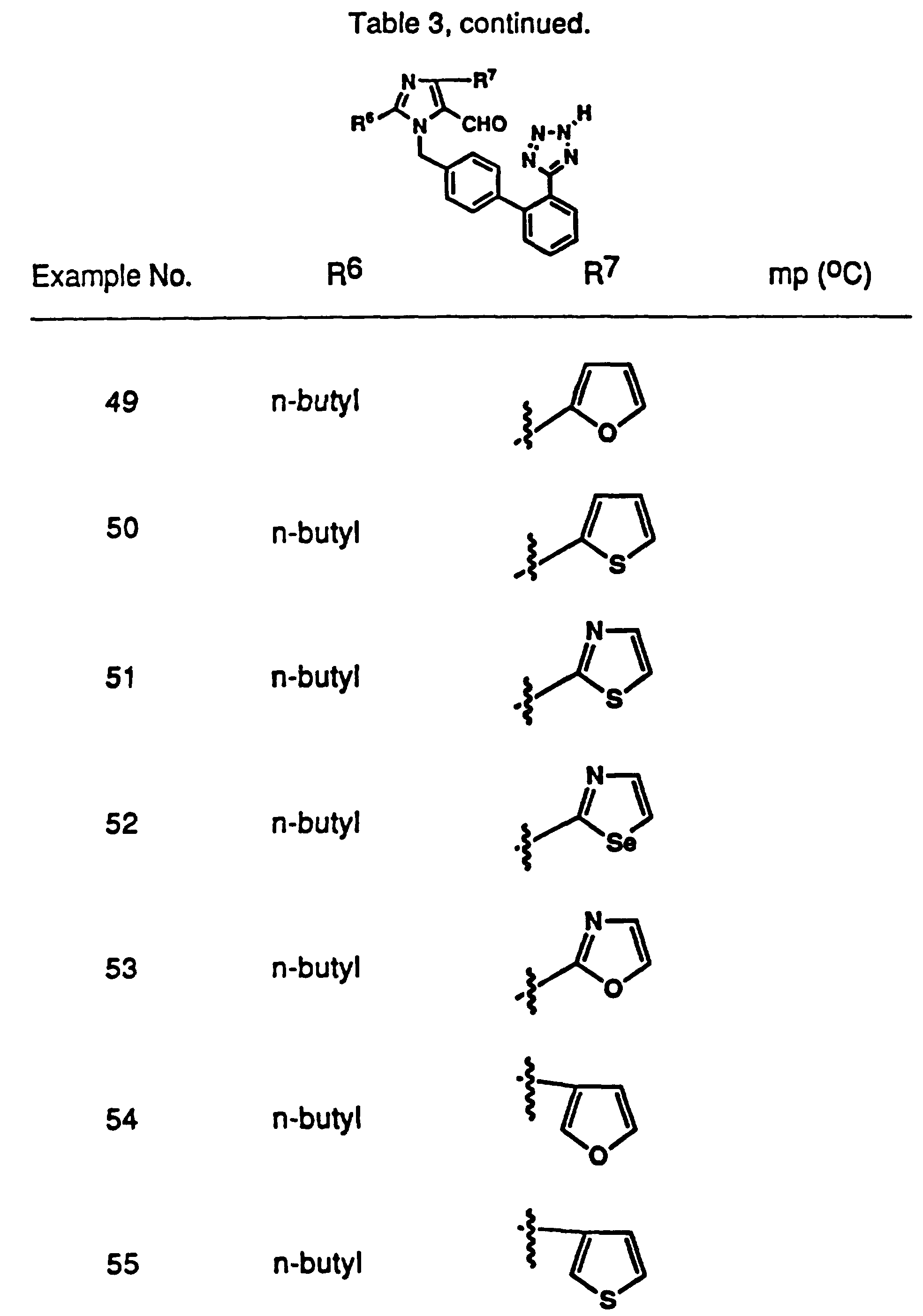

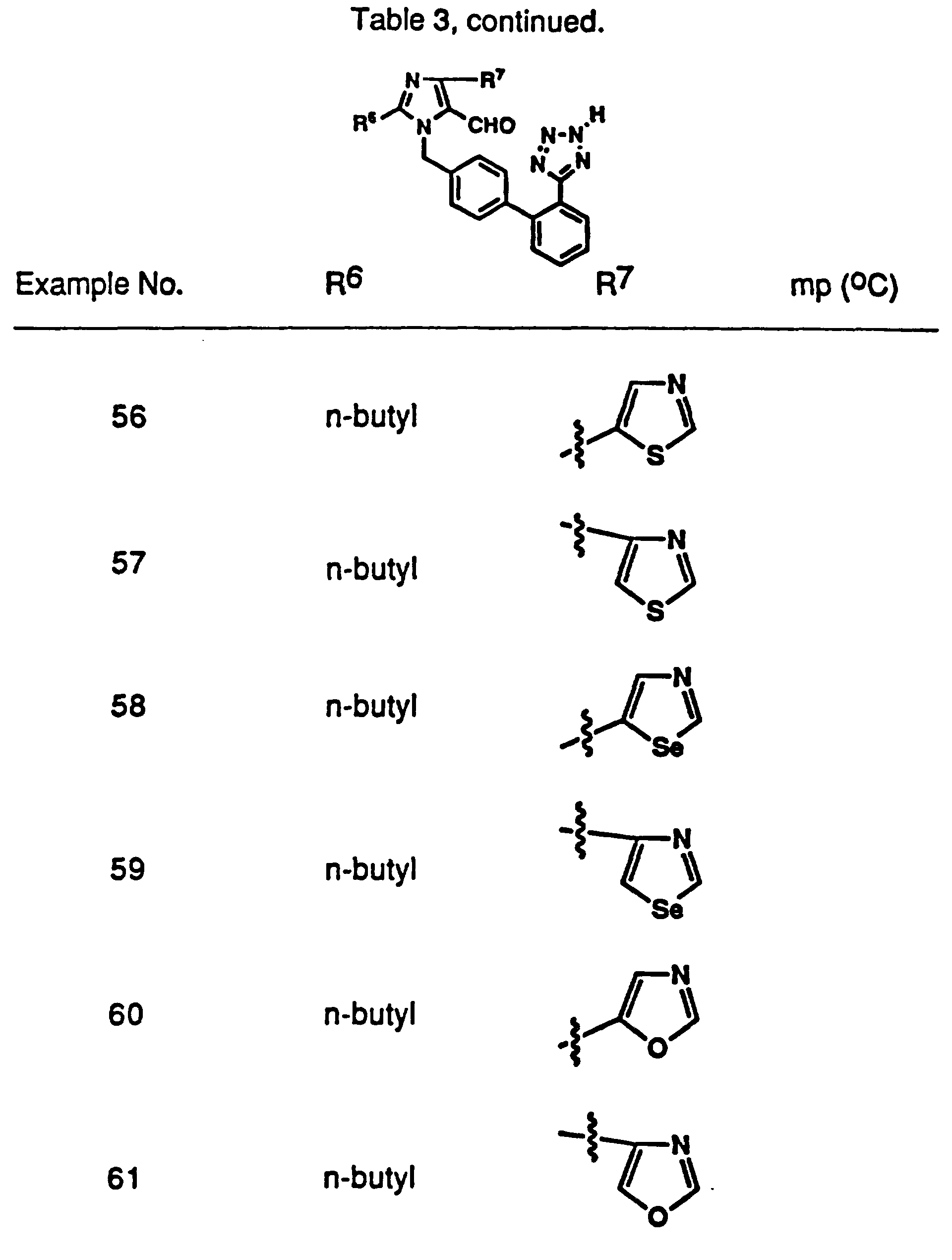

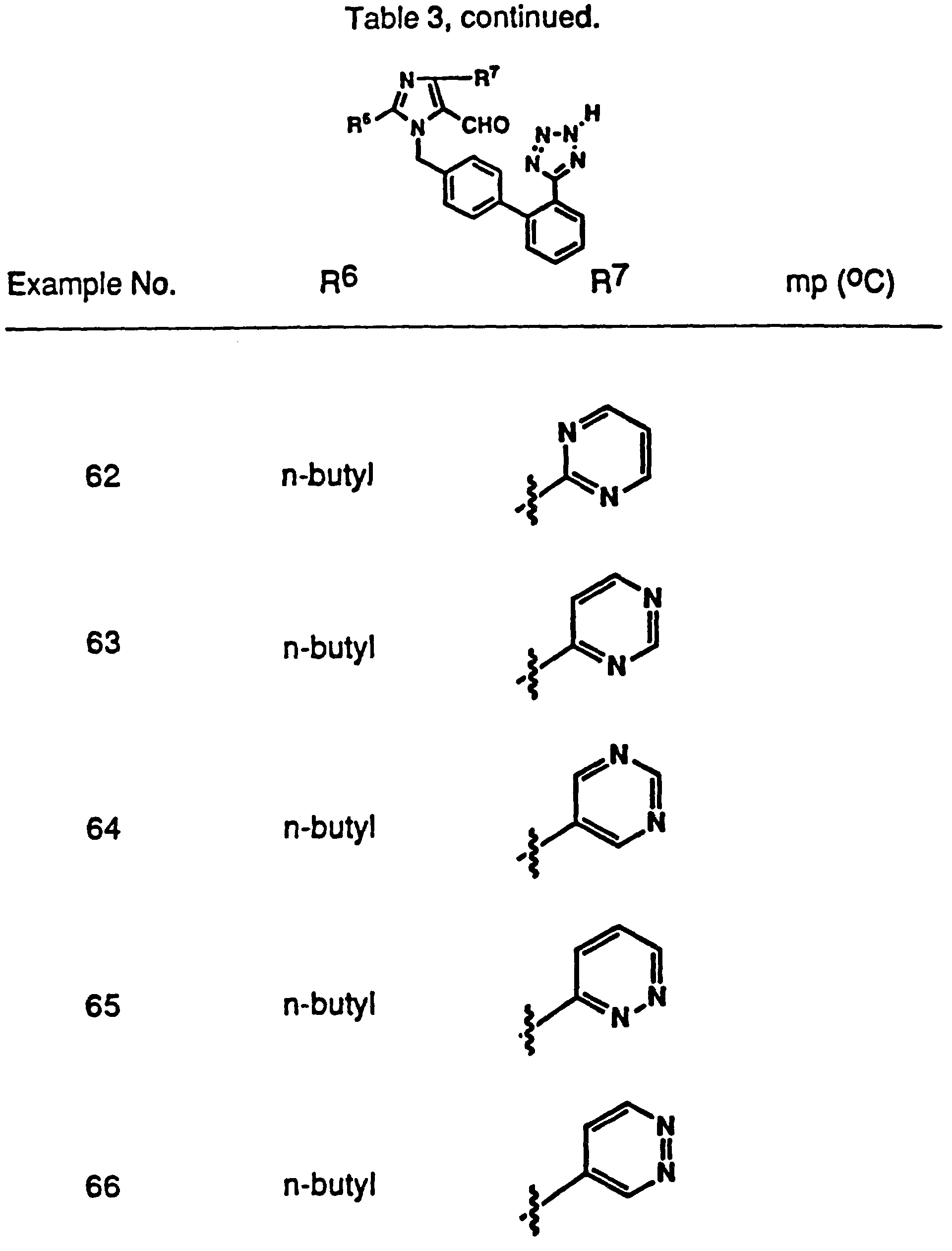

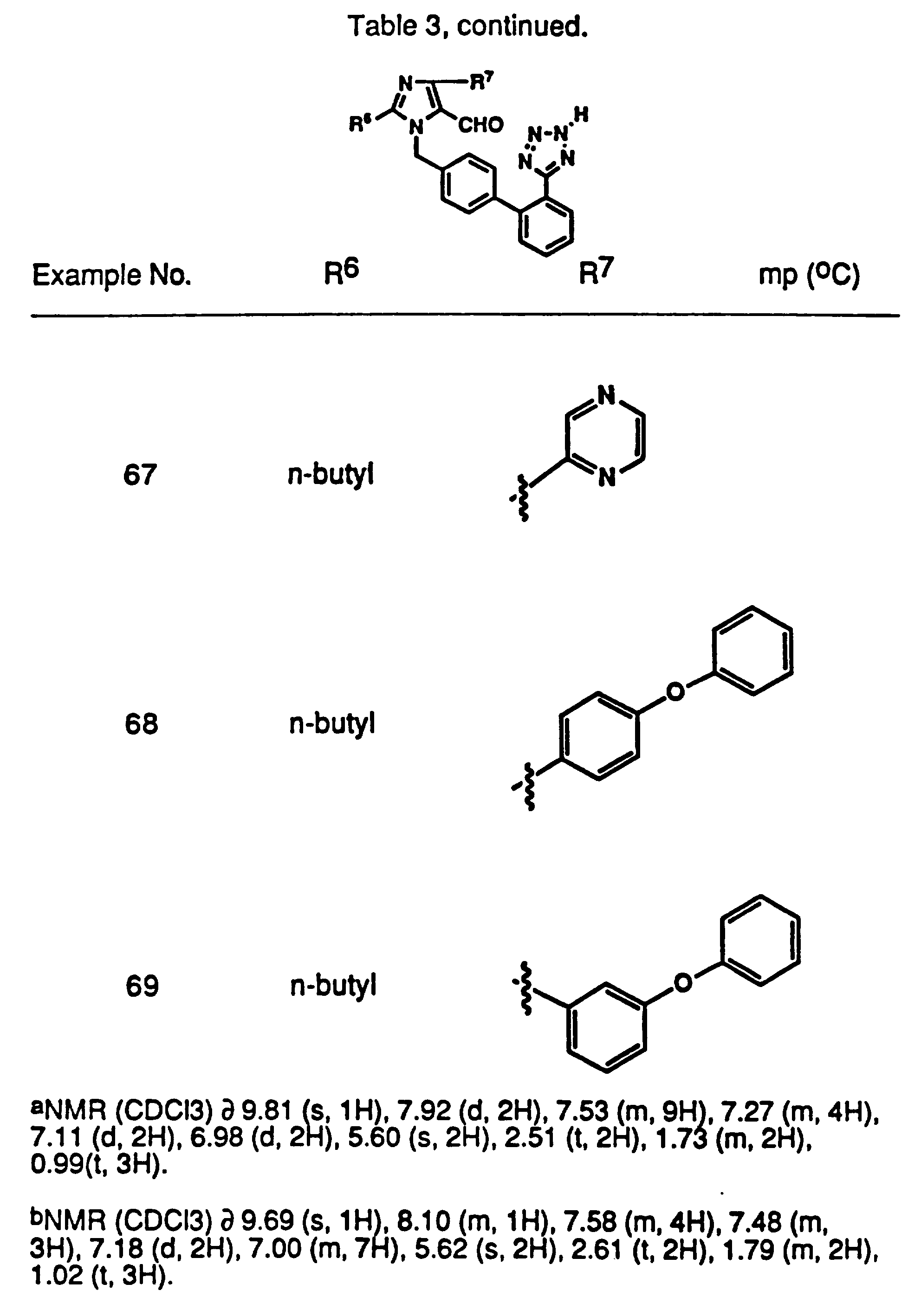

The examples in Tables 4 and 5 can be made by procedures described in examples 1, 2, or 27 using the biphenyl starting materials disclosed in this patent or by other methods familiar to one skilled in the art.

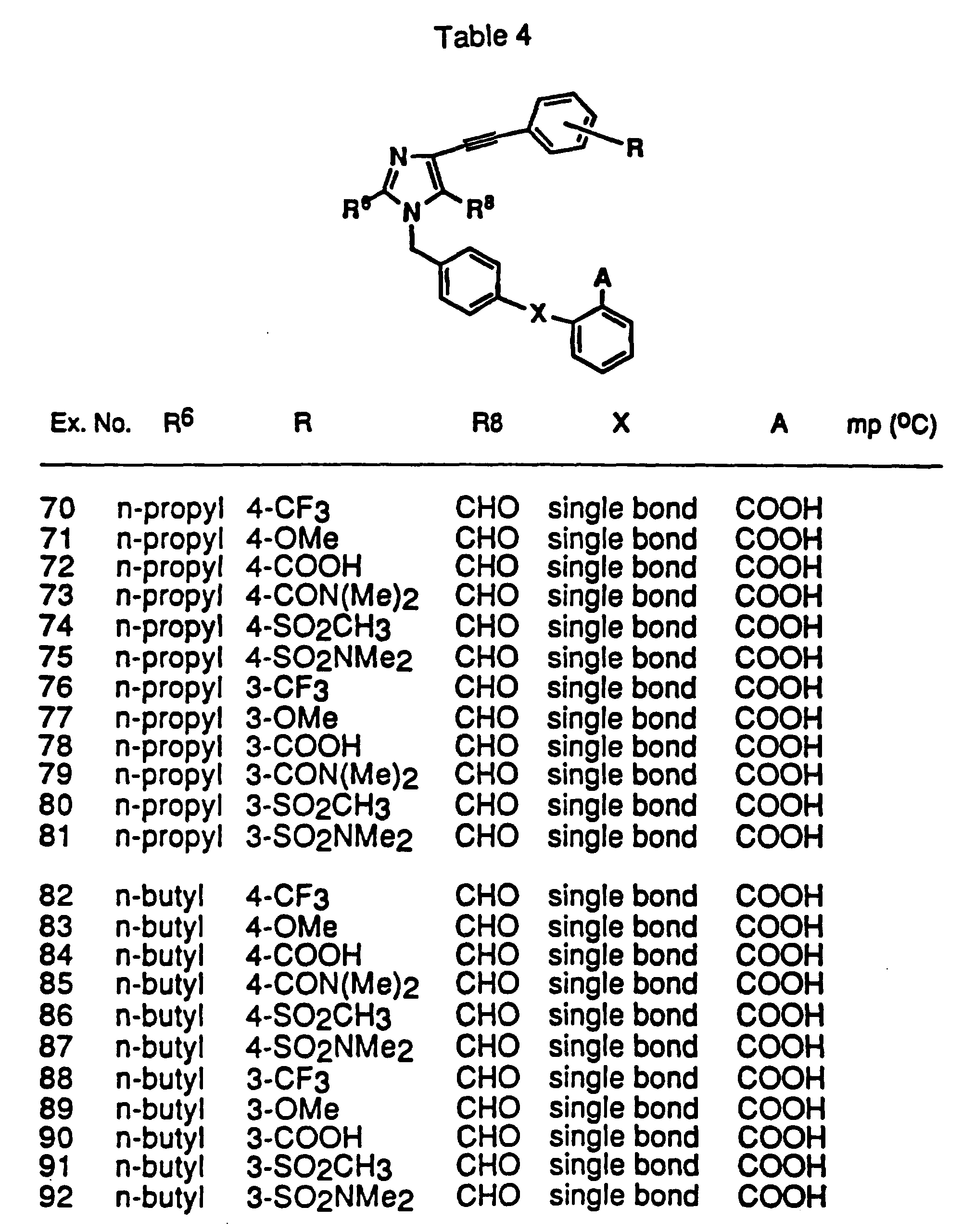

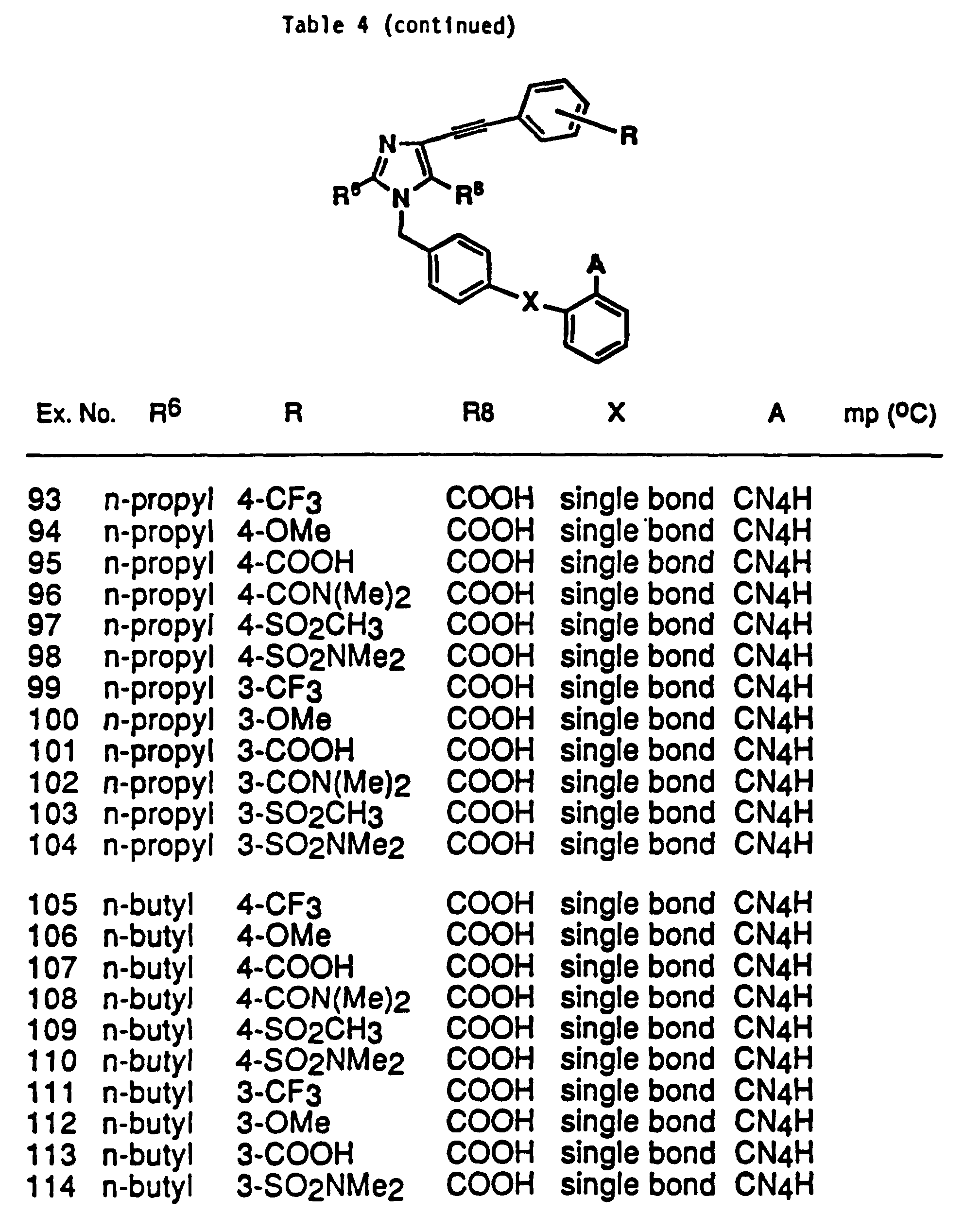

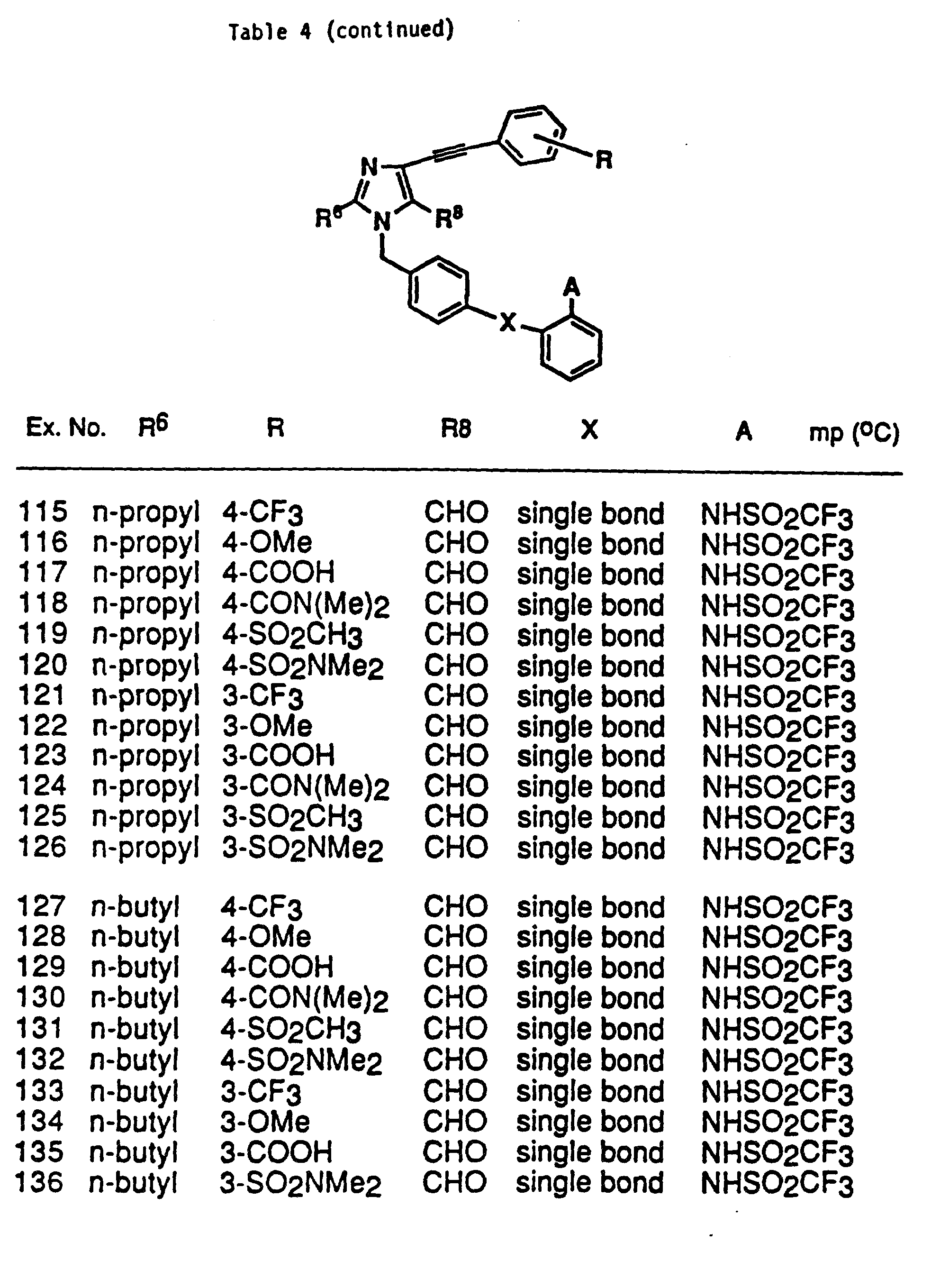

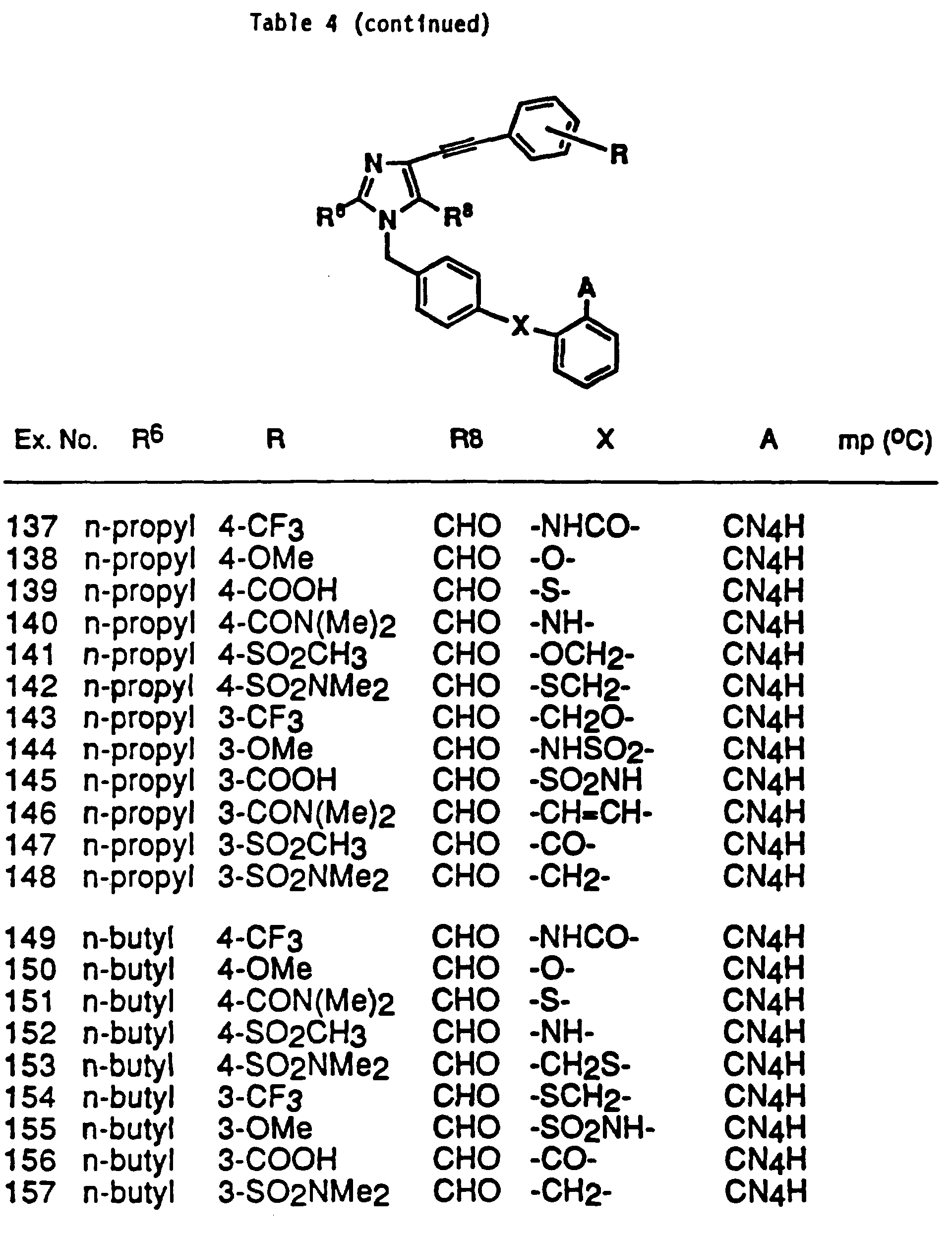

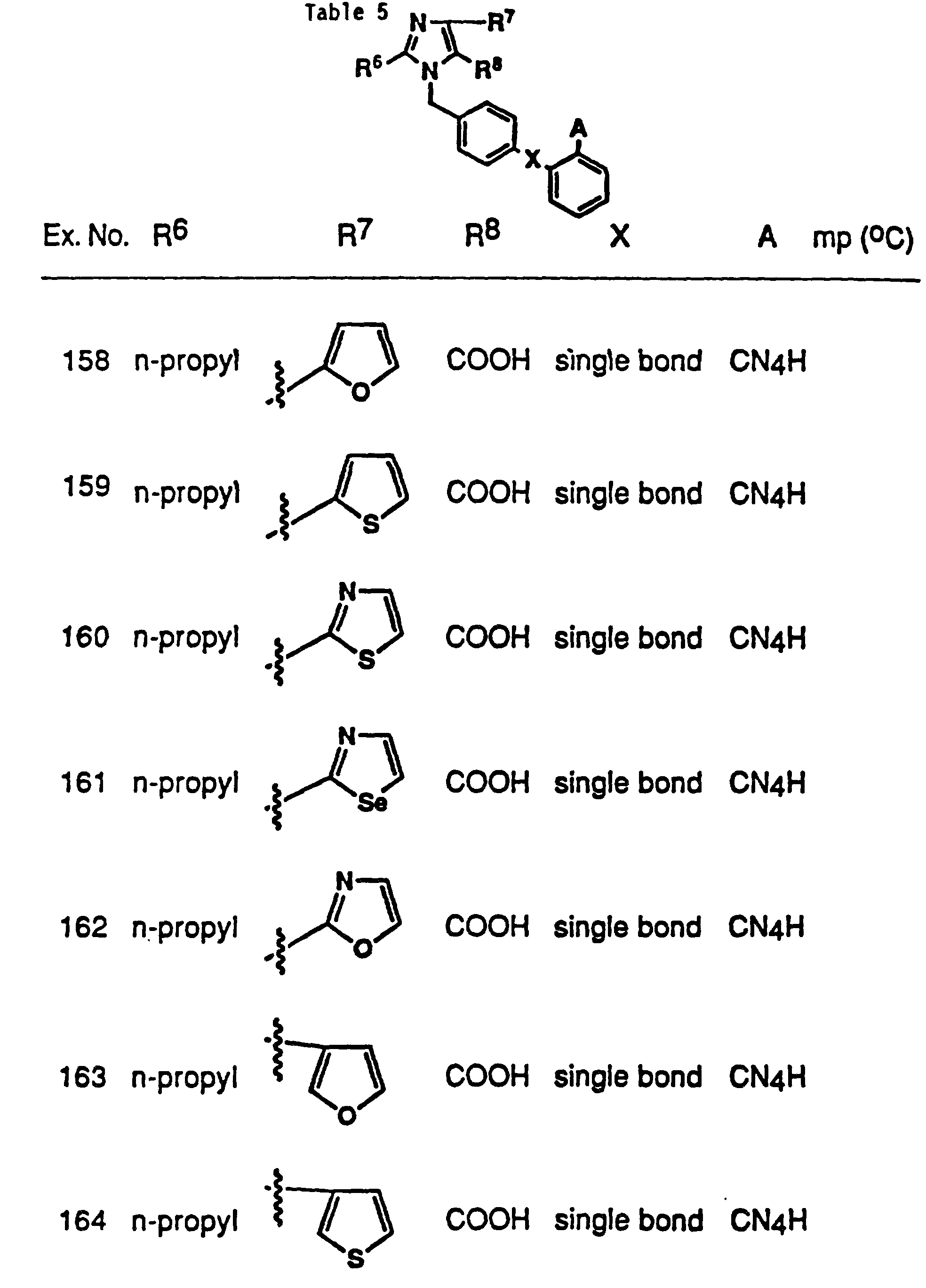

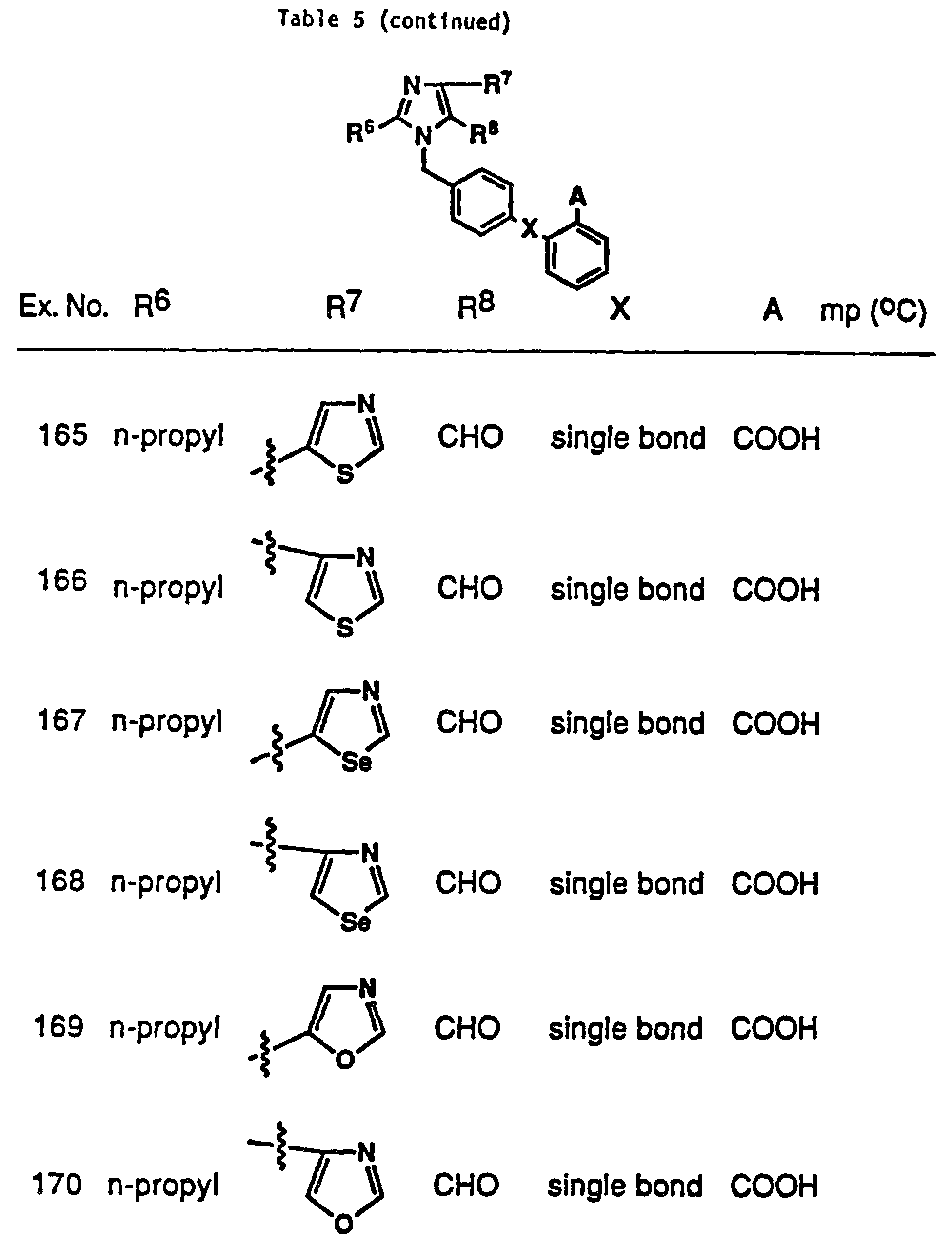

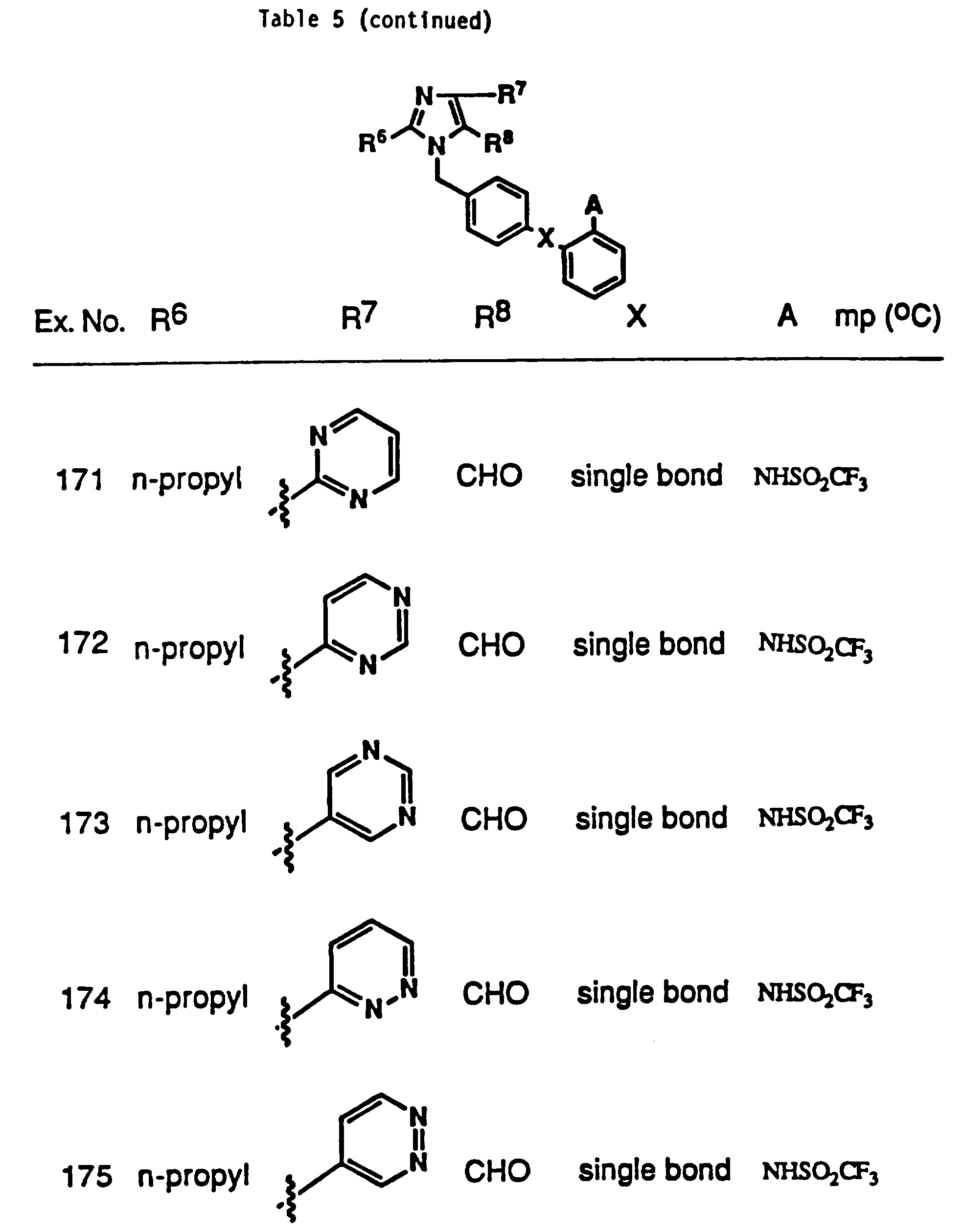

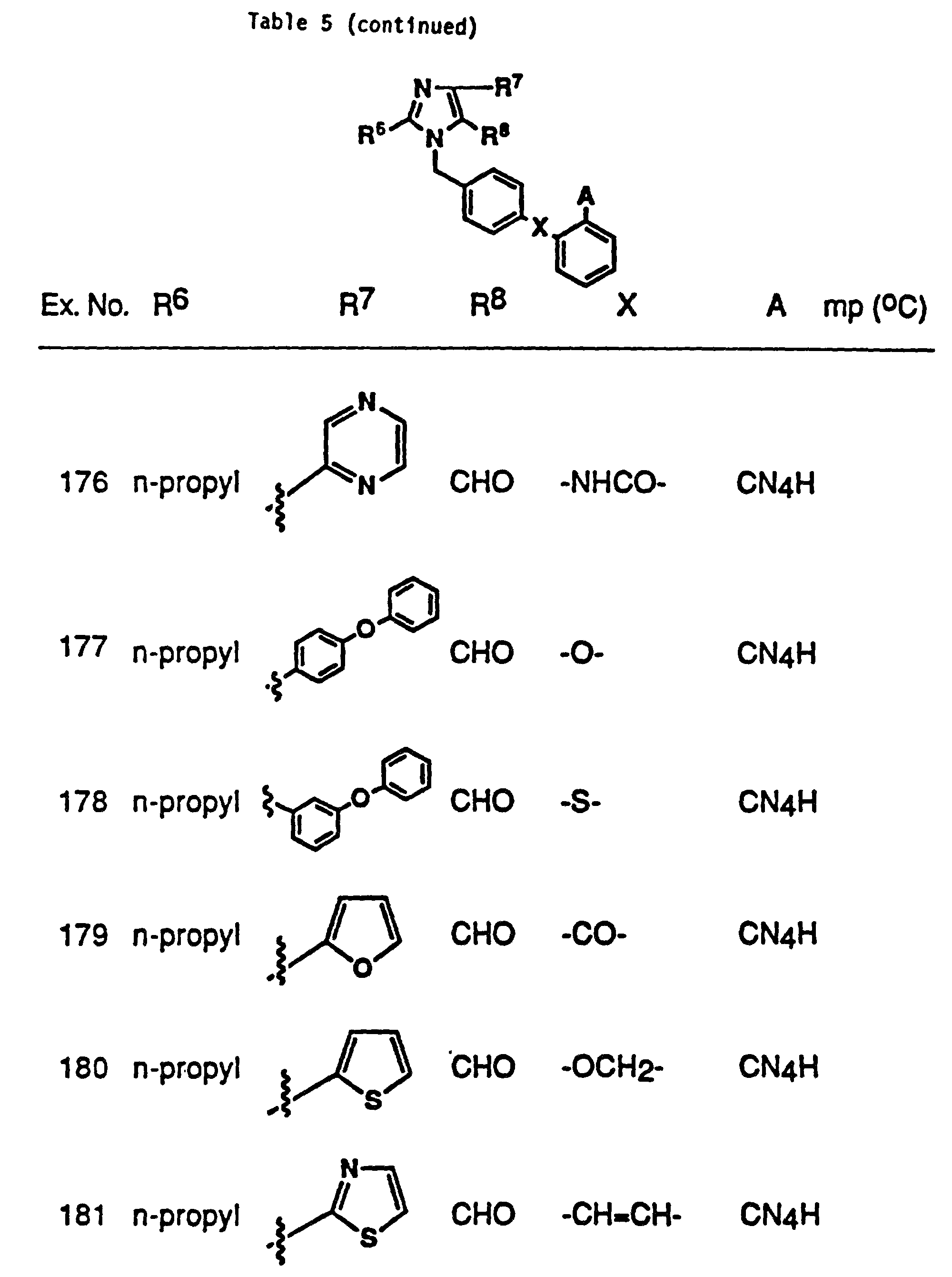

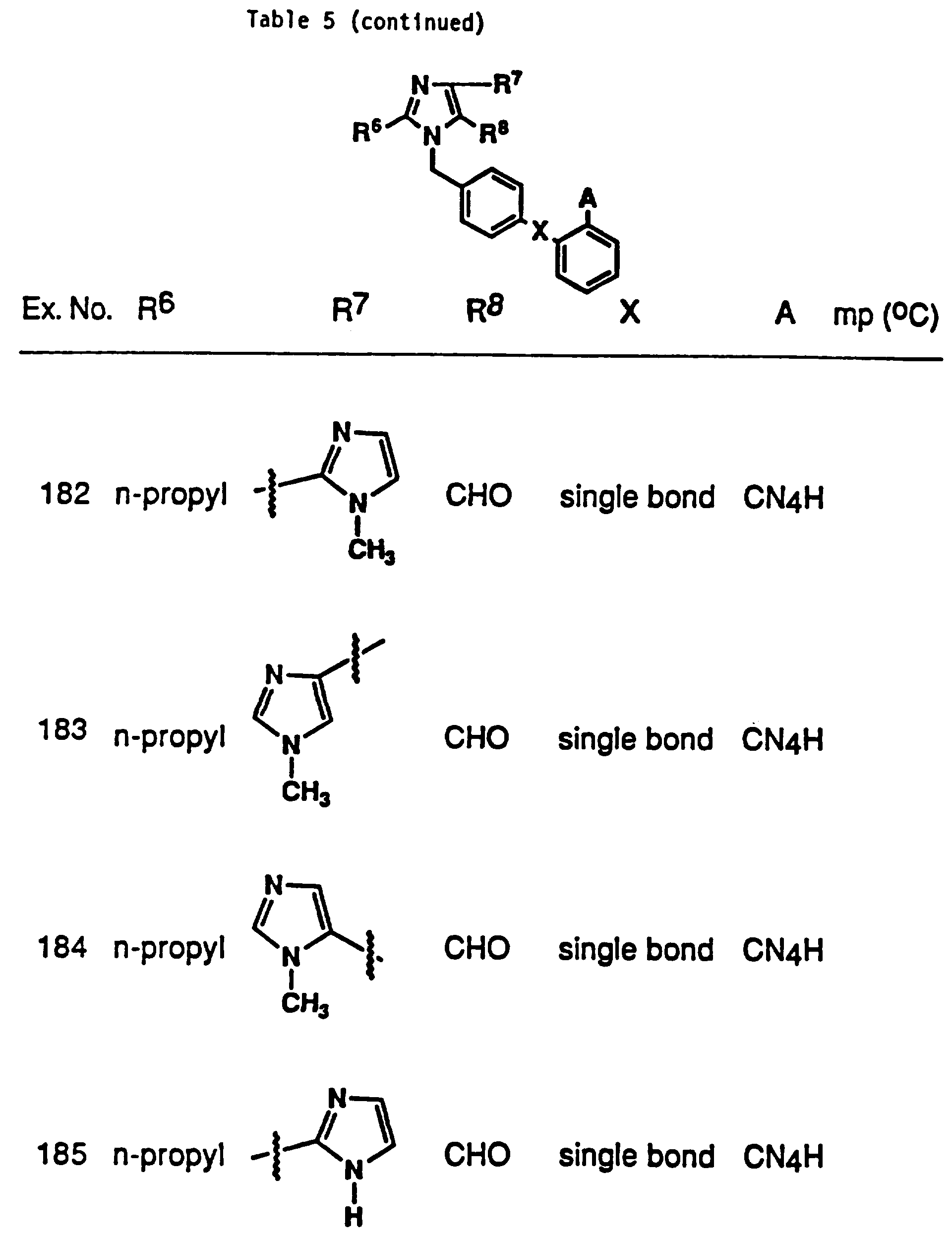

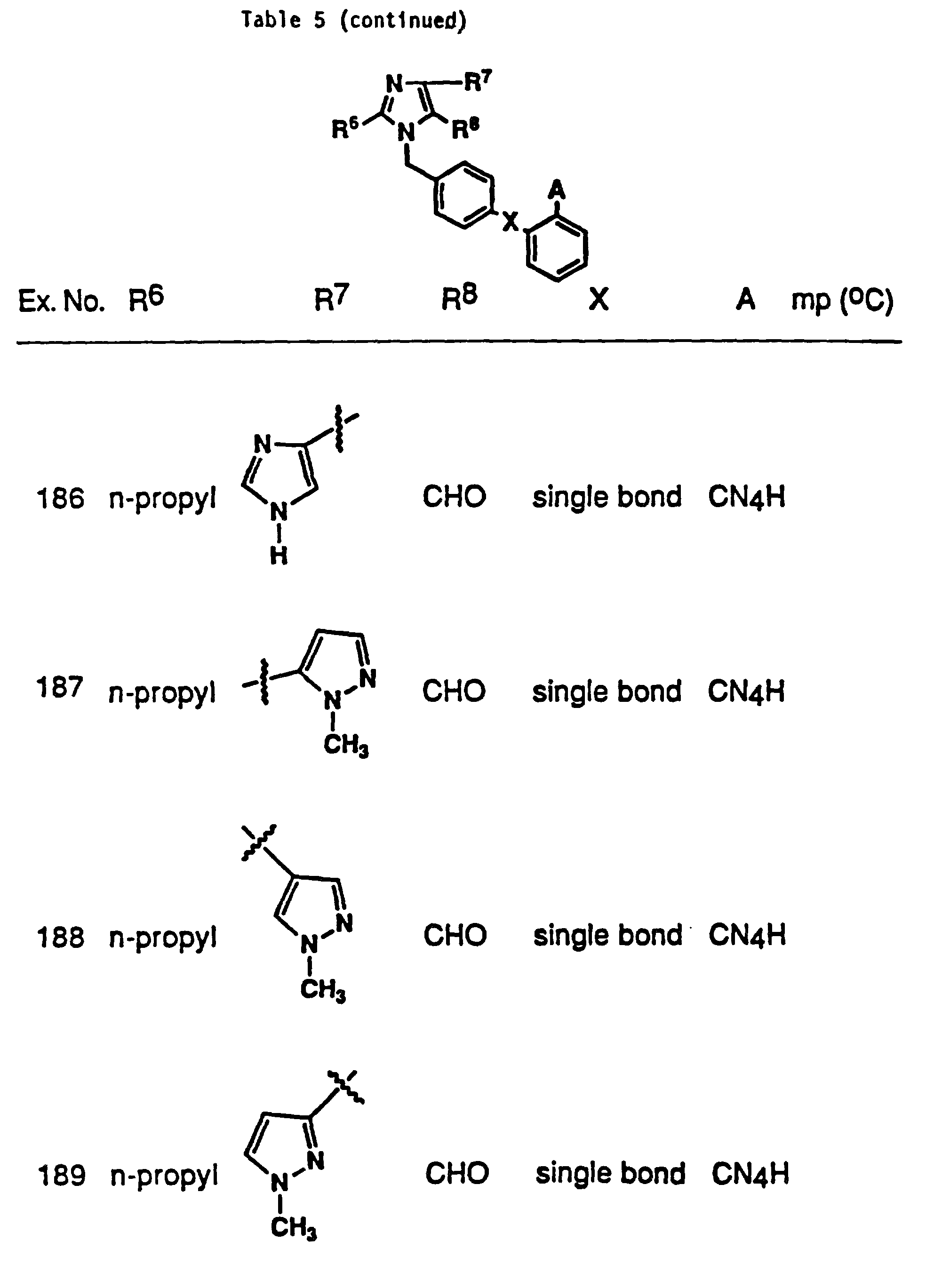

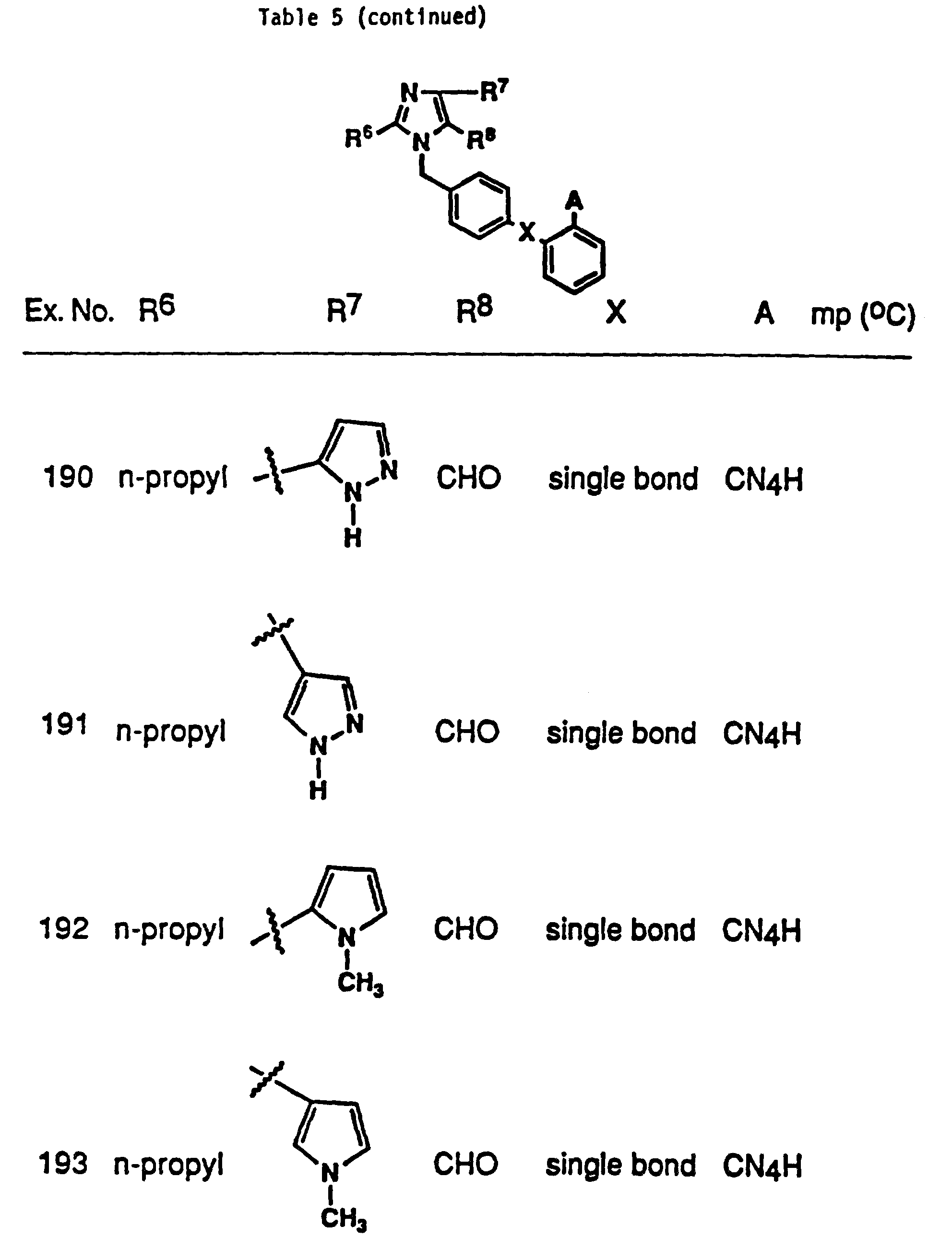

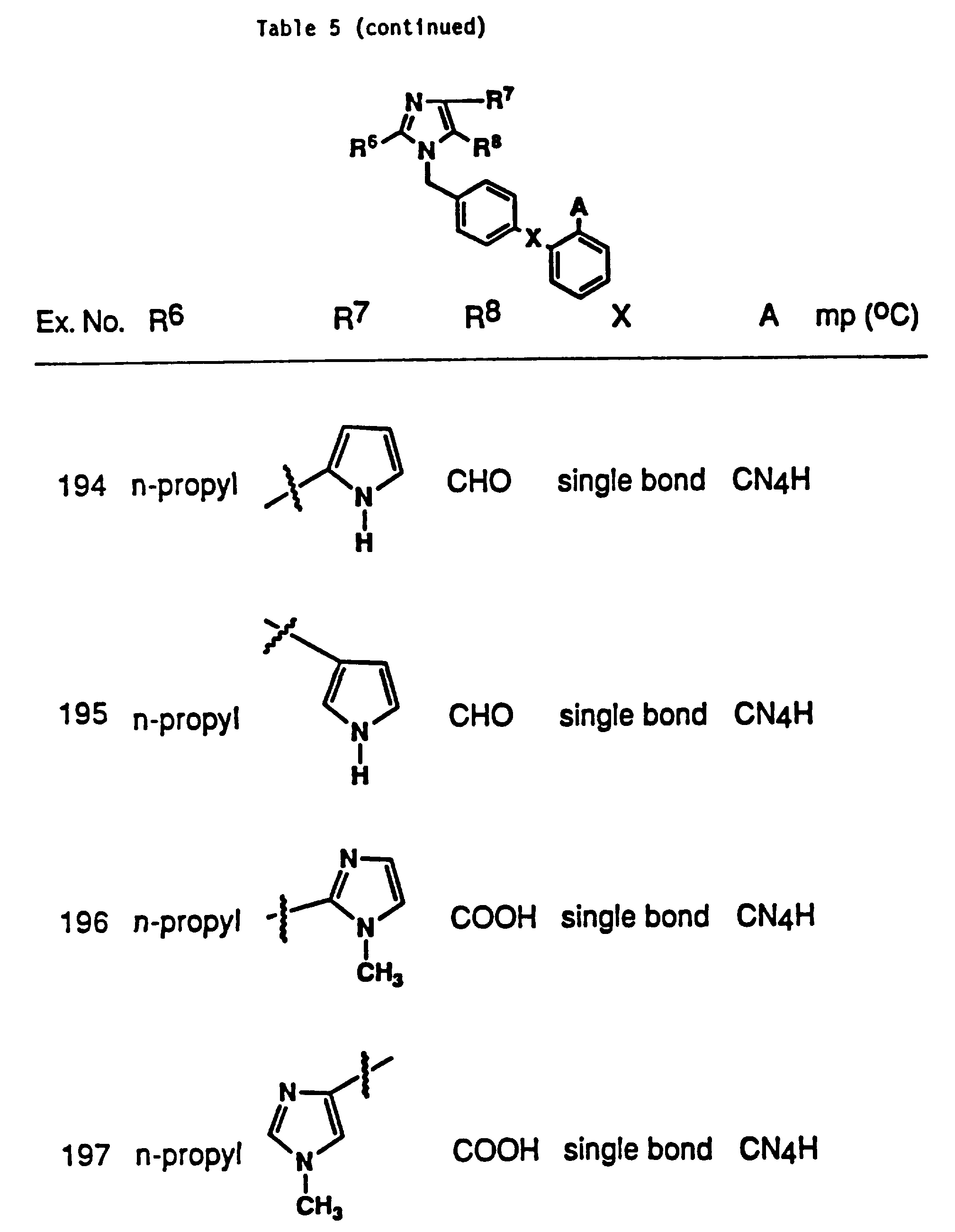

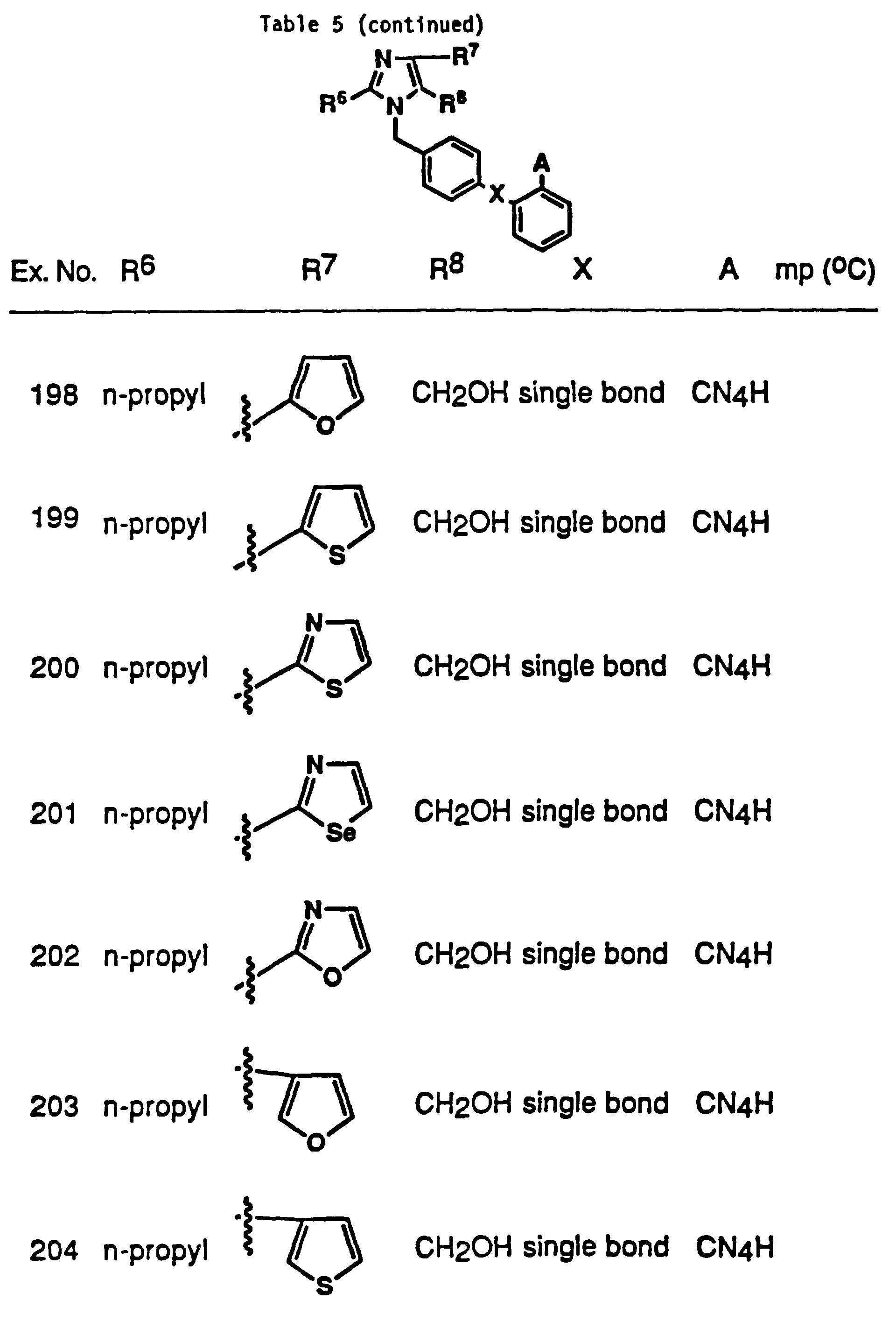

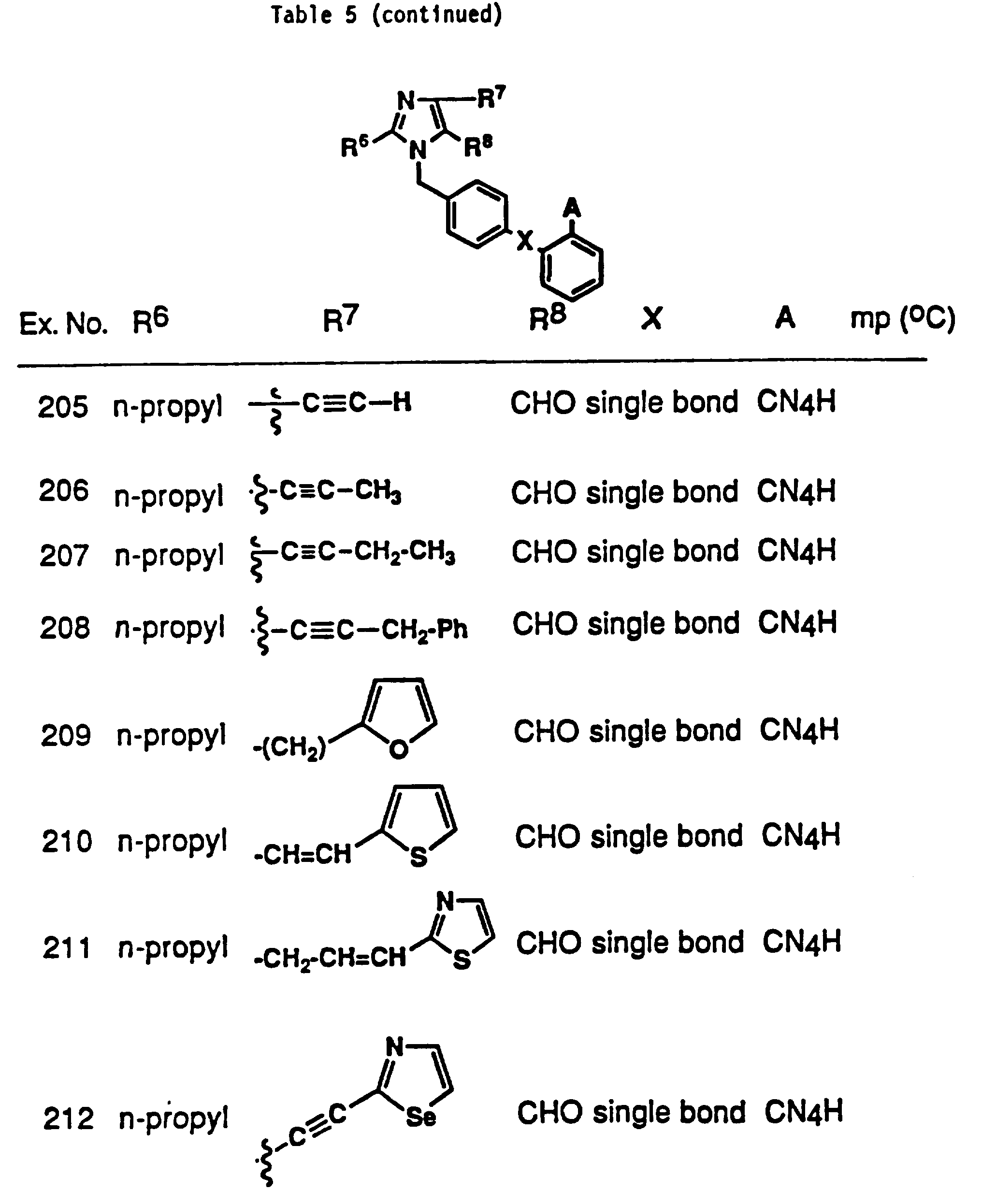

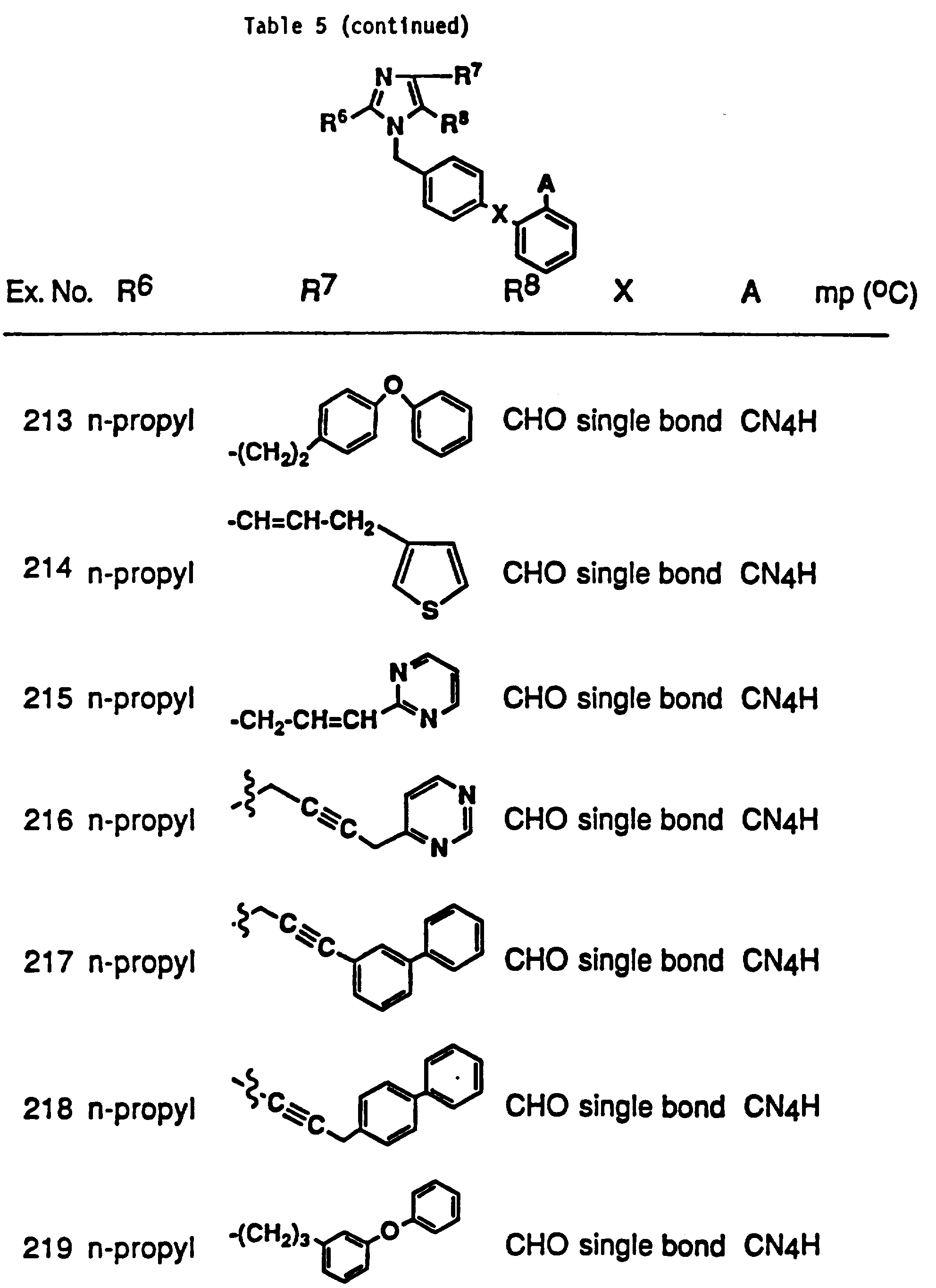

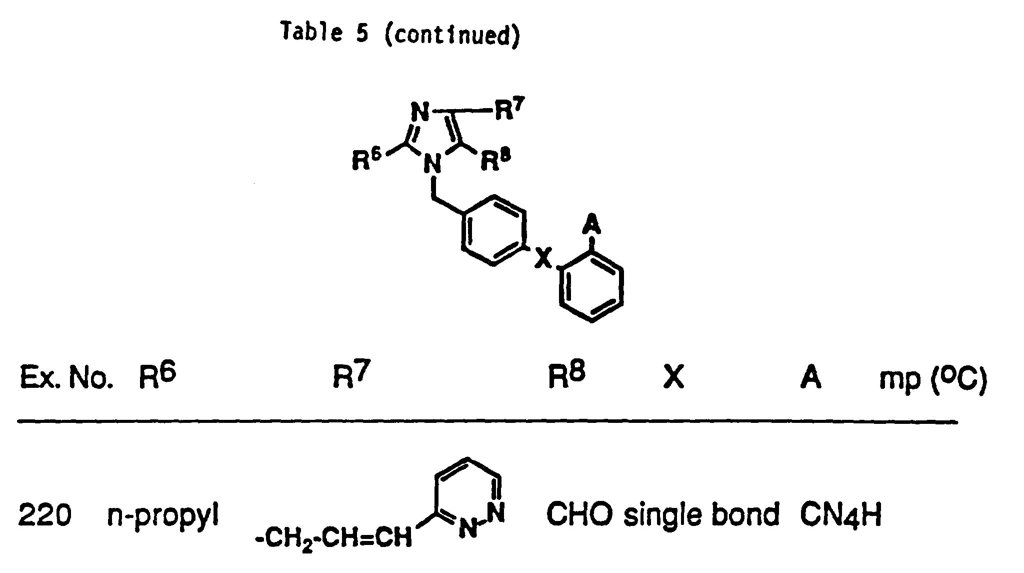

The following compounds in Table 6 were prepared or could be prepared by the procedure in example 1:

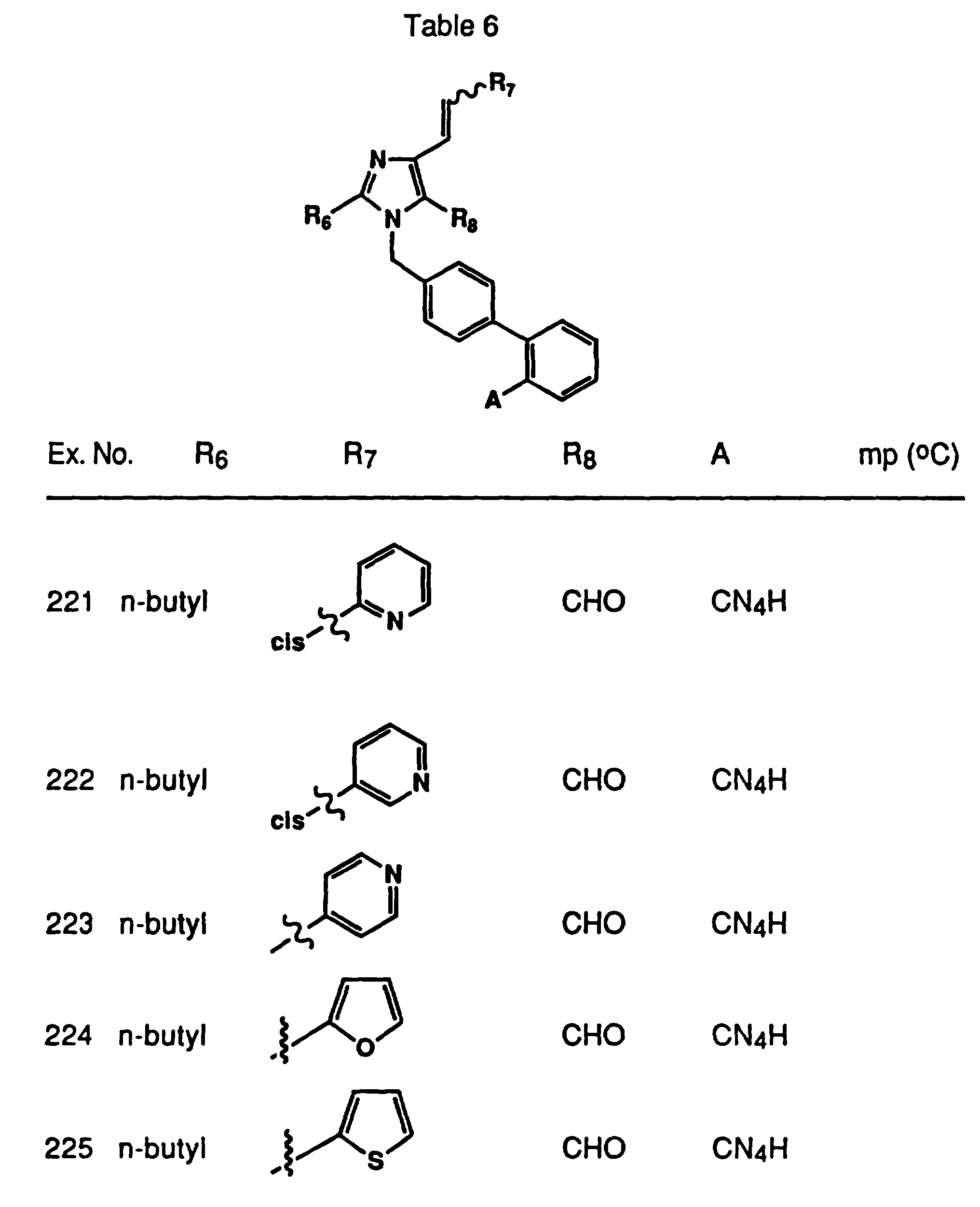

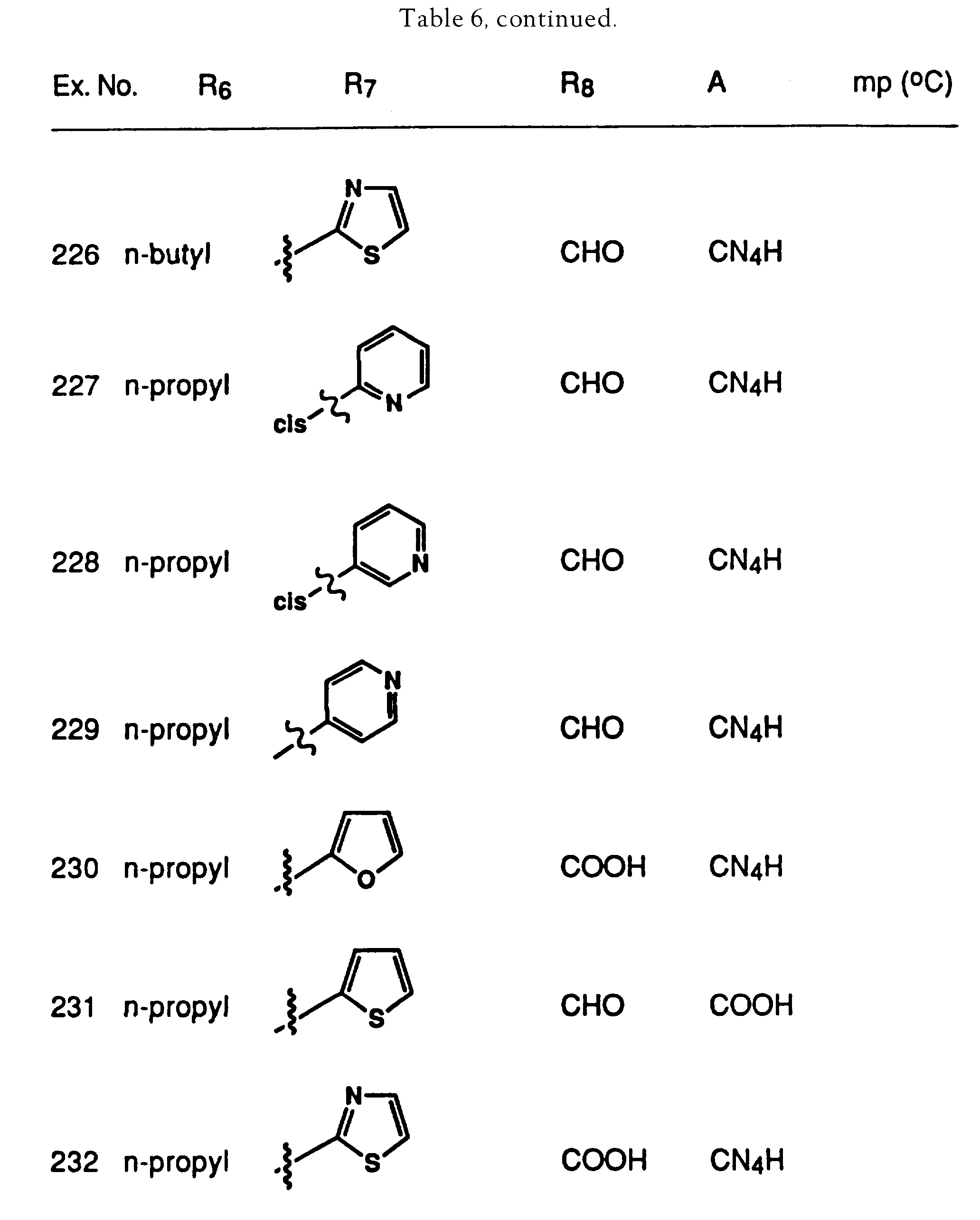

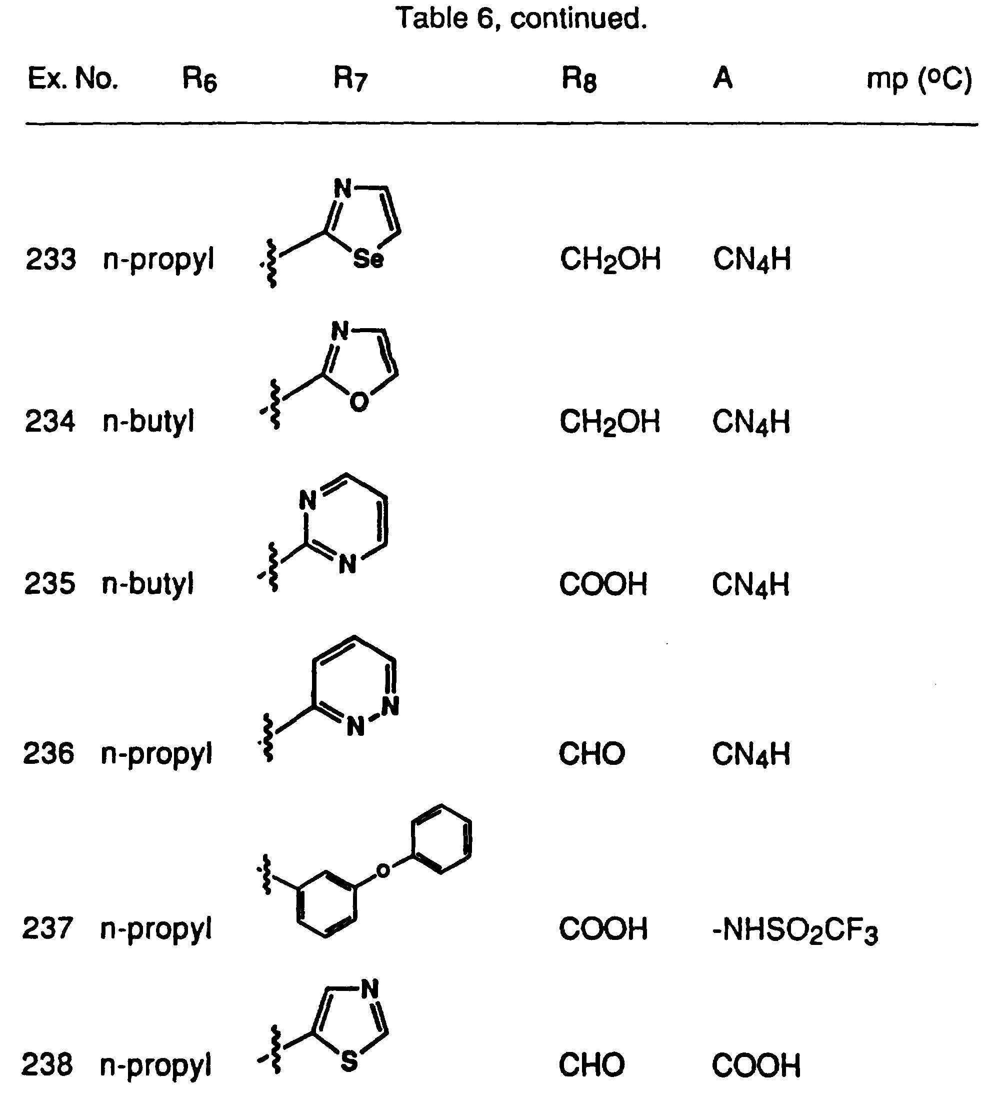

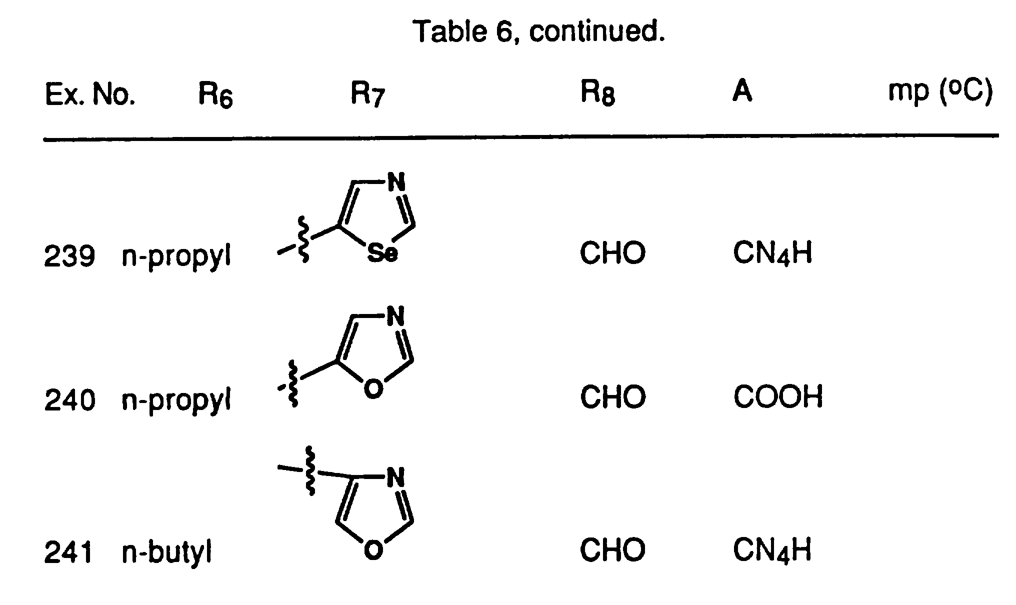

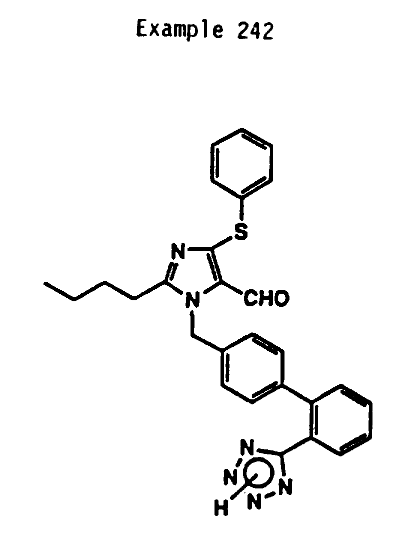

### Preparation of 2-n-Butyl-4-phenylthio-1-[(2'-(1H-tetrazol-5-yl)biphenyl-4-yl)methyl]imidazole-5-carboxyaldehyde

2-n-Butyl-4-chloro-1-[(2'-N-triphenyl-methyl(1H-tetrazol-5-yl)biphenyl-4-yl)methyl]-imidazole-5-carboxyaldehyde (synthesized as described in European Patent Application Number 89100144.8, published 7.19.89) (590 mg, 0.89 mmol, 1 eq), and thiophenol (0.91 mL, 8.9 mmol, 10 eq) were added to a freshly prepared solution of sodium methoxide in methanol (sodium: 205 mg,8.9 mmol, 10 eq; methanol, 40 mL) and the mixture refluxed overnight under N₂. The solvent was removed in vacuo and the residue dissolved in water (50 mL). The pH was adjusted to 10-12 with 10 N NaOH. Gummy solids (trityl group-containing compound) formed which were dissolved by the addition of ethyl ether (50 mL). The layers were separated and the aqueous layer extracted with ethyl ether (2 x 50 mL). The aqueous layer was then extracted with ethyl acetate (6 x 50 mL). The ethyl acetate layers were collected, dried (MgSO₄), and the solvent removed in vacuo to yield a residue which was redissolved in water (50 mL). The pH was adjusted to 1 with conc. HCl. A gummy precipitate containing product formed which was dissolved in ethyl acetate (50 mL). The layers were separated and the aqueous layer was extracted with ethyl acetate (2 x 50 mL). The ethyl acetate layers were collected, dried (MgSO₄), and the solvent removed in vacuo to yield a white glass (200 mg). Crystallization from hot n-butylchloride yielded a white solid (142 mg): mp 1435-145.5°C. NMR (DMSO-d₆) δ 9.82 (s, 1H); 7.80-761 (m, 2H); 7.58 (d, 1H, J=8Hz); 7.52 (d, 1H, J=8Hz); 7.45-7.20 (m, 5H); 7.09 (d, 1H, J=8Hz); 7.03 (d, 2H, J=8Hz); 5.62 (s, 2H); 2.64 (t, 2H, J=7Hz); 1.50 (t of t, 1H, J=7,7Hz); 1.25 (t of q, 2H, J=7,7Hz); 0.80( t, 3H, J=7Hz). Anal. calcd. for CC₂₈H₂₆N₆OS·(H₂O)_{0.4}: C, 67.02; H, 5.38; N, 16.75; S, 6.39. Found: C, 66.90; H, 5.20; N, 16.75; S, 6.00.

Examples 243-253 in Table 7 can be made by procedures described in example 242 and other examples in this patent application and in EP 89100144.8 (published 7.19.89) or by other methods familiar to one skilled in the art.

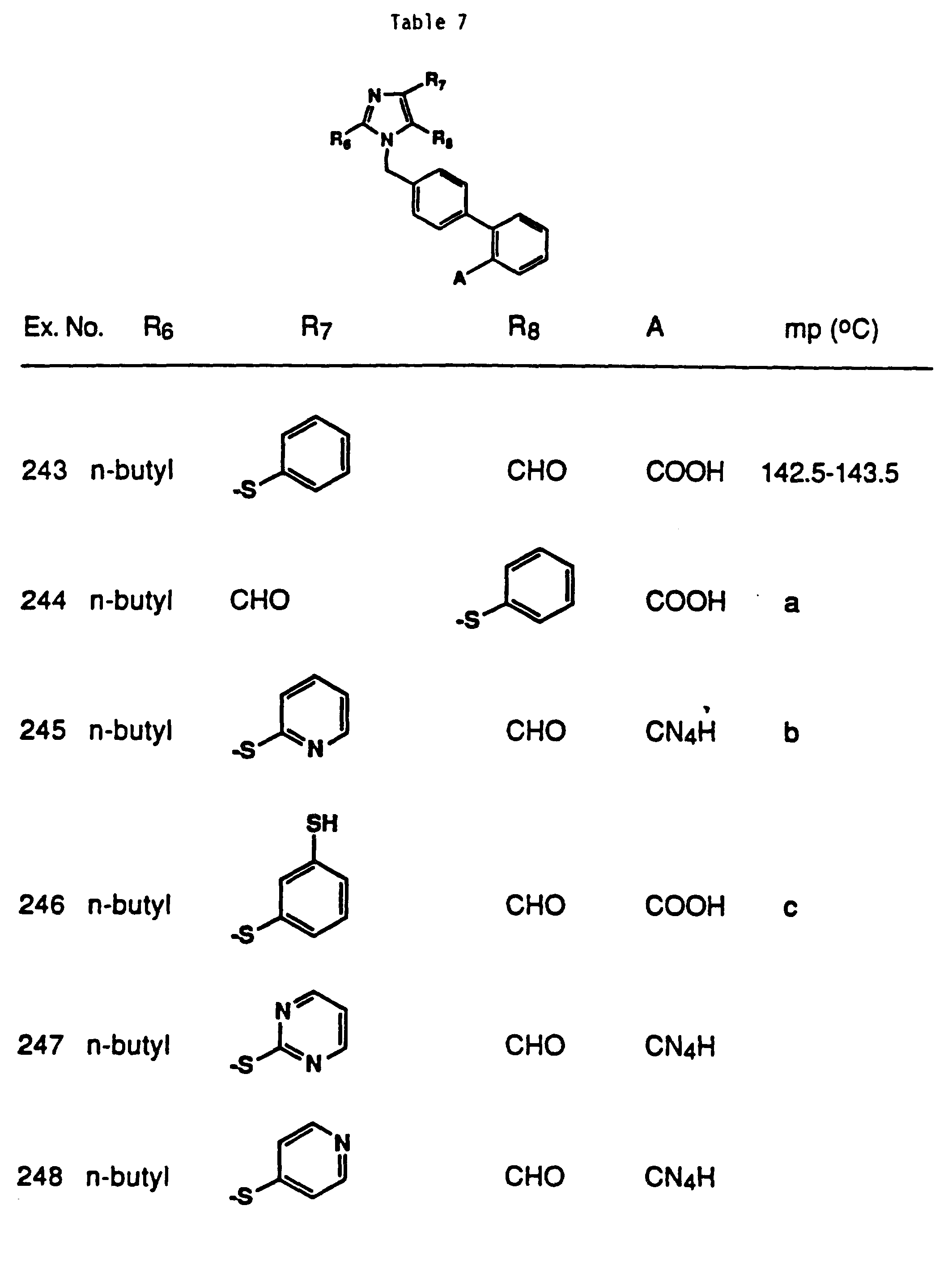

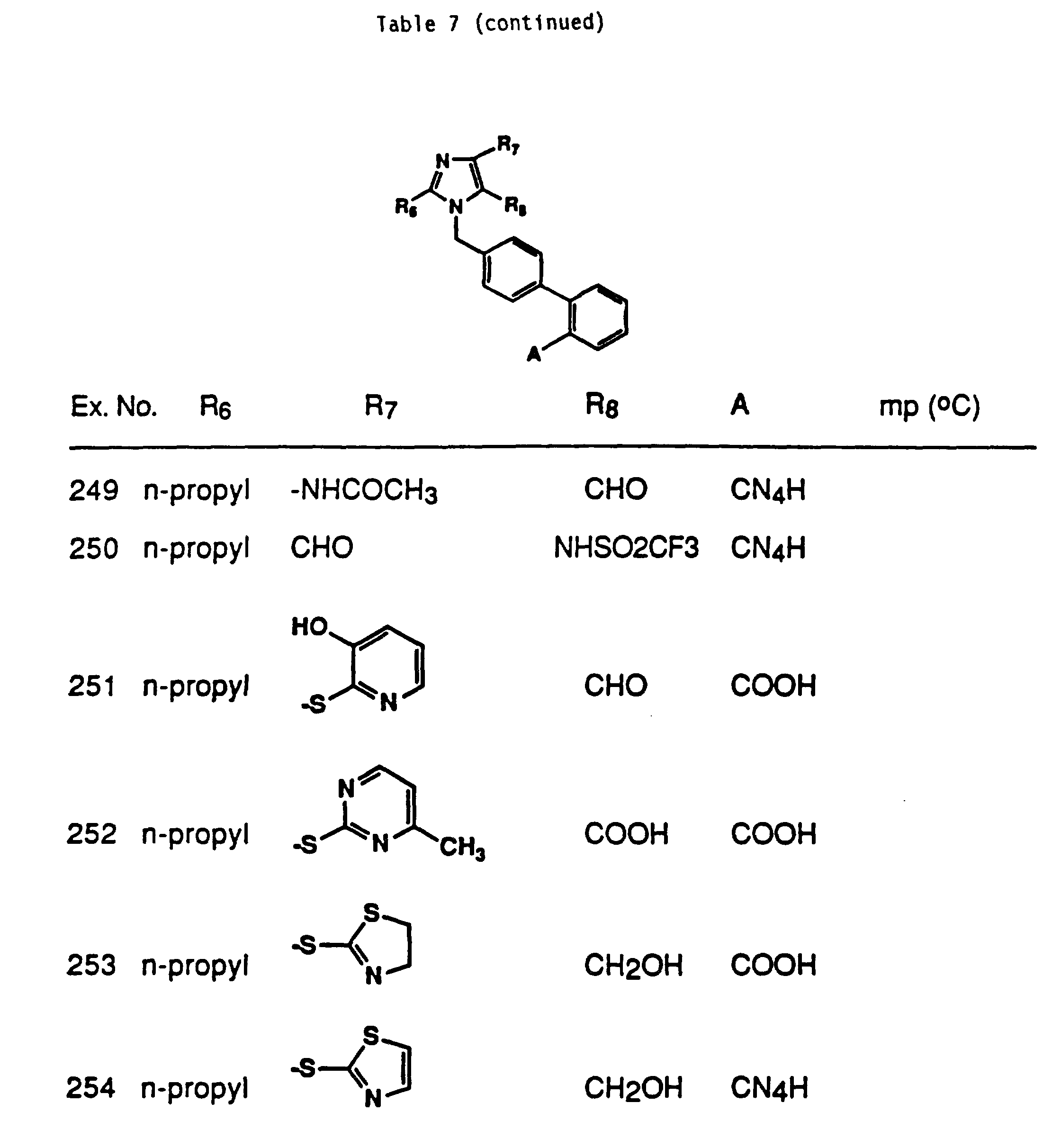

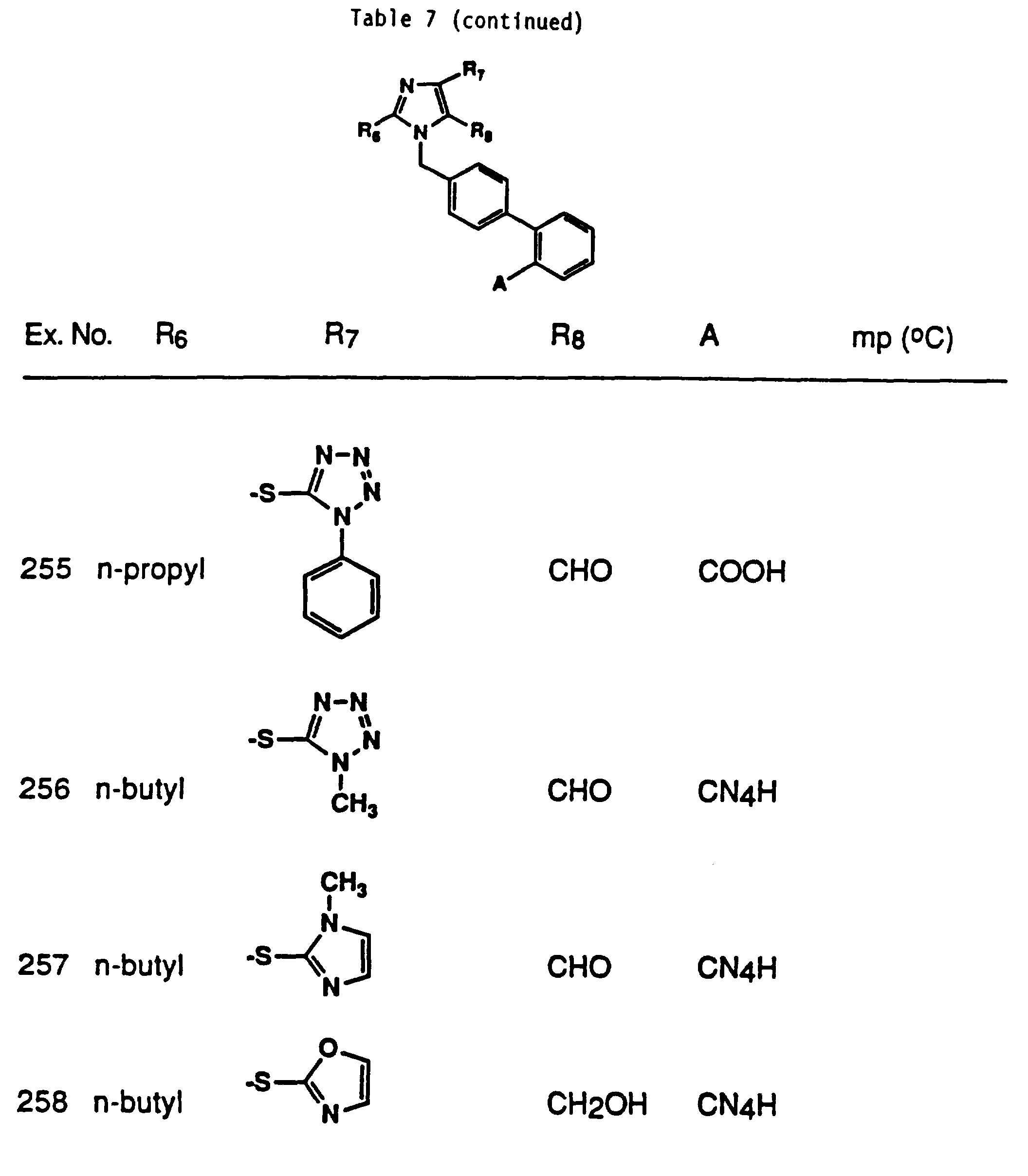

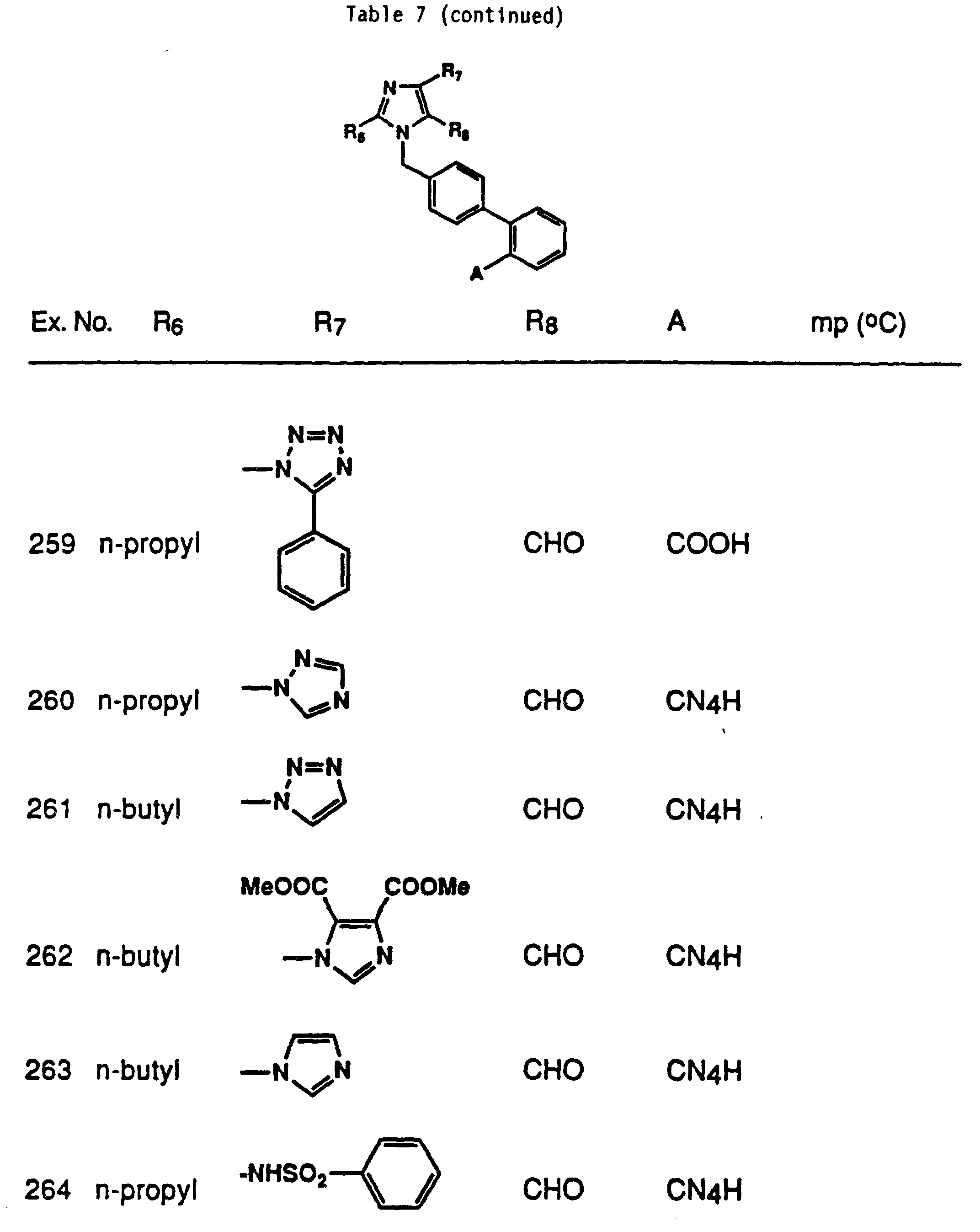

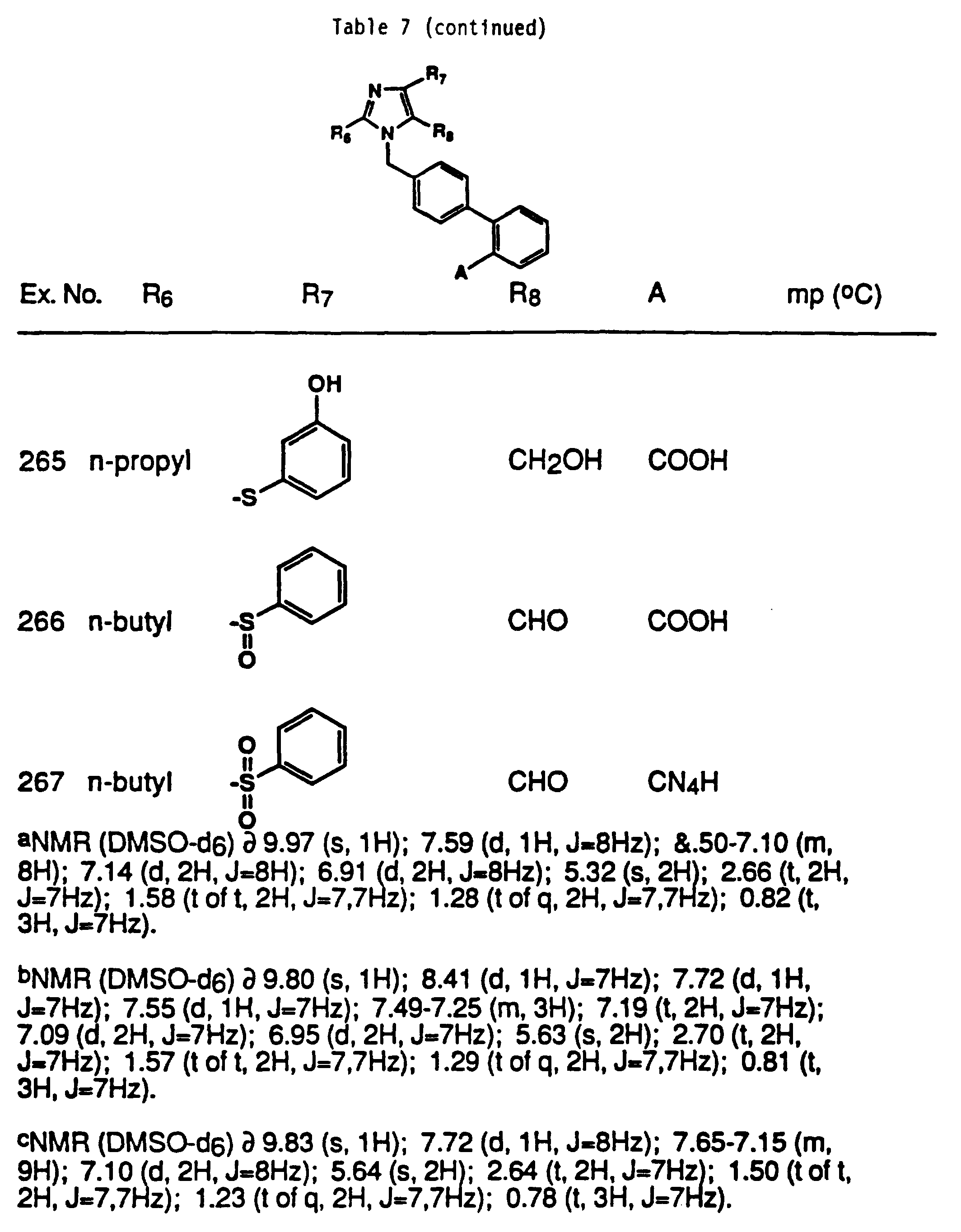

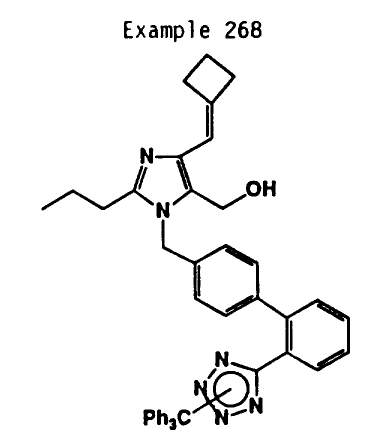

### PART A: Preparation of 2-n-propyl-4-cyclobutylidenyl-5-hydroxymethyl-1-[(2'-(N-triphenylmethyl-(1H-tetrazol-5-yl))biphenyl-4-yl)methyl]imidazole.

(∂-Bromo-n-butyl)triphenylphosphonium bromide (7.42 g, 0.0155 mmol, 2 eq) was suspended in THF (125 mL) and 0.75 M potassium hexamethyldisilazane (41.4 mL, 0.031 mmol, 4 eq) was added at room temperature. The mixture turned blood red. After 0.5 h, 2-n-propyl-5-hydroxymethyl-1-[(2'-(N-triphenylmethyl-(1H-tetrazol-5-yl))biphenyl-4-yl-methyl]imidazole-4-carboxaldehyde (5.00 g, 7.75 mmol, 1 eq) as a slurry in THF was added. The mixture eventually turned into a yellow-orange suspension. After 24 h, the reaction was worked up by adding a little methanol to quench, followed by ethyl acetate and water. The layers were seperated and the organic layer was washed with water (2X) and brine (1X). The organic layer was dried (MgSO₄), and solvent removed in vacuo, and the residue flash chromatographed in 60:40 pentane/ethyl acetate to 100% ethyl acetate to yield 4.12 g (78%) of a white solid: mp 181.5-182.5°C. NMR (DMSO-d₆) δ 7.78 (m,1H); 7.61 (t,1H, J=7Hz); 7.54 (t,1H,J=7Hz); 7.48-7.20 (m,10H); 7.03 (d,2H,J=8Hz); 6.96-6.70 (m,8H); 5.99 (s,1H); 5.13 (s,2H); 4.97 (t,1H,J=7Hz); 4.21 (d,2H,J=7Hz); 3.05 (m,2H); 2.79 (m,2H); 2.31 (t,2H,J=7Hz); 1.98 (m,2H); 1.48 (t of q, 2H,J=7,7Hz); 0.77 (t,3H,J=7Hz). Anal. ((C₄₅H₄₂N₆O·(H₂O)_{0.75}) C, H, N.

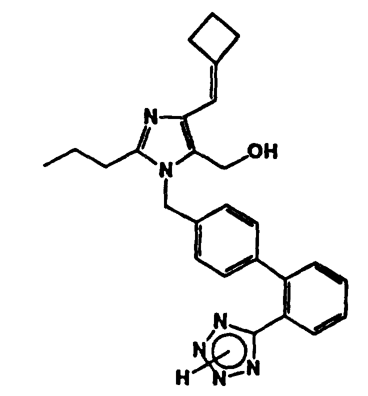

### PART B: Preparation of 2-n-propyl-4-cyclobutylidenyl-5-hydroxymethyl-1-[(2'-(1H-tetrazol-5-yl)biphenyl-4-yl)methyl]imidazole.

2-n-Propyl-4-cyclobutylildenyl-5-hydroxymethyl-1-[(2'-(N-triphenylmethyl-(1H-tetrazol-5-yl))biphenyl-4-yl)methyl]imidazole (1.00 g), methanol (25 mL) and THF (15 mL) were mixed and refluxed for 24 h. The solvents were removed in vacuo and the residue Immediately flash chromatographed quickly in 1:1 pentane/ethyl acetate to 100% isopropanol and eventually to 100% ethanol to yield 320 mg of a light yellow glass: NMR (DMSO-d₆) δ 7.59 (d,1H,J=7Hz); 7.54 (t,1H,J=7Hz); 7.46 (t,1H,J=7Hz); 7.42 (d,1H,J=7Hz); 7.06 (d,2H,J=7Hz); 6.90 (d,2H,J=7Hz); 5.97 (s,1H); 5.17 (s,2H); 4.31 (s,2H); 3.04 (m,2H); 2.77 (m,2H); 2.42 (t,2H,J=7Hz); 1.97 (m,2H); 1.53 (t of q,2H,J=7,7Hz); 0.86 (t 3H,J=7Hz). Anal. (C₂₆H₂₈N₆O) C, H, N.

### Utility

The hormone angiotensin II (AII) produces numerous biological responses (e.g. vasoconstriction) through stimulation of its receptors on cell membranes. For the purpose of identifying compounds such as All antagonists which are capable of interacting with the AII receptor, a ligand-receptor binding assay was utilized for the initial screen. The assay was carried out according to the method described by [Glossmann et al., J. Biol. Chem., 249, 825 (1974)], but with some modifications. The reaction mixture contained rat adrenal cortical microsomes (source of AII receptor) in Tris buffer and 2 nM of ³H-AII with or without potential AII antagonist. This mixture was incubated for 1 hour at room temperature and the reaction was subsequently terminated by rapid filtration and rinsing through glass micro-fibre filter. Receptor-bound ³H-AII trapped in filter was quantitated by scintillation counting. The inhibitory concentration (IC₅₀) of potential AII antagonist which gives 50% displacement of the total specifically bound ³H-AII is a measure of the affinity of such compound for the AII receptor. Compounds of this invention which were tested in this binding assay exhibited IC₅₀ of 10⁻⁵M or less (Table 8).

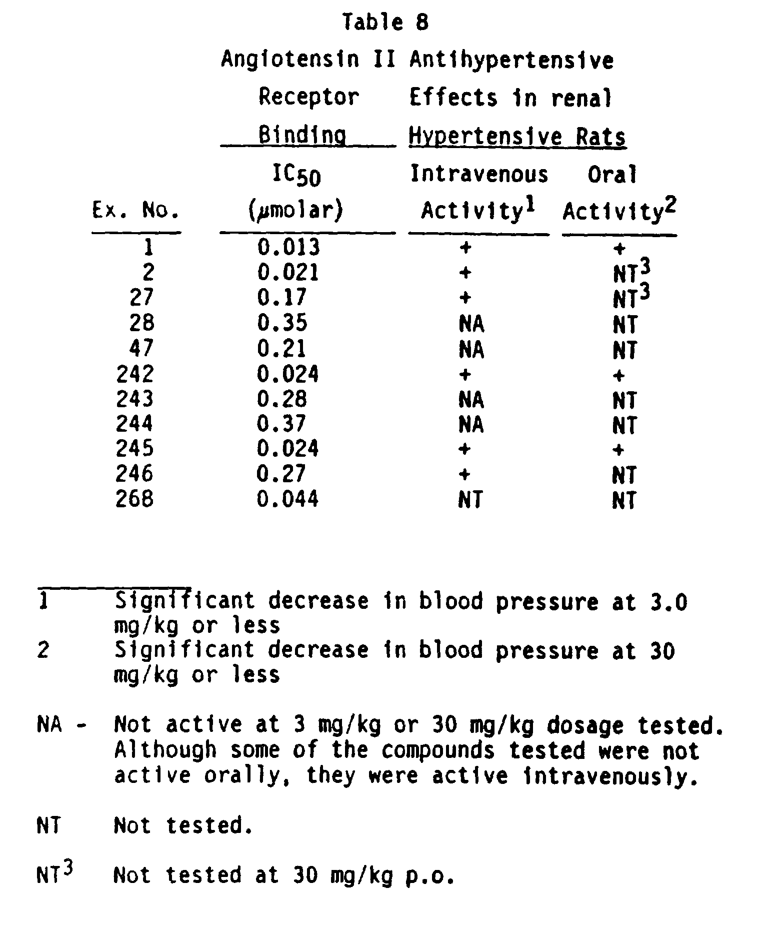

The potential antihypertensive effects of the compounds of this invention may be demonstrated by administering the compounds to awake rats made hypertensive by ligation of the left renal artery [Cangiano et al., J. Pharmacol. Exp. Ther., 208, 310 (1979)]. This procedure increases blood pressure by increasing renin production with consequent elevation of AII levels. Compounds are administered orally at 30 mg/kg and/or intravenously via a cannula in the jugular vein at 3 mg/kg. Arterial blood pressure Is continuously measured directly through a carotid artery cannula and recorded using a pressure transducer and a polygraph. Blood pressure levels after treatment are compared to pretreatment levels to determine the antihypertensive effects of the compounds which were tested. Some compounds of this invention exhibited intravenous activity at 3 mg/kg and some exhibited oral activity at 30 mg/kg (Table 8).

### Dosage Forms

The compounds of this invention can be administered for the treatment of hypertension according to the invention by any means that effects contact of the active ingredient compound with the site of action in the body of a warm-blooded animal. For example, administration can be parenteral, i.e., subcutaneous, intravenous, intramuscular, or intra peritoneal. Alternatively, or concurrently, in some cases administration can be by the oral route.

The compounds can be administered by any conventional means available for use in conjunction with pharmaceuticals, either as individual therapeutic agents or in a combination of therapeutic agents. They can be administered alone, but are generally administered with a pharmaceutical carrier selected on the basis of the chosen route of administration and standard pharmaceutical practice.

For the purpose of this disclosure, a warm-blooded animal is a member of the animal kingdom possessed of a homeostatic mechanism and includes mammals and birds.

The dosage administered will be dependent on the age, health and weight of the recipient, the extent of disease, kind of concurrent treatment, if any, frequency of treatment and the nature of the effect desired. Usually, a dally dosage of active ingredient compound will be from about 1-500 milligrams per day. Ordinarily, from 10 to 100 milligrams per day in one or more applications is effective to obtain desired results. These dosages are the effective amounts both for treatment of hypertension and for treatment of congestive heart failure, i.e., for lowering blood pressure and for correcting the hemodynamic burden on the heart to relieve the congestion.

The active ingredient can be administered orally in solid dosage forms, such as capsules, tablets, and powders, or in liquid dosage forms, such as elixirs syrups, and suspensions. It can also be administered parenterally, in sterile liquid dosage forms.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and If necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propylparaben, and chlorobutanol.

Suitable pharmaceutical carriers are described in Remington's Pharmaceutical Sciences, A. Osol, a standard reference text in this field.

Useful pharmaceutical dosage-forms for administration of the compounds of this invention can be illustrated as follows:

### Capsules

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

### Soft Gelatin Capsules

A mixture of active ingredient in a digestible oil such as soybean oil, cottonseed oil or olive oil is prepared and injected by means of a positive displacement pump into gelatin to form soft gelatin capsules containing 100 milligrams of the active ingredient. The capsules are washed and dried.

### Tablets

A large number of tablets are prepared by conventional procedures so that the dosage unit is 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

### Injectable

A parenteral composition suitable for administration by injection is prepared by stirring 1.5% by weight of active ingredient in 10% by volume propylene glycol. The solution is made to volume with water for injection and sterilized.

### Suspension

An aqueous suspension is prepared for oral administration so that each 5 milliliters contain 100 milligrams of finely divided active ingredient, 100 milligrams of sodium carboxymethyl cellulose, 5 milligrams of sodium benzoate, 1.0 grams of sorbitol solution, U.S.P., and 0.025 milliliters of vanillin.

The same dosage forms can generally be used when the compounds of this invention are administered stepwise in conjunction with another therapeutic agent. When the drugs are administered in physical combination, the dosage form and administration route should be selected for compatibility with both drugs. Suitable dosages, dosage forms and administration routes are illustrated in the following tables.

| Examples of NSAID's that can be combined with AII blockers of this invention : | | | |
|---|---|---|---|
| Drug | Dose (mg) | Formulation | Route |
| Indomethacin | 25 | Tablet | Oral |
| | (2/3 times daily) | | |
| Meclofenamate | 50-100 | Tablet | Oral |
| | (2/3 times daily) | | |
| Ibuprofen | 300-400 | Tablet | Oral |
| | (3/4 times daily) | | |
| Piroxicam | 10-20 | Tablet | Oral |
| | (1/2 times daily) | | |
| Sulindac | 150-200 | Tablet | Oral |
| | (2 times daily) | | |
| Azapropazone | 200-500 | Tablet | Oral |
| | (3/4 times daily) | | |

| Examples of diuretics that can be combined with AII blockers of this invention : | | | |
|---|---|---|---|
| Drug | Dose (mg) | Formulation | Route |
| Benzothiadizides | 25-100 (daily) | Tablet | Oral |
| (e.g. hydrochlorothiazide) | | | |
| | | | |
| Loop diuretics | 50-80 (daily) | Tablet | Oral |
| (e.g. furosemide) | | | |

When used with an NSAID, the dosage of AII blockers will generally be the same as when the AII blocker is used alone, i.e., 1-500 milligrams per day, ordinarily from 10 to 100 milligrams per day in one or more applications. When used with diuretics, the initial dose of All blocker can be less, e.g., 1-100 milligrams per day and for the more active compounds 1-10 milligrams per day.

It is expected that the compounds of this invention will also be useful in the treatment of chronic renal failure.

## Claims (Claims for the following Contracting State(s): AT, BE, CH, DE, DK, FR, GB, IT, LI, LU, NL, SE)

1. An antihypertensive compound of the formula: 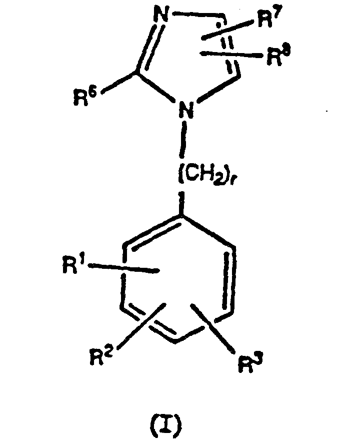 wherein
R¹ is 4 - CO₂H ; 4-CO₂R^{9:} 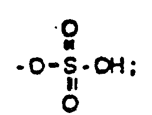 -SO₃H ; - C(CF₃)₂OH ; 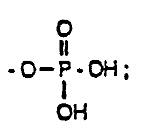 -PO₃H₂ ; 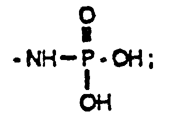 4-NHSO₂CH₃: 4-NHSO₂CF₃; -CONHOR^{12;} -SO₂NH₂; 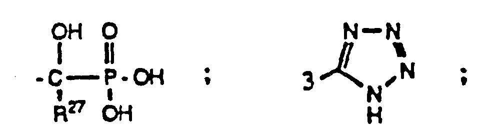 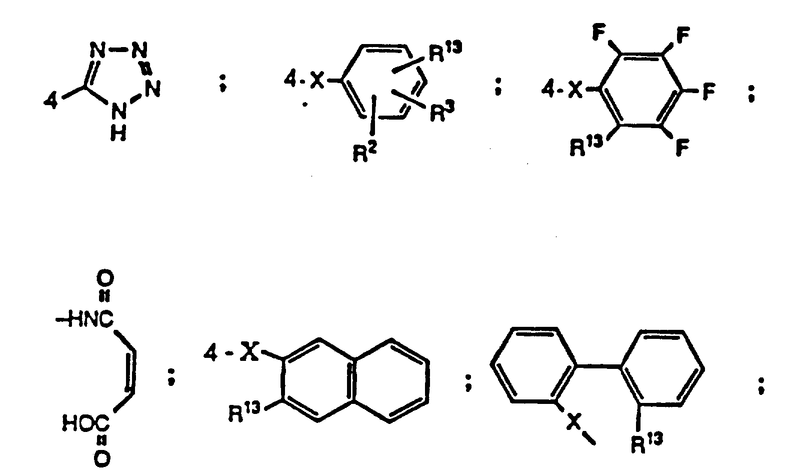 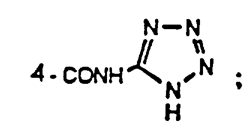 4-CONHNHSO₂CF₃; 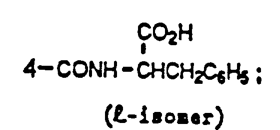 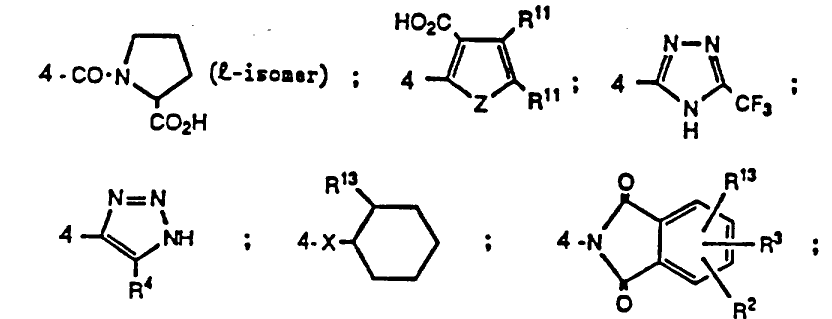 or 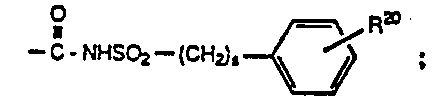
R is H; Cl; Br; I; F; NO₂; CN; alkyl of 1 to 4 carbon atoms; acyloxy of 1 to 4 carbon atoms; alkoxy of 1 to 4 carbon atoms; CO₂H; CO₂R⁹; NHSO₂CH₃; NHSO₂CF₃; CONHOR¹; SO₂NH₂ ; 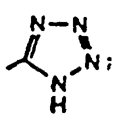 phenyl; or furyl;
R³ is H; Cl, Br, I or F; alkyl of 1 to 4 carbon atoms or alkoxy of 1 to 4 carbon atoms;
R⁴ is CN, NO₂ or CO₂R¹¹;
R⁵ is H, alkyl of 1 to 6 carbon atoms, cycloalkyl of 3 to 6 carbon atoms, alkenyl or alkynyl of 2 to 4 carbon atoms;
R⁶ is alkyl of 2 to 10 carbon atoms, alkenyl or alkynyl of 3 to 10 carbon atoms or the same groups substituted with F or CO₂R¹⁴; cycloalkyl of 3 to 8 carbon atoms, cycloalkylalkyl of 4 to 10 carbon atoms; cycloalkylalkenyl or cycloalkylalkynyl of 5 to 10 carbon atoms; (CH₂)ₛZ(CH₂)ₘR⁵ optionally substituted with F or CO₂R¹⁴; benzyl or benzyl substituted on the plienyl ring with 1 or 2 halogens, alkoxy of 1 to 4 carbon atoms, alkyl of 1 to 4 carbon atoms or nitro;
R⁷ is vinyl; cycloalkylidenyl; alkynyl of 2-10 carbon atoms; phenylalkynyl where the alkynyl portion is 2-6 carbon atoms; heteroaryl selected from 2- and 3-thienyl, 2- and 3-furyl, 2-, 3-, and 4-pyridyl, 2-pyrazinyl, 2-, 4-, and 5-pyrimidinyl, 3- and 4-pyridazinyl, 2-, 4- and 5-thiazolyl, 2-, 4-, and 5-selenazolyl, and 2-, 4-, and 5-oxazolyl, 2- or 3-pyrrolyl, 3-, 4- or 5-pyrazolyl, 2-, 4- or 5-imidazolyl; o-, m- or p-biphenylyl; o-, m- or p-phenoxyphenyl; 2-oxazollnyl; 2-thiazolinyl; substituted phenylalkynyl, heteroaryl, biphenylyl or phenoxyphenyl as defined above substituted with 1 or 2 substituents selected from halogen, hydroxy, mercapto, alkoxy of 1-5 carbon atoms, alkyl of 1-5 carbon atoms, -NO₂, -CN, -CF₃, -COR¹⁶, -CH₂OR¹⁷, -NHCOR¹⁷, -CONR¹⁸R¹⁹, S(O)ᵣR¹⁷, and SO₂NR¹⁸R¹⁹; pyrrolyl, pyrazolyl or imidazolyl as defined above substituted on ring nitrogen with alkyl of 1-5 carbon atoms, phenyl or benzyl; or substituted alkyl, alkenyl, or alkynyl of 1 to 10 carbon atoms substituted with a substituted or unsubstituted heteroaryl, biphenylyl or phenoxyphenyl group as defined above; -S(O)ᵣ-heteroaryl, -S-(O)ᵣ-biphenylyl, -S(O)ᵣ-phenoxyphenyl, -S-tetrazole, -S(O)ᵣR¹⁷, -NR¹⁸R¹⁹, -NR¹⁸-heteroaryl, -NR¹⁸-phenyl, -NR¹⁸-biphenylyl, -NR¹⁸-phenoxyphenyl, -N-phthalimido, -NH-SO₂-phenoxyphenyl, -NH-SO₂-heteroaryl, -NH-SO₂-biphenylyl, -NH-SO₂-R¹⁷, and -S-(C=0)-R¹⁷, N-imidazolyl, N-1,2,3-triazolyl, N-1,2,4-triazolyl, where heteroaryl is a heterocycle defined in the scope of R⁷ and where the phenyl group in R¹⁷ of -S-(O)ᵣR¹⁷, the N-imidazolyl, N-1,2,3-triazolyl, and N-1,2,4-triazolyls may be substituted with one or two substituents as described above for heteroaryl;
R⁸ is 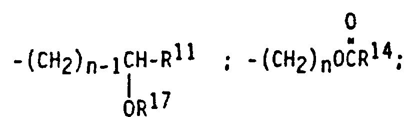 -(CH₂)ₙSR¹⁵; 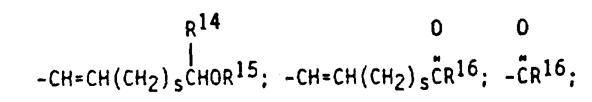 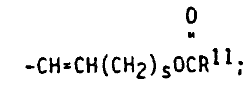 -(CH₂)ₘ-tetrazolyl; 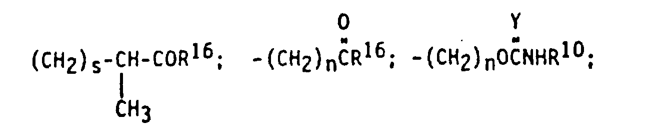 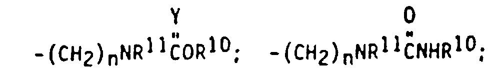 -(CH₂)ₙNR¹¹SO₂R¹⁰; 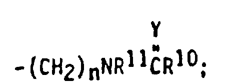
R9 is 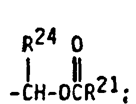
R¹⁰ is alkyl of 1 to 6 carbon atoms or perfluoroalkyl of 1 to 6 carbon atoms, 1-adamantyl, 1-naphthyl, 1-(1-naphthyl)ethyl, or (CH)ₚC₆H₅;
R¹¹ is H, alkyl of 1 to 6 carbon atoms, cycloalkyl of 3 to 6 carbon atoms, phenyl or benzyl;
R¹ is H, methyl or benzyl;
R¹³ is -CO₂H; -CO₂R⁹; -CH₂CO₂H, -CH₂CO₂R⁹; or 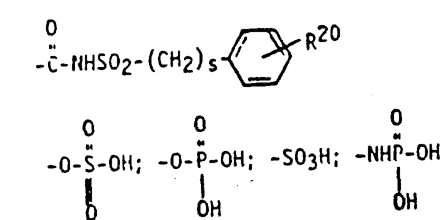 -PO₃H₂; -C(CF₃)₂OH; -NHSO₂CH₃; -NHSO₂CF₃; -NHCOCF₃; -CONHOR¹; -SO₂NH₂; 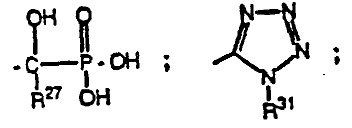 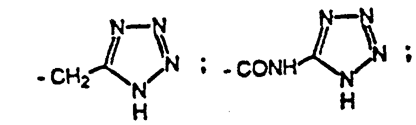 -CONHNHSO₂CF₃ ; 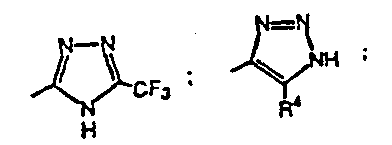
R¹⁴ is H, alkyl or perfluoroalkyl of 1 to 8 carbon atoms, cycloalkyl of 3 to 6 carbon atoms, phenyl or benzyl;
R¹⁵ is H, alkyl of 1 to 6 carbon atoms, cycloalkyl of 3 to 6 carbon atoms, phenyl, benzyl, acyl of 1 to 4 carbon atoms, phenacyl;
R¹⁶ is H, alkyl of 1 to 6 carbon atoms, cycloalkyl of 3 to 6 carbon atoms, (CH₂)ₚC₆H₅, OR¹⁷, or NR¹⁸R¹⁹;
R¹⁷ is H, alkyl of 1 to 6 carbon atoms, cycloalkyl of 3 to 6 carbon atoms, phenyl or benzyl;
R¹⁸ and R¹⁹ independently are H, alkyl of 1 to 4 carbon atoms, phenyl, benzyl, α-methylbenzyl, or taken together with the nitrogen form a ring of the formula 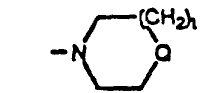 Q is NR⁰, O or CH₂;
R⁰ is H, alkyl of 1-4 carbon atoms, or phenyl;
R¹ is alkyl of 1 to 6 carbon atoms, -NRR³, or 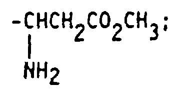
R and R³ independently are H, alkyl of 1 to 6 carbon atoms, benzyl, or are taken together as (CH₂)ᵤ where u is 3-6;
R⁴ is H, CH₃ or -C₆H₅;
R⁵ is NR⁷R⁸, OR⁸, NHCONH₂, NHCSNH₂, 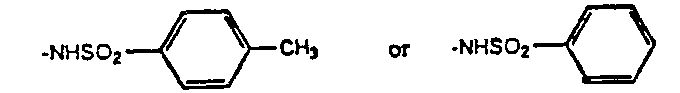
R⁶ is hydrogen, alkyl with from 1 to 6 carbon atoms, benzyl, or allyl;
R⁷ and R⁸ are independently hydrogen, alkyl with from 1 to 5 carbon atoms, or phenyl;
R⁹ and R³⁰ are independently alkyl of 1-4 carbon atoms or taken together are -(CH₂)_{q}-;
R³¹ is H, alkyl of 1 to 4 carbon atoms, -CH₂CH=CH₂ or -CH₂C₆H₄R³;
R³ is H, NO₂, NH₂, OH or OCH₃;
X is a carbon-carbon single bond, -CO-, -CH₂-, -O-, -S-, -NH-, 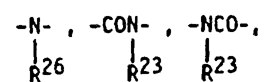 -OCH₂-, -CH₂O-, -SCH₂-, -CH₂S-, -NHC(R⁷)(R⁸)-, -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 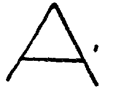 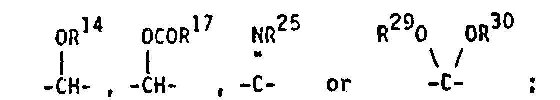
Y is O or S;
Z is O, NR¹¹, or S;
m is 1 to 5;
n is 1 to 10;
p is 0 to 3;
q is 2 to 3;
r is 0 to 2;
s is 0 to 5;
t is 0 or 1;
or a pharmaceutically acceptable salt thereof;
provided that:
(1) the R¹ group is not in the ortho position;
(2) when R¹ is 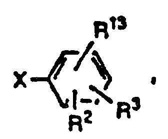 X is a single bond, and R¹³ is CO₂H, or 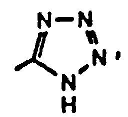 then R¹³ must be in the ortho or meta position; or when R¹ and X are as above and R¹³ is NHSO₂CF₃ or NHSO₂CH₃, R¹³ must be ortho;
(3) when R¹ is 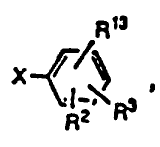 and X is other than a single bond, then R¹³ must be ortho except when X = NR³CO and R¹³ is NHSO₂CF₃ or NHSO₂CH₃, then R¹³ must be ortho or meta;
(4) when R¹ is 4-CO₂H or a salt thereof, R⁶ cannot be S-alkyl;
(5) when R¹ is -4-CO₂H or a salt thereof, the substituent on the 4-position of the imidazole cannot be CH₂OH, CH₂OCOCH₃, or CH₂CO₂H;
(6) when R¹ is 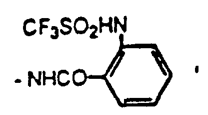 R⁶ is not methoxybenzyl;
(7) the R⁶ group is not 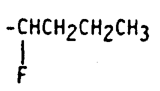 or CH₂OH;

2. A compound of Claim 1 having the formula: 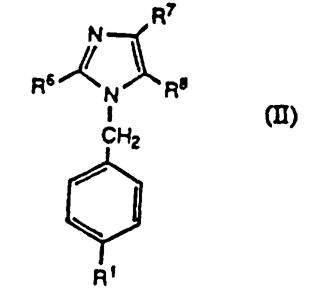 wherein
R¹ is -CO₂H ; -NHSO₂CF₃ ; 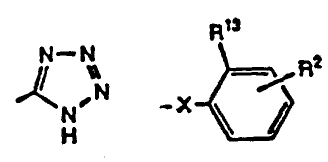 or 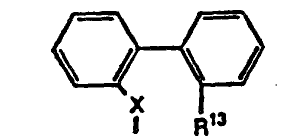
R⁶ is alkyl of 3 to 10 carbon atoms, alkenyl of 3 to 10 carbon atoms, alkynyl of 3 to 10 carbon atoms, cycloalkyl of 3 to 8 carbon atoms, benzyl substituted on the phenyl ring with up to two groups selected from alkoxy of 1 to 4 carbon atoms, halogen, alkyl of 1 to 4 carbon atoms, and nitro;
R⁸ is -(CH₂)ₘ-tetrazolyl, -(CH₂)ₙOR¹¹; 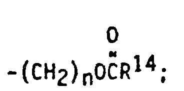 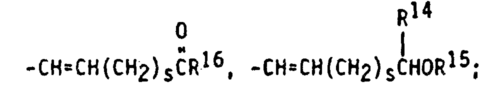 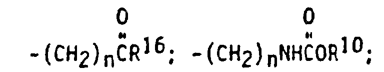 -(CH₂)ₙNHSO₂R¹⁰; or 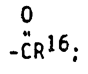
R¹³ is -CO₂H, -CO₂R⁹, NHSO₂CF₃; SO₃H; or 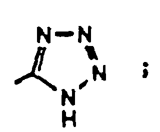
R¹⁶ is H, alkyl of 1 to 5 carbon atoms, OR¹⁷, or NR¹⁸R¹⁹;
X is carbon-carbon single bond, -CO-, 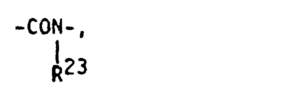 -CH₂CH₂-, 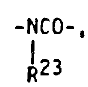 -OCH₂-, -CH₂O-, -O-, -SCH₂-, -CH₂S-, -NHCH₂-, -CH₂NH- or -CH=CH-;
or a pharmaceutically acceptable salt thereof.

3. A compound of Claim 2 wherein:
R is H, alkyl of 1 to 4 carbon atoms, halogen, or alkoxy of 1 to 4 carbon atoms;
R⁶ is alkyl, alkenyl or alkynyl of 3 to 7 carbon atoms;
R⁷ is heteroaryl selected from 2- and 3-thienyl, 2- and 3-furyl, 2-, 3-, and 4-pyridyl, or p-biphenylyl 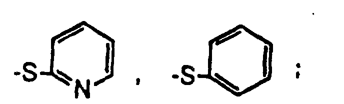
R⁸ is -(CH₂)ₘOR¹¹; 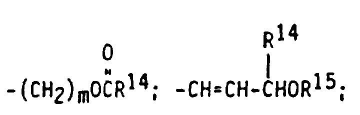 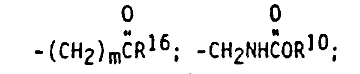 -(CH₂)ₘNHSO₂R¹⁰; or -COR¹⁶;
R¹⁰ is CF₃, alkyl of 1 to 6 carbon atoms or phenyl;
R¹¹ is H, or alkyl of 1 to 4 carbon atoms;
R¹³ is CO₂H; CO₂CH₂OCOC(CH₃)₃; NHSO₂CF₃ or 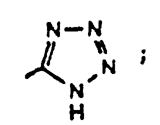
R¹⁴ is H, or alkyl of 1 to 4 carbon atoms;
R¹⁵ is H, alkyl of 1 to 4 carbon atoms, or acyl of 1 to 4 carbon atoms;
R¹⁶ is H, alkyl of 1 to 5 carbon atoms; OR¹⁷; or 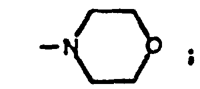 m is 1 to 5;
X = single bond, -O-; -CO-; -NHCO-; or -OCH₂-; or a pharmaceutically acceptable salt thereof.

4. A compound of Claim 3 wherein R¹ is 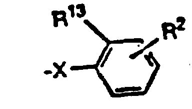 and X is a single bond, or a phamaceutically suitable salt thereof.

5. A pharmaceutical composition comprising a pharmaceutically suitable carrier and a compound of any one of Claims 1 through 4.

6. Pharmaceutical composition of Claim 5 which additionally contains a diuretic or a non-steroidal antiinflammatory drug.

7. A use of a compound of any of claims 1 through 4 for preparing a medicament for preventing renal failure in a warm blooded animal resulting from administration of a non-steroidal anti-inflammatory drug (NSAID).

8. A use of a compound of any of claims 1 through 4 for preparing a medicament for treating hypertension in a warm blodded animal.

9. Use of claim 8 wherein the medicament further comprises a diuretic.

10. A use of a compound of any one of claims 1 through 4 for preparing a medicament for treating congestive heart failure in a warm-blooded animal.

11. A process for the preparation of a compound of Claim 1 wherein r is 1 which comprises contacting an imidazole derivative of Formula 1 with a benzyl derivative of Formula 2 in a solvent in the presence of a base for about 1 to about 10 hours at a temperature in the range of about 20°C to the reflux temperature of the solvent to form a benzylimidazole of Formula 3: 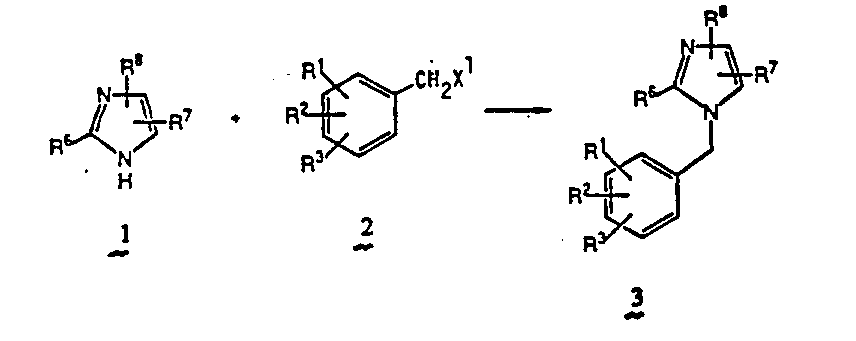 wherein each of R¹, R, R³, R⁶, R⁷ and R⁸ is stable under the reaction conditions and is a group as defined in Claim 1 or an intermediate or protected form thereof which can be transformed to such a group and wherein X¹ is halogen, p-toluenesulfonyloxy or methylsulfonyloxy; and thereafter as necessary transforming said intermediate or protected forms of the R groups to R groups as defined in Claim 1.

12. Process of Claim 11 wherein compounds 1 and 2 are contacted in the presence of a base selected from the group consisting of a metal hydride (MH), a metal alkoxide (MOR), sodium carbonate, potassium carbonate, triethylamine and pyridine, in a dipolar aprotic solvent or, where the base is MOR, the solvent can be an alcohol, ROH, where M is lithium, sodium or potassium and R is methyl, ethyl or t-butyl.

13. Process of Claim 11 wherein a two-phase solvent system, one an organic phase such as methylene chloride and the other an aqueous phase, is used in the presence of a phase transfer catalyst such as tricaprylmethylammonium chloride.

14. Process of Claim 12 wherein: R¹ is 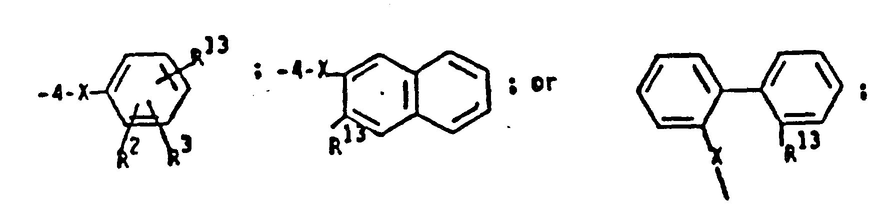
X is a carbon-carbon single bond, -CO-, -O-, -S-, or -NH-;
R and R³ are each independently H, Cl, Br, I, CO₂R¹⁴, F, NO₂, alkyl of 1 to 4 carbon atoms, alkoxy of 1 to 4 carbon atoms, phenyl; or furyl;
R⁶ and R⁷ are as defined in Claim 1;
R⁸ is -(CH₂)ₙOR¹¹; -(CH₂)ₙSR¹⁵; or -(CH₂)ₙCN;
R¹¹ is as defined in Claim 1;
R¹³ is CO₂R¹⁴, CN, NO₂, trialkyltin tetrazole, or trityltetrazole; and
R¹⁴ and R¹⁵ are as defined in Claim 1.

15. Process of Claim 14 wherein R¹³ is -CO₂R¹⁴ and the product of Formula 3 is contacted with an alkali in an aqueous alcoholic solvent or with CF₃CO₂H at a temperature in the range of 20°C to the reflux temperature of the solvent for 1-24 hours, followed by adjustment of the pH of tile mixture to a value in the range of 3 to 7, to convert the product to the corresponding product wherein R¹³ is -CO₂H.

16. Process of Claim 15 wherein at least one of R, R³ or R¹³ in Formula 1 is -CO₂R¹⁴ and is converted to -CO₂H.

17. Process of Claim 15 wherein R¹⁴ is t-butyl and the reaction is conducted in CF₃CO₂H.

18. Process of Claim 14 wherein R¹³ is -CN and the product of Formula 3 is contacted with (i) a strong acid at reflux temperature of the solvent for 2-96 hours or (ii) a strong alkali in an alcohol solvent at a temperature in the range of 20°C and the reflux temperature of the solvent for 2-96 hours followed by adjustment of the pH to 3-7, or (iii) sulfuric acid followed by acid or alkali, to convert the product to the corresponding compound wherein R¹³ is -CO₂H.

19. Process of Claim 18 wherein at least one of R, R³ or R¹³ is -CO₂R¹⁴ and is converted to -CO₂H.

20. Process of Claim 18 wherein R⁸ is -(CH₂)ₙCN and is converted to -(CH₂)ₙCO₂H, or is -(CH₂)ₙOR¹¹ and is converted to (CH₂)ₙOH when R¹³ is converted to -CO₂H.

21. Process of Claim 14 wherein R¹³ is -CN and tile product of Formula 3 is contacted with a mixture of equimolar amounts of sodium azide and ammonium chloride in a polar aprotic solvent at a temperature in the range of 30°C to the reflux temperature of the solvent, for 1 hour to 10 days, to convert the product to the corresponding compound wherein R¹³ is 5-tetrazolyl.

22. Process of Claim 21 wherein R⁸ is -(CH₂)ₘCN and is converted to -(CH₂)ₘ-tetrazolyl when R¹³ is converted to 5-tetrazolyl.

23. Process of Claim 14 wherein R¹³ is -CN and the product of Formula 3 is reacted with trialkyltin azide or triaryltin azide followed by acidic or basic hydrolysis to convert the product to the corresponding compound wherein R¹³ is 5-tetrazolyl.

24. Process of Claim 14 wherein R¹³ is -CN and the product of Formula 3 is reacted with trialkyltin azide or triaryltin azide to produce a compound of Formula 3 wherein R¹³ is trialkyl or triaryl stannyl tetrazol-5-yl, the latter compound is reacted with triphenylmethyl chloride to produce a compound of Formula 3 wherein R¹³ is triphenylmethyl-tetrazol-5-yl, and the latter compound is hydrolyzed to produce a compound of Formula 3 wherein R¹³ is 5-tetrazolyl.

25. Process of Claim 23 wherein R⁸ is -(CH₂)ₙCN and is converted to -(CH₂)ₘ-tetrazolyl when R¹³ is converted to 5-tetrazolyl.

26. Process of Claim 14 wherein R¹³ is -NO₂ and the product of Formula 3 is contacted with a reducing agent to form a second intermediate of Formula 3 in which R¹³ is NH₂, and the latter is contacted with an anhydride (CH₃SO₂)₂O or (CF₃SO₂)₂O or a chloride CH₃SO₂Cl or CF₃SO₂Cl of sulfonic acid in a solvent to produce a compound in which R¹³ is -NHSO₂CH₃ or -NHSO₂CF₃.

27. Process of Claim 26 wherein at least one of R, R³, or R¹³ is -NO₂ and is converted to -NHSO₂CH₃ or -NHSO₂CF₃.

28. Process of Claim 15 or 18 wherein the compound of Formula 3 with R¹³=CO₂H either
(a) is contacted with about 1-4 equivalents of thionyl chloride in excess thionyl chloride or another solvent at a temperature in the range of 20°C to the reflux temperature of the solvent for a period of 5 minutes to 2 hours to form an intermediate of Formula 3 wherein R¹³ is COCl, and the latter is contacted with 2-10 equivalents of hydroxylamine derivative H₂NOR¹ in excess hydroxylamine derivative H₂NOR¹ or other solvent, at a temperature in the range of 25-80°C for 2-18 hours, or
(b) is contacted the hydroxylamine derivative H₂NOR¹, dicyclohexylcarbodiimide and 1-hydroxybenzotriazole in a solvent at a temperature in the range of 0-30°C for 1-24 hours;
to provide a compound in which R¹³ is CONHOR¹.

29. process of Claim 11 wherein: R¹ is 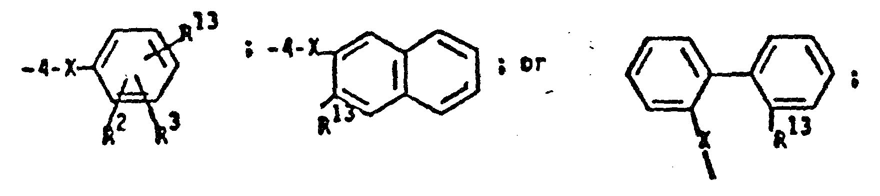
X is a carbon-carbon single bond, -CO-, -O-, -S-, or -NH-;
R, R³, R⁶ and R⁷ are as defined in Claim 1;
and
R⁸ is (CH₂),ₙOR¹¹, (CH₂)ₙOCOR¹⁴, (CH₂)ₙCH(OH)R¹⁶, (CH₂)ₙCOR¹⁶ (CH₂)ₙCl, (CH₂)ₙCN, CHO.

30. Process of Claim 29 wherein R⁸ is (CH₂)ₙOH and the product of Formula 3 is contacted with an alcohol R¹¹OH in the anhydrous state in the presence of a strong acid or a Lewis acid, followed by saponification of any CO₂R¹⁴ groups concomitantly formed or present in intermediate 3, to form the corresponding compound of Formula 3 wherein R⁸ is (CH₂)ₙOR¹¹ and R¹¹ is not H.

31. Process of Claim 29 wherein R⁸ is (CH₂)ₙOR¹¹ and R¹¹ is not H and the product of Formula 3 is contacted with an aqueous acidic medium at a temperature in the range of 25°C and the reflux temperature of the solvent for a period of 0.5-24 hours to form the corresponding compound of Formula 3 wherein R⁸ is (CH₂)ₙOH.

32. Process of Claim 29 wherein R⁸ is (CH₂)ₙOH and the product of Formula 3 is contacted with
(a) a carboxylic acid anhydride (R¹⁴CO)₂O or chloride R¹⁴COCl in a solvent in presence of a base at a temperature in the range of 0°C and the reflux temperature of the solvent for 0.5-24 hours or
(b) a carboxylic acid R¹⁴CO₂H under anhydrous conditions in presence of a strong acid or Lewis acid at 0°-100°C for 0.5 to 24 hours, to form the corresponding compound in which R⁸ is (CH₂)ₙOCOR¹⁴.

33. Process of Claim 29 wherein R⁸ is (CH₂)ₙOCOR¹⁴ and the product of Formula 3 is contacted with aqueous acid or alkali to form the corresponding compound wherein R⁸ is (CH₂)ₙOH.

34. Process of Claim 29 wherein R⁸ is (CH₂)ₙOH and the product of Formula 3 is contacted with an oxidizing agent at a temperature of 25-45°C for 1-200 hours to produce a corresponding compound of Formula 3 in which R⁸ is (CH₂)ₙ₋₁COR¹⁶ and R¹⁶ is H.

35. Process of Claim 29 wherein R⁸ is (CH₂)ₙCOR¹⁶ and R¹⁶ is H and the product of Formula 3 is contacted with an organometallic compound R¹⁶P in which P is MgBr or Li in a solvent at a temperature in the range of -78°C to 100°C for 0.5-24 hours to form a compound of Formula 3 in which R⁸ is (CH₂)ₙCH(OH)R¹⁶ and R¹⁶ is not H.

36. Process of Claim 29 wherein R⁸ is (CH₂)ₙCH(OH)R¹⁶ and R¹⁶ is not H and the product of Formula 3 is contacted with an oxidizing agent in a solvent to form a corresponding compound of Formula 3 in which R⁸ is (CH₂)ₙCOR¹⁶ and R¹⁶ is not H.

37. Process of Claim 29 wherein R⁸ is (CH₂)ₙCOR¹⁶ and R¹⁶ is H and the compound of Formula 3 is contacted with an oxidizing agent in a solvent to form a corresponding compound of Formula 3 in which R⁸ is (CH₂)ₙCOR¹⁶ and R¹⁶ is OH.

38. Process of Claim 29 wherein R⁸ is (CH₂)ₙCOR¹⁶ and R¹⁶ is OH and the compound of Formula 3 is contacted with thionyl chloride in excess or in another solvent at a temperature in the range of 0°C to the reflux temperature of the solvent for 5 minutes to 24 hours to form a corresponding compound of Formula 3 in which R⁸ is (CH₂)ₙCOCl followed by contact of the latter with an amine NHR¹⁸R¹⁹ in excess or in a solvent at temperatures in the range of 0°C and reflux temperature of the solvent for 5 minutes to 24 hours to form a corresponding compound of Formula 3 in which R⁸ is (CH₂)ₙCONR¹⁸R¹⁹.

39. Process of Claim 29 wherein R⁸ is (CH₂)ₙOR¹¹ and R¹¹ is H and the product of Formula 3 is contacted with thionyl chloride in excess or in a solvent at a temperature in the range of 20°C to the reflux temperature of the solvent for 0.5-24 hours to form an intermediate compound of Formula 3 in which R⁸ is (CH₂)ₙCl.

40. Process of Claim 39 wherein the compound of Formula 3 in which R⁸ is (CH₂)ₙCl is contacted with sodium or potassium salt of a mercaptan R¹⁵SH in a solvent at a temperature in the range of 25-100°C for 1-24 hours to form a compound of Formula 3 in which R⁸ is (CH₂)ₙSR¹⁵.

41. Process of Claim 29 wherein the compound of Formula 3 in which R₈ is (CH₂)ₙCl is contacted with an alkali metal cyanide in a solvent at a temperature in the range of 20-100°C for 1-24 hours to form a compound of Formula 3 in which R⁸ is (CH₂)ₙCN and the latter compound is hydrolyzed to the corresponding compound of Formula 3 in which R⁸ is (CH₂)ₙCOR¹⁶ and R¹⁶ is OH.

42. Process of Claim 29 wherein the compound of Formula 3 in which R⁸ is (CH₂)ₙ₋₁Cl is contacted with the sodium or potassium salt of a dialkyl malonate in a solvent at a temperature in the range of 20-100°C for 0.5-24 hours to form a compound of Formula 3 in which R⁸ is (CH₂)ₙ₋₁CH(CO₂alkyl)₂ followed by saponification of the latter with aqueous alkali at a temperature in the range of 25°C to the reflux temperature of the solvent followed by acidification with mineral acid to form a compound of Formula 3 in which R⁸ is (CH₂)ₙ₋₁CH(CO₂H)₂ followed by heating the latter to 120°C or in dilute mineral acid at reflux temperature to form a product of Formula 3 in which R⁸ is (CH₂)ₙCOR¹⁶ and R¹⁶ is OH.

43. Process of Claim 29 wherein R⁸ is -CHO and the compound of Formula 3 is contacted with a methylene phosphorane (C₆H₅)₃P=CH(CH₂)ₛCHR¹⁴OR¹⁵ or (C₆H₅)₃P=CH(CH₂)ₛCOR₁₆ in a solvent at a temperature in the range of 25°C to the reflux temperature of the solvent for 1-24 hours to form a compound of Formula 3 in which R⁸ is -CH=CH(CH₂)ₛCHR¹⁴OR¹⁵ or -CH=CH(CH₂)ₛCOR¹⁶, except where R¹⁵ is H and R¹⁶ is OH, and optionally then contacting the compound of Formula 3 in which R⁸ is -CH=CH(CH₂)ₛCOR¹⁶ with a reducing agent in a solvent at a temperature of 0°-25°C for 0.5-24 hours to form a product of Formula 3 in which R⁸ is -CH=CH(CH₂)ₛCHR¹⁴OH.

44. Process of Claim 29 wherein R⁸ is (CH₂)ₙOH and the compound of Formula 3 is contacted with an isocyanate of Formula R¹⁰NCO in a solvent at a temperature in the range of 25°C to the reflux temperature of the solvent for a period of 5 minutes to 24 hours to form a compound of Formula 3 in which R⁸ is (CH₂)ₙOCONHR¹⁰.

45. Process of Claim 29 wherein the compound in which R⁸ is (CH₂)ₙCl is contacted with an amine R¹¹NH₂ in excess amine or another solvent for a period of 1-24 hours at a temperature in the range of 0°C to the reflux temperature of the solvent to form an intermediate of Formula 3 in which R⁸ is (CH₂)ₙNHR¹¹.

46. Process of Claim 29 in which R⁸ is (CH₂)ₙCl and the compound of Formula 3 is contacted with an alkali metal azide in an aprotic solvent at a temperature in the range of 25-80°C for 1-24 hours to form a compound of Formula 3 in which R⁸ is (CH₂)ₙN₃ and the latter is contacted with a reducing agent to form an intermediate of Formula 3 in which R⁸ is (CH₂)ₙNH₂.

47. Process of Claim 45 or 46 in which R⁸ is (CH₂)ₙNHR¹¹ or (CH₂)ₙNH₂ and the compound of Formula 3 is contacted with a chloroformate of Formula R¹⁰OCOCl or a sulfonyl derivative of Formula R¹⁰SO₂Cl, or (R¹⁰SO₂)₂O in a solvent in the presence of a base at a temperature in the range of 0°C to the reflux temperature of a solvent for 5 minutes to 24 hours to form a compound of Formula 3 in which R⁸ is -(CH₂)ₙNR¹¹CO₂R¹⁰ or -(CH₂)ₙNR¹¹SO₂R¹⁰.

48. Process of Claim 45 or 46 in which the compound of Formula 3 with R⁸ equal to -(CH₂)ₙNHR¹¹ or (CH₂)ₙNH₂ is contacted with an isocyanate or isothiocyanate R¹⁰NCY in a solvent at a temperature in the range of 25°C to the reflux temperature of the solvent for 5 minutes to 24 hours to form a compound of the Formula 3 in which R⁸ is -(CH₂)ₙNR¹¹CYNHR¹⁰.

49. Process of Claim 11 wherein R¹ is NO₂ and R, R³, R⁶, R⁷, and R⁸ are as defined in Claim 34 in which the compound of Formula 3 wherein R¹ is NO₂ is reduced by means of iron and acetic acid, stannous chloride, or hydrogen and palladium to a compound of Formula 3 wherein R¹ is NH₂ and the latter is reacted with an appropriate acid anhydride such as phthalic anhydride or a substituted phthalic anhydride in a solvent or with an appropriate acid chloride such as substituted anthranilic acid chloride in the presence of aqueous alkali or a base or with an appropriately substituted phthalic or anthranilic acid in the presence of dicyclohexylcarbodiimide in a solvent to produce a compound of the Formula 3 in which R¹ is 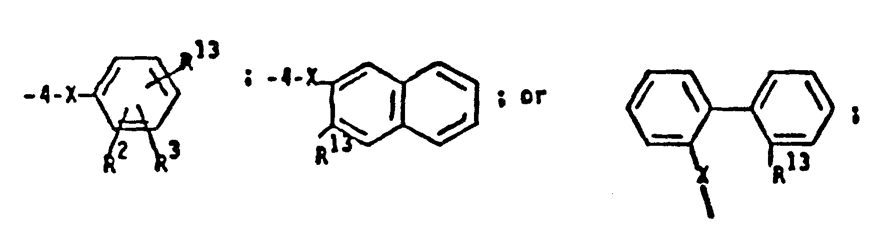 and X is NHCO.

50. Process of Claim 11 wherein R¹ is OCH₂C₆H₅, R and R³ are H and R⁶, R⁷, and R⁸ are as defined in Claim 11 and the resulting compound of Formula 3 with R¹ equal to OCH₂C₆H₅ is contacted with trifluoroacetic acid at reflux temperature for a period of 0.2-1 hour or with hydrogen and palladium to form the corresponding compound of Formula 3 in which R¹ is OH and the latter is contacted with a base at 25°C and a suitable benzyl halide of the formula: 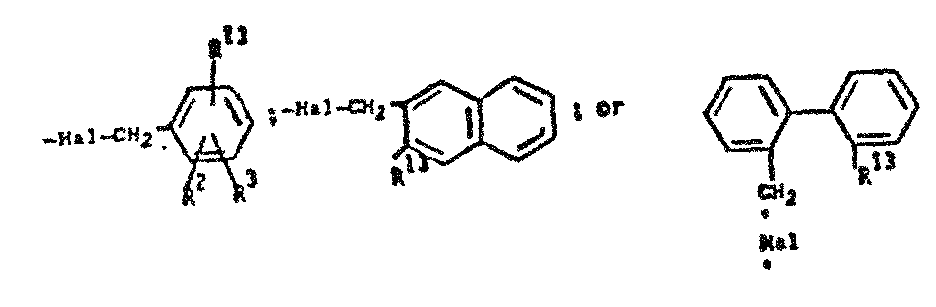 to produce the corresponding compound of Formula 3 wherein R¹ is 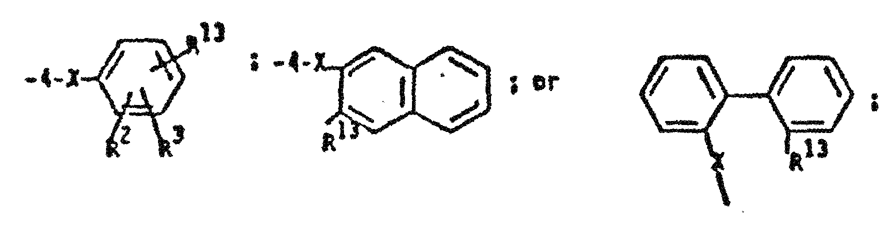 and X is -OCH₂-.

51. Process of Claim 11 wherein R⁸ is -CHO, whereby the benzyl derivative of Formula 2 attaches to the imidazole derivative of Formula 1 preferentially at the nitrogen atom adjacent the carbon atom of the imidazole ring to which R⁸ is attached.

52. A process for the preparation of a compound of Claim 1 wherein r is 0 which comprises contacting an imidazole derivative of Formula 1 or its metallic salt with 4-fluoro-1-nitrobenzene in a solvent, in the presence of a base if the free imidazole is used, for 1-10 days at a temperature of 25-150°C to form an N-phenylimidazole followed by elaboration to compounds wherein X=NHCO by the process described in Claim 49.

53. A process of Claim 29 wherein R⁸ is CHO, and X is a carbon-carbon single bond, and the product of Formula 3 is contacted with an organometallic reagent such as R¹¹MgBr or R¹¹Li in the presence of an anhydrous nonhydroxylic solvent such as ether, THF or dimethoxyethane at -78 to 25°C followed by aqueous work-up followed by acid hydrolysis of any CO₂R¹⁴ groups where R¹⁴ is t-butyl or hydrolysis of any trityl-protected tetrazole groups to form the corresponding compound of Forumula 3 wherein R⁸ is 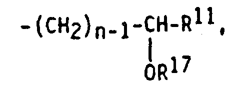 where R¹¹ ≠ H.

54. A process for the preparation of the compounds of claim 1 where R⁷ = substituted or unsubstituted biphenylyl, phenoxyphenyl, or heteroaryl, as defined in claim 1 characterized in that haloimidazole 237 is coupled with an arylmetal derivative ArM, where M=ZnBr, Me₃Sn, or B(OH)₂, in the presence of a transition metal catalyst such as palladium, platinum, nickel, or zirconium, to form an arylimidazole 238: 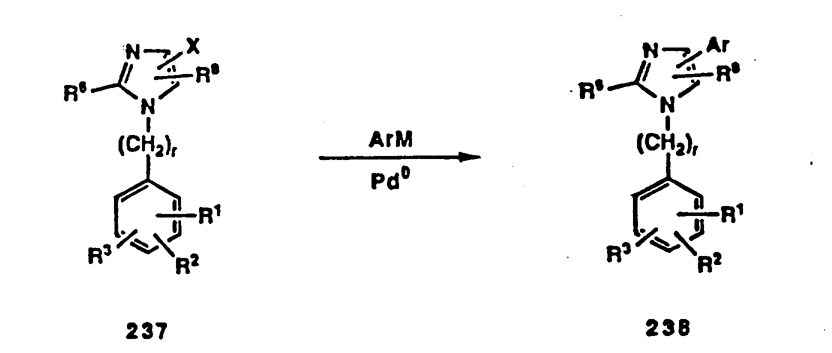
where R¹, R, R³, R⁶, R⁸ and r have the meanings given in Claim 1, X is Br or I, M is ZnBr, Me₃Sn, B(OH)₂, and Ar is substituted or unsubstituted biphenylyl, phenoxyphenyl, or heteroaryl as defined for R⁷ in claim 1.

55. A process for the preparation of the compounds of Claim 1 where R⁷=substituted or unsubstituted biphenylylmethyl, phenoxyphenylmethyl, or heteroarylmethyl as defined in claim 1 characterized in that a haloimidazole 237 is coupled with an arylmethylmetal derivative ArCH₂M' in the presence of a transition metal catalyst to form an arylmethylimidazole 240: 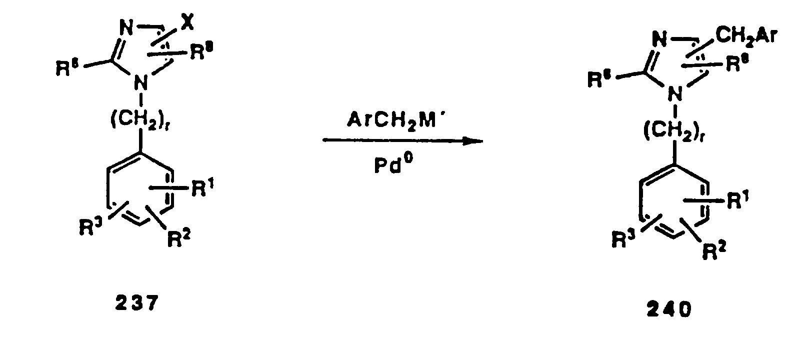
where R¹, R, R³, R⁶, R⁸ and r have the meanings given in Claim 1, X is Br or I, M' is ZnBr, and Ar is substituted or unsubstituted heteroarylmethyl, biphenylmethyl, phenoxyphenylmethyl as defined for R⁷ in claim 1.

56. A process for the preparation of the compounds of Claim 1 where R⁷=vinyl, alkynyl, substituted alkenyl, or substituted alkynyl characterized in that a haloimidazole 237 is coupled with an 1-alkenylmetal or an 1-alkynylmetal derivative (AM), or an 1-alkene, or an 1-alkyne (AH) in the presence of a transition metal catalyst to form a 1-alkenyl- or 1-alkynylimidazole 241: 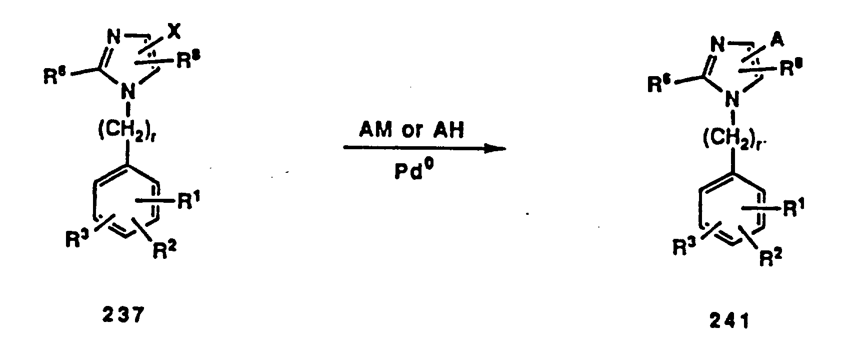
where R¹, R, R³, R⁶, R⁸ and r have the meanings given in Claim 1, X is Br or I, M is a metal, A is vinyl, CH=CH(CH₂)ₓAr, C≡C(CH₂)_{y}CH₃, C≡C(CH₂)_{z}Ph', or C≡C(CH₂)ₓAr, Ph' is phenyl or substituted phenyl, and Ar is substituted or insubstituted biphenylyl, phenoxyphenyl or heteroaryl as defined in claim 1, x=0-8, y=0-7 and z=0-4.

57. A process for the preparation of the compounds of claim 1 where R⁷=vinyl or substituted alkenyl characterized in that imidazole aldehyde 253 is reacted with methylenetriphenylphosphorane or a substituted methylenetriphenylphosphorane to form a vinylimidazole or a substituted alkenylimidazole 254: 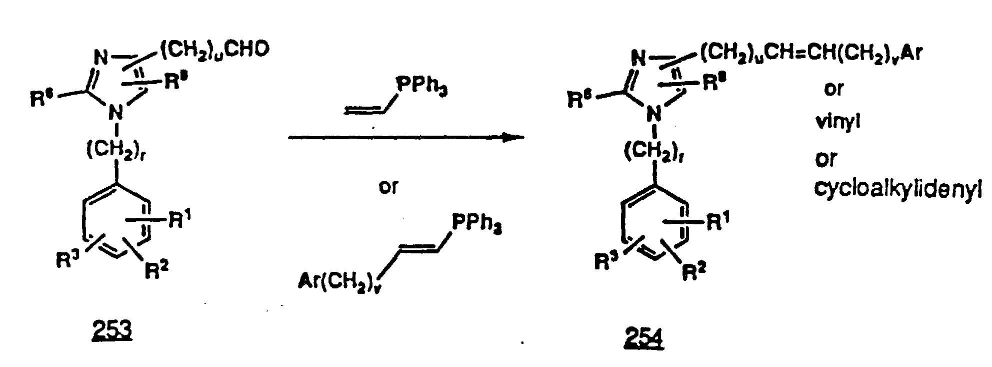 or 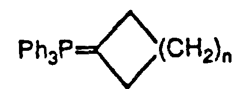 where n=O-5
where R¹, R, R³, R⁶, R⁸ and r have the meanings given in Claim 1, Ar is substituted or unsubstituted biphenylyl, phenoxyphenyl or heteroaryl as defined in claim 1, and u + v = 0 to 8.

58. A process for the preparation of the compounds of Formula I wherein R⁷ is -S(O)ᵣ-heteroaryl, -S-(O)ᵣ-biphenylyl, -S(O)ᵣ-phenoxyphenyl, -S-tetrazole, -S(O)ᵣ-R¹⁷, -NR¹⁸R¹⁹, -NR¹⁸-heteroaryl, -NR¹⁸-phenyl, -NR¹⁸-biphenylyl, -NR¹⁸phenoxyphenyl, -N-phthalimido, -NH-SO₂-phenoxyphenyl, -NH-SO₂-heteroaryl, -NH-SO₂-biphenylyl, -NH-SO₂-R¹⁷, and -S-(C=O)-R¹⁷, N-imidazolyl, N-1,2,3-triazolyl, and N-1,2,4-triazolyl, wherein heteroaryl is as defined in claim 1, characterized in that imidazole containing compounds 255 substituted with an electron withdrawing group E are reacted with nucleophiles in a suitable solvent at room temperature to the reflux temperature of the solvent resulting in an aromatic substitution reaction whereby the leaving group X is substituted with a nucleophile Nu such as sulfur or nitrogen to produce compounds of structure 256:

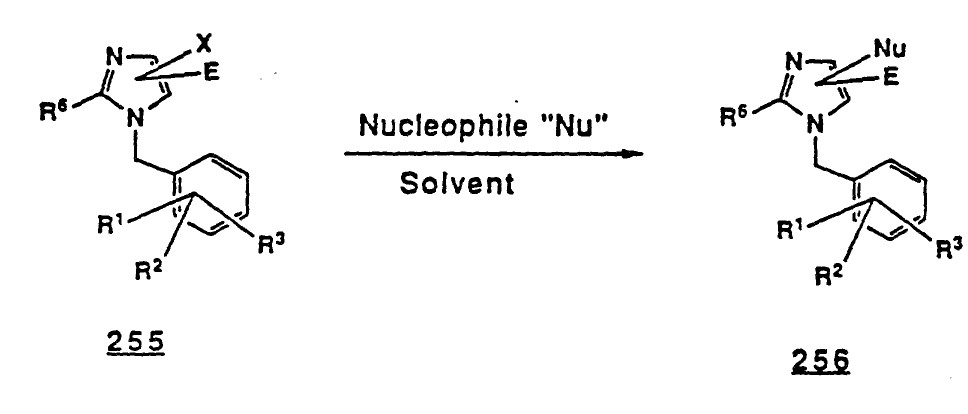

## Claims (Claims for the following Contracting State(s): ES)

1. A process for the preparation of an antihypertensive compound of the formula: 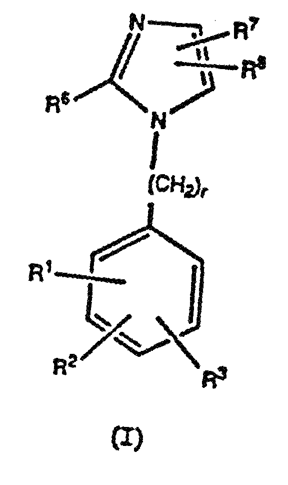 wherein
R¹ is 4-CO₂H; 4-CO₂R^{9;} 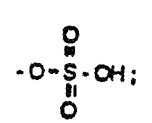 -SO₃H; -C(CF₃)₂OH ; 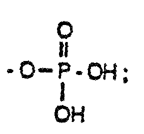 -PO₃H₂; 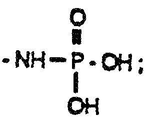 4 -NHSO₂CH₃ ; 4-NHSO₂CF₃ ; - CONHOR^{12;} -SO₂NH₂; 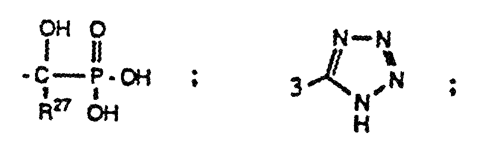 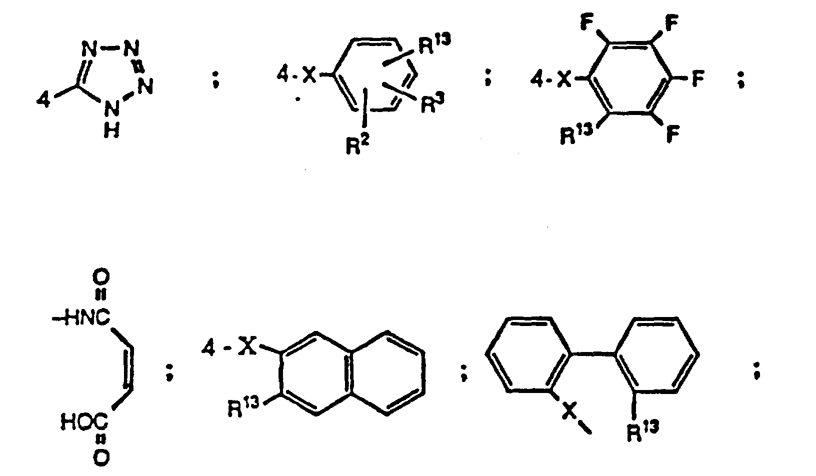 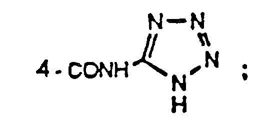 4-CONHNHSO₂CF₃; 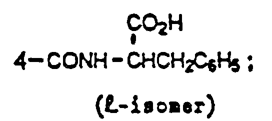 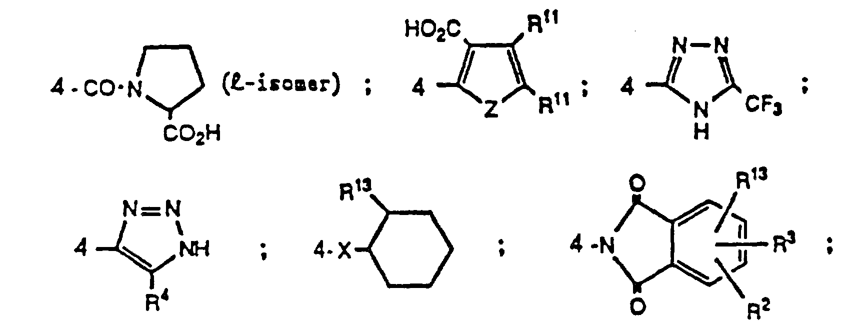 or 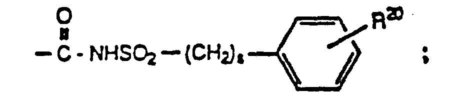
R is H; Cl; Br; I; F; NO₂; CN; alkyl of 1 to 4 carbon atoms; acyloxy of 1 to 4 carbon atoms; alkoxy of 1 to 4 carbon atoms; CO₂H; CO₂R⁹; NHSO₂CH₃; NHSO₂CF₃; CONHOR¹; SO₂NH₂ ; 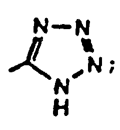 phenyl; or furyl;
R³ is H; Cl, Br, I or F; alkyl of 1 to 4 carbon atoms or alkoxy of 1 to 4 carbon atoms;
R⁴ is CN, NO₂ or CO₂R¹¹;
R⁵ is H, alkyl of 1 to 6 carbon atoms, cycloalkyl of 3 to 6 carbon atoms, alkenyl or alkynyl of 2 to 4 carbon atoms;
R⁶ is alkyl of 2 to 10 carbon atoms, alkenyl or alkynyl of 3 to 10 carbon atoms or the same groups substituted with F or CO₂R¹⁴; cycloalkyl of 3 to 8 carbon atoms, cycloalkylalkyl of 4 to 10 carbon atoms; cycloalkylalkenyl or cycloalkylalkynyl of 5 to 10 carbon atoms; (CH₂)ₛZ(CH₂)ₘR⁵ optionally substituted with F or CO₂R¹⁴; benzyl or benzyl substituted on the phenyl ring with 1 or 2 halogens, alkoxy of 1 to 4 carbon atoms, alkyl of 1 to 4 carbon atoms or nitro;
R⁷ is vinyl; cycloalkylidenyl; alkynyl of 2-10 carbon atoms; phenylalkynyl where the alkynyl portion is 2-6 carbon atoms; heteroaryl selected from 2- and 3-thienyl, 2- and 3-furyl, 2-, 3-, and 4-pyridyl, 2-pyrazinyl, 2-, 4-, and 5-pyrimidinyl, 3- and 4-pyridazinyl, 2-, 4- and 5-thiazolyl, 2-, 4-, and 5-selenazolyl, and 2-, 4-, and 5-oxazolyl, 2- or 3-pyrrolyl, 3-, 4- or 5-pyrazolyl, 2-, 4- or 5-imidazolyl; o-, m- or p-biphenylyl; o-, m- or p-phenoxyphenyl; 2-oxazolinyl; 2-thiazolinyl; substituted phenylalkynyl, heteroaryl, biphenylyl or phenoxyphenyl as defined above substituted with 1 or 2 substituents selected from halogen, hydroxy, mercapto, alkoxy of 1-5 carbon atoms, alkyl of 1-5 carbon atoms, -NO₂, -CN, -CF₃, -COR¹⁶, -CH₂OR¹⁷, -NHCOR¹⁷, -CONR¹⁸R¹⁹, S(O)ᵣR¹⁷, and SO₂NR¹⁸R¹⁹; pyrrolyl, pyrazolyl or imidazolyl as defined above substituted on ring nitrogen with alkyl of 1-5 carbon atoms, plienyl or benzyl; or substituted alkyl, alkenyl, or alkynyl of 1 to 10 carbon atoms substituted with a substituted or unsubstituted heteroaryl, biphenylyl or phenoxyphenyl group as defined above; -S(O)ᵣ-heteroaryl, -S-(O)ᵣ-biphenylyl, -S(O)ᵣ-phenoxyphenyl, -S-tetrazole, -S(O)ᵣR¹⁷, -NR¹⁸R¹⁹, -NR¹⁸-heteroaryl, -NR¹⁸-phenyl, -NR¹⁸-biphenylyl, -NR¹⁸-phenoxyphenyl, -N-phthalimido, -NH-SO₂-phenoxyphenyl, -NH-SO₂-heteroaryl, -NH-SO₂-biphenylyl, -NH-SO₂-R¹⁷, and -S-(C=O)-R¹⁷, N-imidazolyl, N-1,2,3-triazolyl, N-1,2,4-triazolyl, where heteroaryl is a heterocycle defined in the scope of R⁷ and where the phenyl group in R¹⁷ of -S-(O)ᵣR¹⁷, the N-imidazolyl, N-1,2,3-triazolyl, and N-1,2,4-triazolyls may be substituted with one or two substituents as described above for heteroaryl;
R⁸ is 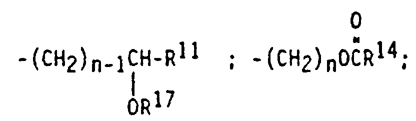 -(CH₂)ₙSR¹⁵; 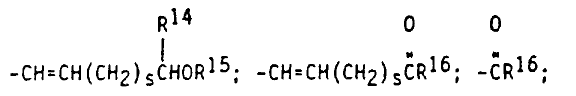 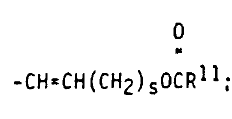 -(CH₂)ₘ-tetrazolyl; 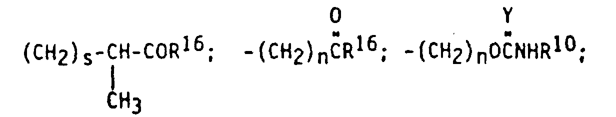 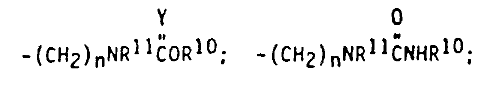 -(CH₂)ₙNR¹¹SO₂R¹⁰; 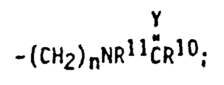
R⁹ is 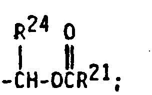
R¹⁰ is alkyl of 1 to 6 carbon atoms or perfluoroalkyl of 1 to 6 carbon atoms, 1-adamantyl, 1-naphthyl, 1-(1-naphthyl)ethyl, or (CH₂)ₚC₆H₅;
R¹¹ is H, alkyl of 1 to 6 carbon atoms, cycloalkyl of 3 to 6 carbon atoms, phenyl or benzyl;
R¹ is H, methyl or benzyl;
R¹³ is -CO₂H; -CO₂R⁹; -CH₂CO₂H, -CH₂CO₂R⁹; or 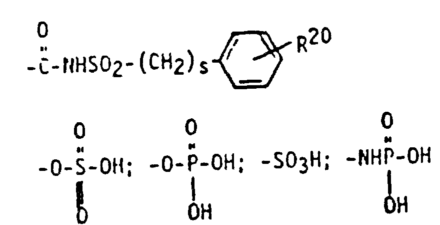 -PO₃H₂; -C(CF₃)₂OH; -NHSO₂CH₃; -NHSO₂CF₃; -NHCOCF₃; -CONHOR¹; -SO₂NH₂; 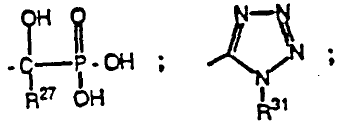 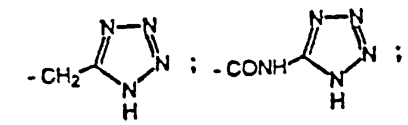 - CONHNHSO₂CF₃ ; 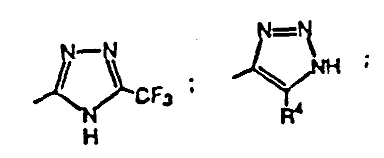
R¹⁴ is H, alkyl or perfluoroalkyl of 1 to 8 carbon atoms, cycloalkyl of 3 to 6 carbon atoms, phenyl or benzyl;
R¹⁵ is H, alkyl of 1 to 6 carbon atoms, cycloalkyl of 3 to 6 carbon atoms, phenyl, benzyl, acyl of 1 to 4 carbon atoms, phenacyl;
R¹⁶ is H, alkyl of 1 to 6 carbon atoms, cycloalkyl of 3 to 6 carbon atoms, (CH₂)ₚC₆H₅, OR¹⁷, or NR¹⁸R¹⁹;
R¹⁷ is H, alkyl of 1 to 6 carbon atoms, cycloalkyl of 3 to 6 carbon atoms, phenyl or benzyl;
R¹⁸ and R¹⁹ independently are H, alkyl of 1 to 4 carbon atoms, phenyl, benzyl, α-methylbenzyl, or taken together with the nitrogen form a ring of the formula 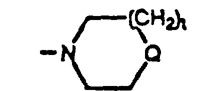
Q is NR⁰, O or CH₂;
R⁰ is H, alkyl of 1-4 carbon atoms, or phenyl;
R¹ is alkyl of 1 to 6 carbon atoms, -NRR³, or 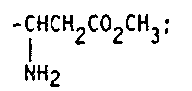
R and R³ independently are H, alkyl of 1 to 6 carbon atoms, benzyl, or are taken together as (CH₂)ᵤ where u is 3-6;
R⁴ is H, CH₃ or -C₆H₅;
R⁵ is NR⁷R⁸, OR⁸, NHCONH₂, NHCSNH₂, 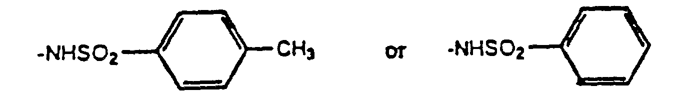
R⁶ is hydrogen, alkyl with from 1 to 6 carbon atoms, benzyl, or allyl;
R⁷ and R⁸ are independently hydrogen, alkyl with from 1 to 5 carbon atoms, or phenyl;
R⁹ and R³⁰ are independently alkyl of 1-4 carbon atoms or taken together are -(CH₂)_{q}-;
R³¹ is H, alkyl of 1 to 4 carbon atoms, -CH₂CH=CH₂ or -CH₂C₆H₄R³;
R³ is H, NO₂, NH₂, OH or OCH₃;
X is a carbon-carbon single bond, -CO-, -CH₂-, -O-, -S-, -NH-, 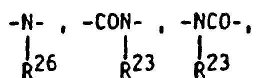 -OCH₂-, -CH₂O-, -SCH₂-, -CH₂S-, -NHC(R⁷)(R⁸)-, -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 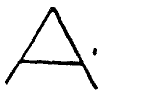 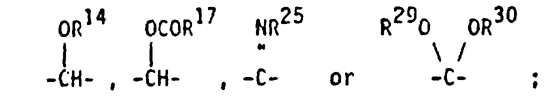
Y is 0 or S;
Z is 0, NR¹¹, or S;
m is 1 to 5;
n is 1 to 10;
p is 0 to 3;
q is 2 to 3;
r is 0 to 2;
s is 0 to 5;
t is 0 or 1;
or a pharmaceutically acceptable salt thereof;
provided that:
(1) the R¹ group is not in the ortho position;
(2) when R¹ is 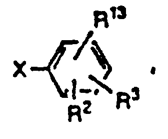 X is a single bond, and R¹³ is CO₂H, or 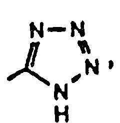 then R¹³ must be in the ortho or meta position; or when R¹ and X are as above and R¹³ is NHSO₂CF₃ or NHSO₂CH₃, R¹³ must be ortho;
(3) when R¹ is 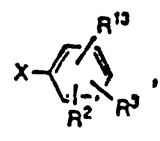 and X is other than a single bond, then R¹³ must be ortho except when X = NR³CO and R¹³ is NHSO₂CF₃ or NHSO₂CH₃, then R¹³ must be ortho or meta;
(4) when R¹ is 4-CO₂H or a salt thereof, R⁶ cannot be S-alkyl;
(5) when R¹ is 4-CO₂H or a salt thereof, the substituent on the 4-position of the imidazole cannot be CH₂OH, CH₂OCOCH₃, or CH₂CO₂H;
(6) when R¹ is 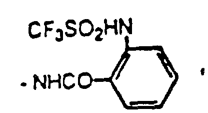 R⁶ is not methoxybenzyl;
(7) the R⁶ group is not 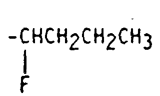 or CH₂OH; said process comprises
A) for the preparation of a compound wherein r is 1: contacting an imidazole derivative of Formula 1 with a benzyl derivative of Formula 2 in a solvent in the presence of a base for 1 to 10 hours at a temperature in the range of 20°C to the reflux temperature of the solvent to form a benzylimidazole of Formula 3: 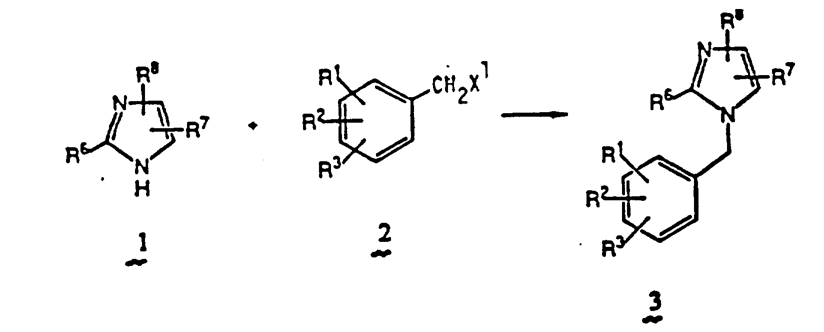 wherein each of R¹, R, R³, R⁶, R⁷ and R⁸ is stable under the reaction conditions and is a group as defined above or an intermediate or protected form thereof which can be transformed to such a group and wherein X¹ is halogen, p-toluenesulfonyloxy or methylsulfonyloxy; and thereafter as necessary transforming said intermediate or protected forms of the R groups to R groups as defined above;
B) for the preparation of a compound wherein r is 0: contacting an imidazole derivative of Formula 1 or its metallic salt with 4-fluoro-1-nitrobenzene in a solvent, in the presence of a base if the free imidazole is used for 1-10 days at a temperature of 25-150°C to form an N-phenylimidazole;
C) for the preparation of a compound where R⁷ = substituted or unsubstituted biphenylyl, phenoxyphenyl, or heteroaryl, as defined above: coupling haloimidazole 237 with an arylmetal derivative ArM, where H=ZnBr, Me₃Sn, or B(OH)₂, in the presence of a transition metal catalyst such as palladium, platinum, nickel, or zirconium, to form an arylimidazole 238: 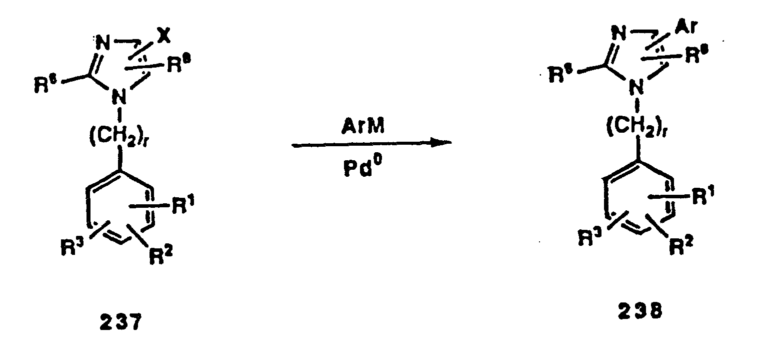
where R¹, R, R³, R⁶, R⁸ and r have the meanings given above, X is Br or I, M is ZnBr, Me₃Sn, B(OH)₂, and Ar is substituted or unsubstituted biphenylyl, phenoxyphenyl, or heteroaryl as defined for R⁷ above;
D) for the preparation of a compound where R⁷=substituted or unsubstituted biphenylylmethyl, phenoxyphenylmethyl, or heteroarylmethyl as defined above:coupling haloimidazole 237 with an arylmethylmetal derivative ArCH₂M' in the presence of a transition metal catalyst to form an arylmethylimidazole 240: 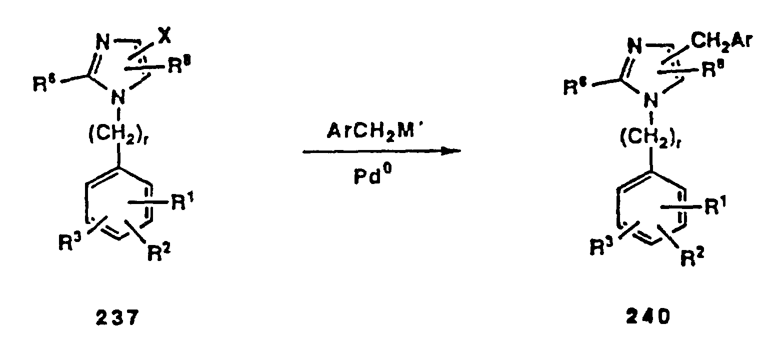
where R¹, R, R³, R⁶, R⁸ and r have the meanings given above,
X is Br or I, M' is ZnBr, and Ar is substituted or unsubstituted heteroarylmethyl, biphenylmethyl, phenoxyphenylmethyl as defined for R⁷ above;
E) for the preparation of a compound where R⁷=vinyl, alkynyl, substituted alkenyl, or substituted alkynyl:
coupling haloimidazole 237 with an 1-alkenylmetal or an 1-alkynylmetal derivative (AM), or an 1-alkene, or an 1-alkyne (AH) in the presence of a transition metal catalyst to form a 1-alkenyl- or 1-alkynylimidazole 241: 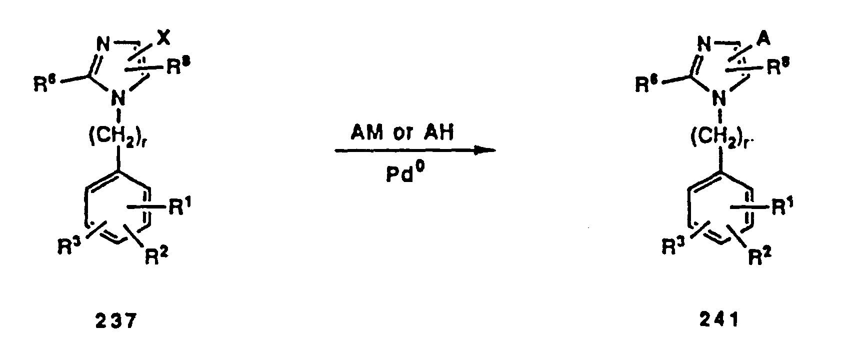
where R¹, R, R³, R⁶, R⁸ and r have the meanings given above,
X is Br or I, M is a metal, A is vinyl, CH=CH(CH₂)ₓAr, C≡C(CH₂)_{y}CH₃, C≡C(CH₂)_{z}Ph', or C≡C(CH₂)ₓAr, Ph' is phenyl or substituted phenyl, and Ar is substituted or unsubstituted biphenylyl, phenoxyphenyl or heteroaryl as defined above, x=8, y=0-7 and z=0-4.
F) for the preparation of a compound where R⁷=vinyl or substituted alkenyl : reacting imidazole aldehyde 253
with methylenetriphenylphosphorane or a substituted methylenetriphenylphosphorane to form a vinylimidazole or a substituted alkenylimidazole 254: 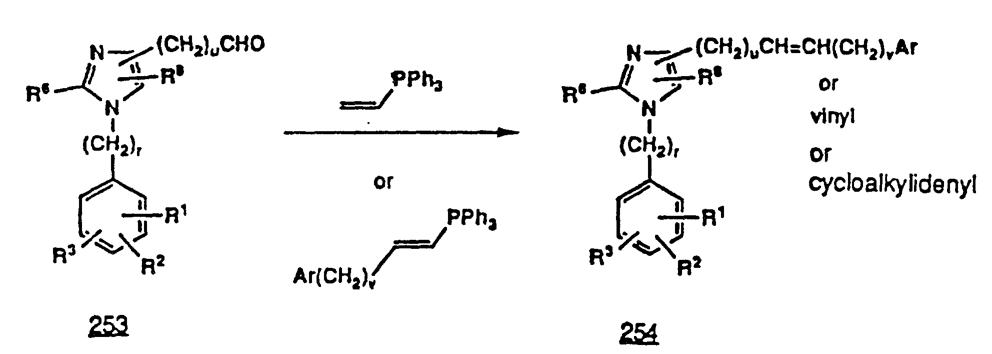 or 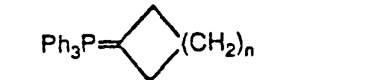 where n=O-5
where R¹, R, R³, R⁶, R⁸ and r have the meanings given above
Ar is substituted or unsubstituted biphenylyl, phenoxyphenyl or heteroaryl as defined above, and u + v = 0 to 8; and
G) for the preparation of a compound of Formula I wherein R⁷ is -S(O)ᵣ-heteroaryl, -S-(O)ᵣ-biphenylyl, -S(O)ᵣ-phenoxyphenyl, -S-tetrazole, -S(O)ᵣR¹⁷, -NR¹⁸R¹⁹, -NR¹⁸-heteroaryl, -NR¹⁸-phenyl, -NR¹⁸-biphenylyl, -NR¹⁸phenoxyphenyl, -N-phthalimido, -NH-SO₂-phenoxyphenyl, -NH-SO₂-heteroaryl, -NH-SO₂-biphenylyl, -NH-SO₂-R¹⁷, and -S-(C=O)-R¹⁷, N-imidazolyl, N-1,2,3-triazolyl, and N-1,2,4-triazolyl, wherein heteroaryl is as defined above : reacting imidazole containing compounds 255 substituted with an electron withdrawing group E
with nucleophiles in a suitable solvent at room temperature to the reflux temperature of the solvent resulting in an aromatic substitution reaction whereby the leaving group X is substituted with a nucleophile Nu such as sulfur or nitrogen to produce compounds of structure 256:

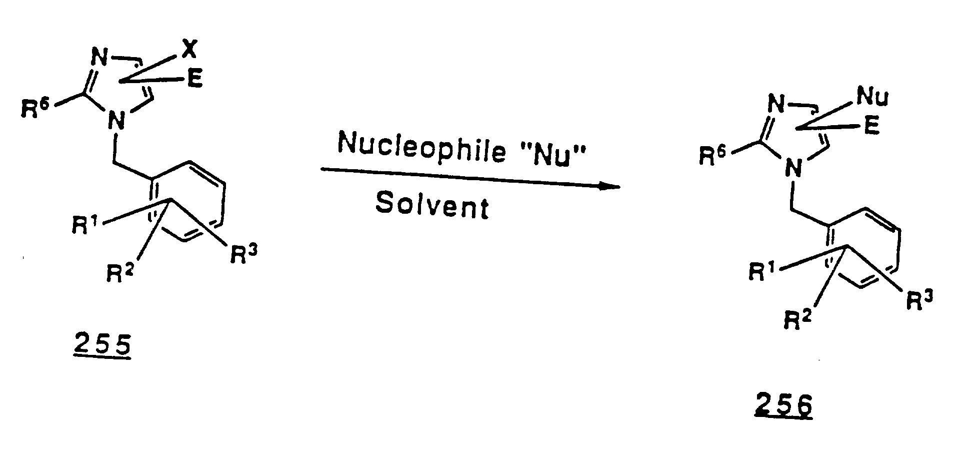

2. Process of Claim 1 wherein in A) compounds 1 and 2 are contacted in the presence of a base selected from the group consisting of a metal hydride (MH), a metal alkoxide (MOR), sodium carbonate, potassium carbonate, triethylamine and pyridine, in a dipolar aprotic solvent or, where the base is MOR, the solvent can be an alcohol, ROH, where M is lithium, sodium or potassium and R is methyl, ethyl or t-butyl.

3. Process of Claim 1 wherein in A) a two-phase solvent system, one an organic phase such as methylene chloride and the other an aqueous phase, is used in the presence of a phase transfer catalyst such as tricaprylmethylammonium chloride.

4. Process of Claim 2 wherein: R¹ is 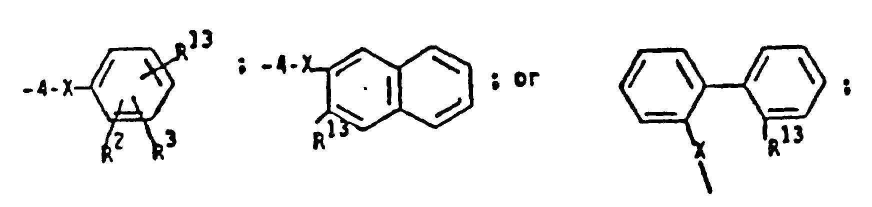
X is a carbon-carbon single bond, -CO-, -O-, -S-, or -NH-;
R and R³ are each independently H, Cl, Br, I, CO₂R¹⁴, F, NO₂, alkyl of 1 to 4 carbon atoms, alkoxy of 1 to 4 carbon atoms, phenyl; or furyl;
R⁶ and R⁷ are as defined in Claim 1;
R⁸ is -(CH₂)ₙOR¹¹; -(CH₂)ₙSR¹⁵; or -(CH₂)ₙCN;
R¹¹ is as defined in Claim 1;
R¹³ is CO₂R¹⁴, CN, NO₂, trialkyltin tetrazole, or trityltetrazole; and
R¹⁴ and R¹⁵ are as defined in Claim 1.

5. Process of Claim 4 wherein R¹³ is -CO₂R¹⁴ and the product of Formula 3 is contacted with an alkali in an aqueous alcoholic solvent or with CF₃CO₂H at a temperature in the range of 20°C to the reflux temperature of the solvent for 1-24 hours, followed by adjustment of the pH of the mixture to a value in the range of 3 to 7, to convert the product to the corresponding product wherein R¹³ is -CO₂H.

6. Process of Claim 5 wherein at least one of R, R³ or R¹³ in Formula 1 is -CO₂R¹⁴ and is converted to -CO₂H.

7. Process of Claim 5 wherein R¹⁴ is t-butyl and the reaction is conducted in CF₃CO₂H.

8. Process of Claim 4 wherein R¹³ is -CN and the product of Formula 3 is contacted with (i) a strong acid at reflux temperature of the solvent for 2-96 hours or (ii) a strong alkali in an alcohol solvent at a temperature in the range of 20°C and the reflux temperature of the solvent for 2-96 hours followed by adjustment of the pH to 3-7, or (iii) sulfuric acid followed by acid or alkali, to convert the product to the corresponding compound wherein R¹³ is -CO₂H.

9. Process of Claim 8 wherein at least one of R, R³ or R¹³ is -CO₂R¹⁴ and is converted to -CO₂H.

10. Process of Claim 8 wherein R⁸ is -(CH₂)ₙCN and is converted to -(CH₂)ₙCO₂H, or is -(CH₂)ₙOR¹¹ and is converted to (CH₂)ₙOH when R¹³ is converted to -CO₂H.

11. Process of Claim 4 wherein R¹³ is -CN and the product of Formula 3 is contacted with a mixture of equimolar amounts of sodium azide and ammonium chloride in a polar aprotic solvent at a temperature in the range of 30°C to the reflux temperature of the solvent, for 1 hour to 10 days, to convert the product to the corresponding compound wherein R¹³ is 5-tetrazolyl.

12. Process of Claim 11 wherein R⁸ is -(CH₂)ₘCN and is converted to -(CH₂)ₘ-tetrazolyl when R¹³ is converted to 5-tetrazolyl.

13. Process of Claim 4 wherein R¹³ is -CN and the product of Formula 3 is reacted with trialkyltin azide or triaryltin azide followed by acidic or basic hydrolysis to convert the product to the corresponding compound wherein R¹³ is 5-tetrazolyl.

14. Process of Claim 4 wherein R¹³ is -CN and the product of Formula 3 is reacted with trialkyltin azide or triaryltin azide to produce a compound of Formula 3 wherein R¹³ is trialkyl or triaryl stannyl tetrazol-5-yl, the latter compound is reacted with triphenylmethyl chloride to produce a compound of Formula 3 wherein R¹³ is triphenylmethyl-tetrazol-5-yl, and the latter compound is hydrolyzed to produce a compound of Formula 3 wherein R¹³ is 5-tetrazolyl.

15. Process of Claim 3 wherein R⁸ is -(CH₂)ₙCN and is converted to -(CH₂)ₘ-tetrazolyl when R¹³ is converted to 5-tetrazolyl.

16. Process of Claim 4 wherein R¹³ is -NO₂ and the product of Formula 3 is contacted with a reducing agent to form a second intermediate of Formula 3 in which R¹³ is NH₂, and the latter is contacted with an anhydride (CH₃SO₂)₂O or (CF₃SO₂)₂O or a chloride CH₃SO₂Cl or CF₃SO₂Cl of sulfonic acid in a solvent to produce a compound in which R¹³ is -NHSO₂CH₃ or -NHSO₂CF₃.

17. Process of Claim 16 wherein at least one of R, R³, or R¹³ is -NO₂ and is converted to -NHSO₂CH₃ or -NHSO₂CF₃.

18. Process of Claim 5 or 8 wherein the compound of Formula 3 with R¹³=CO₂H either
(a) is contacted with about 1-4 equivalents of thionyl chloride in excess thionyl chloride or another solvent at a temperature in the range of 20°C to the reflux temperature of the solvent for a period of 5 minutes to 2 hours to form an intermediate of Formula 3 wherein R¹³ is COCl, and the latter is contacted with 2-10 equivalents of hydroxylamine derivative H₂NOR¹ in excess hydroxylamine derivative H₂NOR¹ or other solvent, at a temperature in the range of 25-80°C for 2-18 hours, or
(b) is contacted the hydroxylamine derivative H₂NOR¹, dicyclohexylcarbodiimide and 1-hydroxybenzotriazole in a solvent at a temperature in the range of 0-30°C for 1-24 hours; to provide a compound in which R¹³ is CONHOR¹.

19. Process of Claim 1 wherein in A) : R¹ is 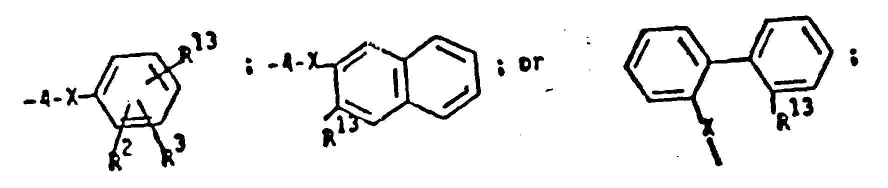
X is a carbon-carbon single bond, -Co-, -O-, -S-, or -NH-;
R, R³, R⁶ and R⁷ are as defined in Claim 1;
and
R⁸ is (CH₂),ₙOR¹¹, (CH₂)ₙOCOR¹⁴, (CH₂)ₙCH(OH)R¹⁶, (CH₂)ₙCOR¹⁶ (CH₂)ₙCl, (CH₂)ₙCN, CHO.

20. Process of Claim 19 wherein R⁸ is (CH₂)ₙOH and the product of Formula 3 is contacted with an alcohol R¹¹OH in the anhydrous state in the presence of a strong acid or a Lewis acid, followed by saponification of any CO₂R¹⁴ groups concomitantly formed or present in intermediate 3, to form the corresponding compound of Formula 3 wherein R⁸ is (CH₂)ₙOR¹¹ and R¹¹ is not H.

21. Process of Claim 19 wherein R⁸ is (CH₂)ₙOR¹¹ and R¹¹ is not H and the product of Formula 3 is contacted with an aqueous acidic medium at a temperature in the range of 25°C and the reflux temperature of the solvent for a period of 0.5-24 hours to form the corresponding compound of Formula 3 wherein R⁸ is (CH₂)ₙOH.

22. Process of Claim 19 wherein R⁸ is (CH₂)ₙOH and the product of Formula 3 is contacted with
(a) a carboxylic acid anhydride (R¹⁴CO)₂O or chloride R¹⁴COCl in a solvent in presence of a base at a temperature in the range of 0°C and the reflux temperature of the solvent for 0.5-24 hours or
(b) a carboxylic acid R¹⁴CO₂H under anhydrous conditions in presence of a strong acid or Lewis acid at 0°-100°C for 0.5 to 24 hours, to form the corresponding compound in which R⁸ is (CH₂)ₙOCOR¹⁴.

23. Process of Claim 19 wherein R⁸ is (CH₂)ₙOCOR¹⁴ and the product of Formula 3 is contacted with aqueous acid or alkali to form the corresponding compound wherein R⁸ is (CH₂)ₙOH.

24. Process of Claim 19 wherein R⁸ is (CH₂)ₙOH and the product of Formula 3 is contacted with an oxidizing agent at a temperature of 25-45°C for 1-200 hours to produce a corresponding compound of Formula 3 in which R⁸ is (CH₂)ₙ₋₁COR¹⁶ and R¹⁶ is H.

25. Process of Claim 19 wherein R⁸ is (CH₂)ₙCOR¹⁶ and R¹⁶ is H and the product of Formula 3 is contacted with an organometallic compound R¹⁶P in which P is MgBr or Li in a solvent at a temperature in the range of -78°C to 100°C for 0.5-24 hours to form a compound of Formula 3 in which R⁸ is (CH₂)ₙCH(OH)R¹⁶ and R¹⁶ is not H.

26. Process of Claim 19, wherein R⁸ is (CH₂)ₙCH(OH)R¹⁶ and R¹⁶ is not H and the product of Formula 3 is contacted with an oxidizing agent in a solvent to form a corresponding compound of Formula 3 in which R⁸ is (CH₂)ₙCOR¹⁶ and R¹⁶ is not H.

27. Process of Claim 19 wherein R⁸ is (CH₂)ₙCOR¹⁶ and R¹⁶ is H and the compound of Formula 3 is contacted with an oxidizing agent in a solvent to form a corresponding compound of Formula 3 in which R⁸ is (CH₂)ₙCOR¹⁶ and R¹⁶ is OH.

28. Process of Claim 19 wherein R⁸ is (CH₂)ₙCOR¹⁶ and R¹⁶ is OH and the compound of Formula 3 is contacted with thionyl chloride in excess or in another solvent at a temperature in the range of 0°C to the reflux temperature of the solvent for 5 minutes to 24 hours to form a corresponding compound of Formula 3 in which R⁸ is (CH₂)ₙCOCl followed by contact of the latter with an amine NHR¹⁸R¹⁹ in excess or in a solvent at temperatures in the range of 0°C and reflux temperature of the solvent for 5 minutes to 24 hours to form a corresponding compound of Formula 3 in which R⁸ is (CH₂)ₙCONR¹⁸R¹⁹.

29. Process of Claim 19 wherein R⁸ is (CH₂)ₙOR¹¹ and R¹¹ is H and the product of Formula 3 is contacted with thionyl chloride in excess or in a solvent at a temperature in the range of 20°C to the reflux temperature of the solvent for 0.5-24 hours to form an intermediate compound of Formula 3 in which R⁸ is (CH₂)ₙCl.

30. Process of Claim 29 wherein the compound of Formula 3 in which R⁸ is (CH₂)ₙCl is contacted with sodium or potassium salt of a mercaptan R¹⁵SH in a solvent at a temperature in the range of 25-100°C for 1-24 hours to form a compound of Formula 3 in which R⁸ is (CH₂)ₙSR¹⁵.

31. Process of Claim 19 wherein the compound of Formula 3 in which R₈ is (CH₂)ₙCl is contacted with an alkali metal cyanide in a solvent at a temperature in the range of 20-100°C for 1-24 hours to form a compound of Formula 3 in which R⁸ is (CH₂)ₙCN and the latter compound is hydrolyzed to the corresponding compound of Formula 3 in which R⁸ is (CH₂)ₙCOR¹⁶ and R¹⁶ is OH.

32. Process of Claim 19 wherein the compound of Formula 3 in which R⁸ is (CH₂)ₙ₋₁Cl is contacted with the sodium or potassium salt of a dialkyl malonate in a solvent at a temperature in the range of 20-100°C for 0.5-24 hours to form a compound of Formula 3 in which R⁸ is (CH₂)ₙ₋₁CH(CO₂alkyl)₂ followed by saponification of the latter with aqueous alkali at a temperature in the range of 25°C to the reflux temperature of the solvent followed by acidification with mineral acid to form a compound of Formula 3 in which R⁸ is (CH₂)ₙ₋₁CH(CO₂H)₂ followed by heating the latter to 120°C or in dilute mineral acid at reflux temperature to form a product of Formula 3 in which R⁸ is (CH₂)ₙCOR¹⁶ and R¹⁶ is OH.

33. Process of Claim 19 wherein R⁸ is -CHO and the compound of Formula 3 is contacted with a methylene phosphorane (C₆H₅)₃P=CH(CH₂)ₛCHR¹⁴OR¹⁵ or (C₆H₅)₃P=CH(CH₂)ₛCOR¹⁶ in a solvent at a temperature in the range of 25°C to the reflux temperature of the solvent for 1-24 hours to form a compound of Formula 3 in which R⁸ is -CH=CH(CH₂)ₛCHR¹⁴OR¹⁵ or -CH=CH(CH₂)ₛCOR¹⁶, except where R¹⁵ is H and R¹⁶ is OH, and optionally then contacting the compound of Formula 3 in which R⁸ is -CH=CH(CH₂)ₛCOR¹⁶ with a reducing agent in a solvent at a temperature of 0°-25°C for 0.5-24 hours to form a product of Formula 3 in which R⁸ is -CH=CH(CH₂)ₛCHR¹⁴OH.

34. Process of Claim 19 wherein R⁸ is (CH₂)ₙOH and the compound of Formula 3 is contacted with an isocyanate of Formula R¹⁰NCO in a solvent at a temperature in the range of 25°C to the reflux temperature of the solvent for a period of 5 minutes to 24 hours to form a compound of Formula 3 in which R⁸ is (CH₂)ₙOCONHR¹⁰.

35. Process of Claim 19 wherein the compound in which R⁸ is (CH₂)ₙCl is contacted with an amine R¹¹NH₂ in excess amine or another solvent for a period of 1-24 hours at a temperature in the range of 0°C to the reflux temperature of the solvent to form an intermediate of Formula 3 in which R⁸ is (CH₂)ₙNHR¹¹.

36. Process of Claim 19 in which R⁸ is (CH₂)ₙCl and the compound of Formula 3 is contacted with an alkali metal azide in an aprotic solvent at a temperature in the range of 25-80°C for 1-24 hours to form a compound of Formula 3 in which R⁸ is (CH₂)ₙN₃ and the latter is contacted with a reducing agent to form an intermediate of Formula 3 in which R⁸ is (CH₂)ₙNH₂.

37. Process of Claim 35 or 36 in which R⁸ is (CH₂)ₙNHR¹¹ or (CH₂)ₙNH₂ and the compound of Formula 3 is contacted with a chloroformate of Formula R¹⁰OCOCl or a sulfonyl derivative of Formula R¹⁰SO₂Cl, or (R¹⁰SO₂)₂O in a solvent in the presence of a base at a temperature in the range of 0°C to the reflux temperature of a solvent for 5 minutes to 24 hours to form a compound of Formula 3 in which R⁸ is -(CH₂)ₙNR¹¹CO₂R¹⁰ or -(CH₂)ₙNR¹¹SO₂R¹⁰.

38. Process of Claim 35 or 36 in which the compound of Formula 3 with R⁸ equal to -(CH₂)ₙNHR¹¹ or (CH₂)ₙNH₂ is contacted with an isocyanate or isothiocyanate R¹⁰NCY in a solvent at a temperature in the range of 25°C to the reflux temperature of the solvent for 5 minutes to 24 hours to form a compound of the Formula 3 in which R⁸ is -(CH₂)ₙNR¹¹CYNHR¹⁰.

39. Process of Claim 1 wherein R¹ in A) and B) is NO₂ and R, R³, R⁶, R⁷, and R⁸ are as defined in Claim 24 in which a compound wherein R¹ is NO₂ is reduced by means of iron and acetic acid, stannous chloride, or hydrogen and palladium to a compound wherein R¹ is NH₂ and the latter is reacted with an appropriate acid anhydride such as phthalic anhydride or a substituted phthalic anhydride in a solvent or with an appropriate acid chloride such as substituted anthranilic acid chloride in the presence of aqueous alkali or a base or with an appropriately substituted phthalic or anthranilic acid in the presence of dicyclohexylcarbodiimide in a solvent to produce a compound in which R¹ is 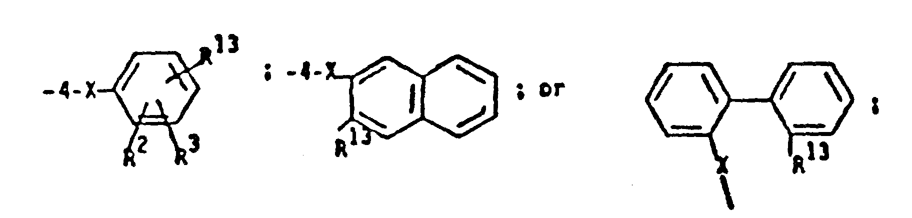 and X is NHCO.

40. Process of Claim 1 wherein in A) R¹ is OCH₂C₆H₅, R and R³ are H and R⁶, R⁷, and R⁸ are as defined in Claim 1 and the resulting compound of Formula 3 with R¹ equal to OCH₂C₆H₅ is contacted with trifluoroacetic acid at reflux temperature for a period of 0.2-1 hour or with hydrogen and palladium to form the corresponding compound of Formula 3 in which R¹ is OH and the latter is contacted with a base at 25°C and a suitable benzyl halide of the formula: 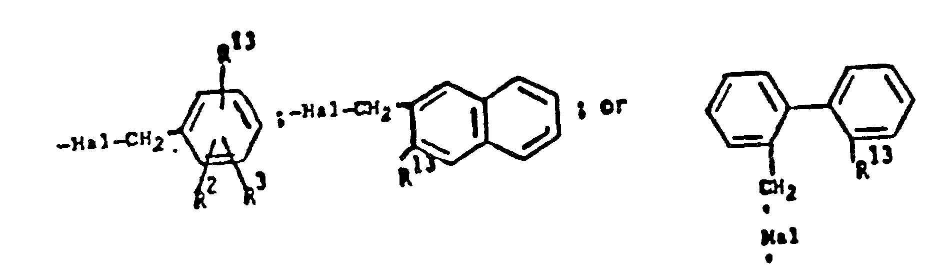 to produce the corresponding compound of Formula 3 wherein R¹ is 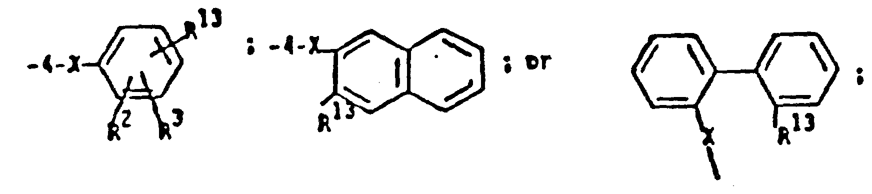 and X is -OCH₂-.

41. Process of Claim 1 wherein R⁸ in A) is -CHO, whereby the benzyl derivative of Formula 2 attaches to the imidazole derivative of Formula 1 preferentially at the nitrogen atom adjacent the carbon atom of the imidazole ring to which R⁸ is attached.

42. A process of Claim 19 wherein R⁸ is CHO, and X is a carbon-carbon single bond, and the product of Formula 3 is contacted with an organometallic reagent such as R¹¹MgBr or R¹¹Li in the presence of an anhydrous nonhydroxylic solvent such as ether, THF or dimethoxyethane at -78 to 25°C followed by aqueous work-up followed by acid hydrolysis of any CO₂R¹⁴ groups where R¹⁴ is t-butyl or hydrolysis of any trityl-protected tetrazole groups to form the corresponding compound of Forumula 3 wherein R⁸ is 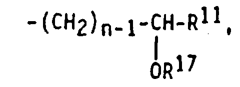 where R¹¹ ≠ H.

43. A process according to any of claims 1-42 wherein the prepared compound is having the formula: 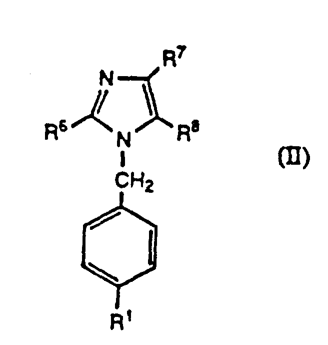 wherein R¹ is -CO₂H ; -NHSO₂CF₃ ; 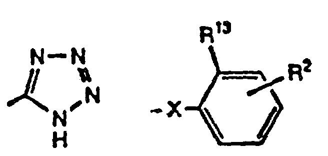 or 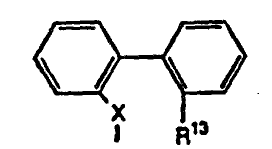
R⁶ is alkyl of 3 to 10 carbon atoms, alkenyl of 3 to 10 carbon atoms, alkynyl of 3 to 10 carbon atoms, cycloalkyl of 3 to 8 carbon atoms, benzyl substituted on the phenyl ring with up to two groups selected from alkoxy of 1 to 4 carbon atoms, halogen, alkyl of 1 to 4 carbon atoms, and nitro;
R⁸ is -(CH₂)ₘ-tetrazolyl, -(CH₂)ₙOR¹¹; 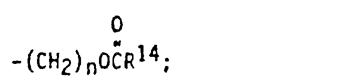 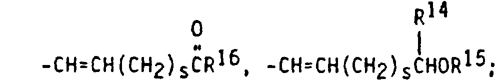 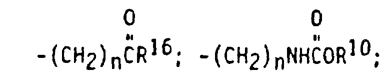 -(CH₂)ₙNHSO₂R¹⁰; or 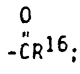
R¹³ is -CO₂H, -CO₂R⁹, NHSO₂CF₃; SO₃H; or 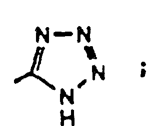
R¹⁶ is H, alkyl of 1 to 5 carbon atoms, OR¹⁷, or NR¹⁸R¹⁹;
X is carbon-carbon single bond, -CO-, 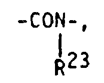 -CH₂CH₂-, 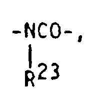 -OCH₂-, -CH₂O-, -O-, -SCH₂-, -CH₂S-, -NHCH₂-, -CH₂NH- or -CH=CH-; or a pharmaceutically acceptable salt thereof.

44. A process of claim 43 wherein:
R is H, alkyl of 1 to 4 carbon atoms, halogen, or alkoxy of 1 to 4 carbon atoms;
R⁶ is alkyl, alkenyl or alkynyl of 3 to 7 carbon atoms;
R⁷ is heteroaryl selected from 2- and 3-thienyl, 2- and 3-furyl, 2-, 3-, and 4-pyridyl, or p-biphenylyl 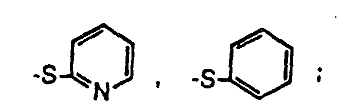
R⁸ is -(CH₂)ₘOR¹¹; 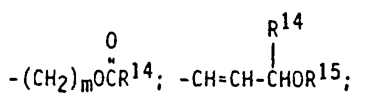 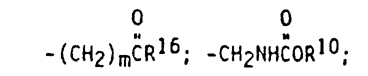 -(CH₂)ₘNHSO₂R¹⁰; or -COR¹⁶;
R¹⁰ is CF₃, alkyl of 1 to 6 carbon atoms or phenyl;
R¹¹ is H, or alkyl of 1 to 4 carbon atoms;
R¹³ is CO₂H; CO₂CH₂OCOC(CH₃)₃; NHSO₂CF₃ or 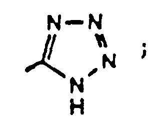
R¹⁴ is H, or alkyl of 1 to 4 carbon atoms;
R¹⁵ is H, alkyl of 1 to 4 carbon atoms, or acyl of 1 to 4 carbon atoms;
R¹⁶ is H, alkyl of 1 to 5 carbon atoms; OR¹⁷; or 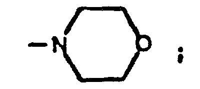
m is 1 to 5;
X = single bond, -O-; -CO-; -NHCO-; or -OCH₂-; or a pharmaceutically acceptable salt thereof.

45. A process of Claim 44 wherein R¹ is 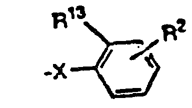 and X is a single bond, or a pharmaceutically suitable salt thereof.

46. A process for preparing a pharmaceutical composition which comprises admixing a pharmaceutically suitable carrier with a compound prepared according to any one of Claims 1 through 45.

47. The process of Claim 46 wherein the pharmaceutical composition additionally contains a diuretic or a non-steroidal antiinflammatory drug.

## Patentansprüche (Patentansprüche für folgende(n) Vertragsstaat(en): AT, BE, CH, DE, DK, FR, GB, IT, LI, LU, NL, SE)

1. Blutdrucksenkende Verbindung der Formel 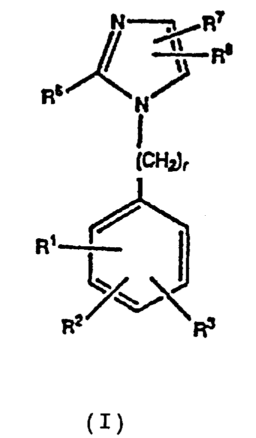 worin
R¹ 4-CO₂H; 4-CO₂R^{9;} 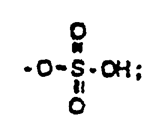 -SO₃H; - C(CF₃)₂OH; 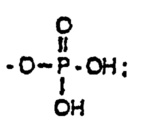 -PO₃H₂; 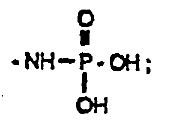 4-NHSO₂CH₃; 4-NHSO₂CF₃; -CONHOR^{12;} -SO₂NH₂; 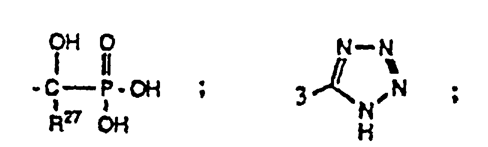 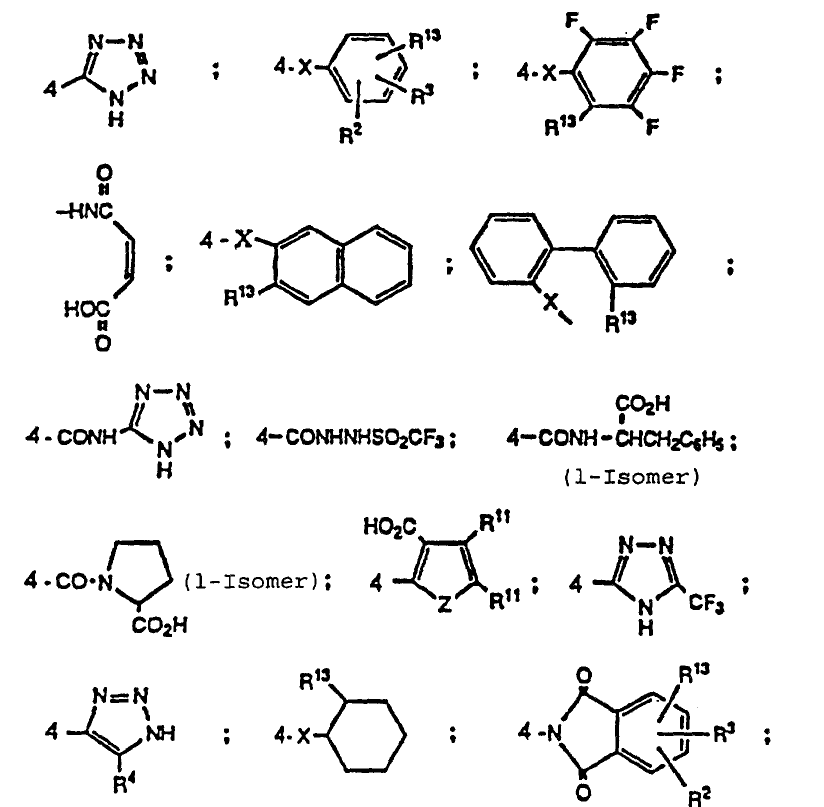 oder 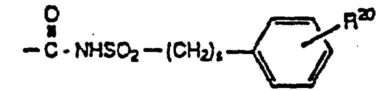 ist,
R H, Cl, Br, I, F, NO₂, CN, Alkyl mit 1 bis 4 Kohlenstoffatomen, Acyloxy mit 1 bis 4 Kohlenstoffatomen, Alkoxy mit 1 bis 4 Kohlenstoffatomen, CO₂H, CO₂R⁹, NHSO₂CH₃, NHSO₂CF₃, CONHOR¹, SO₂NH₂, 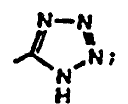 Phenyl oder Furyl ist,
R³ H, Cl, Br, I oder F, Alkyl mit 1 bis 4 Kohlenstoffatomen oder Alkoxy mit 1 bis 4 Kohlenstoffatomen ist,
R⁴ CN, NO₂ oder CO₂R¹¹ ist,
R⁵ H, Alkyl mit 1 bis 6 Kohlenstoffatomen, Cycloalkyl mit 3 bis 6 Kohlenstoffatomen, Alkenyl oder Alkinyl mit 2 bis 4 Kohlenstoff ist,
R⁶ Alkyl mit 2 bis 10 Kohlenstoffatomen, Alkenyl oder Alkinyl mit 3 bis 10 Kohlenstoffatomen oder dieselben, durch F oder CO₂R¹⁴ substituierten Gruppen, Cycloalkyl mit 3 bis 8 Kohlenstoffatomen, Cycloalkylalkyl mit 4 bis 10 Kohlenstoffatomen, Cycloalkylalkenyl oder Cycloalkylalkinyl mit 5 bis 10 Kohlenstoffatomen, gegebenenfalls durch F oder CO₂R¹⁴ substituiertes (CH₂)ₛZ(CH₂)ₘR⁵, Benzyl oder im Phenylring durch 1 oder 2 Halogene, Alkoxy mit 1 bis 4 Kohlenstoffatomen, Alkyl mit 1 bis 4 Kohlenstoffatomen oder Nitro substituiertes Benzyl ist,
R⁷ Vinyl, Cycloalkylidenyl, Alkinyl mit 2-10 Kohlenstoffatomen, Phenylalkinyl, worin der Alkinylteil 2-6 Kohlenstoffatome darstellt, aus 2- und 3-Thienyl, 2- und 3-Furyl, 2-, 3- und 4-Pyridyl, 2-Pyrazinyl, 2-, 4- und 5-Pyrimidinyl, 3- und 4-Pyridazinyl, 2-, 4- und 5-Thiazolyl, 2-, 4- und 5-Selenazolyl und 2-, 4- und 5-Oxazolyl, 2- oder 3-Pyrrolyl, 3-, 4- oder 5-Pyrazolyl, 2-, 4- oder 5-Imidazolyl ausgewähltes Heteroaryl, o-, m- oder p-Biphenylyl, o-, m- oder p-Phenoxyphenyl, 2-Oxazolinyl, 2-Thiazolinyl, substituiertes, vorstehend definiertes Phenylalkinyl, Heteroaryl, Biphenylyl oder Phenoxyphenyl, das durch 1 oder 2 Substituenten substituiert ist, die aus Halogen, Hydroxy, Mercapto, Alkoxy mit 1-5 Kohlenstoffatomen, Alkyl mit 1-5 Kohlenstoffatomen, -NO₂, -CN, -CF₃, -COR¹⁶, -CH₂OR¹⁷, -NHCOR¹⁷, -CONR¹⁸R¹⁹, S(O)ᵣR¹⁷ und SO₂NR¹⁸R¹⁹ ausgewählt sind, vorstehend definiertes Pyrrolyl, Pyrazolyl oder Imidazolyl, das am Ringstickstoff durch Alkyl mit 1-5 Kohlenstoffatomen, Phenyl oder Benzyl substituiert ist, oder substituiertes Alkyl, Alkenyl oder Alkinyl mit 1 bis 10 Kohlenstoffatomen ist, das durch eine substituierte oder unsubstituierte, vorstehend definierte Heteroaryl-, Biphenylyl- oder Phenoxyphenylgruppe, -S(O)ᵣ-Heteroaryl, -S-(O)ᵣ-Biphenylyl, -S(O)ᵣ-Phenoxyphenyl, -S-Tetrazol, -S(O)ᵣR¹⁷, -NR¹⁸R¹⁹, -NR¹⁸-Heteroaryl, -NR¹⁸-Phenyl, -NR¹⁸-Biphenylyl, -NR¹⁸-Phenoxyphenyl, -N-Phthalimido, -NH-SO₂-Phenoxyphenyl, -NH-SO₂-Heteroaryl, -NH-SO₂-Biphenylyl, -NH-SO₂-R¹⁷ und -S-(C=O)-R¹⁷, -N-Imidazolyl, N-1,2,3-Triazolyl, N-1,2,4-Triazolyl substituiert ist, worin Heteroaryl ein im Umfang von R⁷ definierter Heterocyclus ist und worin die Phenylgruppe in R¹⁷ von -S-(O)ᵣR¹⁷, N-Imidazolyl, N-1,2,3-Triazolyl und N-1,2,4Triazolyl durch einen oder zwei vorstehend für Heteroaryl beschriebene Substituenten substituiert sein können,
R⁸ 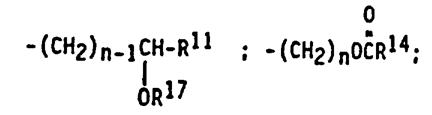 -(CH₂)ₙSR¹⁵_{;} 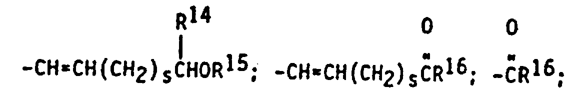 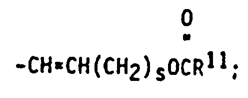 -(CH₂)ₘ-Tetrazolyl; 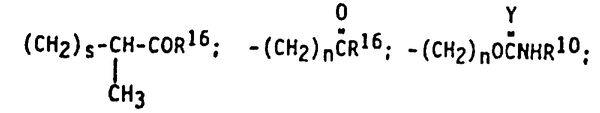 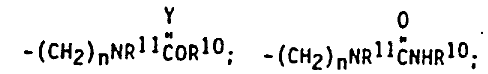 -(CH₂)ₙNR¹¹SO₂R¹⁰; 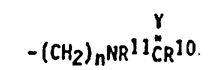 ist,
R⁹ 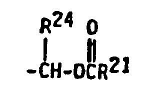 ist,
R¹⁰ Alkyl mit 1 bis 6 Kohlenstoffatomen oder Perfluoralkyl mit 1 bis 6 Kohlenstoffatomen, 1-Adamantyl, 1-Naphthyl, 1-(1-Naphthyl)ethyl oder (CH₂)ₚC₆H₅ ist,
R¹¹ H, Alkyl mit 1 bis 6 Kohlenstoffatomen, Cycloalkyl mit 3 bis 6 Kohlenstoffatomen, Phenyl oder Benzyl ist,
R¹ H, Methyl oder Benzyl ist,
R¹³ -CO₂H, -CO₂R⁹, -CH₂CO₂H, -CH₂CO₂R⁹ oder 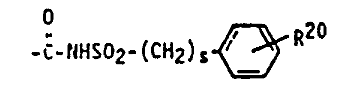 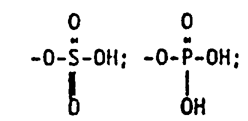 -SO₃H; 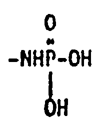 -PO₃H₂; -C(CF₃)₂OH; -NHSO₂CH₃; -NHSO₂CF₃; -NHCOCF₃; -CONHOR¹; -SO₂NH₂; 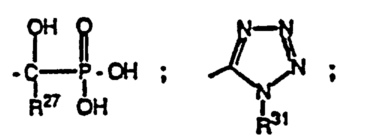 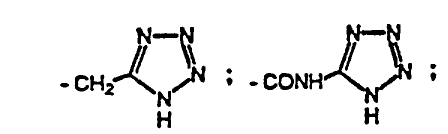 - CONHNHSO₂CF₃ ; 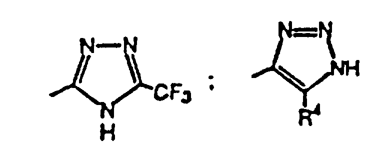 ist,
R¹⁴ H, Alkyl oder Perfluoralkyl mit 1 bis 8 Kohlenstoffatomen, Cycloalkyl mit 3 bis 6 Kohlenstoffatomen, Phenyl oder Benzyl ist,
R¹⁵ H, Alkyl mit 1 bis 6 Kohlenstoffatomen, Cycloalkyl mit 3 bis 6 Kohlenstoffatomen, Phenyl, Benzyl, Acyl mit 1 bis 4 Kohlenstoffatomen, Phenacyl ist,
R¹⁶ H, Alkyl mit 1 bis 6 Kohlenstoffatomen, Cycloalkyl mit 3 bis 6 Kohlenstoffatomen, (CH₂)ₚC₆H₅, OR¹⁷ oder NR¹⁸R¹⁹ ist,
R¹⁷ H, Alkyl mit 1 bis 6 Kohlenstoffatomen, Cycloalkyl mit 3 bis 6 Kohlenstoffatomen, Phenyl oder Benzyl ist,
R¹⁸ und R¹⁹ unabhängig H, Alkyl mit 1 bis 4 Kohlenstoffatomen, Phenyl, Benzyl, α-Methylbenzyl sind oder mit dem Stickstoffatom zusammengenommen einen Ring der Formel 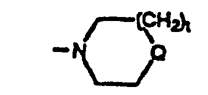 bilden,
Q NR⁰, O oder CH₂ ist,
R⁰ H, Alkyl mit 1-4 Kohlenstoffatomen oder Phenyl ist,
R¹ Alkyl mit 1 bis 6 Kohlenstoffatomen, -NRR³ oder 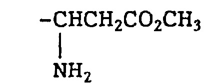 ist,
R und R³ unabhängig H, Alkyl mit 1 bis 6 Kohlenstoffatomen, Benzyl oder zusammengenommen (CH₂)ᵤ sind, worin u 3-6 ist,
R⁴ H, CH₃ oder -C₆H₅ ist,
R⁵ NR⁷R⁸, OR⁸, NHCONH₂, NHCSNH₂, 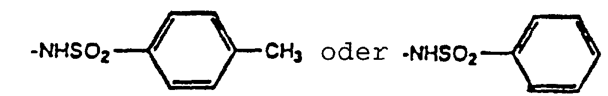 ist,
R⁶ Wasserstoff, Alkyl mit 1 bis 6 Kohlenstoffatomen, Benzyl oder Allyl ist,
R⁷ und R⁸ unabhängig Wasserstoff, Alkyl mit 1 bis 5 Kohlenstoffatomen oder Phenyl sind,
R⁹ und R³⁰ unabhängig Alkyl mit 1-4 Kohlenstoffatomen oder zusammengenommen -(CH₂)_{q}- sind,
R³¹ H, Alkyl mit 1 bis 4 Kohlenstoffatomen, -CH₂CH=CH₂ oder -CH₂C₆H₄R³ ist,
R³ H, NO₂, NH₂, OH oder OCH₃ ist,
X eine Kohlenstoff-Kohlenstoff-Einfachbindung, -CO-, -CH₂-, -O-, -S-, -NH-, 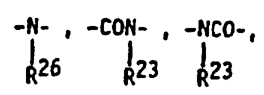 -OCH₂-, -CH₂O-, -SCH₂-, -CH₂S-, -NHC(R⁷)(R⁸)-, -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 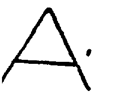 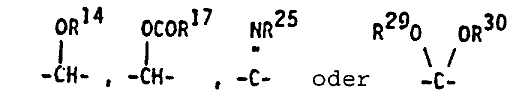 ist,
Y O oder S ist,
Z O, NR¹¹ oder S ist,
m 1 bis 5 ist,
n 1 bis 10 ist,
p 0 bis 3 ist,
q 2 bis 3 ist,
r 0 bis 2 ist,
s 0 bis 5 ist,
t 0 oder 1 ist,
oder ein pharmazeutisch annehmbares Salz derselben, vorausgesetzt, daß
(1) sich die Gruppe R¹ nicht in ortho-Stellung befindet,
(2) wenn R¹ 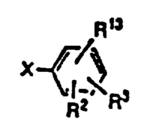 ist, X eine Einfachbindung ist und R¹³ CO₂H oder 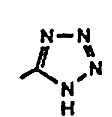 ist, R¹³ sich dann in ortho- oder meta- Stellung befinden muß, oder wenn R¹ und X wie vorstehend sind und R¹³ NHSO₂CF₃ oder NHSO₂CH₃ ist, R¹³ ortho sein muß,
(3) wenn R¹ 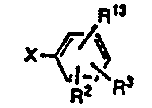 ist und X etwas anderes als eine Einfachbindung ist, R¹³ dann ortho sein muß, außer wenn X = NR³CO und R¹³ NHSO₂CF₃ oder NHSO₂CH₃ ist, R¹³ dann ortho oder meta sein muß,
(4) wenn R¹ 4-CO₂H oder ein Salz davon ist, R⁶ nicht S-Alkyl sein kann,
(5) wenn R¹ 4-CO₂H oder ein Salz davon ist, der Substituent in der 4-Stellung des Imidazols nicht CH₂OH, CH₂OCOCH₃ oder CH₂CO₂H sein kann,
(6) wenn R¹ 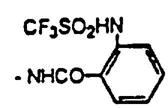 ist, R⁶ nicht Methoxybenzyl ist,
(7) die Gruppe R⁶ nicht 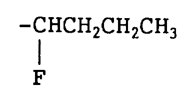 oder CH₂OH ist.

2. Verbindung von Anspruch 1 mit der Formel 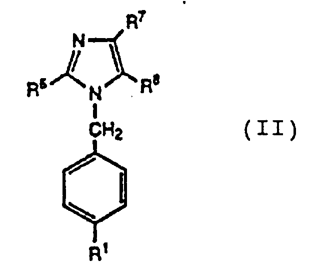 worin
R⁴ - CO₂H ; -NHSO₂CF₃ ; 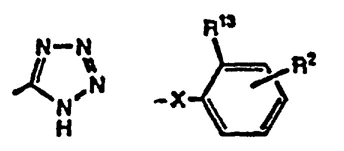 oder 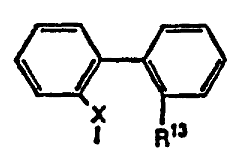 ist,
R⁶ Alkyl mit 3 bis 10 Kohlenstoffatomen, Alkenyl mit 3 bis 10 Kohlenstoffatomen, Alkinyl mit 3 bis 10 Kohlenstoffatomen, Cycloalkyl mit 3 bis 8 Kohlenstoffatomen, Benzyl ist, das am Phenylring mit bis zu zwei Gruppen substituiert ist, die aus Alkoxy mit 1 bis 4 Kohlenstoffatomen, Halogen, Alkyl mit 1 bis 4 Kohlenstoffatomen und Nitro ausgewählt sind,
R⁸ - (CH₂)ₘ- Tetrazolyl, - (CH₂)ₙOR¹¹; 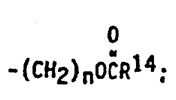 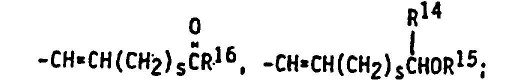 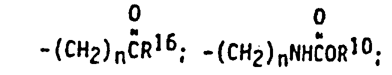 - (CH₂)ₙNHSO₂R¹⁰;
oder 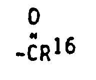 ist,
R¹³ -CO₂H, -CO₂R⁹, NHSO₂CF₃, SO₃H oder 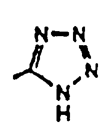 ist,
R¹⁶ H, Alkyl mit 1 bis 5 Kohlenstoffatomen, OR¹⁷ oder NR¹⁸R¹⁹ ist,
X eine Kohlenstoff-Kohlenstoff-Einfachbindung, -CO-, 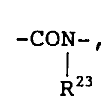 -CH₂CH₂- , 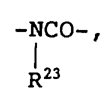 -OCH₂-, -CH₂O- , -O-, -SCH₂- , -CH₂S - , -NHCH₂- , -CH₂NH- oder -CH=CH- ist,
oder ein pharmazeutisch annehmbares Salz derselben.

3. Verbindung von Anspruch 2, worin
R H, Alkyl mit 1 bis 4 Kohlenstoffatomen, Halogen oder Alkoxy mit 1 bis 4 Kohlenstoffatomen ist,
R⁶ Alkyl, Alkenyl oder Alkinyl mit 3 bis 7 Kohlenstoffatomen ist,
R⁷ Heteroaryl ist, das aus 2- und 3-Thienyl, 2- und 3-Furyl, 2-, 3- und 4-Pyridyl oder p-Biphenylyl 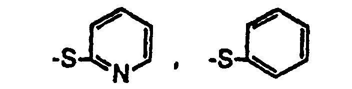 ausgewählt ist,
R⁸ -(CH₂)ₘOR¹¹; 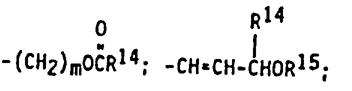 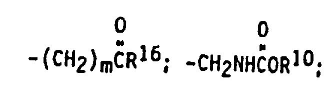 -(CH₂)ₘNHSO₂R¹⁰ oder -COR¹⁶ ist,
R¹⁰ CF₃, Alkyl mit 1 bis 6 Kohlenstoffatomen oder Phenyl ist,
R¹¹ H oder Alkyl mit 1 bis 4 Kohlenstoffatomen ist,
R¹³ CO₂H, CO₂CH₂OCOC (CH₃)₃, NHSO₂CF₃ oder 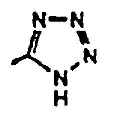 ist,
R¹⁴ H oder Alkyl mit 1 bis 4 Kohlenstoffatomen ist,
R¹⁵ H, Alkyl mit 1 bis 4 Kohlenstoffatomen oder Acyl mit 1 bis 4 Kohlenstoffatomen ist,
R¹⁶ H, Alkyl mit 1 bis 5 Kohlenstoffatomen, OR¹⁷ oder 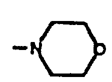 ist,
m 1 bis 5 ist,
X = Einfachbindung, -O-, -CO-, -NHCO- oder -OCH₂-, oder ein pharmazeutisch annehmbares Salz derselben.

4. Verbindung von Anspruch 3, worin R¹ 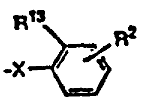 ist und X eine Einfachbindung ist oder ein pharmazeutisch geeignetes Salz derselben.

5. Pharmazeutische Zusammensetzung, umfassend einen pharmazeutisch annehmbaren Träger und eine Verbindung eines der Ansprüche 1 bis 4.

6. Pharmazeutische Zusammensetzung von Anspruch 5, die zusätzlich einen diuretischen oder einen nicht-steroidalen, entzündungshemmenden Wirkstoff enthält.

7. Verwendung einer Verbindung eines der Ansprüche 1 bis 4 zum Herstellen eines Arzneimittels zum Verhüten von Nierenversagen in einem warmblütigen Tier, das aus der Verabreichung eines nicht-steroidalen, entzündungshemmenden Wirkstoffs (NSAID) folgt.

8. Verwendung einer Verbindung eines der Ansprüche 1 bis 4 zum Herstellen eines Arzneimittels zum Behandeln von Bluthochdruck in einem warmblütigen Tier.

9. Verwendung von Anspruch 8, wobei das Arzneimittel weiter ein Diuretikum umfaßt.

10. Verwendung einer Verbindung eines der Ansprüche 1 bis 4 zum Herstellen eines Arzneimittels zum Behandeln von Stauungsinsuffizienz in einem warmblütigen Tier.

11. Verfahren zur Herstellung einer Verbindung von Anspruch 1, worin r 1 ist, welches das Zusammenbringen eines Imidazolderivats der Formel 1 mit einem Benzylderivat der Formel 2 in einem Lösungsmittel in Anwesenheit einer Base über etwa 1 bis etwa 10 Stunden bei einer Temperatur im Bereich von etwa 20°C bis zur Rückflußtemperatur des Lösungsmittels unter Bilden eines Benzylimidazols der Formel 3: 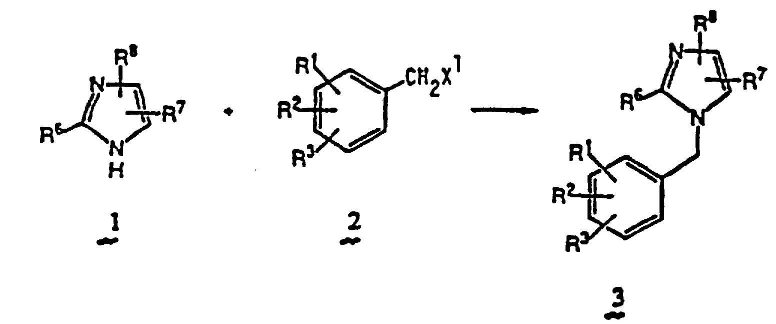 worin R¹, R, R³, R⁶, R⁷ und R⁸ jeweils unter den Reaktionsbedingungen stabil sind und eine in Anspruch 1 definierte Gruppe oder ein Zwischenprodukt oder eine geschützte Form davon sind, welche in eine derartige Gruppe umgewandelt werden kann und worin X¹ Halogen, p-Toluolsulfonyloxy oder Methylsulfonyloxy ist, und danach nötigenfalls das Umwandeln des Zwischenprodukts oder der geschützten Form der in Anspruch 1 definierten Gruppen R in Gruppen R umfaßt.

12. Verfahren von Anspruch 11, wobei Verbindung 1 und 2 in Anwesenheit einer Base, die aus der Gruppe ausgewählt ist, die aus einem Metallhydrid (MH), einem Metallalkoxid (MOR), Natriumcarbonat, Kaliumcarbonat, Triethylamin und Pyridin besteht, in einem dipolaren aprotischen Lösungsmittel zusammengebracht werden oder, wenn die Base MOR ist, das Lösungsmittel ein Alkohol, ROH, sein kann, worin M Lithium, Natrium oder Kalium ist und R Methyl, Ethyl oder t-Butyl ist.

13. Verfahren von Anspruch 11, wobei ein Zweiphasen-Lösungsmittelsystem, eines eine organische Phase wie etwa Methylenchlorid und das andere eine wäßrige Phase, in Anwesenheit eines Phasentransferkatalysators wie etwa Tricaprylmethylammoniumchlorid verwendet wird.

14. Verfahren von Anspruch 12, wobei R¹ 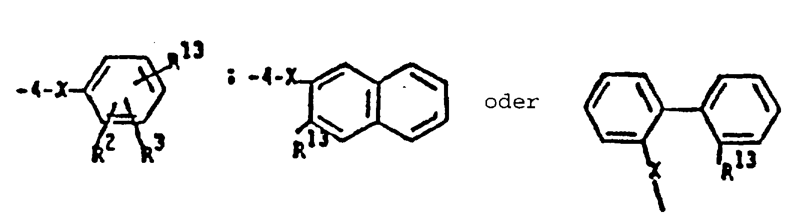 ist,
X eine Kohlenstoff-Kohlenstoff-Einfachbindung, -CO-, -O-, -S- oder -NH- ist,
R und R³ jeweils unabhängig H, Cl, Br, I, CO₂R¹⁴, F, NO₂, Alkyl mit 1 bis 4 Kohlenstoffatomen, Alkoxy mit 1 bis 4 Kohlenstoffatomen, Phenyl oder Furyl sind,
R⁶ und R⁷ wie in Anspruch 1 definiert sind,
R⁸ -(CH₂)ₙOR¹¹, -(CH₂)ₙSR¹⁵ oder -(CH₂)ₙCN ist,
R¹¹ wie in Anspruch 1 ist,
R¹³ CO₂R¹⁴, CN, NO₂, Trialkylzinntetrazol oder Trityltetrazol ist und
R¹⁴ und R¹⁵ wie in Anspruch 1 definiert sind.

15. Verfahren von Anspruch 14, wobei R¹³ -CO₂R¹⁴ ist und das Produkt der Formel 3 1-24 Stunden mit einem Alkali in einem wäßrigalkoholischen Lösungsmittel oder mit CO₃CO₂H bei einer Temperatur im Bereich von 20°C bis zur Rückflußtemperatur des Lösungsmittels zusammengebracht wird, gefolgt von der Einstellung des pH des Gemisches auf einen Wert im Bereich von 3 bis 7 unter Überführen des Produkts in das entsprechende Produkt, worin R¹³ -CO₂H ist.

16. Verfahren von Anspruch 15, wobei wenigstens eines von R, R³ oder R¹³ in Formel 1 -CO₂R¹⁴ ist und in -CO₂H überführt wird.

17. Verfahren von Anspruch 15, wobei R¹⁴ t-Butyl ist und die Reaktion in CF₃CO₂H ausgeführt wird.

18. Verfahren von Anspruch 14, wobei R¹³ -CN ist und das Produkt der Formel 3 mit (i) einer starken Säure 2-96 Stunden bei der Rückflußtemperatur des Lösungsmittels oder (ii) einem starken Alkali in einem Alkohollösungsmittel 2-96 Stunden bei einer Temperatur im Bereich von 20°C und der Rückflußtemperatur des Lösungsmittels, gefolgt von der Einstellung des pH auf 3-7, oder (iii) Schwefelsäure gefolgt von Säure oder Alkali unter Überführen des Produkts in die entsprechende Verbindung, worin R¹³ -CO₂H ist, zusammengebracht wird.

19. Verbindung von Anspruch 18, wobei wenigstens eines von R, R³ oder R¹³ -CO₂R¹⁴ ist, und in -CO₂H überführt wird.

20. Verfahren von Anspruch 18, wobei R⁸ -(CH₂)ₙCN ist und in -(CH₂)ₙCO₂H überführt wird oder -(CH₂)ₙOR¹¹ ist und in (CH₂)ₙOH überführt wird, wenn R¹³ in -CO₂H überführt wird.

21. Verfahren von Anspruch 14, wobei R¹³ -CN ist, und das Produkt der Formel 3 mit einem Gemisch äquimolarer Mengen Natriumazid und Ammoniumchlorid in einem polaren aprotischen Lösungsmittel bei einer Temperatur im Bereich von 30°C bis zur Rückflußtemperatur des Lösungsmittels 1. Stunde bis 10 Tage unter Überführen des Produkts in die entsprechende Verbindung, worin R¹³ 5-Tetrazolyl ist, zusammengebracht wird.

22. Verfahren von Anspruch 21, wobei R⁸ -(CH₂)ₘCN ist und in -(CH₂)ₘ-Tetrazolyl überführt wird, wenn R¹³ in 5-Tetrazolyl überführt wird.

23. Verfahren von Anspruch 14, wobei R¹³ -CN ist, und das Produkt der Formel 3 mit Trialkylzinnazid oder Triarylzinnazid umgesetzt wird, gefolgt von saurer oder basischer Hydrolyse unter Überführen des Produkts in die entsprechende Verbindung, worin R¹³ 5-Tetrazolyl ist.

24. Verfahren von Anspruch 14, wobei R¹³ -CN ist und das Produkt der Formel 3 mit Trialkylzinnazid oder Triarylzinnazid unter Herstellen einer Verbindung der Formel 3 umgesetzt wird, worin R¹³ Trialkyl- oder Triarylstannyltetrazol-5-yl ist, letztere Verbindung mit Triphenylmethylchlorid unter Erzeugen einer Verbindung der Formel 3, worin R¹³ Triphenylmethyltetrazol-5-yl ist, umgesetzt wird und letztere Verbindung unter Erzeugen einer Verbindung der Formel 3, worin R¹³ 5-Tetrazolyl ist, hydrolysiert wird.

25. Verfahren von Anspruch 23, worin R⁸ -(CH₂)ₙCN ist und in -(CH₂)ₘ-Tetrazolyl überführt wird, wenn R¹³ in 5-Tetrazolyl überführt wird.

26. Verfahren von Anspruch 14, wobei R¹³ -NO₂ ist und das Produkt der Formel 3 mit einem Reduktionsmittel unter Bilden eines zweiten Zwischenprodukts der Formel 3, in der R¹³ NH₂ ist, zusammengebracht wird und letztere mit einem Sulfonsäureanhydrid (CH₃SO₂)₂O oder (CF₃SO₂)₂O oder -chlorid CH₃SO₂Cl oder CF₃SO₂Cl in einem Lösungsmittel unter Herstellen einer Verbindung zusammengebracht wird, in welcher R¹³ -NHSO₂CH₃ oder -NHSO₂CF₃ ist.

27. Verfahren von Anspruch 26, wobei wenigstens eines von R, R³ oder R¹³ -NO₂ ist und in -NHSO₂CH₃ oder-NHSO₂CF₃ überführt wird.

28. Verfahren von Anspruch 15 oder 18, wobei die Verbindung der Formel 3 mit R¹³ = CO₂H unter Liefern einer Verbindung, in der R¹³ CONHOR¹ ist, entweder
(a) mit etwa 1-4 Äquivalenten Thionylchlorid in überschüssigem Thionylchlorid oder einem weiteren Lösungsmittel bei einer Temperatur im Bereich von 20°C bis zur Rückflußtemperatur des Lösungsmittels über einen Zeitraum von 5 Minuten bis 2 Stunden unter Bilden eines Zwischenproduktes der Formel 3, worin R¹³ COCl ist, zusammengebracht wird und letzteres 2-18 Stunden bei einer Temperatur im Bereich von 25-80°C mit 2-10 Äquivalenten eines Hydroxylaminderivates H₂NOR¹ in überschüssigem Hydroxylaminderivat H₂NOR¹ oder einem anderen Lösungsmittel zusammengebracht wird, oder
(b) mit dem Hydroxylaminderivat H₂NOR¹, Dicyclohexylcarbodiimid und 1-Hydroxybenzotriazol in einem Lösungsmittel bei einer Temperatur im Bereich von 0-30°C 1-24 Stunden zusammengebracht wird.

29. Verfahren von Anspruch 11, wobei R¹ 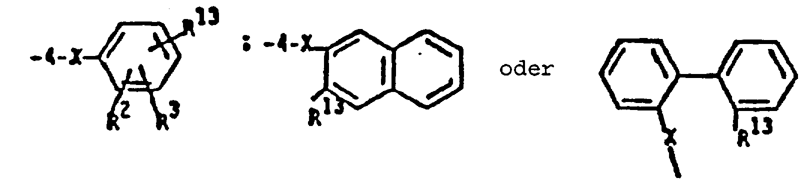 ist,
X eine Kohlenstoff-Kohlenstoff-Einfachbindung, -CO-, -O-, -S- oder -NH- ist,
R, R³, R⁶ und R⁷ wie in Anspruch 1 definiert sind und
R⁸ (CH₂)ₙOR¹¹, (CH₂)ₙOCOR¹⁴, (CH₂)ₙCH(OH)R¹⁶, (CH₂)ₙCOR¹⁶(CH₂)ₙCl, (CH₂)ₙCN, CHO ist.

30. Verfahren von Anspruch 29, wobei R⁸ (CH₂)ₙOH ist und das Produkt der Formel 3 mit einem Alkohol R¹¹OH in wasserfreiem Zustand in Anwesenheit einer starken Säure oder einer Lewissäure zusammengebracht wird, gefolgt von der Verseifung irgendwelcher Gruppen CO₂R¹⁴, die im Zwischenprodukt gleichzeitig gebildet werden oder zugegen sind, unter Bilden der entsprechenden Verbindung der Formel 3, worin R⁸ (CH₂)ₙOR¹¹ ist und R¹¹ nicht H ist.

31. Verfahren von Anspruch 29, wobei R⁸ (CH₂)ₙOR¹¹ ist und R¹¹ nicht H ist und das Produkt der Formel 3 mit einem wäßrigen sauren Medium bei einer Temperatur im Bereich von 25°C und der Rückflußtemperatur des Lösungsmittels über einen Zeitraum von 0,5-24 Stunden unter Bilden der entsprechenden Verbindung der Formel 3, worin R⁸ (CH₂)ₙOH ist, zusammengebracht wird.

32. Verfahren von Anspruch 29, wobei R⁸ (CH₂)ₙOH ist und das Produkt der Formel 3 mit
(a) einem Carbonsäureanhydrid (R¹⁴CO)₂O oder -chlorid R¹⁴COCl 0,5-24 Stunden in einem Lösungsmittel in Anwesenheit einer Base bei einer Temperatur im Bereich von 0°C und der Rückflußtemperatur des Lösungsmittels oder
(b) eine Carbonsäure R¹⁴CO₂H unter wasserfreien Bedingungen in Anwesenheit einer starken Säure oder Lewissäure 0,5 bis 24 Stunden bei 0°-100°C unter Bilden der entsprechenden Verbindung, in der R⁸ (CH₂)ₙOCOR¹⁴ ist, zusammengebracht wird.

33. Verfahren von Anspruch 29, wobei R⁸ (CH₂)ₙOCOR¹⁴ ist und das Produkt der Formel 3 mit wäßriger Säure oder Alkali unter Bilden der entsprechenden Verbindung, worin R⁸ (CH₂)ₙOH ist, zusammengebracht wird.

34. Verfahren von Anspruch 29, wobei R⁸ (CH₂)ₙOH ist und das Produkt der Formel 3 mit einem Oxidationsmittel 1-200 Stunden bei einer Temperatur von 25-45°C unter Herstellen einer entsprechenden Verbindung der Formel 3 zusammengebracht wird, in welcher R⁸ (CH₂)ₙ₋₁COR¹⁶ ist und R¹⁶ H ist.

35. Verfahren von Anspruch 29, wobei R⁸ (CH₂)ₙCOR¹⁶ ist und R¹⁶ H ist und das Produkt der Formel 3 mit einer metallorganischen Verbindung R¹⁶P, in der P MgBr oder Li ist, 0,5-24 Stunden bei einer Temperatur im Bereich von -78°C bis 100°C unter Bilden einer Verbindung der Formel 3 zusammengebracht wird, in welcher R⁸ (CH₂)ₙCH(OH)R¹⁶ ist und R¹⁶ nicht H ist.

36. Verfahren von Anspruch 29, wobei R⁸ (CH₂)ₙCH(OH)R¹⁶ ist und R¹⁶ nicht H ist und das Produkt der Formel 3 mit einem Oxidationsmittel in einem Lösungsmittel unter Bilden einer entsprechenden Verbindung der Formel 3 zusammengebracht wird, in welcher R⁸ (CH₂)ₙCOR¹⁶ ist und R¹⁶ nicht H ist.

37. Verfahren von Anspruch 29, wobei R⁸ (CH₂)ₙCOR¹⁶ ist und R¹⁶ H ist und die Verbindung der Formel 3 mit einem Oxidationsmittel in einem Lösungsmittel unter Bilden einer entsprechenden Verbindung der Formel 3 zusammengebracht wird, in welcher R⁸ (CH₂)ₙCOR¹⁶ ist und R¹⁶ OH ist.

38. Verfahren von Anspruch 29, wobei R⁸ (CH₂)ₙCOR¹⁶ ist und R¹⁶ OH ist und die Verbindung der Formel 3 mit Thionylchlorid im Überschuß oder einem weiteren Lösungsmittel bei einer Temperatur im Bereich von 0°C bis zur Rückflußtemperatur des Lösungsmittels 5 Minuten bis 24 Stunden unter Bilden einer entsprechenden Verbindung der Formel 3, in der R⁸ (CH₂)ₙCOCl ist, zusammengebracht wird, gefolgt vom Zusammenbringen der letzteren mit einem Amin NHR¹⁸R¹⁹ im Überschuß oder in einem Lösungsmittel 5 Minuten bis 24 Stunden bei Temperaturen im Bereich von 0°C und Rückflußtemperatur des Lösungsmittels unter Bilden einer entsprechenden Verbindung der Formel 3, in der R⁸ (CH₂)ₙCONR¹⁸R¹⁹ ist.

39. Verfahren von Anspruch 29, wobei R⁸ (CH₂)ₙOR¹¹ ist und R¹¹ H ist und das Produkt der Formel 3 mit Thionylchlorid im Überschuß oder in einem Lösungsmittel bei einer Temperatur im Bereich von 20°C bis zur Rückflußtemperatur des Lösungsmittels 0,5-24 Stunden unter Bilden einer Zwischenproduktverbindung der Formel 3 zusammengebracht wird, in der R⁸ (CH₂)ₙCl ist.

40. Verfahren von Anspruch 39, wobei die Verbindung der Formel 3, in der R⁸ (CH₂)ₙCl ist, mit dem Natrium- oder Kaliumsalz eines Mercaptans R¹⁵SH 1-24 Stunden in einem Lösungsmittel bei einer Temperatur von 25-100°C unter Bilden einer Verbindung der Formel zusammengebracht wird, in welcher R⁸ (CH₂)ₙSR¹⁵ ist.

41. Verfahren von Anspruch 29, wobei die Verbindung der Formel 3, in der R⁸ (CH₂)ₙCl ist, mit einem Alkalimetallcyanid 1-24 Stunden in einem Lösungsmittel bei einer Temperatur im Bereich von 20-100°C unter Bilden einer Verbindung der Formel 3 zusammengebracht wird, in welcher R⁸ (CH₂)ₙCN ist und die letztere Verbindung zu der entsprechenden Verbindung der Formel 3 hydrolysiert wird, in der R⁸ (CH₂)ₙCOR¹⁶ ist und R¹⁶ OH ist.

42. Verfahren von Anspruch 29, wobei die Verbindung der Formel 3, in der R⁸(CH₂)ₙ₋₁Cl ist, mit dem Natrium- oder Kaliumsalz eines Malonsäuredialkylesters 0,5-24 Stunden in einem Lösungsmittel bei einer Temperatur im Bereich von 20-100°C unter Bilden einer Verbindung der Formel 3 zusammengebracht wird, in welcher R⁸ (CH₂)ₙ₋₁CH(CO₂Alkyl)₂ ist, gefolgt von der Verseifung der letzteren mit wäßrigem Alkali bei einer Temperatur im Bereich von 25 °C bis zur Rückflußtemperatur des Lösungsmittels, gefolgt vom Ansäuern mit Mineralsäure unter Bilden einer Verbindung der Formel 3, in der R⁸ (CH₂)ₙ₋₁CH(CO₂H)₂ ist, gefolgt vom Erhitzen der letzteren auf 120°C oder in verdünnter Mineralsäure bei Rückflußtemperatur unter Bilden eines Produkts der Formel 3, in der R⁸ (CH₂)ₙCOR¹⁶ ist und R¹⁶ OH ist.

43. Verfahren von Anspruch 29, wobei R⁸ -CHO ist und die Verbindung der Formel 3 mit einem Methylenphosphoran (C₆H₅)₃P=CH(CH₂)ₛCHR¹⁴OR¹⁵ oder (C₆H₅)₃P=CH(CH₂)ₛCOR¹⁶ 1-24 Stunden in einem Lösungsmittel bei einer Temperatur im Bereich von 25°C bis zur Rückflußtemperatur des Lösungsmittels unter Bilden einer Verbindung der Formel 3 zusammengebracht wird, in welcher R⁸ -CH=CH(CH₂)ₛCHR¹⁴OR¹⁵ oder -CH=CH(CH₂)ₛCOR¹⁶ ist, außer wenn R¹⁵ H ist und R¹⁶ OH ist, und gegebenenfalls anschließend 0,5-24 Stunden Zusammenbringen der Verbindung der Formel 3, in der R⁸ -CH=CH(CH₂)ₛCOR¹⁶ ist, mit einem Reduktionsmittel in einem Lösungsmittel bei einer Temperatur im Bereich von 0-25°C unter Bilden eines Produkts der Formel 3, in der R⁸ -CH=CH(CH₂)ₛCHR¹⁴OH ist.

44. Verfahren von Anspruch 29, wobei R⁸ (CH₂)ₙOH ist und die Verbindung der Formel 3 mit einem Isocyanat der Formel R¹⁰NCO in einem Lösungsmittel bei einer Temperatur im Bereich von 25°C bis zur Rückflußtemperatur des Lösungsmittels über einen Zeitraum von 5 Minuten bis 24 Stunden unter Bilden einer Verbindung der Formel 3 zusammengebracht wird, in welcher R⁸ (CH₂)ₙOCONHR¹⁰ ist.

45. Verfahren von Anspruch 29, wobei die Verbindung in der R⁸ (CH₂)ₙCl ist, mit einem Amin R¹¹NH₂ in überschüssigem Amin oder einem weiteren Lösungsmittel über einen Zeitraum von 1-24 Stunden bei einer Temperatur im Bereich von 0°C bis zur Rückflußtemperatur des Lösungsmittels unter Bilden eines Zwischenprodukts der Formel 3 zusammengebracht wird, in welcher R⁸ (CH₂)ₙNHR¹¹ ist.

46. Verfahren von Anspruch 29, wobei R⁸ (CH₂)ₙCl ist und die Verbindung der Formel 3 mit einem Alkalimetallazid in einem aprotischen Lösungsmittel 1-24 Stunden bei einer Temperatur im Bereich von 25-80°C unter Bilden einer Verbindung der Formel 3 zusammengebracht wird, in welcher R⁸ (CH₂)ₙN₃ ist und die letztere mit einem Reduktionsmittel unter Bilden eines Zwischenprodukts der Formel 3 zusammengebracht wird, in welcher R⁸ (CH₂)ₙNH₂ ist.

47. Verfahren von Anspruch 45 oder 46, wobei R⁸ (CH₂)ₙNHR¹¹ oder (CH₂)ₙNH₂ ist und die Verbindung der Formel 3 mit einem Chlorameisensäureester der Formel R¹⁰OCOCl oder einem Sulfonylderivat der Formel R¹⁰SO₂Cl oder (R¹⁰SO₂)₂O in einem Lösungsmittel in Anwesenheit einer Base 5 Minuten bis 24 Stunden bei einer Temperatur im Bereich von 0°C bis zur Rückflußtemperatur des Lösungsmittels unter Bilden einer Verbindung der Formel 3 zusammengebracht wird, in welcher R⁸ -(CH₂)ₙNR¹¹CO₂R¹⁰ oder -(CH₂)ₙNR¹¹SO₂R¹⁰ ist.

48. Verfahren von Anspruch 45 oder 46, wobei die Verbindung der Formel 3 mit R⁸ gleich -(CH₂)ₙNHR¹¹ oder (CH₂)ₙNH₂ mit einem Isocyanat oder Isothiocyanat R¹⁰NCY 5 Minuten bis 24 Stunden bei einer Temperatur im Bereich von 25°C bis zur Rückflußtemperatur des Lösungsmittels unter Bilden einer Verbindung der Formel 3 zusammengebracht wird, in welcher R⁸ -(CH₂)ₙNR¹¹CYNHR¹⁰ ist.

49. Verfahren von Anspruch 11, wobei R¹ NO₂ ist und R, R³, R⁶, R⁷ und R⁸ wie in Anspruch 34 definiert sind, wobei die Verbindung der Formel 3, in welcher R¹ NO₂ ist, mittels Eisen und Essigsäure, Zinn(II)-chlorid oder Wasserstoff und Palladium zu einer Verbindung der Formel 3 reduziert wird, worin R¹ NH₂ ist und letztere mit einem entsprechenden Säureanhydrid wie etwa Phthalanhydrid oder einem substituierten Phthalanhydrid in einem Lösungsmittel oder mit einem entsprechenden Säurechlorid wie etwa einem substituierten Anthranilsäurechlorid in Anwesenheit wäßrigen Alkalis oder einer Base oder mit einer entsprechend substituierten Phthal- oder Anthranilsäure in Anwesenheit von Dicyclohexylcarbodiimid unter Herstellen einer Verbindung der Formel 3 umgesetzt wird, in welcher R¹ 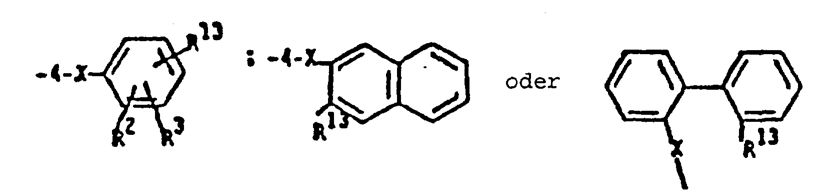 ist, und X NHCO ist.

50. Verfahren von Anspruch 11, wobei R¹ OCH₂C₆H₅ ist, R und R³ H sind und R⁶, R⁷ und R⁸ wie in Anspruch 11 definiert sind und die sich daraus ergebende Verbindung der Formel 3 mit R¹ gleich OCH₂C₆H₅ über einen Zeitraum von 0,2-1 Stunde mit Trifluoressigsäure bei Rückflußtemperatur oder mit Wasserstoff und Palladium unter Bilden der entsprechenden Verbindung der Formel 3 umgesetzt wird, in welcher R¹ OH ist und letztere mit einer Base bei 25°C und einem geeigneten Benzylhalogenid der Formel 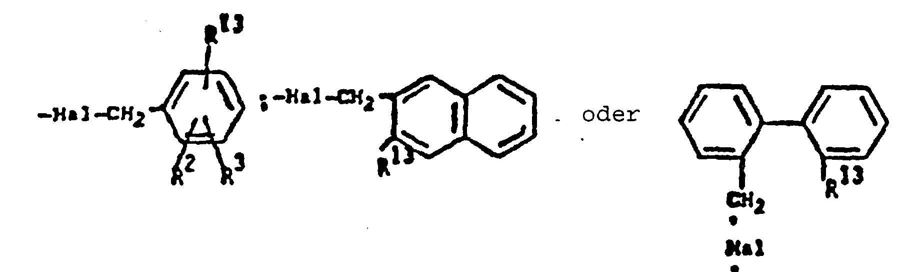 unter Herstellen der entsprechenden Verbindung der Formel 3 zusammengebracht wird, worin R¹ 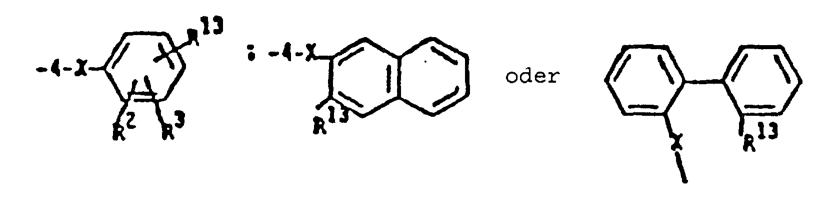 ist,
und X -OCH₂- ist.

51. Verfahren von Anspruch 11, wobei R⁸ -CHO ist, wobei das Benzylderivat der Formel 2 an dem Imidazolderivat der Formel 1 vorzugsweise an das dem Kohlenstoffatom des Imidazolrings, an das R⁸ gebunden ist, benachbarte Stickstoffatom gebunden ist.

52. Verfahren zur Herstellung einer Verbindung von Anspruch 1, worin r 0 ist, welches das Zusammenbringen eines Imidazolderivats der Formel 1 oder seines Metallsalzes mit 4-Fluor-1-nitrobenzol 1-10 Tage in einem Lösungsmittel in Anwesenheit einer Base, falls das freie Imidazol verwendet wird, bei einer Temperatur von 25-150°C unter Bilden eines N-Phenylimidazols umfaßt, gefolgt vom Aufbauen zu Verbindungen, worin X=NHCO, durch das in Anspruch 49 beschriebene Verfahren.

53. Verfahren von Anspruch 29, wobei R⁸ CHO ist und X eine Kohlenstoff-Kohlenstoff-Einfachbindung ist und das Produkt der Formel 3 mit einem metallorganischen Reagenz wie etwa R¹¹MgBr oder R¹¹Li in Anwesenheit eines wasserfreien Lösungsmittels ohne Hydroxylgruppen wie etwa Ether, THF oder Dimethoxyethan bei -78 bis 25°C, gefolgt von wäßriger Aufarbeitung, gefolgt von der sauren Hydrolyse irgendwelcher Gruppen CO₂R¹⁴, worin R¹⁴ t-Butyl ist, oder der Hydrolyse irgendwelcher tritylgeschützter Tetrazolgruppen unter Bilden der entsprechenden Verbindung der Formel 3, worin R⁸ 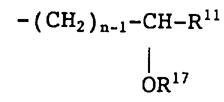 ist, worin R¹¹ ≠ H.

54. Verfahren zur Herstellung der Verbindungen von Anspruch 1, worin R⁷ = in Anspruch 1 definiertes substituiertes oder unsubstituiertes Biphenylyl, Phenoxyphenyl oder Heteroaryl, dadurch gekennzeichnet, daß das Halogenimidazol 237 mit einem Arylmetallderivat ArM, worin M = ZnBr, Me₃Sn oder B(OH)₂, in Anwesenheit eines Übergangsmetallkatalysators wie etwa Palladium, Platin, Nickel oder Zirkonium unter Bilden eines Arylimidazols 238 gekuppelt wird: 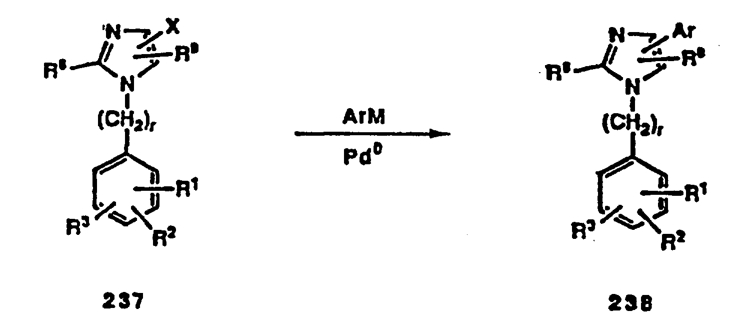 worin R¹, R, R³, R⁶, R⁸ und r die in Anspruch 1 angegebenen Bedeutungen haben, X Br oder I ist, M ZnBr, Me₃Sn, B(OH)₂ ist und Ar für R⁷ in Anspruch 1 definiertes substituiertes oder unsubstituiertes Biphenylyl, Phenoxyphenylmethyl oder Heteroaryl ist.

55. Verfahren zur Herstellung der Verbindungen von Anspruch 1, worin R⁷ = in Anspruch 1 definiertes substituiertes oder unsubstituiertes Biphenylylmethyl, Phenoxyphenylmethyl oder Heteroarylmethyl, dadurch gekennzeichnet, daß ein Halogenimidazol 237 mit einem Arylmethylmetallderivat ArCH₂M' in Anwesenheit eines Übergangsmetallkatalysators unter Bilden eines Arylmethylimidazols 240 gekuppelt wird: 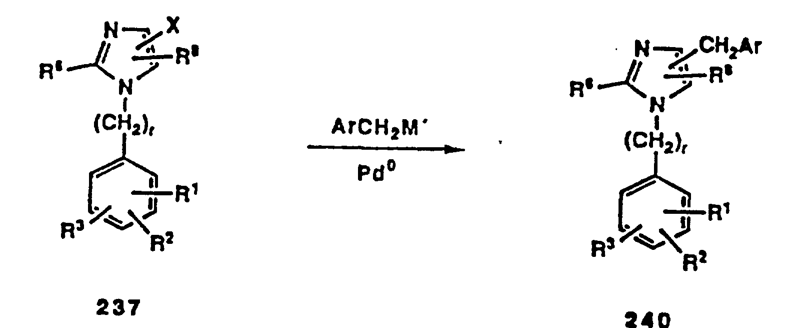 worin R¹, R, R³, R⁶, R⁸ und r die in Anspruch 1 angegebenen Bedeutungen haben, X Br oder I ist, M' ZnBr ist und Ar für R⁷ in Anspruch 1 definiertes substituiertes oder unsubstituiertes Heteroaryl, Biphenylmethyl, Phenoxyphenylmethyl ist.

56. Verfahren zur Herstellung der Verbindungen von Anspruch 1, worin R⁷ = Vinyl, Alkinyl, substituiertes Alkenyl oder substituiertes Alkinyl, dadurch gekennzeichnet, daß ein Halogenimidazol 237 mit einem 1-Alkenylmetall- oder einem 1-Alkinylmetallderivat (AM) oder einem 1-Alken oder einem 1-Alkin (AH) in Anwesenheit eines Übergangsmetallkatalysators unter Bilden eines 1-Alkenyl- oder 1-Alkinylimidazols 241 gekuppelt wird: 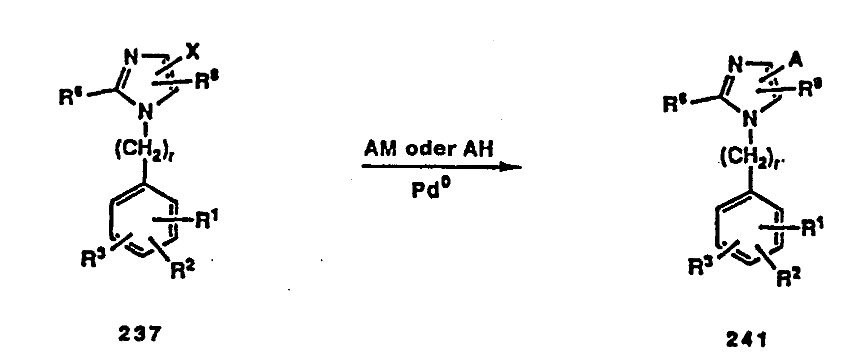 worin R¹, R, R³, R⁶, R⁸ und r die in Anspruch 1 angegebenen Bedeutungen haben, X Br oder I ist, M ein Metall ist, A Vinyl, CH=CH(CH₂)ₓAr, C≡C(CH₂)_{y}CH₃, C≡C(CH₂)_{z}Ph' oder C≡C(CH₂)ₓAr ist, Ph' Phenyl oder substituiertes Phenyl ist und Ar in Anspruch 1 definiertes substituiertes oder unsubstituiertes Biphenylyl, Phenoxyphenyl oder Heteroaryl ist, x = 0-8, y = 0-7 und z = 0-4.

57. Verfahren zur Herstellung der Verbindungen von Anspruch 1, worin R⁷ = Vinyl oder substituiertes Alkenyl, dadurch gekennzeichnet, daß der Imidazolaldehyd 253 mit Methylentriphenylphosphoran oder einem substituierten Methylentriphenylphosphoran unter Bilden eines Vinylimidazols oder eines substituierten Alkenylimidazols 254 umgesetzt wird: 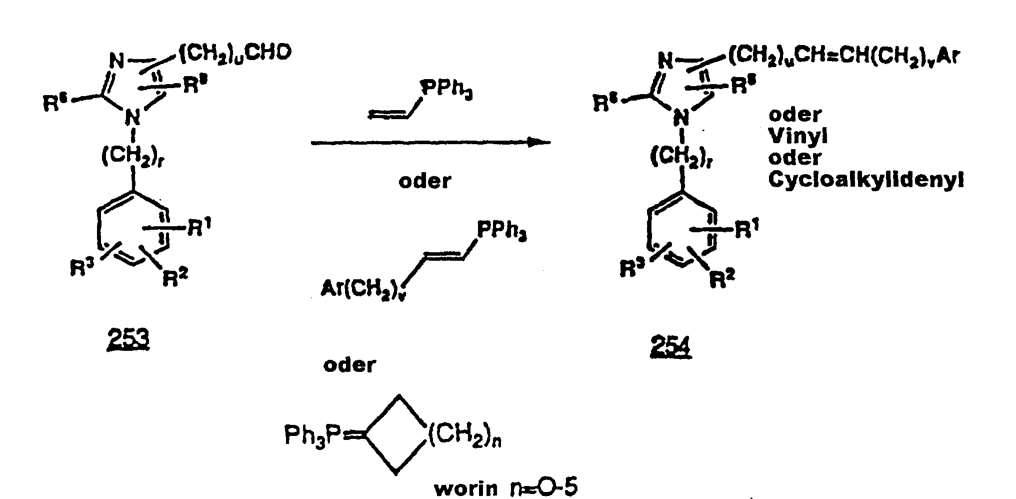 worin R¹, R, R³, R⁶, R⁸ und r die in Anspruch 1 angegebenen Bedeutungen haben, Ar in Anspruch 1 definiertes substituiertes oder unsubstituiertes Biphenylyl, Phenoxyphenyl oder Heteroaryl ist und u + v = 0 bis 8.

58. Verfahren zur Herstellung der Verbindungen der Formel I, worin R⁷ -S(O)ᵣ-Heteroaryl, -S-(O)ᵣ-Biphenylyl, -S(O)ᵣ-Phenoxyphenyl, -S-Tetrazol, -S(O)ᵣR¹⁷, -NR¹⁸R¹⁹, -NR¹⁸-Heteroaryl, -NR¹⁸-Phenyl, -NR¹⁸-Biphenylyl, -NR¹⁸-Phenoxyphenyl, -N-Phthalimido, -NH-SO₂-Phenoxyphenyl, -NH-SO₂-Heteroaryl, -NH-SO₂-Biphenilyl, -NH-SO₂-R¹⁷ und -S-(C=O)-R¹⁷, N-Imidazolyl, N-1,2,3-Triazolyl und N-1,2,4-Triazolyl ist, wobei Heteroaryl wie in Anspruch 1 definiert ist, dadurch gekennzeichnet, daß mit einer elektronenziehenden Gruppe E substituierte imidazolhaltige Verbindungen 255 mit Nukleophilen in einem geeigneten Lösungsmittel bei Raumtemperatur bis zur Rückflußtemperatur des Lösungsmittels umgesetzt werden, was eine aromatische Substitutionsreaktion zur Folge hat, wodurch die Abgangsgruppe X durch ein Nukleophil Nu wie etwa Schwefel oder Stickstoff unter Erzeugen von Verbindungen der Struktur 256 substituiert wird:

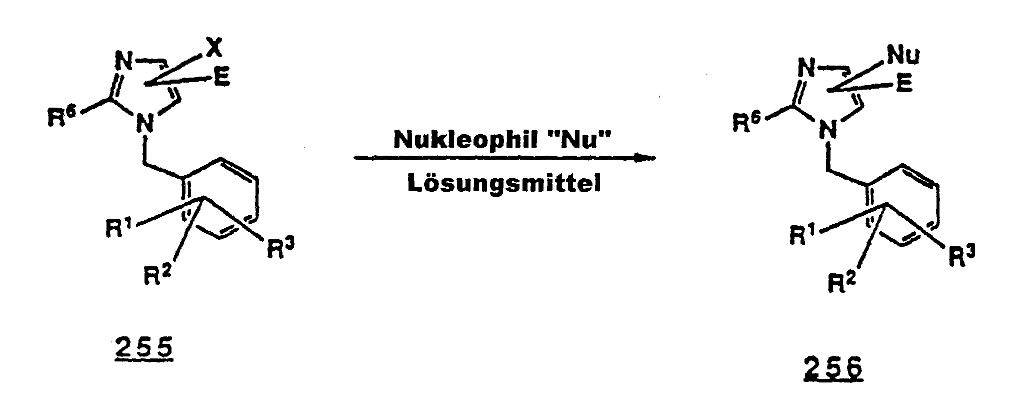

## Patentansprüche (Patentansprüche für folgende(n) Vertragsstaat(en): ES)

1. Verfahren zur Herstellung einer blutdrucksenkenden Verbindung der Formel 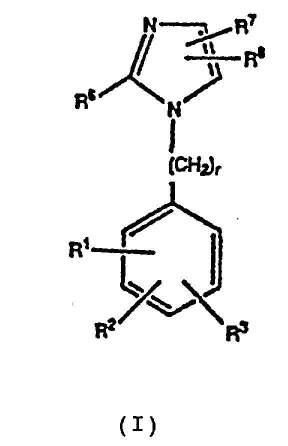 worin
R¹ 4-CO₂H; 4-CO₂R⁹; 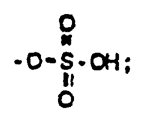 -SO₃H ; -C(CF₃)₂OH ; 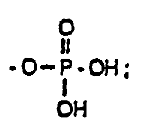 -PO₃H₂ ; 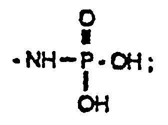 4-NHSO₂CH₃ ; 4-NHSO₂CF₃ ; -CONHOR^{12;} -SO₂NH₂; 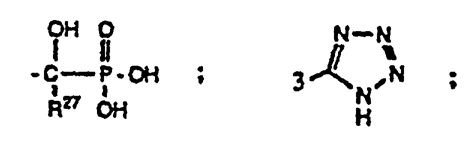 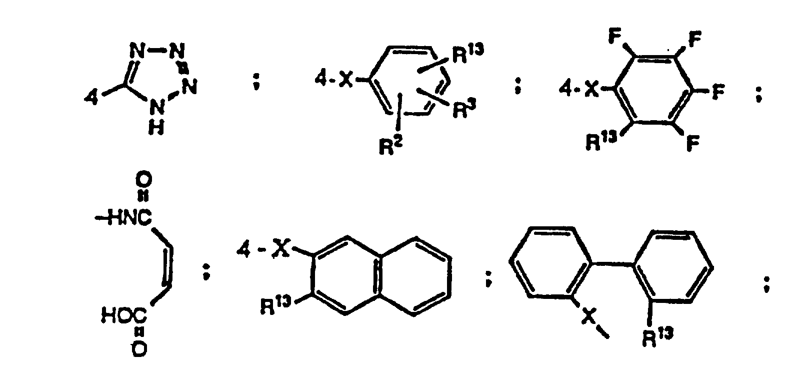 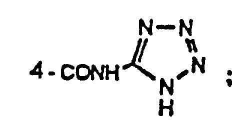 4-CONHNHSO₂CF₃; 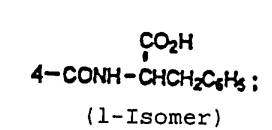 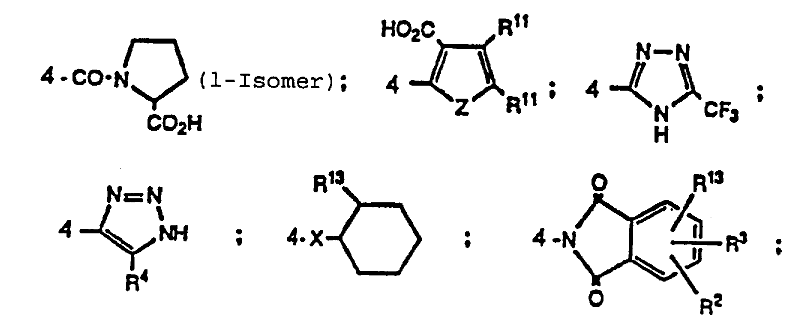 oder 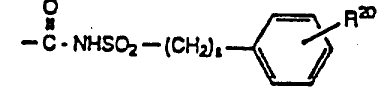 ist,
R H, Cl, Br, I, F, NO₂, CN, Alkyl mit 1 bis 4 Kohlenstoffatomen, Acyloxy mit 1 bis 4 Kohlenstoffatomen, Alkoxy mit 1 bis 4 Kohlenstoffatomen, CO₂H, CO₂R⁹, NHSO₂CH₃, NHSO₂CF₃, CONHOR¹, SO₂NH₂, 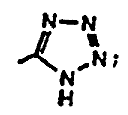 Phenyl oder Furyl ist,
R³ H, Cl, Br, I oder F, Alkyl mit 1 bis 4 Kohlenstoffatomen oder Alkoxy mit 1 bis 4 Kohlenstoffatomen ist,
R⁴ CN, NO₂ oder CO₂R¹¹ ist,
R⁵ H, Alkyl mit 1 bis 6 Kohlenstoffatomen, Cycloalkyl mit 3 bis 6 Kohlenstoffatomen, Alkenyl oder Alkinyl mit 2 bis 4 Kohlenstoff ist,
R⁶ Alkyl mit 2 bis 10 Kohlenstoffatomen, Alkenyl oder Alkinyl mit 3 bis 10 Kohlenstoffatomen oder dieselben, durch F oder CO₂R¹⁴ substituierten Gruppen, Cycloalkyl mit 3 bis 8 Kohlenstoffatomen, Cycloalkylalkyl mit 4 bis 10 Kohlenstoffatomen, Cycloalkylalkenyl oder Cycloalkylalkinyl mit 5 bis 10 Kohlenstoffatomen, gegebenenfalls durch F oder CO₂R¹⁴ substituiertes (CH₂)ₛZ(CH₂)ₘR⁵, Benzyl oder im Phenylring durch 1 oder 2 Halogene, Alkoxy mit 1 bis 4 Kohlenstoffatomen, Alkyl mit 1 bis 4 Kohlenstoffatomen oder Nitro substituiertes Benzyl ist,
R⁷ Vinyl, Cycloalkylidenyl, Alkinyl mit 2-10 Kohlenstoffatomen, Phenylalkinyl, worin der Alkinylteil 2-6 Kohlenstoffatome darstellt, aus 2- und 3-Thienyl, 2- und 3-Furyl, 2-, 3- und 4-Pyridyl, 2-Pyrazinyl, 2-, 4- und 5-Pyrimidinyl, 3- und 4-Pyridazinyl, 2-, 4- und 5-Thiazolyl, 2-, 4- und 5-Selenazolyl und 2-, 4- und 5-Oxazolyl, 2- oder 3-Pyrrolyl, 3-, 4- oder 5-Pyrazolyl, 2-, 4- oder 5-Imidazolyl ausgewähltes Heteroaryl, o-, m- oder p-Biphenylyl, o-, m- oder p-Phenoxyphenyl, 2-Oxazolinyl, 2-Thiazolinyl, substituiertes, vorstehend definiertes Phenylalkinyl, Heteroaryl, Biphenylyl oder Phenoxyphenyl, das durch 1 oder 2 Substituenten substituiert ist, die aus Halogen, Hydroxy, Mercapto, Alkoxy mit 1-5 Kohlenstoffatomen, Alkyl mit 1-5 Kohlenstoffatomen, -NO₂, -CN, -CF₃, -COR¹⁶, -CH₂OR¹⁷, -NHCOR¹⁷, -CONR¹⁸R¹⁹, S(O)ᵣR¹⁷ und SO₂NR¹⁸R¹⁹ ausgewählt sind, vorstehend definiertes Pyrrolyl, Pyrazolyl oder Imidazolyl, das am Ringstickstoff durch Alkyl mit 1-5 Kohlenstoffatomen, Phenyl oder Benzyl substituiert ist, oder substituiertes Alkyl, Alkenyl oder Alkinyl mit 1 bis 10 Kohlenstoffatomen ist, das durch eine substituierte oder unsubstituierte, vorstehend definierte Heteroaryl-, Biphenylyl- oder Phenoxyphenylgruppe, -S(O)ᵣ-Heteroaryl, -S-(O)ᵣ-Biphenylyl, -S (O)ᵣ-Phenoxyphenyl, -S-Tetrazol, -S(O)ᵣR¹⁷, -NR¹⁸R¹⁹, -NR¹⁸-Heteroaryl, -NR¹⁸-Phenyl, -NR¹⁸-Biphenylyl, -NR¹⁸-Phenoxyphenyl, -N-Phthalimido, -NH-SO₂-Phenoxyphenyl, -NH-SO₂-Heteroaryl, -NH-SO₂-Biphenylyl, -NH-SO₂-R¹⁷ und -S-(C=O)-R¹⁷, -N-Imidazolyl, N-1,2,3-Triazolyl, N-1,2,4-Triazolyl substituiert ist, worin Heteroaryl ein im Umfang von R⁷ definierter Heterocyclus ist und worin die Phenylgruppe in R¹⁷ von -S-(O)ᵣR¹⁷, N-Imidazolyl, N-1,2,3-Triazolyl und N-1,2,4-Triazolyl durch einen oder zwei vorstehend für Heteroaryl beschriebene Substituenten substituiert sein können,
R⁸ 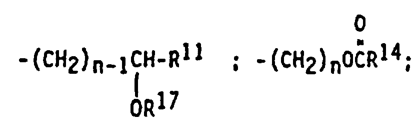 -(CH₂)ₙSR¹⁵; 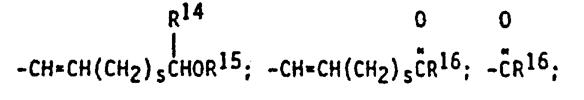 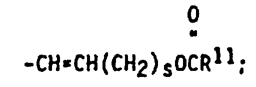 -(CH₂)ₘ-Tetrazolyl; 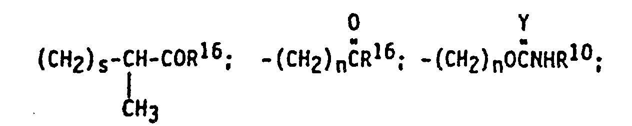 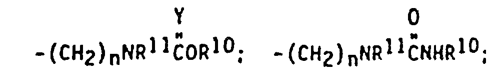 -(CH₂)ₙNR¹¹SO₂R¹⁰; 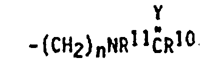 ist,
R⁹ 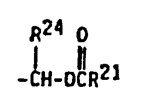 ist,
R¹⁰ Alkyl mit 1 bis 6 Kohlenstoffatomen oder Perfluoralkyl mit 1 bis 6 Kohlenstoffatomen, 1-Adamantyl, 1-Naphthyl, 1-(1-Naphthyl)ethyl oder (CH₂)ₚC₆H₅ ist,
R¹¹ H, Alkyl mit 1 bis 6 Kohlenstoffatomen, Cycloalkyl mit 3 bis 6 Kohlenstoffatomen, Phenyl oder Benzyl ist,
R¹ H, Methyl oder Benzyl ist,
R¹³ -CO₂H, -CO₂R⁹, -CH₂CO₂H, -CH₂CO₂R⁹ oder 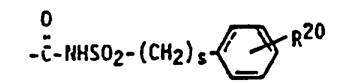 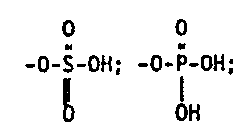 -SO₃H; 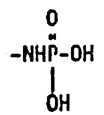 -PO₃H₂; -C(CF₃)₂OH; -NHSO₂CH₃; -NHSO₂CF₃; -NHCOCF₃; -CONHOR¹; -SO₂NH₂; 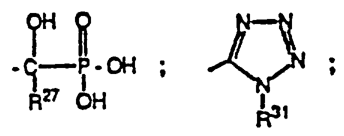 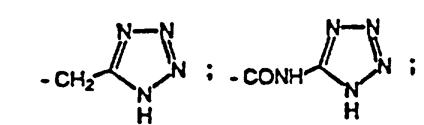 - CONHNHSO₂CF₃ ; 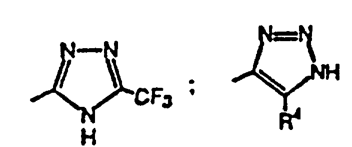 ist,
R¹⁴ H, Alkyl oder Perfluoralkyl mit 1 bis 8 Kohlenstoffatomen, Cycloalkyl mit 3 bis 6 Kohlenstoffatomen, Phenyl oder Benzyl ist,
R¹⁵ H, Alkyl mit 1 bis 6 Kohlenstoffatomen, Cycloalkyl mit 3 bis 6 Kohlenstoffatomen, Phenyl, Benzyl, Acyl mit 1 bis 4 Kohlenstoffatomen, Phenacyl ist,
R¹⁶ H, Alkyl mit 1 bis 6 Kohlenstoffatomen, Cycloalkyl mit 3 bis 6 Kohlenstoffatomen, (CH₂)ₚC₆H₅, OR¹⁷ oder NR¹⁸R¹⁹ ist,
R¹⁷ H, Alkyl mit 1 bis 6 Kohlenstoffatomen, Cycloalkyl mit 3 bis 6 Kohlenstoffatomen, Phenyl oder Benzyl ist,
R¹⁸ und R¹⁹ unabhängig H, Alkyl mit 1 bis 4 Kohlenstoffatomen, Phenyl, Benzyl, α-Methylbenzyl sind oder mit dem Stickstoffatom zusammengenommen einen Ring der Formel 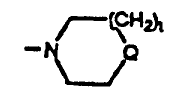 bilden,
Q NR⁰, O oder CH₂ ist,
R⁰ H, Alkyl mit 1-4 Kohlenstoffatomen oder Phenyl ist,
R¹ Alkyl mit 1 bis 6 Kohlenstoffatomen, -NRR³ oder 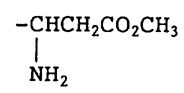 ist,
R und R³ unabhängig H, Alkyl mit 1 bis 6 Kohlenstoffatomen, Benzyl oder zusammengenommen (CH₂)ᵤ sind, worin u 3-6 ist,
R⁴ H, CH₃ oder -C₆H₅ ist,
R⁵ NR⁷R⁸, OR⁸, NHCONH₂, NHCSNH₂, 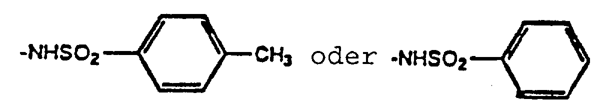 ist,
R⁶ Wasserstoff, Alkyl mit 1 bis 6 Kohlenstoffatomen, Benzyl oder Allyl ist,
R⁷ und R⁸ unabhängig Wasserstoff, Alkyl mit 1 bis 5 Kohlenstoffatomen oder Phenyl sind,
R⁹ und R³⁰ unabhängig Alkyl mit 1-4 Kohlenstoffatomen oder zusammengenommen -(CH₂)_{q}- sind,
R³¹ H, Alkyl mit 1 bis 4 Kohlenstoffatomen, -CH₂CH=CH₂ oder -CH₂C₆H₄R³ ist,
R³ H, NO₂, NH₂, OH oder OCH₃ ist,
X eine Kohlenstoff-Kohlenstoff-Einfachbindung, -CO-, -CH₂-, -O-, -S-, -NH-, 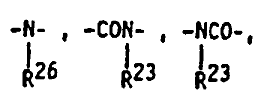 -OCH₂-, -CH₂O-, -SCH₂-, -CH₂S-, -NHC(R⁷)(R⁸)-, -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 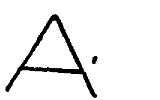 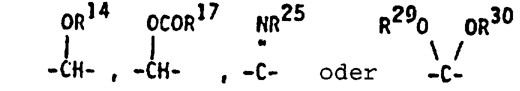 ist,
Y O oder S ist,
Z O, NR¹¹ oder S ist,
m 1 bis 5 ist,
n 1 bis 10 ist,
p 0 bis 3 ist,
q 2 bis 3 ist,
r 0 bis 2 ist,
s 0 bis 5 ist,
t 0 oder 1 ist,
oder eines pharmazeutisch annehmbaren Salzes derselben, vorausgesetzt, daß
(1) sich die Gruppe R¹ nicht in ortho-Stellung befindet,
(2) wenn R¹ 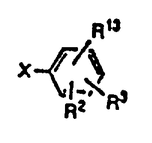 ist, X eine Einfachbindung ist und R¹³ CO₂H oder 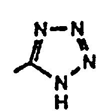 ist, R¹³ sich dann in ortho- oder meta- Stellung befinden muß, oder wenn R¹ und X wie vorstehend sind und R¹³ NHSO₂CF₃ oder NHSO₂CH₃ ist, R¹³ ortho sein muß,
(3) wenn R¹ 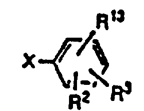 ist und X etwas anderes als eine Einfachbindung ist, R¹³ dann ortho sein muß, außer wenn X = NR³CO und R¹³ NHSO₂CF₃ oder NHSO₂CH₃ ist, R¹³ dann ortho oder meta sein muß,
(4) wenn R¹ 4-CO₂H oder ein Salz davon ist, R⁶ nicht S-Alkyl sein kann,
(5) wenn R¹ 4-CO₂H oder ein Salz davon ist, der Substituent in der 4-Stellung des Imidazols nicht CH₂OH, CH₂OCOCH₃ oder CH₂CO₂H sein kann,
(6) wenn R¹ 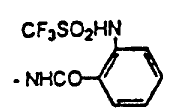 ist, R⁶ nicht Methoxybenzyl ist,
(7) die Gruppe R⁶ nicht 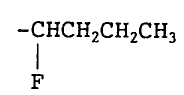 oder CH₂OH ist, wobei das Verfahren
A) zur Herstellung einer Verbindung, worin r 1 ist, das Zusammenbringen eines Imidazolderivats der Formel 1 mit einem Benzylderivat der Formel 2 in einem Lösungsmittel in Anwesenheit einer Base über 1 bis 10 Stunden bei einer Temperatur im Bereich von 20°C bis zur Rückflußtemperatur des Lösungsmittels unter Bilden eines Benzylimidazols der Formel 3: 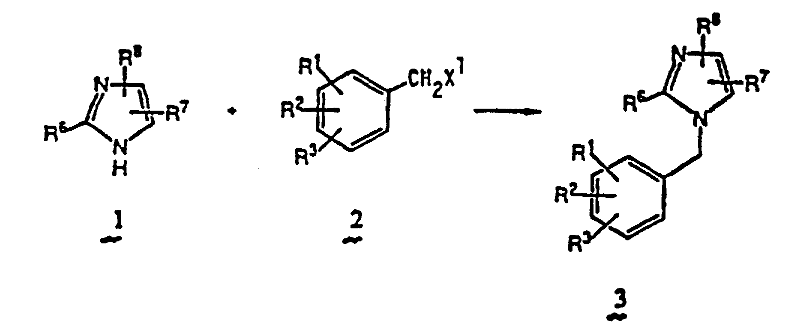 worin R¹, R, R³, R⁶, R⁷ und R⁸ jeweils unter den Reaktionsbedingungen stabil sind und eine in Anspruch 1 definierte Gruppe oder ein Zwischenprodukt oder eine geschützte Form davon sind, welche in eine derartige Gruppe umgewandelt werden kann und worin X¹ Halogen, p-Toluolsulfonyloxy oder Methylsulfonyloxy ist, und danach nötigenfalls das Umwandeln des Zwischenprodukts oder der geschützten Form der vorstehend definierten Gruppen R in Gruppen R umfaßt,
B) zur Herstellung einer Verbindung, worin r 0 ist, das Zusammenbringen eines Imidazolderivats der Formel 1 oder seines Metallsalzes mit 4-Fluor-1-nitrobenzol in einem Lösungsmittel in Anwesenheit einer Base, falls das freie Imidazol verwendet wird, während 1-10 Tagen bei einer Temperatur von 25-150°C unter Bilden eines N-Phenylimidazols,
C) zur Herstellung einer Verbindung, worin R⁷ = vorstehend definiertes substituiertes oder unsubstituiertes Biphenylyl, Phenoxyphenyl oder Heteroaryl, das Kuppeln des Halogenimidazols 237 mit einem Arylmetallderivat ArM, worin M = ZnBr, Me₃Sn oder B(OH)₂, in Anwesenheit eines Übergangsmetallkatalysators wie etwa Palladium, Platin, Nickel oder Zirkonium unter Bilden eines Arylimidazols 238: 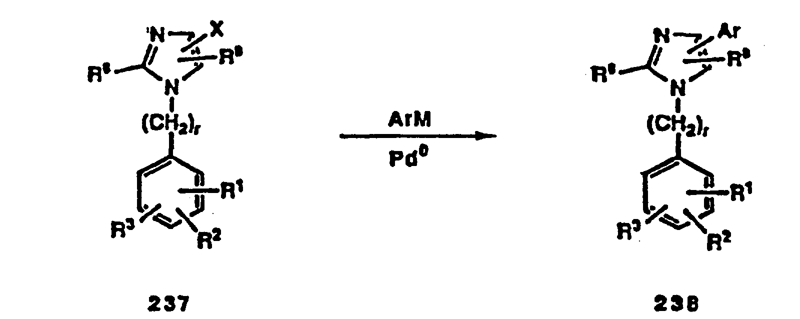 worin R¹, R, R³, R⁶, R⁸ und r die vorstehend angegebenen Bedeutungen haben, X Br oder I ist, M ZnBr, Me₃Sn, B(OH)₂ ist und Ar für R⁷ vorstehend definiertes substituiertes oder unsubstituiertes Biphenylyl, Phenoxyphenylmethyl oder Heteroaryl ist,
D) zur Herstellung einer Verbindung, worin R⁷ = vorstehend definiertes substituiertes oder unsubstituiertes Biphenylylmethyl, Phenoxyphenylmethyl oder Heteroarylmethyl, das Kuppeln eines Halogenimidazols 237 mit einem Arylmethylmetallderivat ArCH₂M' in Anwesenheit eines Übergangsmetallkatalysators unter Bilden eines Arylmethylimidazols 240: 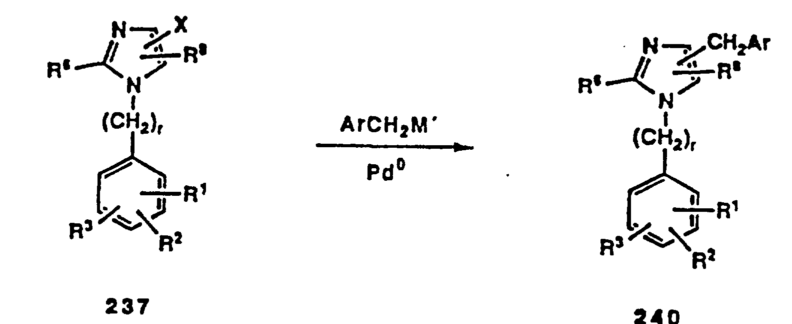 worin R¹, R, R³, R⁶, R⁸ und r die vorstehend angegebenen Bedeutungen haben, X Br oder I ist, M' ZnBr ist und Ar für R⁷ vorstehend definiertes substituiertes oder unsubstituiertes Heteroaryl, Biphenylmethyl, Phenoxyphenylmethyl ist,
E) zur Herstellung einer Verbindung, worin R⁷ = Vinyl, Alkinyl, substituiertes Alkenyl oder substituiertes Alkinyl, das Kuppeln eines Halogenimidazols 237 mit einem 1-Alkenylmetall- oder einem 1-Alkinylmetallderivat (AM) oder einem 1-Alken oder einem 1-Alkin (AH) in Anwesenheit eines Übergangsmetallkatalysators unter Bilden eines 1-Alkenyl- oder 1-Alkinylimidazols 241: 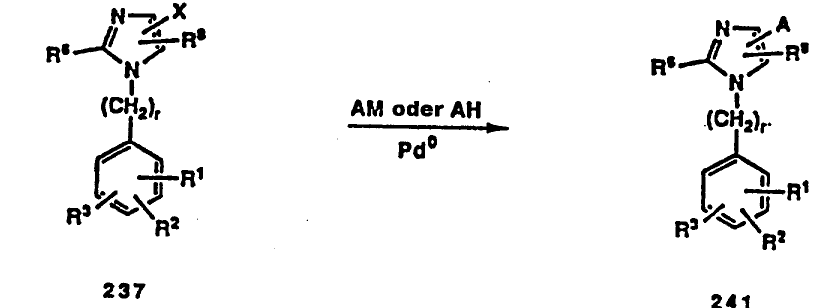 worin R¹, R, R³, R⁶, R⁸ und r die vorstehend angegebenen Bedeutungen haben, X Br oder I ist, M ein Metall ist, A Vinyl, CH=CH(CH₂)ₓAr, C≡C(CH₂)_{y}CH₃, C≡C(CH₂)_{z}Ph' oder C≡C(CH₂)ₓAr ist, Ph' Phenyl oder substituiertes Phenyl ist und Ar vorstehend definiertes substituiertes oder unsubstituiertes Biphenylyl, Phenoxyphenyl oder Heteroaryl ist, x = 0-8, y = 0-7 und z = 0-4,
F) zur Herstellung einer Verbindungen, worin R⁷ = Vinyl oder substituiertes Alkenyl, das Umsetzen eines Imidazolaldehyds 253 mit Methylentriphenylphosphoran oder einem substituierten Methylentriphenylphosphoran unter Bilden eines Vinylimidazols oder eines substituierten Alkenylimidazols 254: 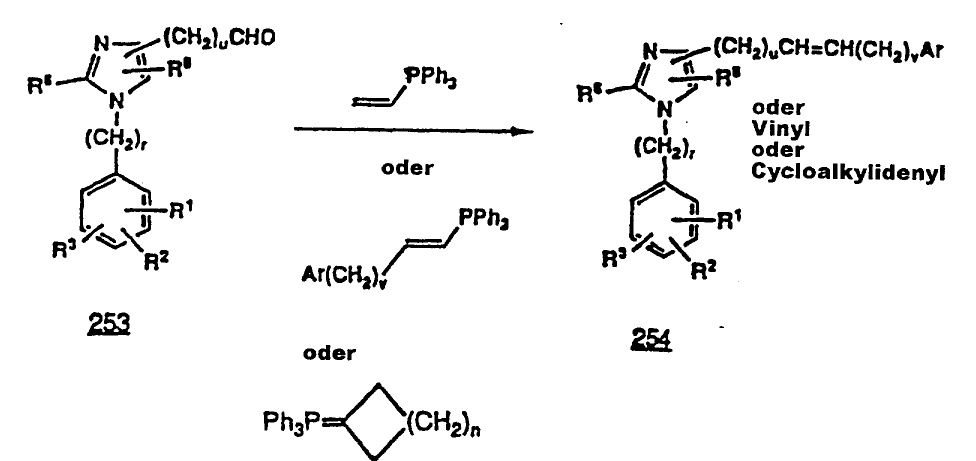 worin n=O-5 worin R¹, R, R³, R⁶, R⁸ und r die vorstehend angegebenen Bedeutungen haben, Ar vorstehend definiertes substituiertes oder unsubstituiertes Biphenylyl, Phenoxyphenyl oder Heteroaryl ist und u + v = 0 bis 8,
G) zur Herstellung einer Verbindung der Formel I, worin R⁷-S(O)ᵣ-Heteroaryl, -S-(O)ᵣ-Biphenylyl, -S(O)ᵣ-Phenoxyphenyl, -S-Tetrazol, -S(O)ᵣR¹⁷, -NR¹⁸R¹⁹, -NR¹⁸-Heteroaryl, -NR¹⁸-Phenyl, -NR¹⁸-Biphenylyl, -NR¹⁸-Phenoxyphenyl, -N-Phthalimido, -NH-SO₂-Phenoxyphenyl, -NH-SO₂-Heteroaryl, -NH-SO₂-Biphenylyl, -NH-SO₂-R¹⁷ und -S-(C=O)R¹⁷, N-Imidazolyl, N-1,2,3-Triazolyl und N-1,2,4-Triazolyl ist, wobei Heteroaryl wie vorstehend definiert ist, das Umsetzen mit einer elektronenziehenden Gruppe E substituierter imidazolhaltiger Verbindungen 255 mit Nukleophilen in einem geeigneten Lösungsmittel bei Raumtemperatur bis zur Rückflußtemperatur des Lösungsmittels umfaßt, was eine aromatische Substitutionsreaktion zur Folge hat, wodurch die Abgangsgruppe X durch ein Nukleophil Nu wie etwa Schwefel oder Stickstoff unter Erzeugen von Verbindungen der Struktur 256 substituiert wird:

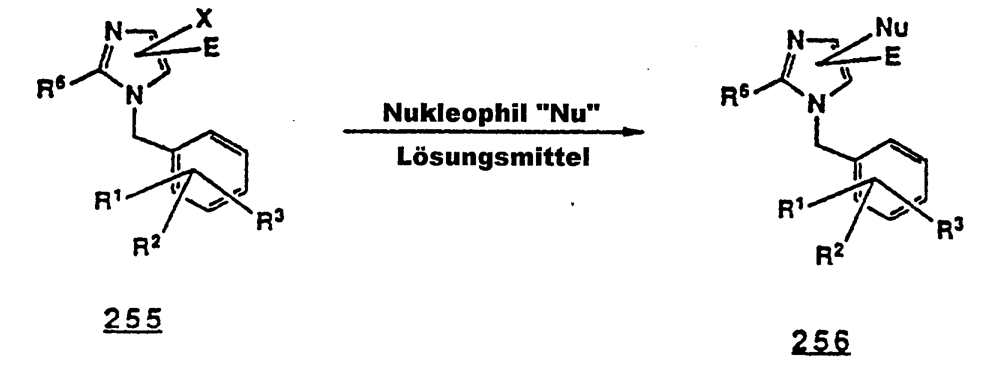

2. Verfahren von Anspruch 1, wobei in A Verbindung 1 und 2 in Anwesenheit einer Base, die aus der Gruppe ausgewählt ist, die aus einem Metallhydrid (MH), einem Metallalkoxid (MOR), Natriumcarbonat, Kaliumcarbonat, Triethylamin und Pyridin besteht, in einem dipolaren aprotischen Lösungsmittel zusammengebracht werden oder, wenn die Base MOR ist, das Lösungsmittel ein Alkohol, ROH, sein kann, worin M Lithium, Natrium oder Kalium ist und R Methyl, Ethyl oder t-Butyl ist.

3. Verfahren von Anspruch 1, wobei in A ein Zweiphasen-Lösungsmittelsystem, eines eine organische Phase wie etwa Methylenchlorid und das andere eine wäßrige Phase, in Anwesenheit eines Phasentransferkatalysators wie etwa Tricaprylmethylammoniumchlorid verwendet wird.

4. Verfahren von Anspruch 2, wobei R¹ 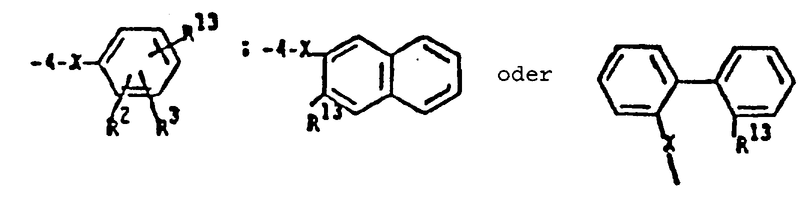 ist,
X eine Kohlenstoff-Kohlenstoff-Einfachbindung, -CO-, -O-, -S- oder -NH- ist,
R und R³ jeweils unabhängig H, Cl, Br, I, CO₂R¹⁴, F, NO₂, Alkyl mit 1 bis 4 Kohlenstoffatomen, Alkoxy mit 1 bis 4 Kohlenstoffatomen, Phenyl oder Furyl sind,
R⁶ und R⁷ wie in Anspruch 1 definiert sind,
R⁸ -(CH₂)ₙOR¹¹, -(CH₂)ₙSR¹⁵ oder -(CH₂)ₙCN ist,
R¹¹ wie in Anspruch 1 ist,
R¹³ CO₂R¹⁴, CN, NO₂, Trialkylzinntetrazol oder Trityltetrazol ist und
R¹⁴ und R¹⁵ wie in Anspruch 1 definiert sind.

5. Verfahren von Anspruch 4, wobei R¹³ -CO₂R¹⁴ ist und das Produkt der Formel 3 1-24 Stunden mit einer Alkalie in einem wäßrig-alkoholischen Lösungsmittel oder mit CF₃CO₂H bei einer Temperatur im Bereich von 20°C bis zur Rückflußtemperatur des Lösungsmittels zusammengebracht wird, gefolgt von der Einstellung des pH des Gemisches auf einen Wert im Bereich von 3 bis 7 unter Überführen des Produkts in das entsprechende Produkt, worin R¹³ -CO₂H ist.

6. Verfahren von Anspruch 5, wobei wenigstens eines von R, R³ oder R¹³ in Formel 1 -CO₂R¹⁴ ist und in -CO₂H überführt wird.

7. Verfahren von Anspruch 5, wobei R¹⁴ t-Butyl ist und die Reaktion in CF₃CO₂H ausgeführt wird.

8. Verfahren von Anspruch 4, wobei R¹³ -CN ist und das Produkt der Formel 3 mit (i) einer starken Säure 2-96 Stunden bei der Rückflußtemperatur des Lösungsmittels oder (ii) einem starken Alkali in einem Alkohollösungsmittel 2-96 Stunden bei einer Temperatur im Bereich von 20°C und der Rückflußtemperatur des Lösungsmittels, gefolgt von der Einstellung des pH auf 3-7, oder (iii) Schwefelsäure gefolgt von Säure oder Alkali unter Überführen des Produkts in die entsprechende Verbindung, worin R¹³ -CO₂H ist, zusammengebracht wird.

9. Verfahren von Anspruch 8, wobei wenigstens eines von R, R³ oder R¹³ -CO₂R¹⁴ ist, und in -CO₂H überführt wird.

10. Verfahren von Anspruch 8, wobei R⁸ -(CH₂)ₙCN ist und in -(CH₂)ₙCO₂H überführt wird oder -(CH₂)ₙOR¹¹ ist und in (CH₂)ₙOH überführt wird, wenn R¹³ in -CO₂H überführt wird.

11. Verfahren von Anspruch 4, wobei R¹³ -CN ist, und das Produkt der Formel 3 mit einem Gemisch äquimolarer Mengen Natriumazid und Ammoniumchlorid in einem polaren aprotischen Lösungsmittel bei einer Temperatur im Bereich von 30°C bis zur Rückflußtemperatur des Lösungsmittels 1 Stunde bis 10 Tage unter Überführen des Produkts in die entsprechende Verbindung, worin R¹³ 5-Tetrazolyl ist, zusammengebracht wird.

12. Verfahren von Anspruch 11, wobei R⁸ -(CH₂)ₘCN ist und in -(CH₂)ₘ-Tetrazolyl überführt wird, wenn R¹³ in 5-Tetrazolyl überführt wird.

13. Verfahren von Anspruch 4, wobei R¹³ -CN ist, und das Produkt der Formel 3 mit Trialkylzinnazid oder Triarylzinnazid umgesetzt wird, gefolgt von saurer oder basischer Hydrolyse unter Überführen des Produkts in die entsprechende Verbindung, worin R¹³ 5-Tetrazolyl ist.

14. Verfahren von Anspruch 4, wobei R¹³ -CN ist und das Produkt der Formel 3 mit Trialkylzinnazid oder Triarylzinnazid unter Herstellen einer Verbindung der Formel 3 umgesetzt wird, worin R¹³ Trialkyl- oder Triarylstannyltetrazol-5-yl ist, letztere Verbindung mit Triphenylmethylchlorid unter Erzeugen einer Verbindung der Formel 3, worin R¹³ Triphenylmethyltetrazol-5-yl ist, umgesetzt wird und letztere Verbindung unter Erzeugen einer Verbindung der Formel 3, worin R¹³ 5-Tetrazolyl ist, hydrolysiert wird.

15. Verfahren von Anspruch 3, worin R⁸ -(CH₂)ₙCN ist und in -(CH₂)ₘ-Tetrazolyl überführt wird, wenn R¹³ in 5-Tetrazolyl überführt wird.

16. Verfahren von Anspruch 4, wobei R¹³ -NO₂ ist und das Produkt der Formel 3 mit einem Reduktionsmittel unter Bilden eines zweiten Zwischenprodukts der Formel 3, in der R¹³ NH₂ ist, zusammengebracht wird und letztere mit einem Sulfonsäureanhydrid (CH₃So₂)₂O oder (CF₃SO₂)₂O oder -chlorid CH₃SO₂Cl oder CF₃SO₂Cl in einem Lösungsmittel unter Herstellen einer Verbindung zusammengebracht wird, in welcher R¹³ -NHSO₂CH₃ oder -NHSO₂CF₃ ist.

17. Verfahren von Anspruch 16, wobei wenigstens eines von R, R³ oder R¹³ -NO₂ ist und in -NHSO₂CH₃ oder-NHSO₂CF₃ überführt wird.

18. Verfahren von Anspruch 5 oder 8, wobei die Verbindung der Formel 3 mit R¹³ = CO₂H unter Liefern einer Verbindung, in der R¹³ CONHOR¹ ist, entweder
(a) mit etwa 1-4 Äquivalenten Thionylchlorid in überschüssigem Thionylchlorid oder einem weiteren Lösungsmittel bei einer Temperatur im Bereich von 20°C bis zur Rückflußtemperatur des Lösungsmittels über einen Zeitraum von 5 Minuten bis 2 Stunden unter Bilden eines Zwischenproduktes der Formel 3, worin R¹³ COCl ist, zusammengebracht wird und letzteres 2-18 Stunden bei einer Temperatur im Bereich von 25-80°C mit 2-10 Äquivalenten eines Hydroxylaminderivates H₂NOR¹ in überschüssigem Hydroxylaminderivat H₂NOR¹ oder einem anderen Lösungsmittel zusammengebracht wird, oder
(b) mit dem Hydroxylaminderivat H₂NOR¹, Dicyclohexylcarbodiimid und 1-Hydroxybenzotriazol in einem Lösungsmittel bei einer Temperatur im Bereich von 0-30°C 1-24 Stunden zusammengebracht wird.

19. Verfahren von Anspruch 1, wobei in A) R¹ 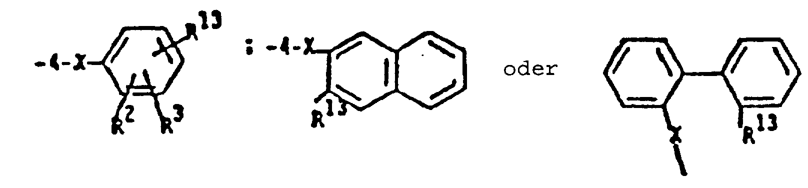 ist,
X eine Kohlenstoff-Kohlenstoff-Einfachbindung, -CO-, -O-, -S- oder -NH- ist,
R, R³, R⁶ und R⁷ wie in Anspruch 1 definiert sind und
R⁸ (CH₂)ₙOR¹¹, (CH₂)ₙOCOR¹⁴, (CH₂)ₙCH(OH)R¹⁶, (CH₂)ₙCOR¹⁶(CH₂)ₙCl, (CH₂)ₙCN, CHO ist.

20. Verfahren von Anspruch 19, wobei R⁸ (CH₂)ₙOH ist und das Produkt der Formel 3 mit einem Alkohol R¹¹OH in wasserfreiem Zustand in Anwesenheit einer starken Säure oder einer Lewissäure zusammengebracht wird, gefolgt von der Verseifung irgendwelcher Gruppen CO₂R¹⁴, die im Zwischenprodukt gleichzeitig gebildet werden oder zugegen sind, unter Bilden der entsprechenden Verbindung der Formel 3, worin R⁸ (CH₂)ₙOR¹¹ ist und R¹¹ nicht H ist.

21. Verfahren von Anspruch 19, wobei R⁸ (CH₂)ₙOR¹¹ ist und R¹¹ nicht H ist und das Produkt der Formel 3 mit einem wäßrigen sauren Medium bei einer Temperatur im Bereich von 25°C und der Rückflußtemperatur des Lösungsmittels über einen Zeitraum von 0,5-24 Stunden unter Bilden der entsprechenden Verbindung der Formel 3, worin R⁸ (CH₂)ₙOH ist, zusammengebracht wird.

22. Verfahren von Anspruch 19, wobei R⁸ (CH₂)ₙOH ist und das Produkt der Formel 3 mit
(a) einem Carbonsäureanhydrid (R¹⁴CO)₂O oder -chlorid R¹⁴COCl 0,5-24 Stunden in einem Lösungsmittel in Anwesenheit einer Base bei einer Temperatur im Bereich von 0°C und der Rückflußtemperatur des Lösungsmittels oder
(b) eine Carbonsäure R¹⁴CO₂H unter wasserfreien Bedingungen in Anwesenheit einer starken Säure oder Lewissäure 0,5 bis 24 Stunden bei 0°-100°C unter Bilden der entsprechenden Verbindung, in der R⁸ (CH₂)ₙOCOR¹⁴ ist, zusammengebracht wird.

23. Verfahren von Anspruch 19, wobei R⁸ (CH₂)ₙOCOR¹⁴ ist und das Produkt der Formel 3 mit wäßriger Säure oder Alkali unter Bilden der entsprechenden Verbindung, worin R⁸ (CH₂)ₙOH ist, zusammengebracht wird.

24. Verfahren von Anspruch 19, wobei R⁸ (CH₂)ₙOH ist und das Produkt der Formel 3 mit einem Oxidationsmittel 1-200 Stunden bei einer Temperatur von 25-45°C unter Herstellen einer entsprechenden Verbindung der Formel 3 zusammengebracht wird, in welcher R⁸ (CH₂)ₙ₋₁COR¹⁶ ist und R¹⁶ H ist.

25. Verfahren von Anspruch 19, wobei R⁸ (CH₂)ₙCOR¹⁶ ist und R¹⁶ H ist und das Produkt der Formel 3 mit einer metallorganischen Verbindung R¹⁶P, in der P MgBr oder Li ist, 0,5-24 Stunden bei einer Temperatur im Bereich von -78°C bis 100°C unter Bilden einer Verbindung der Formel 3 zusammengebracht wird, in welcher R⁸ (CH₂)ₙCH(OH)R¹⁶ ist und R¹⁶ nicht H ist.

26. Verfahren von Anspruch 19, wobei R⁸ (CH₂)ₙCH(OH)R¹⁶ ist und R¹⁶ nicht H ist und das Produkt der Formel 3 mit einem Oxidationsmittel in einem Lösungsmittel unter Bilden einer entsprechenden Verbindung der Formel 3 zusammengebracht wird, in welcher R⁸ (CH₂)ₙCOR¹⁶ ist und R¹⁶ nicht H ist.

27. Verfahren von Anspruch 19, wobei R⁸ (CH₂)ₙCOR¹⁶ ist und R¹⁶ H ist und die Verbindung der Formel 3 mit einem Oxidationsmittel in einem Lösungsmittel unter Bilden einer entsprechenden Verbindung der Formel 3 zusammengebracht wird, in welcher R⁸ (CH₂)ₙCOR¹⁶ ist und R¹⁶ OH ist.

28. Verfahren von Anspruch 19, wobei R⁸ (CH₂)ₙCOR¹⁶ ist und R¹⁶ OH ist und die Verbindung der Formel 3 mit Thionylchlorid im Überschuß oder einem weiteren Lösungsmittel bei einer Temperatur im Bereich von 0°C bis zur Rückflußtemperatur des Lösungsmittels 5 Minuten bis 24 Stunden unter Bilden einer entsprechenden Verbindung der Formel 3, in der R⁸ (CH₂)ₙCOCl ist, zusammengebracht wird, gefolgt vom Zusammenbringen der letzteren mit einem Amin NHR¹⁸R¹⁹ im Überschuß oder in einem Lösungsmittel 5 Minuten bis 24 Stunden bei Temperaturen im Bereich von 0°C und Rückflußtemperatur des Lösungsmittels unter Bilden. einer entsprechenden Verbindung der Formel 3, in der R⁸ (CH₂)ₙCONR¹⁸R¹⁹ ist.

29. Verfahren von Anspruch 19, wobei R⁸ (CH₂)ₙOR¹¹ ist und R¹¹ H ist und das Produkt der Formel 3 mit Thionylchlorid im Überschuß oder in einem Lösungsmittel bei einer Temperatur im Bereich von 20°C bis zur Rückflußtemperatur des Lösungsmittels 0,5-24 Stunden unter Bilden einer Zwischenproduktverbindung der Formel 3 zusammengebracht wird, in der R⁸ (CH₂)ₙCl ist.

30. Verfahren von Anspruch 29, wobei die Verbindung der Formel 3, in der R⁸ (CH₂)ₙCl ist, mit dem Natrium- oder Kaliumsalz eines Mercaptans R¹⁵SH 1-24 Stunden in einem Lösungsmittel bei einer Temperatur von 25-100°C unter Bilden einer Verbindung der Formel zusammengebracht wird, in welcher R⁸ (CH₂)ₙSR¹⁵ ist.

31. Verfahren von Anspruch 19, wobei die Verbindung der Formel 3, in der R⁸ (CH₂)ₙCl ist, mit einem Alkalimetallcyanid 1-24 Stunden in einem Lösungsmittel bei einer Temperatur im Bereich von 20-100°C unter Bilden einer Verbindung der Formel 3 zusammengebracht wird, in welcher R⁸ (CH₂)ₙCN ist und die letztere Verbindung zu der entsprechenden Verbindung der Formel 3 hydrolysiert wird, in der R⁸ (CH₂)ₙCOR¹⁶ ist und R¹⁶ OH ist.

32. Verfahren von Anspruch 19, wobei die Verbindung der Formel 3, in der R⁸(CH₂)ₙ_₁Cl ist, mit dem Natrium- oder Kaliumsalz eines Malonsäuredialkylesters 0,5-24 Stunden in einem Lösungsmittel bei einer Temperatur im Bereich von 20-100°C unter Bilden einer Verbindung der Formel 3 zusammengebracht wird, in welcher R⁸ (CH₂)ₙ₋₁CH(CO₂Alkyl)₂ ist, gefolgt von der Verseifung der letzteren mit wäßrigem Alkali bei einer Temperatur im Bereich von 25 °C bis zur Rückflußtemperatur des Lösungsmittels, gefolgt vom Ansäuern mit Mineralsäure unter Bilden einer Verbindung der Formel 3, in der R⁸ (CH₂)ₙ₋₁CH(CO₂H)₂ ist, gefolgt vom Erhitzen der letzteren auf 120°C oder in verdünnter Mineralsäure bei Rückflußtemperatur unter Bilden eines Produkts der Formel 3, in der R⁸ (CH₂)ₙCOR¹⁶ ist und R¹⁶ OH ist.

33. Verfahren von Anspruch 19, wobei R⁸ -CHO ist und die Verbindung der Formel 3 mit einem Methylenphosphoran (C₆H₅)₃P=CH(CH₂)ₙCHR¹⁴OR¹⁵ oder (C₆H₅)₃P=CH(CH₂)ₛCOR¹⁶ 1-24 Stunden in einem Lösungsmittel bei einer Temperatur im Bereich von 25°C bis zur Rückflußtemperatur des Lösungsmittels unter Bilden einer Verbindung der Formel 3 zusammengebracht wird, in welcher R⁸ -CH=CH(CH₂)ₛCHR¹⁴OR¹⁵ oder -CH=CH(CH₂)ₛCOR¹⁶ ist, außer wenn R¹⁵ H ist und R¹⁶ OH ist, und gegebenenfalls anschließend 0,5-24 Stunden Zusammenbringen der Verbindung der Formel 3, in der R⁸ -CH=CH(CH₂)ₛCOR¹⁶ ist, mit einem Reduktionsmittel in einem Lösungsmittel bei einer Temperatur im Bereich von 0-25°C unter Bilden eines Produkts der Formel 3, in der R⁸ -CH=CH(CH₂)ₛCHR¹⁴OH ist.

34. Verfahren von Anspruch 19, wobei R⁸ (CH₂)ₙOH ist und die Verbindung der Formel 3 mit einem Isocyanat der Formel R¹⁰NCO in einem Lösungsmittel bei einer Temperatur im Bereich von 25°C bis zur Rückflußtemperatur des Lösungsmittels über einen Zeitraum von 5 Minuten bis 24 Stunden unter Bilden einer Verbindung der Formel 3 zusammengebracht wird, in welcher R⁸ (CH₂)ₙOCONHR¹⁰ ist.

35. Verfahren von Anspruch 19, wobei die Verbindung in der R⁸ (CH₂)ₙCl ist, mit einem Amin R¹¹NH₂ in überschüssigem Amin oder einem weiteren Lösungsmittel über einen Zeitraum von 1-24 Stunden bei einer Temperatur im Bereich von 0°C bis zur Rückflußtemperatur des Lösungsmittels unter Bilden eines Zwischenprodukts der Formel 3 zusammengebracht wird, in welcher R⁸ (CH₂)ₙNHR¹¹ ist.

36. Verfahren von Anspruch 19, wobei R⁸ (CH₂)ₙCl ist und die Verbindung der Formel 3 mit einem Alkalimetallazid in einem aprotischen Lösungsmittel 1-24 Stunden bei einer Temperatur im Bereich von 25-80°C unter Bilden einer Verbindung der Formel 3 zusammengebracht wird, in welcher R⁸ (CH₂)ₙN₃ ist und die letztere mit einem Reduktionsmittel unter Bilden eines Zwischenprodukts der Formel 3 zusammengebracht wird, in welcher R⁸ (CH₂)ₙNH₂ ist.

37. Verfahren von Anspruch 35 oder 36, wobei R⁸ (CH₂)ₙNHR¹¹ oder (CH₂)ₙNH₂ ist und die Verbindung der Formel 3 mit einem Chlorameisensäureester der Formel R¹⁰OCOCl oder einem Sulfonylderivat der Formel R¹⁰SO₂Cl oder (R¹⁰SO₂)₂O in einem Lösungsmittel in Anwesenheit einer Base 5 Minuten bis 24 Stunden bei einer Temperatur im Bereich von 0°C bis zur Rückflußtemperatur des Lösungsmittels unter Bilden einer Verbindung der Formel 3 zusammengebracht wird, in welcher R⁸ -(CH₂)ₙNR¹¹CO₂R¹⁰ oder -(CH₂)ₙNR¹¹SO₂R¹⁰ ist.

38. Verfahren von Anspruch 35 oder 36, wobei die Verbindung der Formel 3 mit R⁸ gleich -(CH2)ₙNHR¹¹ oder (CH₂)ₙNH₂ mit einem Isocyanat oder Isothiocyanat R¹⁰NCY 5 Minuten bis 24 Stunden bei einer Temperatur im Bereich von 25°C bis zur Rückflußtemperatur des Lösungsmittels unter Bilden einer Verbindung der Formel 3 zusammengebracht wird, in welcher R⁸ -(CH₂)ₙNR¹¹CYNHR¹⁰ ist.

39. Verfahren von Anspruch 1, wobei R¹ in A) und B) NO₂ ist und R, R³, R⁶, R⁷ und R⁸ wie in Anspruch 24 definiert sind, wobei eine Verbindung, in welcher R¹ NO₂ ist, mittels Eisen und Essigsäure, Zinn(II)-chlorid oder Wasserstoff und Palladium zu einer Verbindung reduziert wird, worin R¹ NH₂ ist und letztere mit einem entsprechenden Säureanhydrid wie etwa Phthalanhydrid oder einem substituierten Phthalanhydrid in einem Lösungsmittel oder mit einem entsprechenden Säurechlorid wie etwa einem substituierten Anthranilsäurechlorid in Anwesenheit wäßrigen Alkalis oder einer Base oder mit einer entsprechend substituierten Phthal- oder Anthranilsäure in Anwesenheit von Dicyclohexylcarbodiimid unter Herstellen einer Verbindung umgesetzt wird, in welcher R¹ 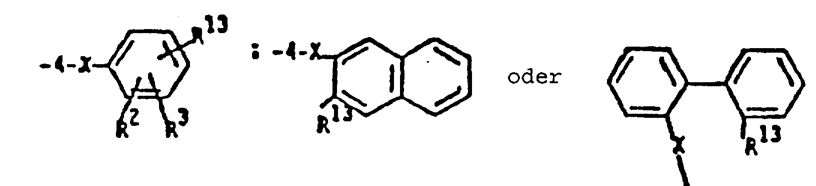 ist, und X NHCO ist.

40. Verfahren von Anspruch 1, wobei.in A) R¹ OCH₂C₆H₅ ist, R und R³ H sind und R⁶, R⁷ und R⁸ wie in Anspruch 1 definiert sind und die sich daraus ergebende Verbindung der Formel 3 mit R¹ gleich OCH₂C₆H₅ über einen Zeitraum von 0,2-1 Stunde mit Trifluoressigsäure bei Rückflußtemperatur oder mit Wasserstoff und Palladium unter Bilden der entsprechenden Verbindung der Formel 3 umgesetzt wird, in welcher R¹ OH ist und letztere mit einer Base bei 25°C und einem geeigneten Benzylhalogenid der Formel 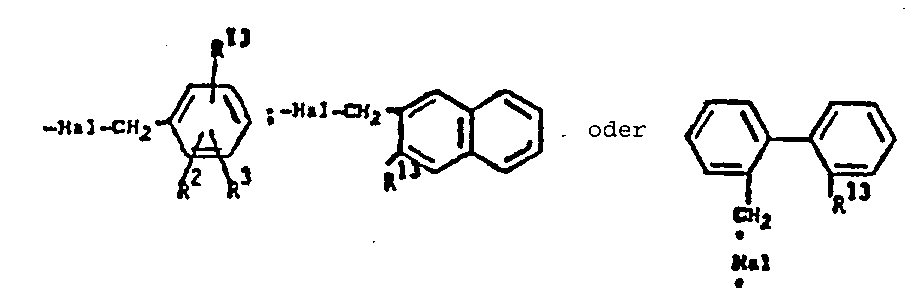 unter Herstellen der entsprechenden Verbindung der Formel 3 zusammengebracht wird, worin R¹ 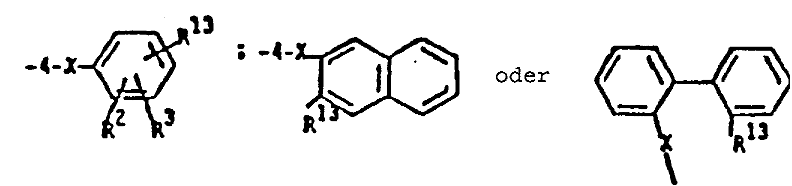 ist,
und X -OCH₂- ist.

41. Verfahren von Anspruch 1, wobei R⁸ in A) -CHO ist, wobei das Benzylderivat der Formel 2 an dem Imidazolderivat der Formel 1 vorzugsweise an das dem Kohlenstoffatom des Imidazolrings, an das R⁸ gebunden ist, benachbarte Stickstoffatom gebunden ist.

42. Verfahren von Anspruch 19, wobei R⁸ CHO ist und X eine Kohlenstoff-Kohlenstoff-Einfachbindung ist und das Produkt der Formel 3 mit einem metallorganischen Reagenz wie etwa R¹¹MgBr oder R¹¹Li in Anwesenheit eines wasserfreien Lösungsmittels ohne Hydroxylgruppen wie etwa Ether, THF oder Dimethoxyethan bei -78 bis 25°C, gefolgt von wäßriger Aufarbeitung, gefolgt von der sauren Hydrolyse irgendwelcher Gruppen CO₂R¹⁴, worin R¹⁴ t-Butyl ist, oder der Hydrolyse irgendwelcher tritylgeschützter Tetrazolgruppen unter Bilden der entsprechenden Verbindung der Formel 3, worin R⁸ 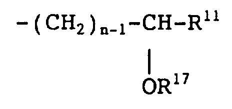 ist, worin R¹¹ ≠ H.

43. Verfahren gemäß einem der Ansprüche 1-42, wobei die hergestellte Verbindung die Formel 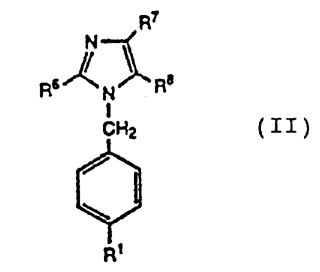 worin
R¹ - CO₂H ; -NHSO₂CF₃ ; 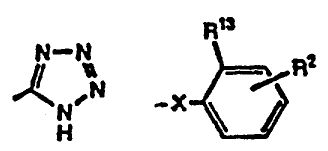 oder 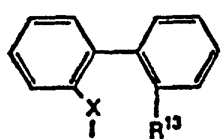 ist,
R⁶ Alkyl mit 3 bis 10 Kohlenstoffatomen, Alkenyl mit 3 bis 10 Kohlenstoffatomen, Alkinyl mit 3 bis 10 Kohlenstoffatomen, Cycloalkyl mit 3 bis 8 Kohlenstoffatomen, Benzyl ist, das am Phenylring mit bis zu zwei Gruppen substituiert ist, die aus Alkoxy mit 1 bis 4 Kohlenstoffatomen, Halogen, Alkyl mit 1 bis 4 Kohlenstoffatomen und Nitro ausgewählt sind,
R⁸ -(CH₂)ₘ-Tetrazolyl, -(CH₂)ₙOR¹¹; 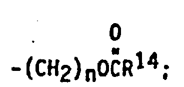 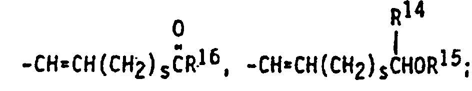 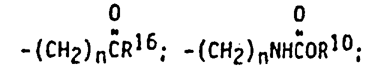 -(CH₂)ₙNHSO₂R¹⁰ ; oder 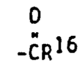 ist,
R¹³ -CO₂H, -CO₂R⁹, NHSO₂CF₃, SO₃H oder 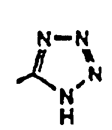 ist,
R¹⁶ H, Alkyl mit 1 bis 5 Kohlenstoffatomen, OR¹⁷ oder NR¹⁸R¹⁹ ist,
X eine Kohlenstoff-Kohlenstoff-Einfachbindung, -CO-, 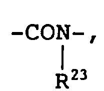 -CH₂CH₂- , 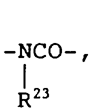 -OCH₂- , -CH₂O-, -O-, -SCH₂- , -CH₂S - , -NHCH₂- , -CH₂NH- oder -CH=CH- ist,
oder eines pharmazeutisch annehmbaren Salzes derselben besitzt.

44. Verfahren von Anspruch 43, wobei
R H, Alkyl mit 1 bis 4 Kohlenstoffatomen, Halogen oder Alkoxy mit 1 bis 4 Kohlenstoffatomen ist,
R⁶ Alkyl, Alkenyl oder Alkinyl mit 3 bis 7 Kohlenstoffatomen ist,
R⁷ Heteroaryl ist, das aus 2- und 3-Thienyl, 2- und 3-Furyl, 2-, 3- und 4-Pyridyl oder p-Biphenylyl 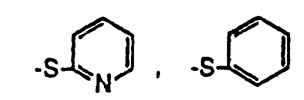 ausgewählt ist,
R⁸ -(CH₂)ₘOR¹¹ ; 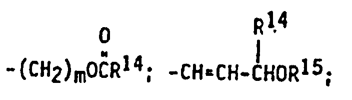 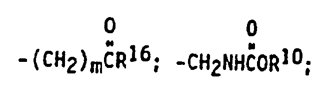 -(CH₂)ₘNHSO₂R¹⁰ oder -COR¹⁶ ist,
R¹⁰ CF₃, Alkyl mit 1 bis 6 Kohlenstoffatomen oder Phenyl ist,
R¹¹ H oder Alkyl mit 1 bis 4 Kohlenstoffatomen ist,
R¹³ CO₂H, CO₂CH₂OCOC(CH₃)₃, NHSO₂CF₃ oder 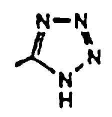 ist,
R¹⁴ H oder Alkyl mit 1 bis 4 Kohlenstoffatomen ist,
R¹⁵ H, Alkyl mit 1 bis 4 Kohlenstoffatomen oder Acyl mit 1 bis 4 Kohlenstoffatomen ist,
R¹⁶ H, Alkyl mit 1 bis 5 Kohlenstoffatomen, OR¹⁷ oder 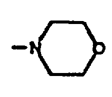 ist,
m 1 bis 5 ist,
X = Einfachbindung, -O-, -CO-, -NHCO- oder -OCH₂-, oder ein pharmazeutisch annehmbares Salz davon.

45. Verfahren von Anspruch 44, wobei R¹ 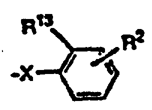 ist und X eine Einfachbindung ist oder ein pharmazeutisch geeignetes Salz davon.

46. Verfahren zur Herstellung einer pharmazeutischen Zusammensetzung, welches das Vermischen eines pharmazeutisch geeigneten Trägers mit einer gemäß einem der Ansprüche 1 bis 45 hergestellten Verbindung umfaßt.

47. Verfahren von Anspruch 46, wobei die pharmazeutische Zusammensetzung zusätzlich einen diuretischen oder einen nicht-steroidalen, entzündungshemmenden Wirkstoff enthält.

## Revendications (Revendications pour l'(les) Etat(s) contractant(s) suivant(s): AT, BE, CH, DE, DK, FR, GB, IT, LI, LU, NL, SE)

1. Un dérivé anti-hypertensif de formule: 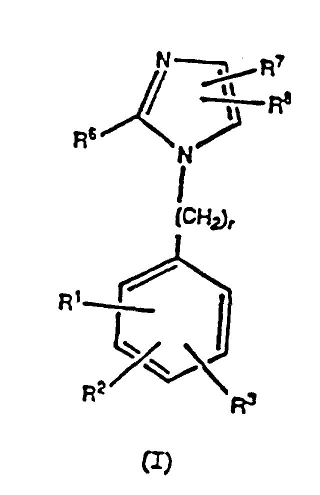 dans laquelle:
R¹ est 4-CO₂H; 4-CO₂R⁹; 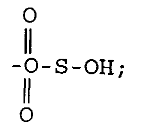 -SO₃H; -C(CF₃)₂OH; 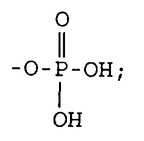 -PO₃H₂; 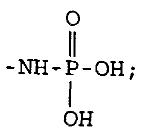 4-NHSO₂CH₃; 4-NHSO₂CF₃; -CONHOR¹; -SO₂NH₂; 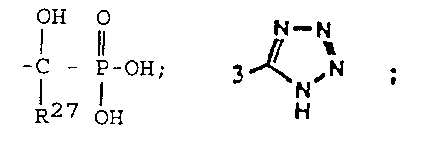 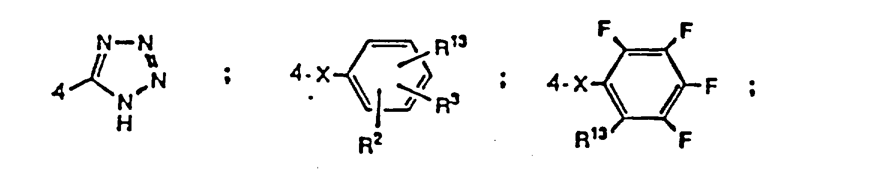 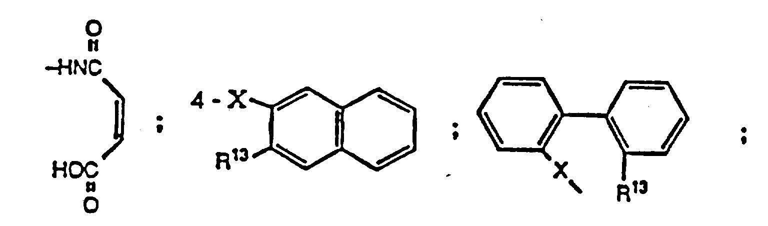 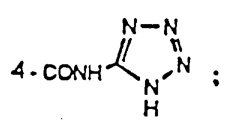 4-CONHNHSO₂CF₃; 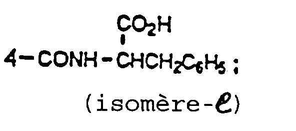 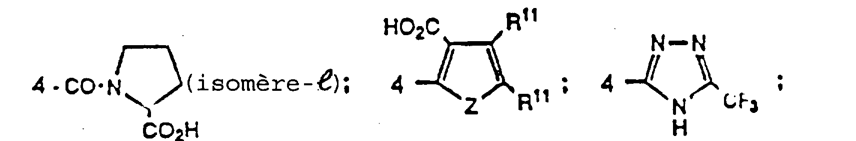 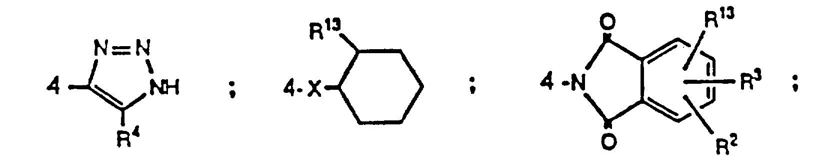 ou 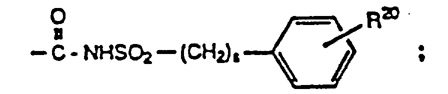
R est H; Cl; Br, I; F; NO₂; CN; un alkyle comportant 1 à 4 atomes de carbone; un acyloxy comportant 1 à 4 atomes de carbone; un alcoxy comportant 1 à 4 atomes de carbone; CO₂H; CO₂R⁹; NHSO₂CH₃; NHSO₂CF₃; CONHOR¹; SO₂NH₂; 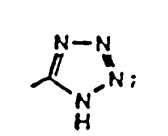 phényle ou furyle;
R³ est H, Cl, Br, I ou F; un alkyle comportant 1 à 4 atomes de carbone ou un alcoxy comportant 1 à 4 atomes de carbone;
R⁴ est CN, NO₂ ou CO₂R¹¹;
R⁵ est H, un alkyle comportant 1 à 6 atomes de carbone, un cycloalkyle comportant 3 à 6 atomes de carbone, un alkényle ou un alkynyle comportant 2 à 4 atomes de carbone;
R⁶ est un alkyle comportant 2 à 10 atomes de carbone, un alkényle ou un alkynyle comportant 3 à 10 atomes de carbone ou ces mêmes groupes substitués par F ou CO₂R¹⁴; un cycloalkyle comportant 3 à 8 atomes de carbone, cycloalkylalkyle comportant 4 à 10 atomes de carbone; un cycloalkylalkényle ou un cycloalkylalkynyle comportant 5 à 10 atomes de carbone; (CH₂)ₛZ(CH₂)ₘR⁵ éventuellement substitué par F ou CO₂R¹⁴; un benzyle ou un benzyle substitué sur le noyau phényle par 1 ou 2 halogènes, un alcoxy comportant 1 à 4 atomes de carbone, un alkyle comportant 1 à 4 atomes de carbone ou nitro;
R⁷ est un vinyle, cycloalkylidényle; alkynyle comportant 2 à 10 atomes de carbone; phénylalkynyle dont la partie alkynyle comporte 2 à 6 atomes de carbone; hétéroaryle choisi parmi 2- et 3-thiényle, 2- et 3-furyle, 2-, 3- et 4-pyridyle, 2-pyrazinyle, 2-, 4- et 5-pyrimidinyle, 3- et 4-pyridazinyle, 2-, 4- et 5-thiazolyle, 2-, 4- et 5-sélénazolyle, et 2- et 4- et 5-oxazolyle, 2- ou 3-pyrrolyle, 3-, 4- ou 5-pyrazolyle, 2-, 4- ou 5-imidazolyle; o-, m- ou p-biphénylyle; o-, m- ou p-phénoxyphényle; 2-oxazolinyle; 2-thiazolinyle; phénylalkynyle substitué, hétéroaryle, biphénylyle ou phénoxyphényle tel que défini ci-dessus, substitué par un ou deux substituants choisis parmi halogène, hydroxy, mercapto, alcoxy comportant 1 à 5 atomes de carbone, alkyle comportant 1 à 5 atomes de carbone, -NO₂, -CN, -CF₃; -COR¹⁶, -CH₂OR¹⁷, -NHCOR¹⁷, -CONR¹⁸R¹⁹, S(O)ᵣR¹⁷ et SO₂NR¹⁸R¹⁹; pyrrolyle, pyrazolyle ou imidazolyle tels que définis ci-dessus, substitués sur les azotes du cycle par des alkyles comportant 1 à 5 atomes de carbone, phényle ou benzyle; ou alkyle substitué, alkényle, ou alkynyle comportant 1 à 10 atomes de carbone substitué par un groupe hétéroaryle, biphénylyle ou phénoxyphényle substitués ou non substitués tel que définis ci-dessus; -S(O)ᵣ-hétéroaryle, -S(O)ᵣ-biphénylyle, -S(O)ᵣ-phénoxyphényle, -S-tétrazole, -S(O)ᵣR¹⁷, -NR¹⁸R¹⁹, -NR¹⁸-hétéroaryle, -NR¹⁸-phényle, -NR¹⁸-biphényle, -NR¹⁸-phénoxyphényle, -N-phtalimido, -NH-SO₂-phénoxyphényle, -NH-SO₂-hétéroaryle, -NH-SO₂-biphényle, -NH-SO₂-R¹⁷, et -S-(C=O)-R¹⁷, N-imidazolyle, N-1,2,3-triazolyle, N-1,2,4-triazolyle, où l'hétéroaryle est un hétérocycle défini à propos de R⁷ et où le groupe phényle dans R¹⁷ de -S-(O)ᵣR¹⁷, le N-imidazolyle, le N-1,2,3-triazolyle et le N-1,2,4-triazolyle peuvent être substitués par un ou deux substituants tels que décrit ci-dessus pour les hétéroaryles:
R⁸ est 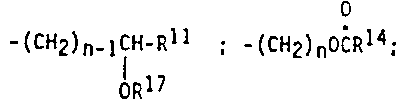 -(CH₂)ₙSR¹⁵; 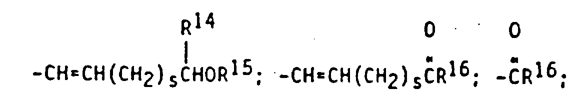 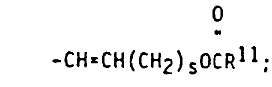 -(CH₂)ₘ-tetrazolyl; 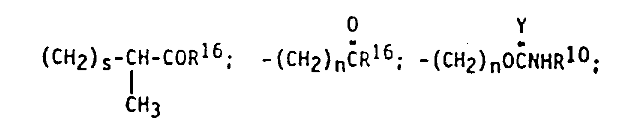 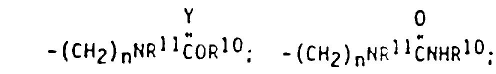 -(CH₂)ₙNR¹¹SO₂R¹⁰; 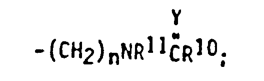
R⁹ est 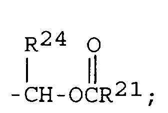
R¹⁰ est un radical alkyle comportant de 1 à 6 atomes de carbone ou perfluoroalkyle comportant 1 à 6 atomes de carbone, 1-adamantyle, 1-naphtyle, 1-(1-naphtyl)--éthyle, ou (CH₂)ₚC₆H₅;
R¹¹ est H, un alkyle comportant 1 à 6 atomes de carbone, cycloalkyle comportant 3 à 6 atomes de carbone, phényle ou benzyle;
R¹ est H, méthyle ou benzyle;
R¹³ est -CO₂H; -CO₂R⁹; -CH₂CO₂H, -CH₂CO₂R⁹; ou 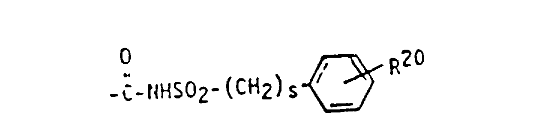 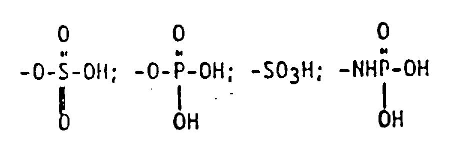 -PO₃H₂; -C(CF₃)₂OH; -NHSO₂CH₃; -NHSO₂CF₃; -NHCOCF₃; -CONHOR¹; -SO₂NH₂; 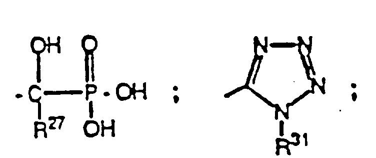 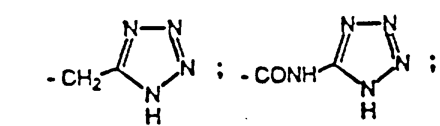 -CONHNHSO₂CF₃ ; 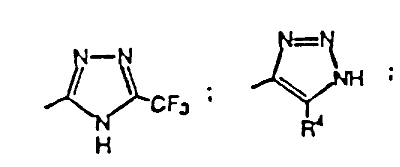
R¹⁴ est un hydrogène, un alkyle ou un perfluoroalkyle comportant 1 à 8 atomes de carbone, cycloalkyle comportant 3 à 6 atomes de carbone, phényle ou benzyle;
R¹⁵ est un hydrogène, un alkyle comportant 1 à 6 atomes de carbone, cycloalkyle comportant 3 à 6 atomes de carbone, phényle, benzyle, acyle comportant 1 à 5 atomes de carbone; phénacyle;
R¹⁶ est un hydrogène, un alkyle comportant 1 à 6 atomes de carbone, cycloalkyle comportant 3 à 6 atomes de carbone; (CH₂)ₚC₆H₅, OR¹⁷, ou bien NR¹⁸R¹⁹;
R¹⁷ est un hydrogène, un alkyle comportant 1 à 6 atomes de carbone, cycloalkyle comportant 3 à 6 atomes de carbone, phényle ou benzyle;
R¹⁸ et R¹⁹ représentent indépendamment l'un de l'autre un hydrogène, un alkyle comportant 1 à 4 atomes de carbone, phényle, benzyle, α-méthylbenzyle, ou pris ensemble avec l'atome d'azote, forment un cycle de formule: 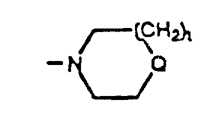
Q est NR⁰, O ou CH₂;
R⁰ est un hydrogène, un alkyle comportant 1 à 4 atomes de carbone, ou phényle;
R¹ est un alkyle comportant 1 à 6 atomes de carbone, -NRR³, ou 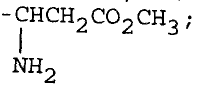
R et R³ représentent indépendamment l'un de l'autre un hydrogène, un alkyle comportant 1 à 6 atomes de carbone, un benzyle, ou forment ensemble (CH₂)ᵤ où u est compris entre 3 et 6;
R⁴ est H, CH₃ ou C₆H₅;
R⁵ est NR⁷R⁸, OR⁸, NHCONH₂, NHCSNH₂, 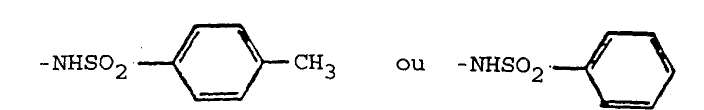
R⁶ est de l'hydrogène, un alkyle comportant 1 à 6 atomes de carbone, benzyle ou allyle;
R⁷ et R⁸ sont, indépendamment l'un de l'autre, des hydrogène, des alkyles comportant 1 à 5 atomes de carbone, ou phényle;
R⁹ et R³⁰ sont indépendamment des alkyles comportant 1 à 4 atomes de carbone, ou pris ensemble, forment -(CH₂)_{q}-;
R³¹ est H, un alkyle comportant 1 à 4 atomes de carbone, -CH₂CH=CH₂ ou -CH₂C₆H₄R³;
R³ est H, NO₂, NH₂, OH ou OCH₃;
X est une liaison simple carbone-carbone, -CO-, -CH₂-, -O-, -S-, -NH-, 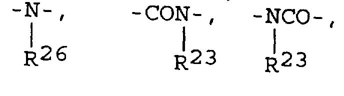 -OCH₂-, CH₂O-, -SCH₂-, -CH₂S-, -NHC(R⁷)(R⁸)-, -NR³ SO₂-, -SO₂NE³-, -C(R⁷)(R⁸)NH-, -CH=CH-, -CF=CF-, -CH=CF-, -CF=CH-, -CH₂CH₂-, -CF₂CF₂-, 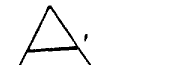 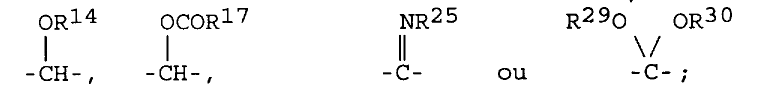
Y est O ou S;
Z est O, NR¹¹, ou S;
m vaut de 1 à 5;
n vaut de 1 à 10;
p vaut de 0 à 3;
q vaut de 2 à 3;
r vaut de 0 à 2;
s vaut de 0 à 5;
t vaut 0 ou 1;
ou un de leurs sels pharmaceutiquement acceptable; avec cette condition que:
(1) le groupe R¹ n'est pas en position ortho;
(2) lorsque R¹ est 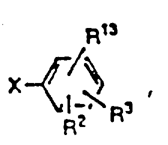 X est une simple liaison, et R¹³ est CO₂H, ou 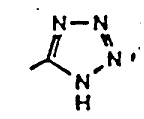 et R¹³ doit être en position ortho ou méta;
ou si R¹ et X sont tels que ci-dessus et R¹³ est NHSO₂CF₃ ou NHSO₂CH₃, R¹³ doit être en ortho;
(3) si R¹ est 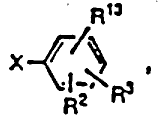 et X est autre qu'une simple liaison,
alors, R¹³ doit être ortho, sauf si X = NR³CO et R¹³ est NHSO₂CF₃ ou NHSO₂CH₃, alors R¹³ doit être en ortho ou en méta;
(4) si R¹ est 4-CO₂H ou un de ses sels, R⁶ ne peut pas être S-alkyle;
(5) si R⁵ est 4-CO₂H ou un de ses sels, le substituant en position-4 de l'imidazole ne peut pas être CH₂OH, ^{CH}2^{OCOCH}3^{, ni CH}2^{CO}2^{H;}
(6) si R¹ est 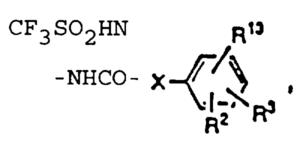 R⁶ n'est pas un méthoxybenzyle;
(7) le groupe R⁶ n'est pas 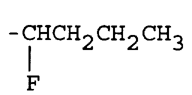 ou CH₂OH

2. Un composé selon la revendication 1, répondant à la formule: 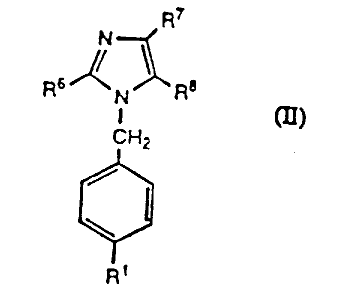 dans laquelle
R¹ est -CO₂H; -NHSO₂CF₃; 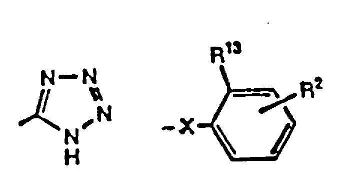 ou 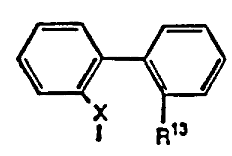
R⁶ est un alkyle comportant 3 à 10 atomes de carbone, alkényle comportant 3 à 10 atomes de carbone, alkynyle comportant 3 à 10 atomes de carbone, cycloalkyle comportant 3 à 8 atomes de carbone, benzyle substitué sur le noyau phényle par un à deux groupes choisis parmi alcoxy comportant 1 à 4 atomes de carbone, halogène, alkyle comportant 1 à 4 atomes de carbone, et nitro;
R⁸ est -(CH₂)ₘ-tétrazolyle, 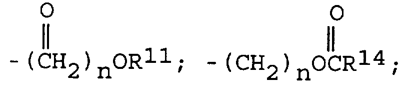 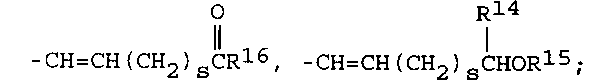 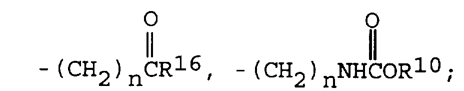 -(CH₂)ₙNHSO₂R¹⁰; ou 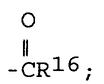
R¹³ est -CO₂H, -CO₂R⁹, NHSO₂CF₃; SO₃H; ou 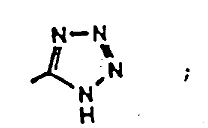
R¹⁶ est H, un alkyle comportant 1 à 5 atomes de carbone, OR¹⁷ ou NR¹⁸R¹⁹;
X est une liaison simple carbone, -CO-, 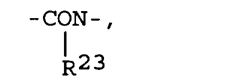 -CH₂CH₂-, 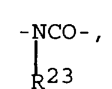 -OCH₂-, -CH₂O, -O-, -SCH₂-, -CH₂S-, -NHCH₂-, -CH₂NH- ou -CH=CH-;
ou un de leurs sels pharmaceutiquement acceptable.

3. Un composé selon la revendication 2, dans lequel:
R est H, un alkyle comportant 1 à 4 atomes de carbone, un halogène ou un alcoxy comportant 1 à 4 atomes de carbone;
R⁶ est un alkyle, un alkényle ou un alkynyle comportant 3 à 7 atomes de carbone;
R⁷ est un hétéroaryle choisi parmi 2- et 3-thiényle, 2- et 3-furyle, 2-, 3- et 4-pyridyle, ou parabiphénylyle, 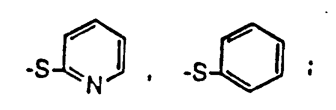
R⁸ est -(CH₂)ₘOR¹¹; 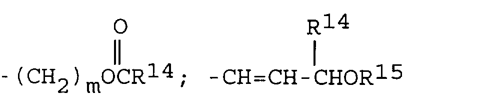 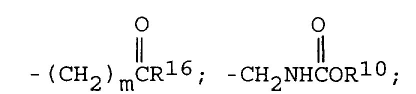 -(CH₂)ₘNHSO₂R¹⁰; ou -COR¹⁶;
R¹⁰ est CF₃, un alkyle comportant 1 à 6 atomes de carbone ou un phényle;
R¹¹ est H, ou un alkyle comportant 1 à 4 atomes de carbone;
R¹³ est CO₂H; CO₂CH₂OCOC(CH₃)₃; NHSO₂CF₃ ou 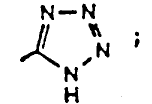
R¹⁴ est H, ou un alkyle comportant 1 à 4 atomes de carbone;
R¹⁵ est H, un alkyle comportant 1 à 4 atomes de carbone, ou acyle comportant 1 à 4 atomes de carbone;
R¹⁶ est H, un alkyle comportant 1 à 5 atomes de carbone; OR¹⁷ ou 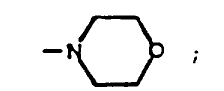
m vaut de 1 à 5;
X est égal à une liaison simple, -O-; -CO-; -NHCO-; ou -OCH₂-;
ou bien un de leurs sels pharmaceutiquement acceptable.

4. Un composé selon la revendication 3, dans lequel R¹ représente: 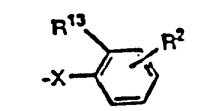 et X représente une liaison simple ou un de ses sels pharmaceutiquement acceptable

5. Une composition pharmaceutique comprenant un support pharmaceutiquement acceptable et un composé selon l'une quelconque des revendications 1 à 4.

6. Une composition pharmaceutique selon la revendication 5, qui contient en outre un médicament diurétique ou un médicament anti-inflammatoire non stéroïdien.

7. Utilisation d'un composé selon l'une quelconque des revendications 1 à 4 pour la préparation d'un médicament pour prévenir l'insuffisance rénale chez un animal à sang chaud, due à l'administration d'un médicament anti-inflammatoire non-stéroidien (NSAID).

8. Utilisation d'un composé selon l'une quelconque des revendications 1 à 4 pour la préparation d'un médicament destiné au traitement de l'hypertension chez un animal à sang chaud.

9. Utilisation selon la revendication 8, dans laquelle le médicament comprend en outre une diurétique.

10. Utilisation d'un composé selon l'une quelconque des revendications 1 à 4 pour la préparation d'un médicament destiné au traitement de l'insuffisance cardiaque chez un animal à sang chaud.

11. Un procédé de préparation d'un composé selon la revendication 1, dans lequel r vaut 1, qui comprend la mise en contact d'un dérivé d'imidazole de formule 1 avec un dérivé benzylique de formule 2 dans un solvant en présence d'une base pendant environ 1 à environ 10 heures, à une température de l'ordre d'environ 20°C à la température de reflux du solvant, pour former une benzylimidazole de formule 3: 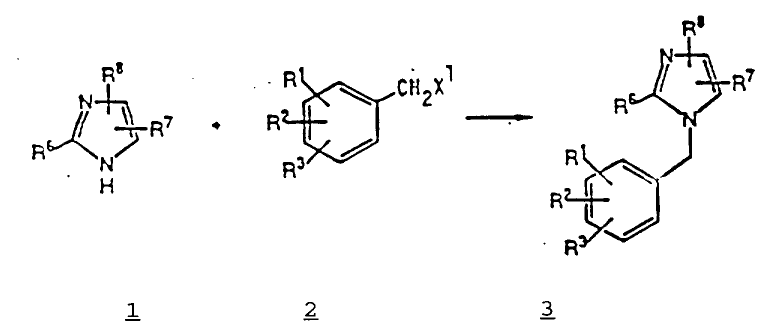 où chacun des R¹, R, R³, R⁶, R⁷ et R⁸ est stable dans les conditions réactionnelles et est un groupe tel que défini dans la revendication 1 ou une de ses formes intermédiaires ou protégées qui peut être transformée en un tel groupe et où X¹ est un halogène, p-toluènesulfonyloxy ou méthylsulfonyloxy; et ensuite, si nécessaire, transformation de ladite forme intermédiaire ou protégée des groupes R en groupe R tel que défini dans la revendication 1.

12. Procédé selon la revendication 11, dans lequel les composés 1 et 2 sont mis en contact en présence d'une base choisie parmi le groupe consistant en hydrure métallique (MH), alcoxyde métallique (MOR), carbonate de sodium, carbonate de potassium, diéthylamine et pyridine, dans un solvant aprotique dipolaire, ou, si la base est MOR, le solvant peut être un alcool, ROH, où M est le lithium, le sodium ou le potassium, et R est du méthyle ou du tert-butyle.

13. Procédé selon la revendication 11, dans lequel on utilise un solvant à deux phases, l'un étant une phase organique de chlorure de méthylène et l'autre étant une phase aqueuse, en présence d'un catalyseur de transfert de phase tel que chlorure de tricaprylméthylammonium.

14. Procédé selon la revendication 12, dans lequel:
R¹ est 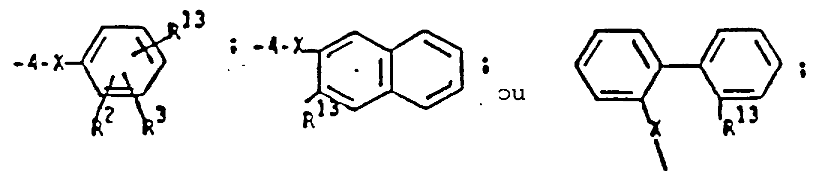
X représente une liaison simple carbone-carbone, -CO-, -O-, -S-, -O- ou -NH-;
R et R³, représentent indépendamment H, Cl, Br, I, CO₂R¹⁴, F, NO₂, un alkyle comportant 1 à 4 atomes de carbone; alcoxy comportant 1 à 4 atomes de carbone, phényle ou furyle;
R⁶ et R⁷ sont tels que définis dans la revendication 1;
R⁸ représente -(CH₂)ₙOR¹¹; -(CH₂)ₙSR¹⁵; ou -(CH₂)ₙCN;
R¹¹ est tel que défini dans la revendication 1;
R¹³ représente CO₂R¹⁴, CN, NO₂, trialkylétain, tétrazole ou trityltétrazole; et
R¹⁴ et R¹⁵ sont tels que définis dans la revendication 1.

15. Procédé selon la revendication 14, dans lequel R¹³ représente -CO₂R¹⁴ et le produit de formule 3 est mis en contact avec un alcali dans un solvant hydroalcoolique ou de CF₃CO₂H à une température comprise entre environ 20°C et la température de reflux du solvant pendant 1 à 24 heures, suivi par l'ajustement du pH du mélange à une valeur comprise entre 3 et 7 pour transformer le produit en le produit correspondant où R¹³ représente -CO₂H.

16. Procédé selon la revendication 15, dans lequel au moins l'un des R, R³ ou R¹³ dans la formule 1 représente -CO₂R¹⁴ et est transformé en -CO₂H.

17. Procédé selon la revendication 15, dans lequel R¹⁴ représente du t-butyle et la réaction est effectuée dans CF₃CO₂H.

18. Procédé selon la revendication 14, dans lequel R¹³ représente -CN et le produit de formule 3 est mis en contact avec:
(i) un acide fort à la température de reflux du solvant pendant 2 à 96 heures; ou
(ii) une base forte dans un solvant alcoolique à une température comprise entre 20°C et la température de reflux du solvant pendant 2 à 96 heures, suivi par l'ajustement du pH entre 3 et 7; ou
(iii) de l'acide sulfurique, suivi par un acide ou un alcali, pour transformer le produit en le composé correspondant où R¹³ représente -CO₂H.

19. Procédé selon la revendication 18, dans lequel au moins des R, R³ ou R¹³ représente -CO₂R¹⁴ et est transformé en -CO₂H.

20. Procédé selon la revendication 18, dans lequel R⁸ représente -(CH₂)ₙCN et est transformé en -(CH₂)ₙCO₂H, ou représente -(CH₂)ₙOR¹¹ et est transformé en (CH₂)ₙOH quand R¹³ est transformé en -CO₂H.

21. Procédé selon la revendication 14, dans lequel R¹³ représente -CN et le produit de formule 3 est mis en contact avec un mélange de quantité équimolaire d'azidure de sodium et de chlorure d'ammonium dans un solvant aprotique polaire à une température comprise entre 30°C et la température de reflux du solvant, pendant 1 heure à 10 jours, pour transformer le produit en le dérivé correspondant où R¹³ représente 5-tétrazolyle.

22. Procédé selon la revendication 21, dans lequel R⁸ représente -(CH₂)ₘCN et est transformé en -(CH₂)ₘ-tétrazolyle, quand R¹³ est transformé en 5-tétrazolyle.

23. Procédé selon la revendication 14, dans lequel R¹³ représente -CN et on fait réagir le produit de formule 3 avec un azidure de trialkylétain ou un azidure de triarylétain, puis on effectue l'hydrolyse acide ou basique pour transformer le produit en le dérivé correspondant dans lequel R¹³ représente 5-tétrazolyle.

24. Procédé selon la revendication 14, dans lequel R¹³ représente -CN et dans lequel on fait réagir le produit de formule 3 avec un azidure de trialkylétain ou un azidure de triarylétain pour obtenir un composé de formule 3 où R¹³ représente un trialkyle ou triarylstannyltétrazol-5-yle, et on fait réagir ce dernier composé avec le chlorure de triphénylméthyle pour former un composé de formule 3 où R¹³ représente le triphénylméthyltétrazol-5-yle et on hydrolyse ce dernier composé pour obtenir un composé de formule 3 où R¹³ représente le 5-tétrazolyle.

25. Procédé selon la revendication 23, dans lequel R⁸ représente -(CH₂)ₘCN et est transformé en -(CH₂)ₘ-tétrazolyle, quand R¹³ est transformé en 5-tétrazolyle.

26. Procédé selon la revendication 14, dans lequel R¹³ représente -NO₂ et on met le produit de formule 3 est mis en contact avec un agent réducteur pour former un second intermédiaire de formule 3 dans lequel R¹³ représente -NH₂ et ce dernier est mis en contact avec un anhydride (CH₃SO₂)₂O ou (CF₃SO₂)₂O ou un chlorure CH₃SO₂Cl ou CF₃SO₂Cl d'un acide sulfonique dans un solvant pour former un composé dans lequel R¹³ -NHSO₂CH₃ ou -NHSO₂CF₃.

27. Procédé selon la revendication 26, dans lequel au moins un des R, R³ ou R¹³ est -NO₂ et est converti en -NHSO₂CH₃ ou -NHSO₂CF₃.

28. Procédé selon la revendication 15 ou 18, dans lequel le composé de formule 3 avec R¹³=CO₂H, soit:
(a) est mis en contact avec environ 1 à 4 équivalents de chlorure de thionyle dans un excès de chlorure de thionyle ou un autre solvant à une température comprise entre 20°C et la température de reflux du solvant pendant 5 minutes à 2 heures pour former un intermédiaire de formule 3 où R¹³ est COCl, et ce dernier est mis en contact avec 2 à 10 équivalents de dérivé d'hydroxylamine H₂NOR¹ dans un excès de dérivé d'hydroxylamine H₂NOR¹ ou un autre solvant, à une température de 25 à 80°C, pendant 2 à 18 heures, ou
(b) est mis en contact avec du dérivé d'hydroxylamine H₂NOR¹, du dicyclohexylcarbodiimide et du 1-hydroxybenzotriazole dans un solvant à une température de 0 à 30°C, pendant 1 à 24 heures; pour donner un composé dans lequel R¹³ est CONHOR¹.

29. Procédé selon la revendication 11, dans lequel:
R¹ représente: 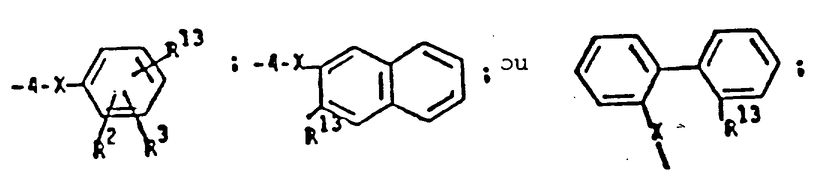
X représente une liaison simple carbone-carbone, -CO-, -O-, -S- ou -NH-;
R, R³, R⁶ et R⁷ sont tels que définis dans la revendication 1; et
R⁸ représente -(CH₂)ₙOR¹¹; -(CH₂)ₙOCR¹⁴; (CH₂)ₙCH(OH)R¹⁶, (CH₂)ₙCOR¹⁶, (CH₂)ₙCl, (CH₂)ₙCN, CHO.

30. Procédé selon la revendication 29, dans lequel R⁸ représente (CH₂)ₙOH et le produit de formule 3 est mis en contact avec un alcool R¹¹OH à l'état anhydre en présence d'un acide fort ou d'un acide de Lewis, suivi d'une saponification de tout groupe CO₂R¹⁴ formé en même temps ou présent dans l'intermédiaire 3 pour former le dérivé correspondant de formule 3 où R⁸ représente (CH₂)ₙOR¹¹ et R¹¹ n'est pas H.

31. Procédé selon la revendication 29, dans lequel R⁸ représente (CH₂)ₙOR¹¹, et R¹¹ n'est pas H et le produit de formule 3 est mis en contact avec un milieu acide aqueux à une température de 25°C à la température de reflux du solvant pendant 0,5 à 24 heures pour former le dérivé correspondant de formule 3 où R⁸ représente (CH₂)ₙOH.

32. Procédé selon la revendication 29, dans lequel R⁸ représente (CH₂)ₙOH et le produit de formule 3 est mis en contact avec:
(a) un anhydride d'acide carboxylique (R¹⁴CO)₂O ou un chlorure R¹⁴COCl dans un solvant en présence d'une base à une température comprise entre 0°C et la température de reflux du solvant pendant 0,5 à 24 heures, ou
(b) un acide carboxylique R¹⁴CO₂ dans des conditions anhydre, en présence d'un acide fort ou d'un acide de Lewis, à une température de 0°C à 100°C pendant 0,5 à 24 heures, pour former le composé correspondant dans lequel R⁸ représente (CH₂)ₙOCOR¹⁴.

33. Procédé selon la revendication 29, dans lequel R⁸ représente (CH₂)ₙOCOR¹⁴ et le produit de formule 3 est mis en contact avec un acide ou d'un alcali aqueux pour former le dérivé correspondant où R⁸ représente (CH₂)ₙOH.

34. Procédé selon la revendication 29, dans lequel R⁸ représente (CH₂)ₙOH et le produit de formule 3 est mis en contact avec un agent d'oxydation à une température de 25°C à 45°C pendant 1 à 200 heures pour obtenir un composé correspondant de formule 3 dans lequel R⁸ représente (CH₂)ₙ₋₁COR¹⁶ et R¹⁶ représente H.

35. Procédé selon la revendication 29, dans lequel R⁸ représente (CH₂)ₙOCOR¹⁶ et R¹⁶ représente H et le produit de formule 3 est mis en contact avec un dérivé organométallique R¹⁶P dans lequel P représente MgBr ou Li dans un solvant à une température de -78°C à 100°C pendant 0,5 à 24 heures, pour obtenir un composé de formule 3 dans lequel R⁸ représente (CH₂)ₙCH(OH)R¹⁶ et R¹⁶ ne représente pas H.

36. Procédé selon la revendication 29, dans lequel R⁸ représente (CH₂)ₙCH(OH)R¹⁶ et R¹⁶ ne représente pas H, et le produit de formule 3 est mis en contact avec un agent d'oxydation dans un solvant pour former un dérivé correspondant de formule 3 dans lequel R⁸ représente (CH₂)ₙCOR¹⁶ et R¹⁶ ne représente pas H.

37. Procédé selon la revendication 29, dans lequel R⁸ représente (CH₂)ₙCOR¹⁶ et R¹⁶ représente H, et le composé de formule 3 est mis en contact avec un agent d'oxydation dans un solvant pour former un composé correspondant de formule 3 dans lequel R⁸ représente (CH₂)ₙCOR¹⁶ et R¹⁶ représente OH.

38. Procédé selon la revendication 29, dans lequel R⁸ représente (CH₂)ₙCOR¹⁶ et R¹⁶ représente OH, et le composé de formule 3 est mis en contact avec du chlorure de thiényle en excès ou dans un autre solvant à une température comprise entre 0°C et la température de reflux du solvant pendant 5 minutes à 24 heures pour former un dérivé correspondant de formule 3, dans lequel R⁸ représente (CH₂)ₙCOCl, suivi du contact de ce dernier avec une amine NHR¹⁸R¹⁹ en excès ou dans un solvant à une température comprise entre 0°C et la température de reflux du solvant, pendant 5 minutes à 24 heures, pour former un dérivé correspondant de formule 3 dans lequel R⁸ représente (CH₂)ₙCONR¹⁸R¹⁹.

39. Procédé selon la revendication 29, dans lequel R⁸ représente (CH₂)ₙOR¹¹ et R¹¹ est H et le produit de formule 3 est mis en contact avec du chlorure de thiényle en excès ou dans un solvant à une température entre 20°C et la température de reflux du solvant pendant 0,5 à 24 heures pour former un composé intermédiaire de formule 3 dans lequel R⁸ représente (CH₂)ₙCl.

40. Procédé selon la revendication 39, dans lequel le composé de formule 3 dans lequel R⁸ représente (CH₂)ₙCl est mis en contact avec un sel de sodium ou de potassium d'un mercaptan R¹⁵SH dans un solvant à une température de 25°C à 100°C pendant 1 à 24 heures pour former un composé de formule 3 dans lequel R⁸ représente (CH₂)ₙSR¹⁵.

41. Procédé selon la revendication 29, dans lequel le composé de formule 3 dans lequel R⁸ représente (CH₂)ₙCl est mis en contact avec un cyanure de métal alcalin à une température de 20°C à 100°C, pendant 1 à 24 heures, pour former un composé de formule 3 dans lequel R⁸ représente (CH₂)ₙCN et ce dernier composé est hydrolysé en le dérivé correspondant de formule 3 dans lequel R⁸ représente (CH₂)ₙCOR¹⁶ et R¹⁶ représente OH.

42. Procédé selon la revendication 29, dans lequel le composé de formule 3 dans lequel R⁸ représente (CH₂)ₙ₋₁Cl est mis en contact avec un sel de sodium ou de potassium d'un malonate de dialkyle dans un solvant, à une température de 20°C à 100°C, pendant 0,5 à 24 heures, pour former un composé de formule 3 dans lequel R⁸ représente (CH₂)ₙ₋₁CH(CO₂alkyle)₂ suivi par la saponification de ce dernier à l'aide d'un alcali aqueux à une température comprise entre 25°C et la température de reflux du solvant, suivie par une acidification à l'aide d'un acide minéral pour former un composé de formule 3 dans lequel R⁸ représente (CH₂)ₙ₋₁CH(CO₂H)₂ suivie du chauffage de ce dernier à 120°C ou dans un acide minéral dilué à la température de reflux pour former un produit de formule 3 dans lequel R⁸ représente (CH₂)ₙCOR¹⁶ et R¹⁶ représente OH.

43. Procédé selon la revendication 29, dans lequel R⁸ représente -CHO et le dérivé de formule 3 est mis en contact avec un méthylène phosphorane (C₆H₅)₃P=CH(CH₂)ₛCHR¹⁴OR¹⁵ ou (C₆H₅)₃P=CH(CH₂)ₛCOR¹⁶ dans un solvant à une température de 25°C à la température du solvant pendant 1 à 24 heures, pour former un composé de formule 3 dans lequel R⁸ représente -CH=CH(CH₂)ₛCHR¹⁴OR¹⁵ ou -CH=CH(CH₂)ₛCOR¹⁶, sauf quand R¹⁵ représente H et R¹⁶ représente OH, et éventuellement suivi de la mise au contact du composé de formule 3 dans lequel R⁸ représente -CH=CH(CH₂)ₛCOR¹⁶ avec un agent réducteur dans un solvant, à une température de 0°C à 25°C, pendant 0,5 à 25 heures, pour former un produit de formule 3 dans lequel R⁸ représente -CH=CH(CH₂)ₛCHR¹⁴OH.

44. Procédé selon la revendication 29, dans lequel R⁸ représente (CH₂)ₙOH et le composé de formule 3 est mis en contact avec un isocyanate de formule R¹⁰NCO dans un solvant à une température comprise entre 25°C et la température de reflux du solvant pendant 5 minutes à 24 heures pour former un composé de formule 3, dans lequel R⁸ représente (CH₂)ₙOCONHR¹⁰.

45. Procédé selon la revendication 29, dans lequel le composé dans lequel R⁸ représente (CH₂)ₙCl est mis en contact avec une amine R¹¹NH₂ dans un excès d'amine ou dans un autre solvant pendant 1 à 24 heures à une température de 0°C à la température de reflux du solvant pour former un intermédiaire de formule 3, dans lequel R⁸ représente (CH₂)ₙNHR¹¹.

46. Procédé selon la revendication 29, dans lequel R⁸ représente (CH₂)ₙCl et le composé de formule 3 est mis en contact avec un azidure de métal alcalin dans un solvant aprotique à une température de 25°C à 80°C pendant 1 à 24 heures pour former un composé de formule 3, dans lequel R⁸ représente (CH₂)ₙN₃ et ce dernier est mis en contact avec un agent réducteur pour former un intermédiaire de formule 3, dans lequel R⁸ représente (CH₂)ₙNH₂.

47. Procédé selon la revendication 45 ou 46, dans lequel R⁸ représente (CH₂)ₙNHR¹¹ ou (CH₂)ₙNH₂ et le composé de formule 3 est mis en contact avec un chloroformiate de formule R¹⁰OCOCl ou un dérivé sulfonyle de formule R^{1O}SO₂Cl, ou (R¹⁰SO₂)₂O dans un solvant en présence d'une base à une température de 0°C à la température de reflux du solvant pendant 5 minutes à 24 heures pour former un composé de formule 3 dans lequel R⁸ représente (CH₂)ₙNR¹¹CO₂R¹⁰ ou (CH₂)ₙNR¹¹SO₂R¹⁰.

48. Procédé selon la revendication 45 ou 46, dans lequel le dérivé de formule 3 où R⁸ représente -(CH₂)ₙNHR¹¹ ou (CH₂)ₙNH₂ est mis en contact avec un isocyanate ou isothiocyanate R¹⁰NCY dans un solvant à une température de 25°C à la température de reflux du solvant, pendant 5 minutes à 24 heures, pour former un composé de formule 3, dans lequel R⁸ représente (CH₂)ₙNR¹¹CYNHR¹⁰.

49. Procédé selon la revendication 11, dans lequel R¹ représente NO₂ et R, R³, R⁶, R⁷ et R⁸ sont tels que définis dans la revendication 34, dans lequel le composé de formule 3 dans lequel R¹ est NO₂ est réduit à l'aide de fer et d'acide acétique, de chlorure stanneux ou d'hydrogène et de palladium, en un composé de formule 3 dans lequel R¹ représente NH₂ et ce dernier est mis à réagir avec un anhydride d'acide approprié tel que l'anhydride phtalique ou un anhydride phtalique substitué dans un solvant ou avec un chlorure d'acide approprié tel qu'un chlorure d'acide anthranilique substitué en présence d'un alcali aqueux ou d'une base ou avec un acide phtalique ou anthranilique convenablement substitué en présence de dicyclohexylcarbodiimide dans un solvant, pour obtenir un composé de formule 3 dans lequel R¹ est: 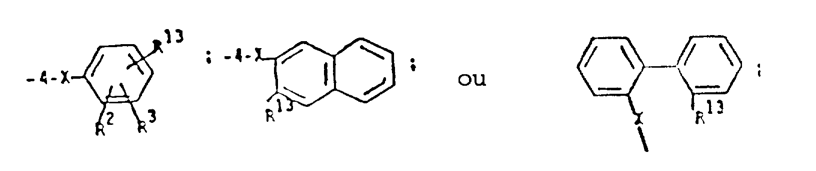 et X représente NHCO.

50. Procédé selon la revendication 11, dans lequel R¹ représente OCH₂C₆H₅, R et R³ sont des hydrogènes et R⁶, R⁷ et R⁸ sont tels que définis dans la revendication 11, et le composé de formule 3 avec R¹ égal à OCH₂C₆H₅ est mis en contact avec de l'acide trifluoroacétique à la température de reflux pendant une période de 0,2 à 1 heure ou en présence d'hydrogène et de palladium pour former le dérivé correspondant de formule 3 dans lequel R¹ représente OH et ce dernier est mis en contact avec une base à 25°C et un halogénure de benzyle convenable de formule: 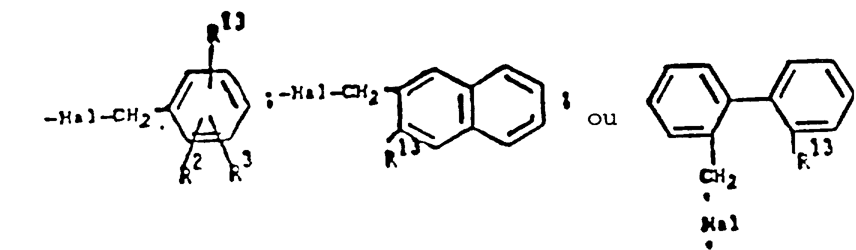 pour obtenir le dérivé correspondant de formule 3 dans lequel R¹ est: 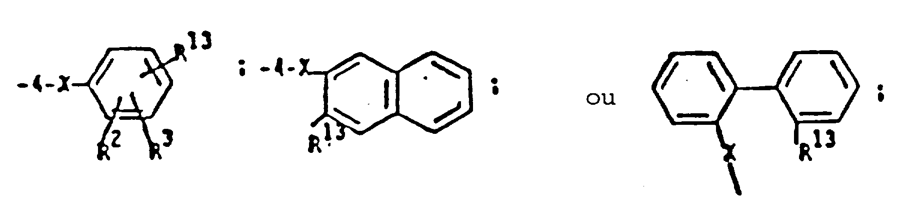 et X représente -OCH₂-.

51. Procédé selon la revendication 11, dans lequel R⁸ représente -CHO, dans lequel le dérivé benzylé de formule 2 se fixe au dérivé imidazolique de formule 1, préférentiellement sur l'atome d'azote adjacent à l'atome de carbone du cycle imidazole auquel R⁸ est fixé.

52. Un procédé de préparation d'un composé selon la revendication 1, dans lequel r représente O, qui comprend la mise en contact d'un dérivé d'imidazole de formule 1 ou un de ses sels métalliques avec le 4-fluoro-1-nitrobenzène dans un solvant, en présence d'une base si on utilise l'imidazole libre, pendant 1 à 10 jours, à une température de 25°C à 150°C, pour former une N-phénylimidazole, puis élaboration des composés où X=NHCO par le procédé décrit dans la revendication 49.

53. Un procédé selon la revendication 29, dans lequel R⁸ représente CHO, et X représente une liaison simple carbone-carbone, et dans lequel on met en contact le produit de formule 3 est mis en contact avec un réactif organométallique tel que R¹¹mgBr ou R¹¹Li en présence d'un solvant non hydroxylé anhydre tel que éther, THF ou diméthoxyéthane, à une température de -78°C à 25°C, suivi par un traitement aqueux, suivi par une hydrolyse acide de tout groupe CO₂R¹⁴ où R¹⁴ représente le t-butyle ou une hydrolyse de tout groupe tétrazole protégé par un trityle, pour former le composé correspondant de formule 3 où R⁸ représente 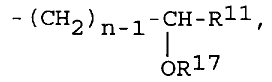 où R¹¹ est différent de H.

54. Un procédé de préparation d'un composé selon la revendication 1, dans lequel R⁷ représente un biphénylyle, phénoxyphényle ou hétéroaryle tel que défini dans la revendication 1, substitué ou non substitué, caractérisé en ce que l'on couple l'haloimidazole 237 avec un dérivé arylmétallique ArM, où M=ZnBr, Me₃Sn ou B(OH)₂, en présence d'un catalyseur à base de métal de transition tel que palladium, platine, nickel ou zirconium, pour former une arylimidazole 238: 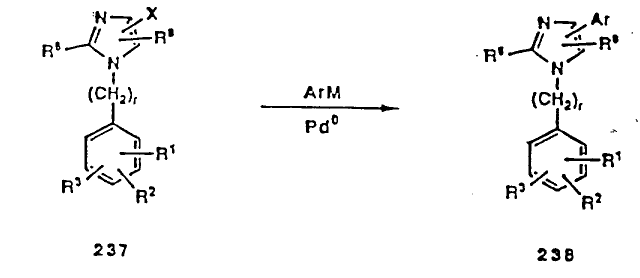 où R¹, R, R³, R⁶, R⁸ et r ont la signification donnée dans la revendication 1, X représente Br ou I, M représente ZnBr, Me₃Sn, B(OH)₂, et Ar est un biphénylyle, phénoxyphényle ou hétéroaryle tel que défini pour R⁷ dans la revendication 1, substitué ou non substitué.

55. Un procédé de préparation des composés selon la revendication 1, où R⁷= un biphénylméthyle, un phénoxyphénylméthyl ou un hétéroarylméthyle, tel que défini dans la revendication 1, substitué ou non substitué, caractérisé en ce que l'on couple un haloimidazole 237 avec un dérivé arylméthyle métallique ArCH₂M' en présence d'un catalyseur à base de métal de transition pour former un arylméthylimidazole 240: 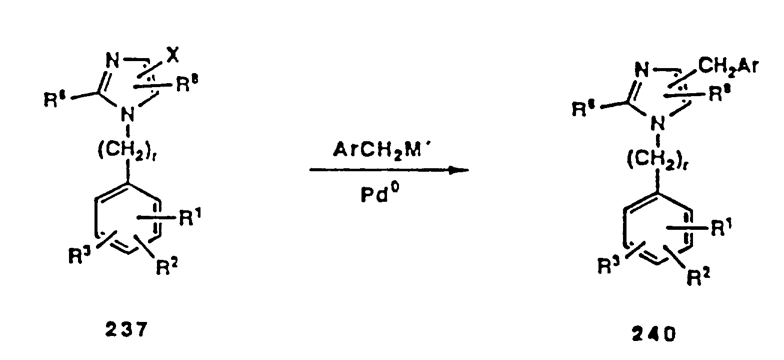 où R¹, R, R³, R⁶, R⁸ et r ont la signification donnée dans la revendication 1, X représente Br ou I, M' représente ZnBr, et Ar est un hétéroarylméthyle, biphénylméthyle, phénoxyphénylméthyle tel que défini pour R⁷ dans la revendication 1, substitué ou non substitué.

56. Un procédé de préparation des composés selon la revendication 1, où R⁷= vinyle, alkynyle, alkényle substitué ou alkynyle substitué, caractérisé en ce que un haloimidazole 237 est couplé avec un dérivé 1-alkénylmétal ou 1-alkynylmétal (AM), ou un 1-alkène, ou un 1-alkyne (1H) en présence d'un catalyseur à base de métal de transition pour former un 1-alkényl- ou 1-alkynylimidazole 241: 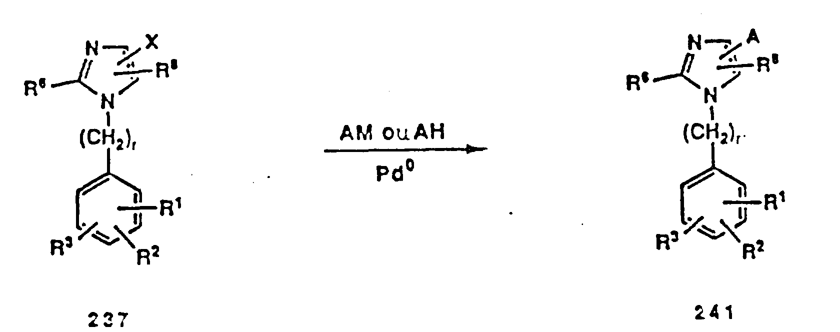 où R¹, R, R³, R⁶, R⁸ et r ont la signification donnée dans la revendication 1, X représente Br ou I, M est un métal, A est un vinyle, CH=CH(CH₂)ₓAr, C≡C(CH₂)_{y}CH₃, C≡C(CH₂)_{z}Ph', ou bien C≡C(CH₂)ₓAr, Ph' est un phényle ou un phényle substitué, et Ar est un biphénylyle substitué ou non substitué, phénoxyphényle ou hétéroaryle, tel que défini dans la revendication 1, x= 0 à 8, y= 0 à 7 et z= 0 à 4.

57. Un procédé de préparation d'un composé selon la revendication 1, où R⁷= vinyle ou alkényle substitué, caractérisé en ce que l'on fait réagir l'imidazol aldéhyde 253 avec le méthylène triphénylphosphorane ou un méthylène triphénylphosphorane substitué pour former un vinylimidazole ou un alkénylimidazole substitué 254: 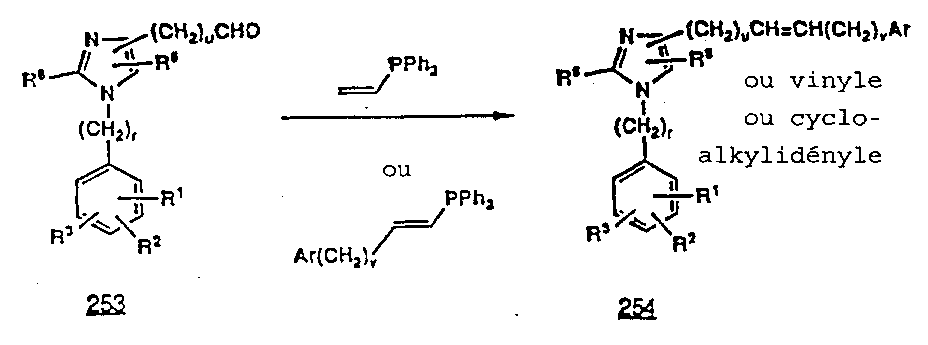 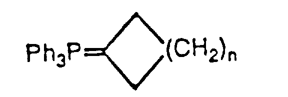 où n= 0 à 5 où R¹, R, R³, R⁶, R⁸ et r ont la signification donnée dans la revendication 1, Ar est un biphényle, phénoxyphényle ou hétéroaryle substitué ou non substitué tel que défini dans la revendication 1, et u+v = 0 à 8.

58. Un procédé de préparation d'un composé de formule I où R⁷ représente -S(O)ᵣ-hétéroaryle, -S(O)ᵣ-biphénylyle, -S(O)ᵣ-phénoxyphényle, -S-tétrazole, -S(O)ᵣ-R¹⁷, -NR¹⁸R¹⁹, -NR¹⁸-hétéroaryle, -NR¹⁸-phényle, -NR¹⁸-biphénylyle, -NR¹⁸-phénoxyphényle, -N-phtalimido, -NH-SO₂-phénoxyphényle, -NH-SO₂-hétéroaryle, -NH-SO₂-biphénylyle, -NH-SO₂-R¹⁷, et -S-(C=O)-R¹⁷, N-imidazolyle, N-1,2,3-triazolyle et N-1,2,4-triazolyle, dans lequel l'hétéroaryle est tel que défini dans la revendication 1, caractérisé en ce que ces composés imidazoliques 255 substitué par un groupe électro-attracteur E sont mis à réagir avec des nucloéphiles dans un solvant convenable à une température comprise entre la température ambiante et la température de reflux du solvant, pour donner une réaction de substitution aromatique dans laquelle le groupe partant X est remplacé par un nucléophile Nu tel que soufre ou azote, pour obtenir des composés de formule développée 256:

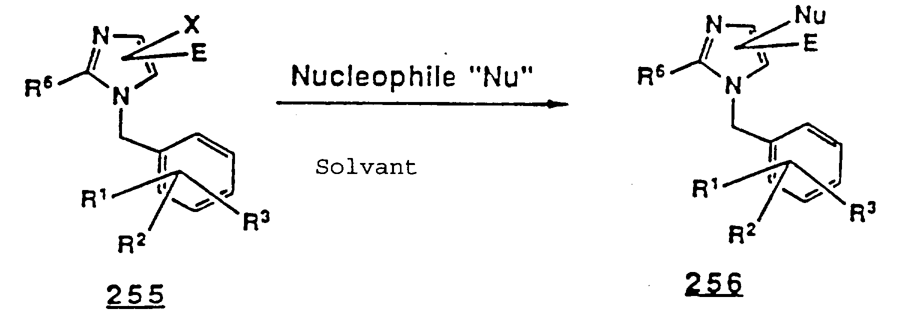

## Revendications (Revendications pour l'(les) Etat(s) contractant(s) suivant(s): ES)

1. Un procédé de préparation d'un dérivé anti-hypertensif répondant à la formule: 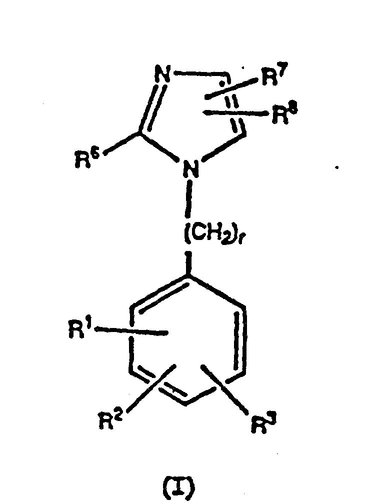 dans laquelle
R¹ est 4-CO₂H; 4-CO₂R⁹; 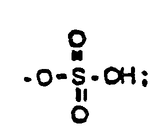 -SO₃H; -C(CF₃)₂OH; 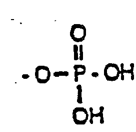 -PO₃H₂; 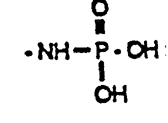 4-NHSO₂CH₃; 4-NHSO₂CF₃ ; -CONHOR^{12;} -SO₂NH₂; 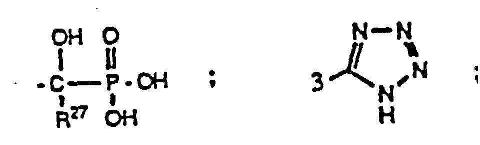 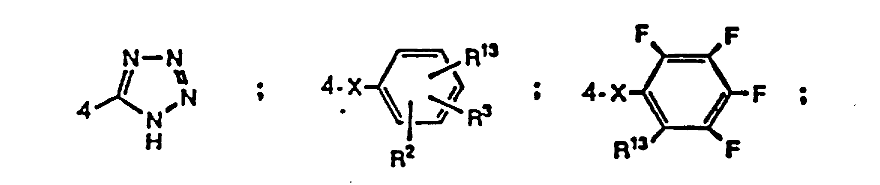 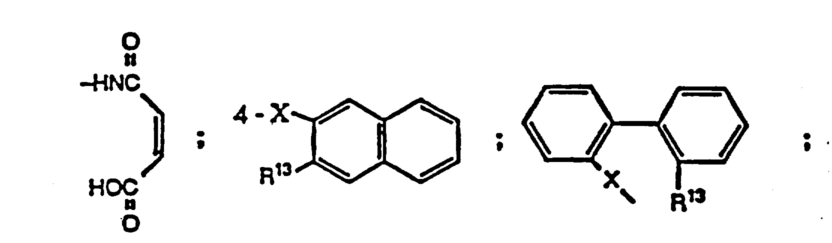 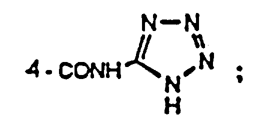 4-CONHNHSO₂CF₃; 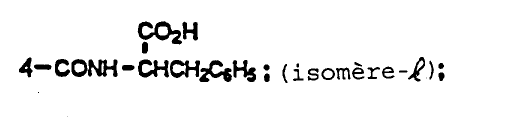 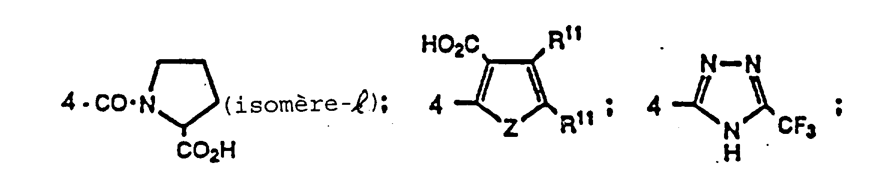 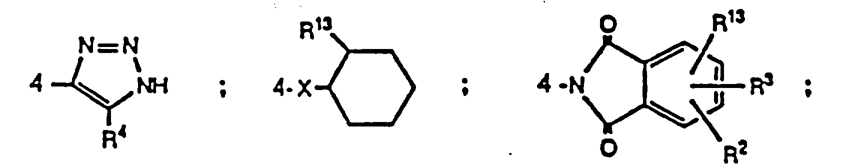 ou 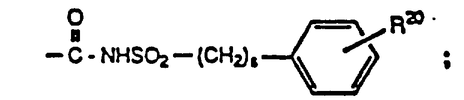
R est H; Cl; Br, I; F; NO₂; CN; un alkyle comportant 1 à 4 atomes de carbone; un acyloxy comportant 1 à 4 atomes de carbone; un alcoxy comportant 1 à 4 atomes de carbone; CO₂H; CO₂R⁹; NHSO₂CH₃; NHSO₂CF₃; CONHOR¹;
SO₂NH₂; 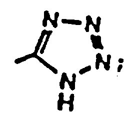 phényle; ou furyle;
R³ est H; Cl, Br, I ou F; un alkyle comportant 1 à 4 atomes de carbone ou un alcoxy comportant 1 à 4 atomes de carbone;
R⁴ est CN, NO₂ ou CO₂R¹¹;
R⁵ est H, un alkyle comportant 1 à 6 atomes de carbone, un cycloalkyle comportant 3 à 6 atomes de carbone, un alkényle ou un alkynyle comportant 2 à 4 atomes de carbone;
R⁶ est un alkyle comportant 2 à 10 atomes de carbone, un alkényle ou un alkynyle comportant 3 à 10 atomes de carbone ou ces mêmes groupes substitués par F ou CO₂R¹⁴; un cycloalkyle comportant 3 à 8 atomes de carbone, cycloalkylalkyle comportant 4 à 10 atomes de carbone; un cycloalkylalkényle ou un cycloalkylalkynyle comportant 5 à 10 atomes de carbone; (CH₂)ₛZ(CH₂)ₘR⁵ éventuellement substitué par F ou CO₂R¹⁴; un benzyle ou un benzyle substitué sur le noyau phényle par 1 ou 2 halogènes, un alcoxy comportant 1 à 4 atomes de carbone, un alkyle comportant 1 à 4 atomes de carbone ou un groupe nitro;
R⁷ est un vinyle; cycloalkylidényle; alkynyle comportant 2 à 10 atomes de carbone; phénylalkynyle dont la partie alkynyle comporte 2 à 6 atomes de carbone; hétéroaryle choisi parmi 2- et 3-thiényle, 2- et 3-furyle, 2-, 3- et 4-pyridyle, 2-pyrazinyle, 2-, 4- et 5-pyrimidinyle, 3- et 4-pyridazinyle, 2-, 4- et 5-thiazolyle, 2-, 4- et 5-sélénazolyle, et 2- et 4- et 5-oxazolyle, 2- ou 3-pyrrolyle, 3-, 4- ou 5-pyrazolyle, 2-, 4- ou 5-imidazolyle; o-, m- ou p-biphénylyle; o-, m- ou p-phénoxyphényle; 2-oxazolinyle; 2-thiazolinyle; phénylalkynyle substitué, hétéroaryle, biphénylyle ou phénoxyphényle tel que défini ci-dessus substitué par un ou deux substituants choisis parmi halogène, hydroxy, mercapto, alcoxy comportant 1 à 5 atomes de carbone, alkyle comportant 1 à 5 atomes de carbone, -NO₂, -CN, -CF₃, -COR¹⁶, -CH₂OR¹⁷, -NHCOR¹⁷, -CONR¹⁸R¹⁹, S(O)ᵣR¹⁷ et SO₂NR¹⁸R¹⁹; pyrrolyle, pyrazolyle ou imidazolyle tels que définis ci-dessus, substitués sur l'azote du cycle par un alkyle comportant 1 à 5 atomes de carbone, phényle ou benzyle; ou alkyle substitué, alkényle, ou alkynyle comportant 1 à 10 atomes de carbone substitué par un groupe hétéroaryle, biphénylyle ou phénoxyphényle substitué ou non substitué tel que défini ci-dessus; -S(O)ᵣ-hétéroaryle, -S(O)ᵣ-biphénylyle, -S(O)ᵣ-phénoxyphényle, -S-tétrazole, -S(O)ᵣR¹⁷, -NR¹⁸R¹⁹, -NR¹⁸-hétéroaryle, -NR¹⁸-phényle, -NR¹⁸-biphénylyle, -NR¹⁸-phénoxyphényle, -N-phtalimido, -NH-SO₂-phénoxyphényle, -NH-SO₂-hétéroaryle, -NH-SO₂-biphéniphée, -NH-SO₂-R¹⁷, et -S-(C=O)-R¹⁷, N-imidazolyle, N-1,2,3-triazolyle, N-1,2,4-triazolyle, où l'hétéroaryle est un hétérocycle défini à propos de R⁷ et où le groupe phényle dans R¹⁷ de -S-(O)ᵣR¹⁷, le N-imidazolyle, le N-1,2,3-triazolyle et le N-1,2,4-triazolyle peuvent être substitués par un ou deux substituants tels que décrits ci-dessus pour l'hétéroaryle:
R⁸ est 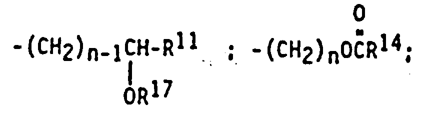 -(CH₂)ₙSR¹⁵_{;} 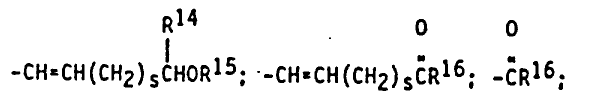 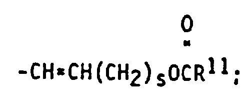 -(CH₂)ₘ-tetrazolyl; 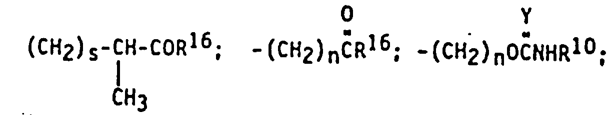 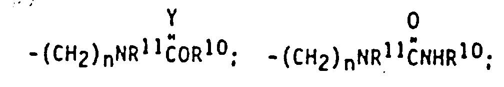 -(CH₂)ₙNR¹¹SO₂R¹⁰; 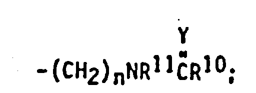
R⁹ est 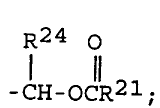
R¹⁰ est un radical alkyle comportant de 1 à 6 atomes de carbone ou perfluoroalkyle comportant 1 à 6 atomes de carbone, 1-adamantyle, 1-naphtyle, 1-(1-naphtyl)-éthyle, ou (CH₂)ₚC₆H₅;
R¹¹ est H, un alkyle comportant 1 à 6 atomes de carbone, cycloalkyle comportant 3 à 6 atomes de carbone, phényle ou benzyle;
R¹ est H, méthyle ou benzyle;
R¹³ est -CO₂H; -CO₂R⁹; -CH₂CO₂H; -CH₂CO₂R⁹; ou 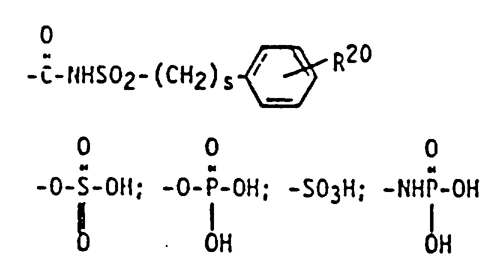 -PO₃H₂; -C(CF₃)₂OH; -NHSO₂CH₃; -NHSO₂CF₃; -NHCOCF₃; -CONHOR¹; -SO₂NH₂; 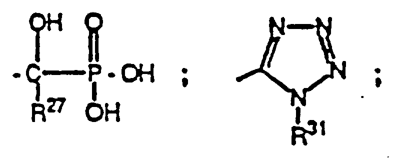 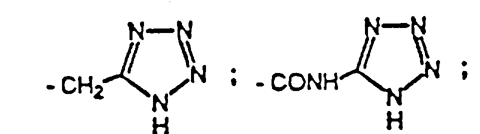 -CONHNHSO₂CF₃ ; 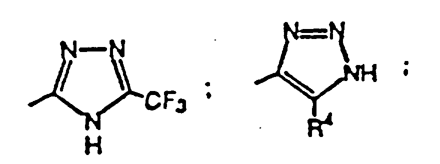
R¹⁴ est un hydrogène, un alkyle ou un perfluoroalkyle comportant 1 à 8 atomes de carbone, cycloalkyle comportant 3 à 6 atomes de carbone, phényle ou benzyle;
R¹⁵ est un hydrogène, un alkyle comportant 1 à 6 atomes de carbone, cycloalkyle comportant 3 à 6 atomes de carbone, phényle, benzyle, acyle comportant 1 à 4 atomes de carbone; phénacyle;
R¹⁶ est un hydrogène, un alkyle comportant 1 à 6 atomes de carbone, cycloalkyle comportant 3 à 6 atomes de carbone; (CH₂)ₚC₆H₅, OR¹⁷, ou bien NR¹⁸R¹⁹;
R¹⁷ est un hydrogène, un alkyle comportant 1 à 6 atomes de carbone, cycloalkyle comportant 3 à 6 atomes de carbone, phényle ou benzyle;
R¹⁸ et R¹⁹ représentent, indépendamment l'un de l'autre, un hydrogène, un alkyle comportant 1 à 4 atomes de carbone, phényle, benzyle, α-méthylbenzyle, ou pris ensemble avec l'atome d'azote, forment un cycle de formule: 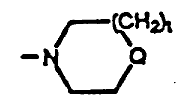
Q est NR⁰, O ou CH₂;
R⁰ est un hydrogène, un alkyle comportant 1 à 4 atomes de carbone, ou phényle;
R¹ est un alkyle comportant 1 à 6 atomes de carbone, -NRR³, ou 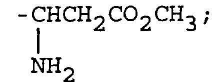
R et R³ représentent, indépendamment l'un de l'autre, un hydrogène, un alkyle comportant 1 à 6 atomes de carbone, un benzyle, ou forment ensemble (CH₂)ᵤ où u est compris entre 3 et 6;
R⁴ est H, CH₃ ou -C₆H₅;
R⁵ est NR⁷R⁸, OR⁸, NHCONH₂, NHCSNH₂, 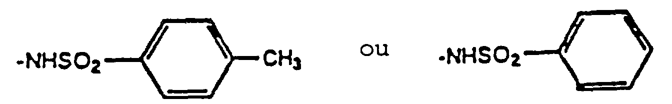
R⁶ est un hydrogène, un alkyle comportant 1 à 6 atomes de carbone, benzyle ou allyle;
R⁷ et R⁸ sont, indépendamment l'un de l'autre, un hydrogène, un alkyle comportant 1 à 5 atomes de carbone, ou phényle;
R⁹ et R³⁰ sont, indépendamment l'un de l'autre, un alkyle comportant 1 à 4 atomes de carbone, ou pris ensemble, forment -(CH₂)_{q}-;
R³¹ est H, un alkyle comportant 1 à 4 atomes de carbone, -CH₂CH=CH₂ ou -CH₂C₆H₄R³;
R³ est H, NO₂, NH₂, OH ou OCH₃;
X est une liaison simple carbone-carbone,-CO-, -CH₂-, -O-, -S-, -NH-, 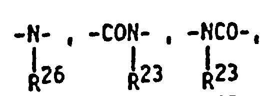 -OCH₂-, -CH₂O-, -SCH₂-, -CH₂S-, -NHC(R⁷)(R⁸)-, -NR³SO₂-, -SO₂NR³-, -C(R⁷)(R⁸)NH-, -CH=CH-, -CF=CF-, -CH=CF-, -CF=CH-, -CH₂CH₂-, -CF₂CF₂-, 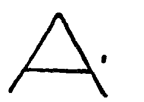 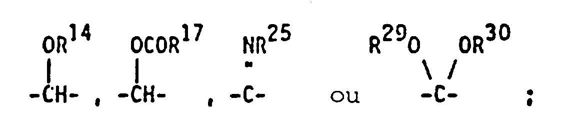
Y est O ou S;
Z est O, NR¹¹, ou S;
m vaut de 1 à 5;
n vaut de 1 à 10;
p vaut de 0 à 3;
q vaut de 2 à 3;
r vaut de 0 à 2;
s vaut de 0 à 5;
t vaut 0 ou 1;
ou un de leurs sels pharmaceutiquement acceptables; avec cette condition que:
(1) le groupe R¹ n'est pas en position ortho;
(2) lorsque 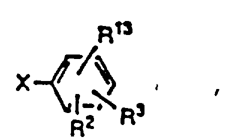 X est une simple liaison, et R¹³ est CO₂H, ou 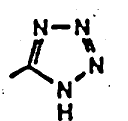 et R¹³ doit être en position ortho ou méta; ou si R¹ et X sont tels que définis ci-dessus et R¹³ est NHSO₂CF₃ ou NHSO₂CH₃, R¹³ doit être en position ortho;
(3) si R¹ est 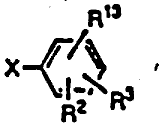 et X est autre qu'une simple liaison,
alors,
R¹³ doit être en position ortho, sauf si X = NR³CO et R¹³ est NHSO₂CF₃ ou NHSO₂CH₃, alors R¹³ doit être en position ortho ou méta;
(4) si R¹ est 4-CO₂H ou un de ses sels, R⁶ ne peut pas être S-alkyle;
(5) si R⁵ est 4-CO₂H ou un de ses sels, le substituant en position-4 de l'imidazole ne peut pas être CH₂OH, CH₂OCOCH₃, ou CH₂CO₂H;
(6) si R¹ est 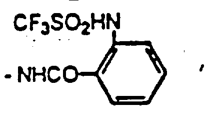 R⁶ n'est pas un méthoxybenzyle;
(7) le groupe R⁶ n'est pas 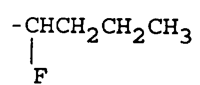 ou CH₂OH;
ledit procédé comprend:
A) pour la préparation d'un composé dans lequel r est 1: la mise en contact d'un dérivé d'imidazole de formule 1 avec un dérivé benzylique de formule 2 dans un solvant, en présence d'une base, pendant 1 à 10 heures, à une température comprise entre 20°C et la température de reflux du solvant, pour obtenir un benzylimidazole de formule 3: 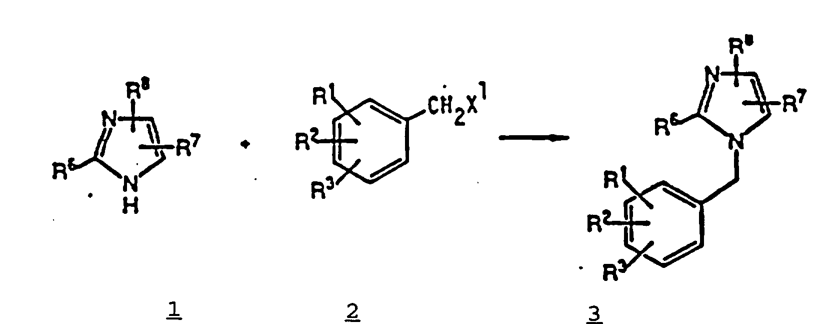 dans lesquelles R¹, R, R³, R⁶, R⁷ et R⁸ sont tous stables dans les conditions réactionnelles et sont des groupes tels que définis ci-dessus ou des formes intermédiaires ou protégées de ceux-ci qui peuvent être transformées en de tels groupes; et dans lesquelles X¹ est un halogène, un groupe paratoluènesulfonyloxy ou méthylsulfonyloxy;
et ensuite, si nécessaire, la transformation desdites formes intermédiaires ou protégées des groupes R en groupes R tels que définis ci-dessus;
B) pour la préparation d'un composé dans lequel r est 0: la mise en contact d'un dérivé d'imidazole de formule 1 ou d'un de ses sels métalliques avec le 4-fluoro-1-nitrobenzène dans un solvant, en présence d'une base, si on utilise de l'imidazole libre, pendant 1 à 10 jours, à une température de 25 à 150°C, pour obtenir un N-phénylimidazole;
C) pour la préparation d'un composé dans lequel R⁷ = biphénylyle, phénoxyphényle, ou hétéroaryle substitué ou non substitué, tel que défini ci-dessus: le couplage d'un haloimidazole 237 avec un dérivé arylmétallique ArM, où M = ZnBr, Me₃Sn, ou B(OH)₂, en présence d'un catalyseur à base de métal de transition tel que palladium, platine, nickel, ou zirconium, pour obtenir un arylimidazole 238: 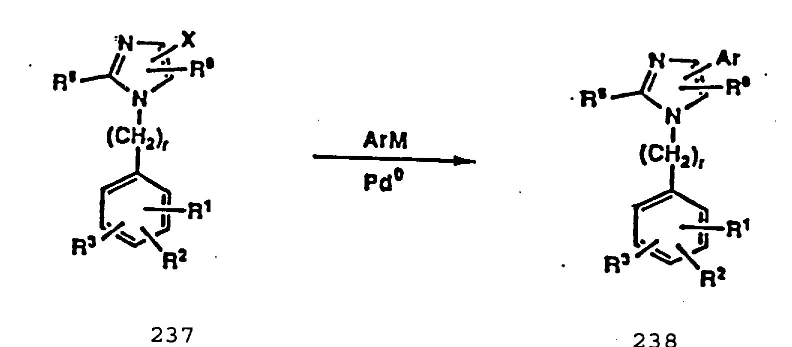
où R¹, R, R³, R⁶, R⁸ et r ont les significations données ci-dessus,
X est Br ou I,
M est ZnBr, Me₃Sn, B(OH)₂, et
Ar est un radical biphénylyle, phénoxyphényle, ou hétéroaryle substitué ou non substitué, tel que défini ci-dessus pour R⁷;
D) pour la préparation d'un dérivé où R⁷ = biphénylméthyle, phénoxyphénylméthyle, ou hétéroarylméthyle, substitué ou non substitué, tel que défini ci-dessus: le couplage d'un haloimididazole 237 avec un dérivé arylméthylmétallique ArCH₂M' en présence d'un catalyseur à base de métal de transition pour obtenir un arylméthylimidazole 240: 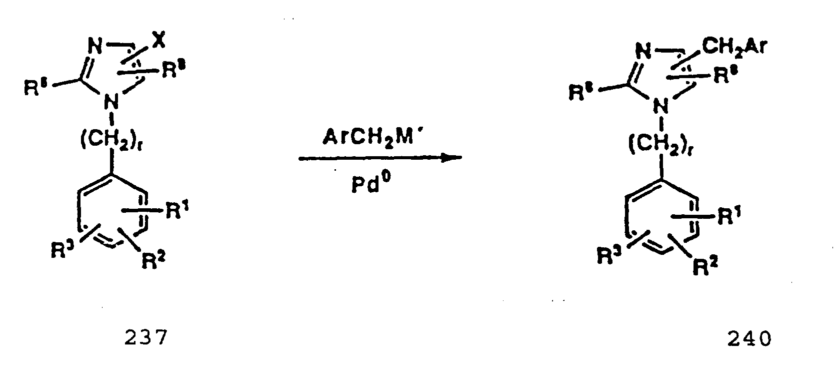
où
R¹, R, R³, R⁶, R⁸ et r ont les significations données ci-dessus,
X est Br ou I,
M' est ZnBr, et
Ar est un radical hétéroarylméthyle, biphénylméthyle, ou phénoxyphénylméthyle, substitué ou non substitué, tel que défini pour R⁷ ci-dessus;
E) pour la préparation d'un dérivé où R⁷ = vinyle, alkynyle, alkényle substitué ou alkynyle substitué: couplage d'un haloimidazole 237 avec un dérivé 1-alkénylmétal ou 1-alkynylmétal (AM), ou un 1-alcène, ou un 1-alcyne (AH), en présence d'un catalyseur à base de métal de transition pour obtenir un 1-alkényl- ou 1-alkynylimidazole 241: 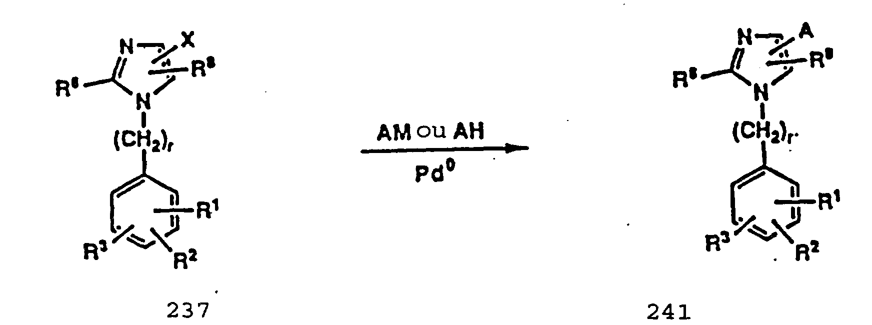 où
R¹, R, R³, R⁶, R⁸ et r ont les significations indiquées ci-dessus,
X est Br ou I,
M est un métal,
A est un groupe vinyle, CH=CH(CH₂)ₓAr, C≡C(CH₂)_{y}CH₃, C≡C(CH₂)_{z}Ph', ou C≡C(CH₂)ₓAr,
Ph' est un phényle, ou un phényle substitué, et
Ar est un biphénylyle, phénoxyphényle ou hétéroaryle, substitué ou non substitué, tel que défini ci-dessus,
X est égal à 0 à 8,
Y est égal à 0 à 7, et
Z est égal à 0 à 4;
F) pour la préparation d'un composé où R⁷ = vinyle ou alkényle substitué: la réaction d'un imidazole aldéhyde 253 avec le méthylènetriphénylphosphorane ou un méthylènetriphénylphosphorane substitué pour obtenir un vinylimidazole ou un alkénylimidazole substitué 254: 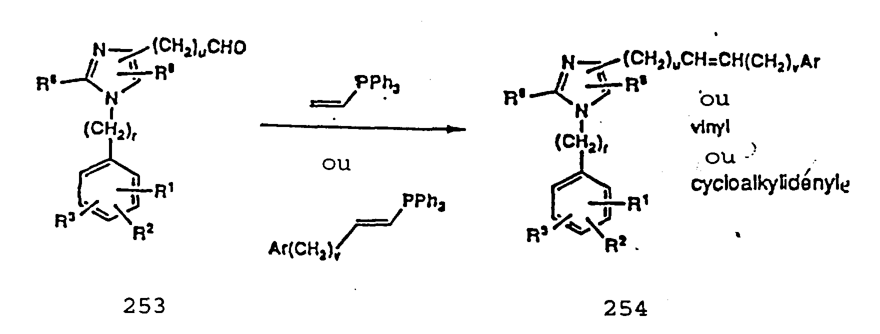 ou 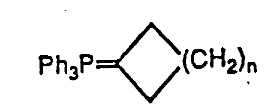 où n=0 à 5 où
R¹, R, R³, R⁶, R⁸ et r ont les significations données ci-dessus,
Ar est un groupe biphénylyle, phénoxyphényle ou hétéroaryle tel que défini ci-dessus, substitué ou non substitué, et
u+v = 0 à 8; et
G) pour la préparation d'un composé de formule I, où R⁷ est un groupe -S(O)ᵣ-hétéroaryle, -S(O)ᵣ-biphénylyle, -S(O)ᵣ-phénoxyphényle, -S-tétrazole, -S(O)ᵣR¹⁷, -NR¹⁸R¹⁹, -NR¹⁸-hétéroaryle, -NR¹⁸-phényle, -NR¹⁸-biphénylyle, -NR¹⁸-hétéroaryle, -NR¹⁸-phényle, -NR¹⁸-biphénylyle, -NR¹⁸-phénoxyphényle, -N-phtalimido, -NH-SO₂-phénoxyphényle, -NH-SO₂-hétéroaryle, -NH-SO₂-biphénylyle, -NH-SO₂-R¹⁷, et -S-(C=O)-R¹⁷, N-imidazolyle, N-1,2,3-triazolyle et N-1,2,4-triazolyle, où l'hétéroaryle est tel que défini ci-dessus: réaction des dérivés contenant un imidazole 255 substitués par un groupe électro-attracteur E avec des nucléophiles dans un solvant convenable à une température comprise entre la température ambiante et la température de reflux du solvant pour effectuer une réaction de substitution aromatique dans laquelle le groupe partant X est remplacé par un nucléophile Nu tel que soufre ou azote, pour obtenir des composés de structure 256:

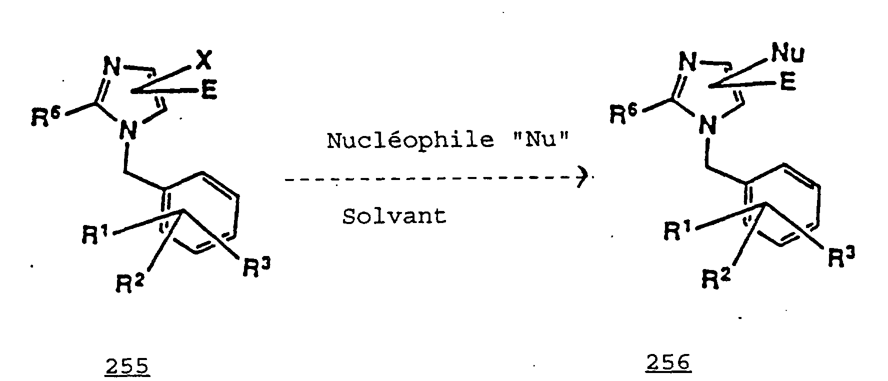

2. Procédé selon la revendication 1, dans lequel, dans A), on met en contact les composés 1 et 2 en présence d'une base choisie dans le groupe consistant en hydrure métallique (MH), alcoxyde métallique (MOR), carbonate de sodium, carbonate de potassium, triéthylamine et pyridine, dans un solvant aprotique dipolaire ou, si la base est MOR, le solvant peut être un alcool, ROH, avec M étant lithium, sodium ou potassium et R étant un méthyle, éthyle ou t-butyle.

3. Procédé selon la revendication 1, dans lequel, dans A), on utilise un système de solvants à deux phases, l'une étant une phase organique telle que chlorure de méthylène et l'autre une phase aqueuse, en présence d'un catalyseur de transfert de phase tel que le chlorure de tricaprylméthylammonium.

4. Procédé selon la revendication 2, dans lequel: est
R¹ est 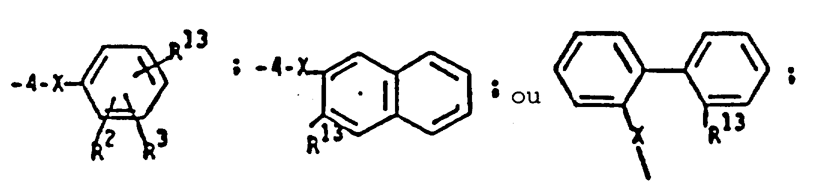
X est une liaison simple carbone-carbone, -CO-, -O-, -S-, ou -NH-;
R et R³, représentent, indépendamment l'un de l'autre, H, Cl, Br, I, CO₂R¹⁴, F, NO₂, un alkyle comportant 1 à 4 atomes de carbone, alcoxy comportant 1 à 4 atomes de carbone, phényle, ou furyle;
R⁶ et R⁷ sont tels que définis dans la revendication 1;
R⁸ représente -(CH₂)ₙOR¹¹; -(CH₂)ₙSR¹⁵; ou -(CH₂)ₙCN;
R¹¹ est tel que défini dans la revendication 1;
R¹³ représente CO₂R¹⁴, CN, NO₂, trialkylétain tétrazole, ou trityltétrazole; et
R¹⁴ et R¹⁵ sont tels que définis dans la revendication 1.

5. Procédé selon la revendication 4, dans lequel R¹³ représente -CO₂R¹⁴ et le produit de formule 3 est mis en contact avec un alcali dans un solvant hydroalcoolique ou avec CF₃CO₂H à une température comprise entre 20°C et la température de reflux du solvant pendant 1 à 24 heures, suivi par l'ajustement du pH du mélange à une valeur comprise entre 3 et 7, pour transformer le produit en le produit correspondant dans lequel R¹³ représente -CO₂H.

6. Procédé selon la revendication 5, dans lequel au moins l'un des groupes R, R³ ou R¹³ de la formule 1 représente -CO₂R¹⁴ et est transformé en -CO₂H.

7. Procédé selon la revendication 5, dans lequel R¹⁴ représente du t-butyle et la réaction est effectuée dans CF₃CO₂H.

8. Procédé selon la revendication 4, dans lequel R¹³ représente -CN et le produit de formule 3 est mis en contact avec:
(i) un acide fort à la température de reflux du solvant pendant 2 à 96 heures, ou
(ii) un alcali fort dans un solvant alcoolique à une température comprise entre 20°C et la température de reflux du solvant pendant 2 à 96 heures, suivi par l'ajustement du pH entre 3 et 7, ou
(iii) de l'acide sulfurique, suivi par un acide ou un alcali, pour transformer le produit en le composé correspondant dans lequel R¹³ représente -CO₂H.

9. Procédé selon la revendication 8, dans lequel au moins l'un des groupes R, R³ ou R¹³ représente -CO₂R¹⁴ et est transformé en -CO₂H.

10. Procédé selon la revendication 8, dans lequel R⁸ représente -(CH₂)ₙCN et est transformé en -(CH₂)ₙCO₂H, ou représente -(CH₂)ₙOR¹¹ et est transformé en (CH₂)ₙOH quand R¹³ est transformé en -CO₂H.

11. Procédé selon la revendication 4, dans lequel R¹³ représente -CN et le produit de formule 3 est mis en contact avec un mélange de quantités équimolaires d'azidure de sodium et de chlorure d'ammonium dans un solvant aprotique polaire à une température comprise entre 30°C et la température de reflux du solvant, pendant 1 heure à 10 jours, pour transformer le produit en le composé correspondant dans lequel R¹³ représente le 5-tétrazolyle.

12. Procédé selon la revendication 11, dans lequel R⁸ représente -(CH₂)ₘCN et est transformé en -(CH₂)ₘ-tétrazolyle, lorsque R¹³ est transformé en 5-tétrazolyle.

13. Procédé selon la revendication 4, dans lequel R¹³ représente -CN et on fait réagir le produit de formule 3 avec un azidure de trialkylétain ou un azidure de triarylétain, puis on effectue l'hydrolyse en milieu acide ou basique pour transformer le produit en le composé correspondant dans lequel R¹³ représente le 5-tétrazolyle.

14. Procédé selon la revendication 4, dans lequel R¹³ représente -CN et dans lequel on fait réagir le produit de formule 3 avec un azidure de trialkylétain ou un azidure de triarylétain pour obtenir un composé de formule 3 dans lequel R¹³ représente un trialkyl ou triarylstannyltétrazol-5-yle, ce dernier composé réagissant avec le chlorure de triphénylméthyle pour former un composé de formule 3 où R¹³ représente le triphénylméthyltétrazol-5-yle et on hydrolyse ce dernier composé pour obtenir un composé de formule 3 dans lequel R¹³ représente le 5-tétrazolyle.

15. Procédé selon la revendication 3, dans lequel R⁸ représente -(CH₂)ₙCN et est transformé en -(CH₂)ₘ-tétrazolyle, quand R¹³ est transformé en 5-tétrazolyle.

16. Procédé selon la revendication 4, dans lequel R¹³ représente -NO₂ et on met le produit de formule 3 en contact avec un agent réducteur pour former un second intermédiaire de formule 3 dans lequel R¹³ représente -NH₂ et ce dernier est mis en contact avec un anhydride (CH₃SO₂)₂O ou (CF₃SO₂)₂O ou un chlorure CH₃SO₂Cl ou CF₃SO₂Cl d'un acide sulfonique dans un solvant pour former un composé dans lequel R¹³ représente -NHSO₂CH₃ ou -NHSO₂CF₃.

17. Procédé selon la revendication 16, dans lequel au moins l'un des groupes R, R³ ou R¹³ est -NO₂ et est transformé en -NHSO₂CH₃ ou -NHSO₂CF₃.

18. Procédé selon la revendication 5 ou 8, dans lequel le composé de formule 3 avec R¹³=CO₂H est soit:
(a) mis en contact avec environ 1 à 4 équivalents de chlorure de thionyle dans un excès de chlorure de thionyle ou un autre solvant à une température comprise entre 20°C et la température de reflux du solvant pendant 5 minutes à 2 heures pour donner un intermédiaire de formule 3 dans lequel R¹³ est COCl, et ce dernier est mis en contact avec 2 à 10 équivalents de dérivé d'hydroxylamine H₂NOR¹ dans un excès d'hydroxylamine H₂NOR¹ ou un autre solvant, à une température de 25 à 80°C, pendant 2 à 18 heures, soit
(b) mis en contact avec un dérivé d'hydroxylamine H₂NOR¹, un dicyclohexylcarbodiimide et un 1-hydroxybenzotriazole, dans un solvant à une température comprise entre 0 et 30°C, pendant 1 à 24 heures; pour former un composé dans lequel R¹³ est CONHOR¹.

19. Procédé selon la revendication 1, dans lequel, dans A):
R¹ représente: 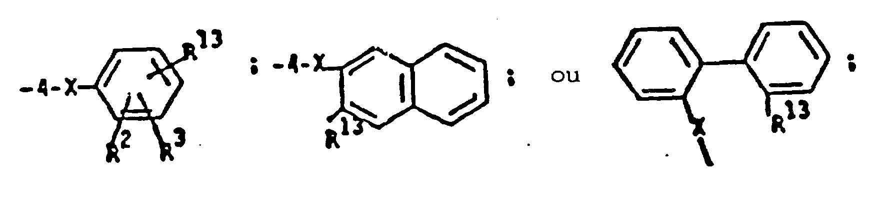
X représente une liaison simple carbone-carbone, -CO-, -O-, -S- ou -NH-;
R, R³, R⁶ et R⁷ sont tels que définis dans la revendication 1; et
R⁸ représente -(CH₂)ₙOR¹¹, -(CH₂)ₙOCOR¹⁴, (CH₂)ₙCH(OH)R¹⁶, (CH₂)ₙCOR¹⁶, (CH₂)ₙCl, (CH₂)ₙCN, CHO.

20. Procédé selon la revendication 19, dans lequel R⁸ représente (CH₂)ₙOH et le produit de formule 3 est mis en contact avec un alcool R¹¹OH à l'état anhydre en présence d'un acide fort ou d'un acide de Lewis, suivi d'une saponification de tout groupe CO₂R¹⁴ formé en même temps ou présent dans l'intermédiaire 3, pour obtenir le dérivé correspondant de formule 3 dans lequel R⁸ représente (CH₂)ₙOR¹¹ et R¹¹ n'est pas H.

21. Procédé selon la revendication 19, dans lequel R⁸ représente (CH₂)ₙOR¹¹, et R¹¹ n'est pas H et le produit de formule 3 est mis en contact avec un milieu acide aqueux à une température comprise entre 25°C et la température de reflux du solvant pendant une période de 0,5 à 24 heures pour former le composé correspondant de formule 3 dans lequel R⁸ représente (CH₂)ₙOH.

22. Procédé selon la revendication 19, dans lequel R⁸ représente (CH₂)ₙOH et le produit de formule 3 est mis en contact avec:
(a) un anhydride d'acide carboxylique (R¹⁴CO)₂O ou un chlorure d'acide carboxylique R¹⁴COCl dans un solvant en présence d'une base à une température comprise entre 0°C et la température de reflux du solvant pendant 0,5 à 24 heures, ou
(b) un acide carboxylique R¹⁴CO₂ dans des conditions anhydres, en présence d'un acide fort ou d'un acide de Lewis, à une température de 0°C à 100°C pendant une période de 0,5 à 24 heures, pour obtenir le composé correspondant dans lequel R⁸ représente (CH₂)ₙOCOR¹⁴.

23. Procédé selon la revendication 19, dans lequel R⁸ représente (CH₂)ₙOCOR¹⁴ et le produit de formule 3 est mis en contact avec un acide ou un alcali aqueux pour former le composé correspondant dans lequel R⁸ représente (CH₂)ₙOH.

24. Procédé selon la revendication 19, dans lequel R⁸ représente (CH₂)ₙOH et le produit de formule 3 est mis en contact avec un agent d'oxydation à une température de 25°C à 45°C pendant 1 à 200 heures pour donner un composé correspondant de formule 3 dans lequel R⁸ représente (CH₂)ₙ₋₁COR¹⁶ et R¹⁶ représente H.

25. Procédé selon la revendication 19, dans lequel R⁸ représente (CH₂)ₙCOR¹⁶ et R¹⁶ représente H et le produit de formule 3 est mis en contact avec un dérivé organométallique R¹⁶P dans lequel P représente MgBr ou Li dans un solvant à une température de -78°C à 100°C pendant 0,5 à 24 heures, pour former un composé de formule 3 dans lequel R⁸ représente (CH₂)ₙCH(OH)R¹⁶ et R¹⁶ ne représente pas H.

26. Procédé selon la revendication 19, dans lequel R⁸ représente (CH₂)ₙCH(OH)R¹⁶ et R¹⁶ ne représente pas H, et le produit de formule 3 est mis en contact avec un agent d'oxydation dans un solvant pour former un composé correspondant de formule 3 dans lequel R⁸ représente (CH₂)ₙCOR¹⁶ et R¹⁶ ne représente pas H.

27. Procédé selon la revendication 19, dans lequel R⁸ représente (CH₂)ₙCOR¹⁶ et R¹⁶ représente H, et le composé de formule 3 est mis en contact avec un agent d'oxydation dans un solvant pour former un composé correspondant de formule 3 dans lequel R⁸ représente (CH₂)ₙCOR¹⁶ et R¹⁶ représente OH.

28. Procédé selon la revendication 19, dans lequel R⁸ représente (CH₂)ₙCOR¹⁶ et R¹⁶ représente OH, et le composé de formule 3 est mis en contact avec du chlorure de thionyle en excès ou dans un autre solvant à une température comprise entre 0°C et la température de reflux du solvant pendant 5 minutes à 24 heures pour former un dérivé correspondant de formule 3 dans lequel R⁸ représente (CH₂)ₙCOCl, suivi de la mise en contact de ce dernier avec une amine NHR¹⁸R¹⁹ en excès ou dans un solvant à une température comprise entre 0°C et la température de reflux du solvant, pendant 5 minutes à 24 heures, pour former un composé correspondant de formule 3 dans lequel R⁸ représente (CH₂)ₙCONR¹⁸R¹⁹.

29. Procédé selon la revendication 19, dans lequel R⁸ représente (CH₂)ₙOR¹¹ et R¹¹ est H et le produit de formule 3 est mis en contact avec du chlorure de thionyle en excès ou dans un solvant à une température comprise entre 20°C et la température de reflux du solvant pendant 0,5 à 24 heures pour former un composé intermédiaire de formule 3 dans lequel R⁸ représente (CH₂)ₙCl.

30. Procédé selon la revendication 29, dans lequel le composé de formule 3 dans lequel R⁸ représente (CH₂)ₙCl est mis en contact avec un sel de sodium ou de potassium d'un mercaptan R¹⁵SH dans un solvant à une température de 25°C à 100°C pendant 1 à 24 heures pour obtenir un composé de formule 3 dans lequel R⁸ représente (CH₂)ₙSR¹⁵.

31. Procédé selon la revendication 19, dans lequel le composé de formule 3 dans lequel R⁸ représente (CH₂)ₙCl est mis en contact avec un cyanure de métal alcalin dans un solvant à une température de 20°C à 100°C, pendant 1 à 24 heures, pour obtenir un composé de formule 3 dans lequel R⁸ représente (CH₂)ₙCN et ce dernier composé est hydrolysé en le composé correspondant de formule 3 dans lequel R⁸ représente (CH₂)ₙCOR¹⁶ et R¹⁶ représente OH.

32. Procédé selon la revendication 19, dans lequel le composé de formule 3 dans lequel R⁸ représente (CH₂)ₙ₋₁Cl est mis en contact avec un sel de sodium ou de potassium d'un malonate de dialkyle dans un solvant, à une température de 20°C à 100°C, pendant 0,5 à 24 heures, pour former un composé de formule 3 dans lequel R⁸ représente (CH₂)ₙ₋₁CH(CO₂alkyle)₂, suivi par la saponification de ce dernier à l'aide d'un alcali aqueux à une température comprise entre 25°C et la température de reflux du solvant, suivie par une acidification à l'aide d'un acide minéral pour former un composé de formule 3 dans lequel R⁸ représente (CH₂)ₙ₋₁CH(CO₂H)₂ suivie du chauffage de ce dernier à 120°C ou dans un acide minéral dilué à la température de reflux pour former un produit de formule 3 dans lequel R⁸ représente (CH₂)ₙCOR¹⁶ et R¹⁶ représente OH.

33. Procédé selon la revendication 19, dans lequel R⁸ représente -CHO et le dérivé de formule 3 est mis en contact avec un méthylène phosphorane (C₆H₅)₃P=CH(CH₂)ₛCHR¹⁴OR¹⁵ ou (C₆H₅)₃P=CH(CH₂)ₛCOR¹⁶ dans un solvant à une température de 25°C à la température de reflux du solvant pendant 1 à 24 heures, pour former un composé de formule 3 dans lequel R⁸ représente -CH=CH(CH₂)ₛCHR¹⁴OR¹⁵ ou -CH=CH(CH₂)ₛCOR¹⁶, sauf quand R¹⁵ représente H et R¹⁶ représente OH, et éventuellement suivi de la mise en contact du composé de formule 3 dans lequel R⁸ représente -CH=CH(CH₂)ₛCOR¹⁶ avec un agent réducteur dans un solvant, à une température de 0°C à 25°C, pendant 0,5 à 24 heures, pour donner un produit de formule 3 dans lequel R⁸ représente -CH=CH(CH₂)ₛCHR¹⁴OH.

34. Procédé selon la revendication 19, dans lequel R⁸ représente (CH₂)ₙOH et le composé de formule 3 est mis en contact avec un isocyanate de formule R¹⁰NCO dans un solvant à une température comprise entre 25°C et la température de reflux du solvant pendant 5 minutes à 24 heures pour former un composé de formule 3, dans lequel R⁸ représente (CH₂)ₙOCONHR¹⁰.

35. Procédé selon la revendication 19, dans lequel le composé dans lequel R⁸ représente (CH₂)ₙCl est mis en contact avec une amine R¹¹NH₂ dans un excès d'amine ou dans un autre solvant pendant 1 à 24 heures à une température de 0°C à la température de reflux du solvant pour former un intermédiaire de formule 3, dans lequel R⁸ représente (CH₂)ₙNHR¹¹.

36. Procédé selon la revendication 19, dans lequel R⁸ représente (CH₂)ₙCl et le composé de formule 3 est mis en contact avec un azidure de métal alcalin, dans un solvant aprotique à une température de 25°C à 80°C, pendant 1 à 24 heures, pour former un composé de formule 3 dans lequel R⁸ représente (CH₂)ₙN₃ et ce dernier est mis en contact avec un agent réducteur pour former un intermédiaire de formule 3 dans lequel R⁸ représente (CH₂)ₙNH₂.

37. Procédé selon la revendication 35 ou 36, dans lequel R⁸ représente (CH₂)ₙNHR¹¹ ou (CH₂)ₙNH₂ et le composé de formule 3 est mis en contact avec un chloroformiate de formule R¹⁰OCOCl ou un dérivé sulfonyle de formule R¹⁰SO₂Cl, ou (R¹⁰SO₂)₂O dans un solvant en présence d'une base à une température de 0°C à la température de reflux du solvant pendant 5 minutes à 24 heures pour former un composé de formule 3 dans lequel R⁸ représente (CH₂)ₙNR¹¹CO₂R¹⁰ ou (CH₂)ₙNR¹¹SO₂R¹⁰.

38. Procédé selon la revendication 35 ou 36, dans lequel le dérivé de formule 3 où R⁸ représente -(CH₂)ₙNHR¹¹ ou (CH₂)ₙNH₂ est mis au contact d'un isocyanate ou isothiocyanate R¹⁰NCY, dans un solvant, à une température comprise entre 25°C à la température de reflux du solvant, pendant 5 minutes à 24 heures, pour former un composé de formule 3 dans lequel R⁸ représente (CH₂)ₙNR¹¹CYNHR¹⁰.

39. Procédé selon la revendication 1, dans lequel R¹ dans A) et B) représente NO₂ et R, R³, R⁶, R⁷ et R⁸ sont tels que définis dans la revendication 24, dans lequel un composé où R¹ est NO₂ est réduit à l'aide de fer et d'acide acétique, de chlorure stanneux, ou d'hydrogène et de palladium, en un composé dans lequel R¹ représente NH₂ et ce dernier est mis à réagir avec un anhydride d'acide approprié tel qu'un anhydride phtalique ou un anhydride phtalique substitué dans un solvant, ou avec un chlorure d'acide approprié tel qu'un chlorure d'acide anthranilique substitué en présence d'un alcali aqueux ou d'une base ou avec un acide phtalique ou anthranilique convenablement substitué en présence de dicyclohexylcarbodiimide dans un solvant, pour former un produit dans lequel R¹ est: 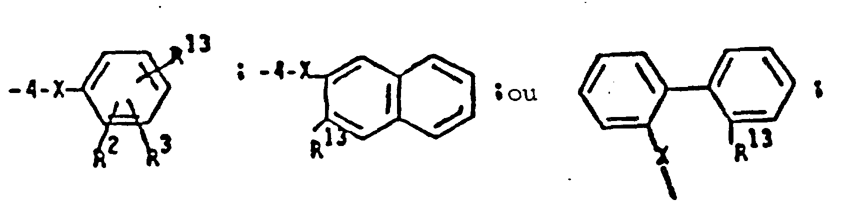 et X représente NHCO.

40. Procédé selon la revendication 1, dans lequel, dans A), R¹ représente OCH₂C₆H₅, R et R³ sont H et R⁶, R⁷ et R⁸ sont tels que définis dans la revendication 1, et on met en contact le composé de formule 3 résultant avec R¹ représentant OCH₂C₆H₅ avec de l'acide trifluoroacétique à la température de reflux, pendant une période de 0,2 à 1 heure, ou avec de l'hydrogène et du palladium pour former le composé correspondant de formule 3 dans lequel R¹ représente OH et ce dernier est mis en contact avec une base à 25°C et un halogénure de benzyle convenable de formule: 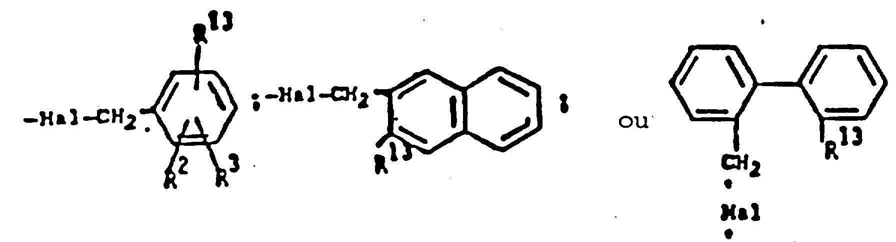 pour former le dérivé correspondant de formule 3 dans lequel R¹ est: 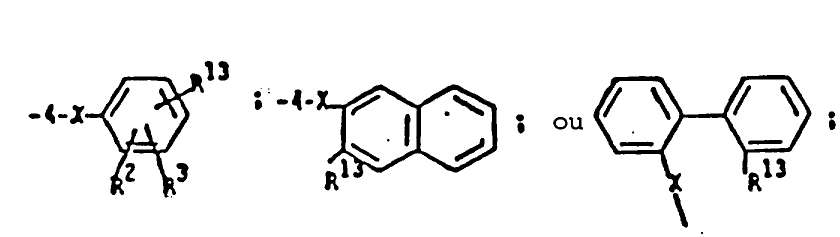 et X représente -OCH₂-.

41. Procédé selon la revendication 1, dans lequel R⁸, dans A), représente -CHO, de sorte que le dérivé benzylique de formule 2 se fixe sur un dérivé d'imidazole de formule 1, de préférence sur l'atome d'azote adjacent à l'atome de carbone du cycle imidazole auquel R⁸ est fixé.

42. Un procédé selon la revendication 19, dans lequel R⁸ représente CHO, et X représente une liaison simple carbone-carbone, et dans lequel on met en contact le produit de formule 3 avec un réactif organométallique tel que R¹¹mgBr ou R¹¹Li en présence d'un solvant non hydroxylé anhydre tel que éther, THF ou diméthoxyéthane, à une température de -78°C à 25°C, suivi par un traitement aqueux, suivi par une hydrolyse acide de tout groupe CO₂R¹⁴ où R¹⁴ représente le t-butyle ou une hydrolyse de tout groupe tétrazole protégé par un trityle, pour former le composé correspondant de formule 3 où R⁸ représente 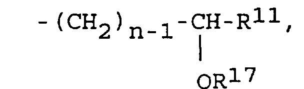 où R¹¹ est différent de H.

43. Un procédé selon l'une quelconque des revendications 1 à 42, dans lequel le composé préparé présente la formule: 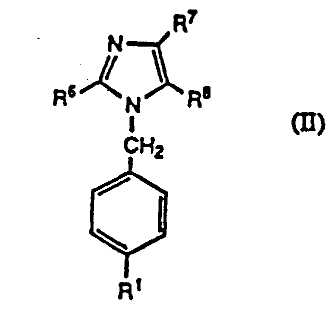 où
R¹ est CO₂H; -NHSO₂CF₃; 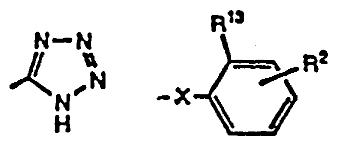 ou 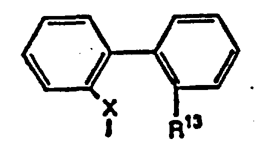
R⁶ est un alkyle comportant 3 à 10 atomes de carbone, alkényle comportant 3 à 10 atomes de carbone, alkynyle comportant 3 à 10 atomes de carbone, cycloalkyle comportant 3 à 8 atomes de carbone, benzyle substitué sur le noyau phényle par un à deux groupes choisis parmi alcoxy comportant 1 à 4 atomes de carbone, halogène, alkyle comportant 1 à 4 atomes de carbone, et nitro;
R⁸ is -(CH₂)ₘ-tetrazolyl, -(CH₂)ₙOR¹¹; 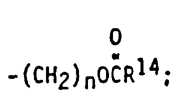 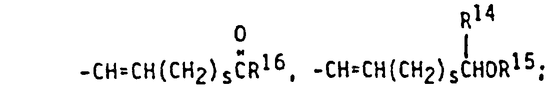 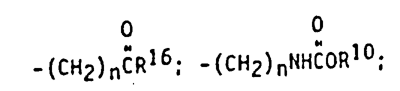 -(CH₂)ₙNHSO₂R¹⁰; ou 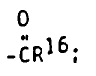
R¹³ est -CO₂H, -CO₂R⁹, NHSO₂CF₃; SO₃H; ou 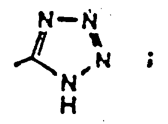
R¹⁶ est H, un alkyle comportant 1 à 5 atomes de carbone, OR¹⁷, ou NR¹⁸R¹⁹;
X est une liaison simple carbone-carbone, -CO-, 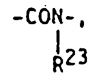 -CH₂CH₂-, 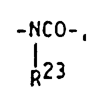 -OCH₂-, -CH₂O-, -O-, -SCH₂-, -CH₂S-, -NHCH₂-, -CH₂NH- ou -CH=CH-;
ou correspond à un de ses sels pharmaceutiquement acceptables.

44. Un procédé selon la revendication 43, dans lequel:
R est H, un alkyle comportant 1 à 4 atomes de carbone, halogène, ou alcoxy comportant 1 à 4 atomes de carbone;
R⁶ est un alkyle, un alkényle ou un alkynyle comportant 3 à 7 atomes de carbone;
R⁷ est un hétéroaryle choisi parmi 2- et 3-thiényle, 2- et 3-furyle, 2-, 3- et 4-pyridyle, ou parabiphénylyle, 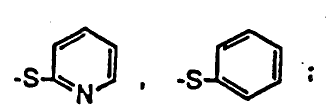
R⁸ est -(CH₂)ₘOR¹¹; 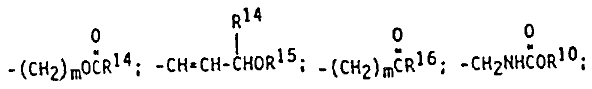 -(CH₂)ₘNHSO₂R¹⁰; ou -COR¹⁶;
R¹⁰ est CF₃, un alkyle comportant 1 à 6 atomes de carbone ou un phényle;
R¹¹ est H, ou un alkyle comportant 1 à 4 atomes de carbone;
R¹³ est CO₂H; CO₂CH₂OCOC(CH₃)₃; NHSO₂CF₃ ou 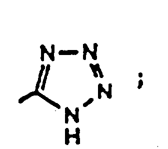
R¹⁴ est H, ou un alkyle comportant 1 à 4 atomes de carbone;
R¹⁵ est H, un alkyle comportant 1 à 4 atomes de carbone, ou un acyle comportant 1 à 4 atomes de carbone;
R¹⁶ est H, un alkyle comportant 1 à 5 atomes de carbone; OR¹⁷; ou 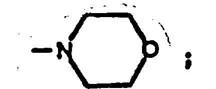
m vaut de 1 à 5;
X est une liaison simple, -O-; -CO-; -NHCO-; ou -OCH₂-; ou bien un de ses sels pharmaceutiquement acceptables.

45. Un procédé selon la revendication 44, dans lequel R¹ représente: 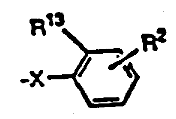 et X représente une liaison simple,
ou un de ses sels pharmaceutiquement acceptables.

46. Un procédé de préparation d'une composition pharmaceutique qui comprend le mélange d'un support pharmaceutiquement acceptable avec un composé préparé selon l'une quelconque des revendications 1 à 45.

47. Le procédé selon la revendication 46, dans lequel la composition pharmaceutique renferme en outre un médicament diurétique ou un médicament anti-inflammatoire non stéroïdien.
